(19) Europäisches Patentamt
European Patent Office
Office européen des brevets

(11) EP 4 556 473 A1

(12) **EUROPEAN PATENT APPLICATION**
published in accordance with Art. 153(4) EPC

(43) Date of publication:
**21.05.2025 Bulletin 2025/21**

(21) Application number: **23838800.3**

(22) Date of filing: **03.07.2023**

(51) International Patent Classification (IPC):
*C07D 471/04* (2006.01) *C07D 471/14* (2006.01)
*C07D 491/048* (2006.01) *C07D 491/052* (2006.01)
*C07D 491/147* (2006.01) *C07D 495/04* (2006.01)
*A61K 31/55* (2006.01) *A61K 31/4375* (2006.01)
*A61K 31/519* (2006.01) *A61K 31/437* (2006.01)
*A61K 31/4365* (2006.01) *A61P 35/00* (2006.01)

(52) Cooperative Patent Classification (CPC):
**A61K 31/4365; A61K 31/437; A61K 31/4375; A61K 31/519; A61K 31/55; A61P 35/00; C07D 471/04; C07D 471/14; C07D 491/048; C07D 491/052; C07D 491/147; C07D 495/04**

(86) International application number:
**PCT/CN2023/105579**

(87) International publication number:
**WO 2024/012308 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **15.07.2022 CN 202210836367**
**10.11.2022 CN 202211414992**

(71) Applicant: **Abbisko Therapeutics Co., Ltd.**
**Shanghai 201203 (CN)**

(72) Inventors:
- **DENG, Haibing**
  **Shanghai 201203 (CN)**
- **YANG, Fei**
  **Shanghai 201203 (CN)**
- **LIU, Zhaomin**
  **Shanghai 201203 (CN)**
- **LIU, Jian**
  **Shanghai 201203 (CN)**
- **YU, Hongping**
  **Shanghai 201203 (CN)**
- **CHEN, Zhui**
  **Shanghai 201203 (CN)**
- **XU, Yaochang**
  **Shanghai 201203 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

# (54) PRMT5 INHIBITOR, PREPARATION METHOD THEREFOR, AND PHARMACEUTICAL USE THEREOF

(57) A PRMT5 inhibitor, a preparation method therefor, and the pharmaceutical use thereof. In particular, the present invention relates to a PRMT5 inhibitor having a structure of formula (I), a preparation method therefor, a pharmaceutical composition containing same, and the use of same as a PRMT5 inhibitor and the use of same for treatment and/or prevention of PRMT5-mediated diseases. Each substituent of formula (I) is as defined in the description.

EP 4 556 473 A1

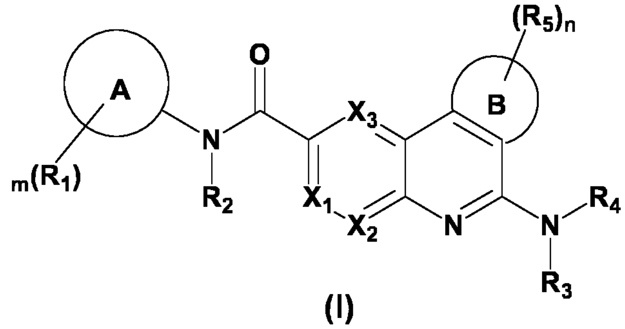

(I)

## Description

### TECHNICAL FIELD

The present invention belongs to the field of pharmaceutical synthesis, and particularly relates to a PRMT5 inhibitor, preparation method therefor, and pharmaceutical use thereof.

### BACKGROUND

Epigenetic gene regulation is an important biological regulatory mechanism for protein synthesis and cell differentiation, and plays an important role in many human diseases.

Epigenetic regulation involves regulation of inheritable genetic material without altering its nucleic acid sequence. Generally, epigenetic regulation is a selective and reversible modification (such as methylation) of DNA and proteins (such as histones) to control the transition between transcriptionally active state and inactive state in chromatin conformation. Modifications of these covalent bonds can be controlled by enzymes, such as methyltransferases (e.g., PRMT5), many of which are associated with specific gene changes in many human pathogenic genes. PRMT5 plays an important role in many diseases such as tumors, metabolic diseases and hematological diseases.

Homozygous deletion of tumor suppressor genes is the driver of tumors and often leads to deletions of passenger genes near the suppressor genes. The deletion of these passenger genes creates tumor cell-specific weaknesses that can be targeted by targeted therapies. Homozygous deletion of chromosome 9p21 locus, including the well-known tumor suppressor gene CDKN2A, occurs in 15% of tumors and often contains deletion of the passenger gene MTAP. MTAP is a key enzyme in the methionine and adenine recycling pathways. The deletion of MTAP leads to the accumulation of its substrate, MTA. MTA and S-adenosylmethionine (SAM) are structurally similar, and the latter is a methyl substrate donor of the type II methyltransferase PRMT5. Due to the increase in MTA levels caused by MTAP deletion, it will selectively compete with SAM for binding of PRMT5, leaving methyltransferase in an inactivation state and more likely to be affected by PRMT5 inhibition. shRNA screening of a wide range of tumor cell lines across many different genomic ranges has shown a correlation between MTAP deletion and dependence of the cell line on PRMT5, thus putting the impact of this metabolic susceptibility in the spotlight. However, PRMT5 is a very important gene for cells, and studies on conditional knockout of PRMT5 or siRNA knockout suggest that inhibition of PRMT5 in normal tissues will have significant side effects. (e.g., cytopenia, infertility, decreased skeletal muscle, myocardial hypertrophy, etc.). Therefore, there is a need for new strategies to apply and explore this metabolic susceptibility to selectively target PRMT5 in MTAP-deleted tumors while avoiding its effect on PRMT5 in normal tissues (MTAP wild-type).

Small-molecule inhibitors targeting PRMT5 that works together with MTA can selectively target PRMT5 only in the MTA-bound state, and this PRMT5 is only enriched in MTAP-deleted tumor cells. Therefore, PRMT5 will not be targeted when MTA levels are very low in normal cells with intact MTAP, thus providing a better treatment window.

### SUMMARY

The purpose of the present invention is to provide a PRMT5 inhibitor, a preparation method therefor, and a pharmaceutical use thereof. The series of compounds of the present invention have a strong inhibitory effect on PRMT5 and can be widely used in the preparation of drugs for the treatment and/or prevention of PRMT5-mediated diseases, thereby promising the development of a new generation of PRMT5 inhibitors.

The first aspect of the present invention provides a compound of formula (I), a stereoisomer or pharmaceutically acceptable salt thereof:

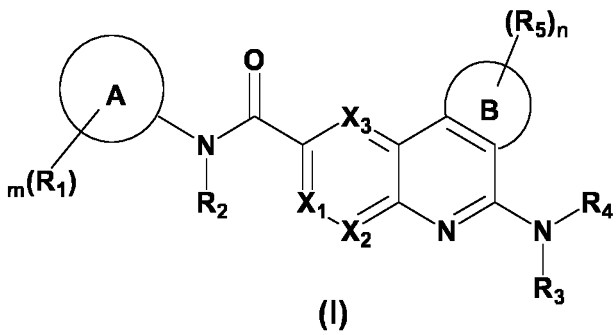

(I) ,

wherein, $X_1$ is $CR_6$ or N; $X_2$ is $CR_7$ or N; $X_3$ is $CR_8$ or N;

ring A is $C_{3-10}$ cycloalkyl, 4-10 membered heterocyclyl, $C_{6-10}$ aryl or 5-10 membered heteroaryl, and the $C_{3-10}$ cycloalkyl or 4-10 membered heterocyclyl is further fused to $C_{6-10}$ aryl or 5-10 membered heteroaryl, the $C_{6-10}$ aryl or 5-10 membered heteroaryl is further fused to $C_{3-10}$ cycloalkyl or 4-10 membered heterocyclyl;

ring B is $C_{4-12}$ cycloalkyl, 4-12 membered heterocyclyl, $C_{6-10}$ aryl or 5-10 membered heteroaryl;

each $R_1$ is independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{11}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{11}$;

$R_2$ is selected from the group consisting of hydrogen, deuterium, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -S(O)$_r$$R_9$, -O-$R_{10}$, -C(O)O$R_{10}$, -C(O)$R_{11}$ and -C(O)N$R_{12}R_{13}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{11}$;

$R_3$ and $R_4$ are each independently selected from the group consisting of hydrogen, deuterium, hydroxy, $C_{1-10}$ alkyl, $C_{2-10}$ alkenyl, $C_{2-10}$ alkynyl, $C_{3-12}$ cycloalkyl and 3-12 membered heterocyclyl, or, $R_3$ and $R_4$, together with a nitrogen atom directly attached thereto, form a 4-10 membered heterocyclyl or 5-10 membered heteroaryl, and above groups are independently optionally further substituted by one or more substituents selected from the group consisting of deuterium, halogen, cyano, hydroxy, =O, =S, $C_{1-10}$ alkyl, $C_{1-10}$ haloalkyl, $C_{1-10}$ deuterioalkyl, $C_{2-10}$ alkenyl, $C_{2-10}$ alkynyl, $C_{1-10}$ alkoxy, $C_{3-12}$ cycloalkyl, $C_{3-12}$ cycloalkoxy, 3-12 membered heterocyclyl, 3-12 membered heterocyclyloxy and -N$R_{12}R_{13}$;

each $R_5$ is independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{11}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{11}$;

$R_6$, $R_7$ and $R_8$ are each independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$C_{0-8}$ alkyl-$SF_5$, -$C_{0-8}$ alkyl-O-S(O)$_2$$R_9$, -$C_{0-8}$ alkyl-S(O)$_r$$R_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)($R_{11}$)$_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O)$R_{13}$;

each $R_9$ is independently selected from the group consisting of hydrogen, deuterium, hydroxy, $C_{1-10}$ alkyl, $C_{2-10}$ alkenyl, $C_{3-12}$ cycloalkyl, 3-12 membered heterocyclyl, $C_{6-10}$ aryl, 5-10 membered heteroaryl and -N$R_{12}R_{13}$, and above groups are independently optionally further substituted by one or more substituents selected from the group consisting of deuterium, halogen, hydroxy, =O, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and -N$R_{12}R_{13}$;

each $R_{10}$ is independently selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, and above groups are independently optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered

heteroaryloxy and $-NR_{12}R_{13}$;

each $R_{11}$ is independently selected from the group consisting of hydrogen, deuterium, hydroxy, $C_{1-10}$ alkyl, $C_{1-10}$ alkoxy, $C_{2-10}$ alkenyl, $C_{2-10}$ alkynyl, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{12}R_{13}$, and above groups are independently optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and $-NR_{12}R_{13}$;

each $R_{12}$ and each $R_{13}$ are independently selected from the group consisting of hydrogen, deuterium, hydroxy, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and $C_{1-10}$ alkanoyl, and above groups are independently optionally further substituted by one or more substituents selected from the group consisting of deuterium, halogen, hydroxy, =O, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono $C_{1-10}$ alkylamino, di $C_{1-10}$ alkylamino and $C_{1-10}$ alkanoyl, or,

$R_{12}$ and $R_{13}$, together with a nitrogen atom directly attached thereto, form a 4-10 membered heterocyclyl or 5-10 membered heteroaryl, and the 4-10 membered heterocyclyl or 5-10 membered heteroaryl is further optionally substituted by one or more substituents selected from the group consisting of deuterium, halogen, hydroxy, =O, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono $C_{1-10}$ alkylamino, di $C_{1-10}$ alkylamino and $C_{1-10}$ alkanoyl;

each r is independently 0, 1 or 2;

m is 0, 1, 2, 3, 4, 5 or 6; and

n is 0, 1, 2, 3, 4 or 5.

As a preferred embodiment, in the compound of formula (I), the stereoisomer or pharmaceutically acceptable salt thereof, ring A is $C_{3-8}$ cycloalkyl, 4-8 membered heterocyclyl, $C_{6-8}$ aryl or 5-8 membered heteroaryl, and the $C_{3-8}$ cycloalkyl or 4-8 membered heterocyclyl is further fused to $C_{6-8}$ aryl or 5-8 membered heteroaryl, the $C_{6-8}$ aryl or 5-8 membered heteroaryl is further fused to $C_{3-8}$ cycloalkyl or 4-8 membered heterocyclyl;

ring B is $C_{4-6}$ cycloalkyl, 4-6 membered heterocyclyl, $C_{6-8}$ aryl or 5-8 membered heteroaryl;

each $R_1$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-C_{0-4}$ alkyl-$SF_5$, $-C_{0-4}$ alkyl-O-$S(O)_2R_9$, $-C_{0-4}$ alkyl-$S(O)_rR_9$, $-C_{0-4}$ alkyl-O-$R_{10}$, $-C_{0-4}$ alkyl-C(O)$OR_{10}$, $-C_{0-4}$ alkyl-C(O)$SR_{10}$, $-C_{0-4}$ alkyl-S-C(O)$R_{11}$, $-C_{0-4}$ alkyl-C(O)$R_{11}$, - $C_{0-4}$ alkyl-O-C(O)$R_{11}$, $-C_{0-4}$ alkyl-P(O)$(R_{11})_2$, $-C_{0-4}$ alkyl-$NR_{12}R_{13}$, $-C_{0-4}$ alkyl-C(O)$NR_{12}R_{13}$ and - $C_{0-4}$ alkyl-N($R_{12}$)-C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-C_{0-4}$ alkyl-$SF_5$, $-C_{0-4}$ alkyl-O-$S(O)_2R_9$, $-C_{0-4}$ alkyl-$S(O)_rR_9$, $-C_{0-4}$ alkyl-O-$R_{10}$, $-C_{0-4}$ alkyl-C(O)$OR_{10}$, $-C_{0-4}$ alkyl-C(O)$SR_{10}$, $-C_{0-4}$ alkyl-S-C(O)$R_{11}$, $-C_{0-4}$ alkyl-C(O)$R_{11}$, $-C_{0-4}$ alkyl-O-C(O)$R_{11}$, $-C_{0-4}$ alkyl-P(O)$(R_{11})_2$, $-C_{0-4}$ alkyl-$NR_{12}R_{13}$, $-C_{0-4}$ alkyl-C(O)$NR_{12}R_{13}$ and $-C_{0-4}$ alkyl-N($R_{12}$)-C(O)$R_{11}$;

$R_2$ is selected from the group consisting of hydrogen, deuterium, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-S(O)_rR_9$, $-O-R_{10}$, $-C(O)OR_{10}$, $-C(O)R_{11}$ and $-C(O)NR_{12}R_{13}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-C_{0-4}$ alkyl-$SF_5$, $-C_{0-4}$ alkyl-O-$S(O)_2R_9$, $-C_{0-4}$ alkyl-$S(O)_rR_9$, $-C_{0-4}$ alkyl-O-$R_{10}$, $-C_{0-4}$ alkyl-C(O)$OR_{10}$, - $C_{0-4}$ alkyl-C(O)$SR_{10}$, $-C_{0-4}$ alkyl-S-C(O)$R_{11}$, $-C_{0-4}$ alkyl-C(O)$R_{11}$, $-C_{0-4}$ alkyl-O-C(O)$R_{11}$, $-C_{0-4}$ alkyl-P(O)$(R_{11})_2$, $-C_{0-4}$ alkyl-$NR_{12}R_{13}$, $-C_{0-4}$ alkyl-C(O)$NR_{12}R_{13}$ and $-C_{0-4}$ alkyl-N($R_{12}$)-C(O)$R_{11}$;

$R_3$ and $R_4$ are each independently selected from the group consisting of hydrogen, deuterium, hydroxy, $C_{1-4}$ alkyl, $C_{2-4}$ alkenyl, $C_{2-4}$ alkynyl, $C_{3-6}$ cycloalkyl and 3-6 membered heterocyclyl, or, $R_3$ and $R_4$, together with a nitrogen atom directly attached thereto, form a 4-6 membered heterocyclyl or 5-8 membered heteroaryl, and above groups are independently optionally further substituted by one or more substituents selected from the group consisting of deuterium, halogen, cyano, hydroxy, =O, =S, $C_{1-4}$ alkyl, $C_{1-4}$ haloalkyl, $C_{1-4}$ deuterioalkyl, $C_{2-4}$ alkenyl, $C_{2-4}$ alkynyl, $C_{1-4}$ alkoxy, $C_{3-6}$ cycloalkyl, $C_{3-6}$ cycloalkoxy, 3-6 membered heterocyclyl, 3-6 membered heterocyclyloxy and $-NR_{12}R_{13}$;

each $R_5$ is independently selected from the group consisting of hydrogen, deuterium, halogen, cyano, $C_{1-4}$ alkyl, $C_{2-4}$

alkenyl, $C_{2-4}$ alkynyl, $C_{3-6}$ cycloalkyl, 3-6 membered heterocyclyl, $C_{6-8}$ aryl, 5-8 membered heteroaryl, -$C_{0-4}$ alkyl-$SF_5$, -$C_{0-4}$ alkyl-O-S(O)$_2R_9$, -$C_{0-4}$ alkyl-S(O)$_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, - $C_{0-4}$ alkyl-O-C(O)$R_{11}$, -$C_{0-4}$ alkyl-P(O)($R_{11}$)$_2$, -$C_{0-4}$ alkyl-N$R_{12}R_{13}$, -$C_{0-4}$ alkyl-C(O)N$R_{12}R_{13}$ and - $C_{0-4}$ alkyl-N($R_{12}$)-C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$C_{0-4}$ alkyl-$SF_5$, -$C_{0-4}$ alkyl-O-S(O)$_2R_9$, -$C_{0-4}$ alkyl-S(O)$_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)($R_{11}$)$_2$, -$C_{0-4}$ alkyl-N$R_{12}R_{13}$, -$C_{0-4}$ alkyl-C(O)N$R_{12}R_{13}$ and -$C_{0-4}$ alkyl-N($R_{12}$)-C(O)$R_{11}$;

$R_6$, $R_7$ and $R_8$ are each independently selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$C_{0-4}$ alkyl-$SF_5$, -$C_{0-4}$ alkyl-O-S(O)$_2R_9$, -$C_{0-4}$ alkyl-S(O)$_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)($R_{11}$)$_2$, -$C_{0-4}$ alkyl-N$R_{12}R_{13}$, -$C_{0-4}$ alkyl-C(O)N$R_{12}R_{13}$ and -$C_{0-4}$ alkyl-N($R_{12}$)-C(O)$R_{11}$;

wherein, $R_9$, $R_{10}$, $R_{11}$, $R_{12}$, $R_{13}$ and r are defined as in the compound of formula (I).

As a preferred embodiment, in the compound of formula (I), the stereoisomer or pharmaceutically acceptable salt thereof, each $R_9$ is independently selected from the group consisting of hydrogen, deuterium, hydroxy, $C_{1-4}$ alkyl, $C_{2-4}$ alkenyl, $C_{3-6}$ cycloalkyl, 3-6 membered heterocyclyl, $C_{6-8}$ aryl, 5-8 membered heteroaryl and -N$R_{12}R_{13}$, and above groups are independently optionally further substituted by one or more substituents selected from the group consisting of deuterium, halogen, hydroxy, =O, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}R_{13}$;

each $R_{10}$ is independently selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, and above groups are independently optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}R_{13}$;

each $R_{11}$ is independently selected from the group consisting of hydrogen, deuterium, hydroxy, $C_{1-4}$ alkyl, $C_{1-4}$ alkoxy, $C_{2-4}$ alkenyl, $C_{2-4}$ alkynyl, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}R_{13}$, and above groups are independently optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}R_{13}$;

each $R_{12}$ and each $R_{13}$ are independently selected from the group consisting of hydrogen, deuterium, hydroxy, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and $C_{1-4}$ alkanoyl, and above groups are independently optionally further substituted by one or more substituents selected from the group consisting of deuterium, halogen, hydroxy, =O, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono $C_{1-4}$ alkylamino, di $C_{1-4}$ alkylamino and $C_{1-4}$ alkanoyl, or,

$R_{12}$ and $R_{13}$, together with a nitrogen atom directly attached thereto, form a 4-6 membered heterocyclyl or 5-8 membered heteroaryl, and the 4-6 membered heterocyclyl or 5-8 membered heteroaryl is further optionally substituted by one or more substituents selected from the group consisting of deuterium, halogen, hydroxy, =O, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono $C_{1-4}$ alkylamino, di $C_{1-4}$ alkylamino and $C_{1-4}$ alkanoyl.

As a preferred embodiment, in the compound of formula (I), the stereoisomer or pharmaceutically acceptable salt thereof, the compound of formula (I) is a compound of formula (II):

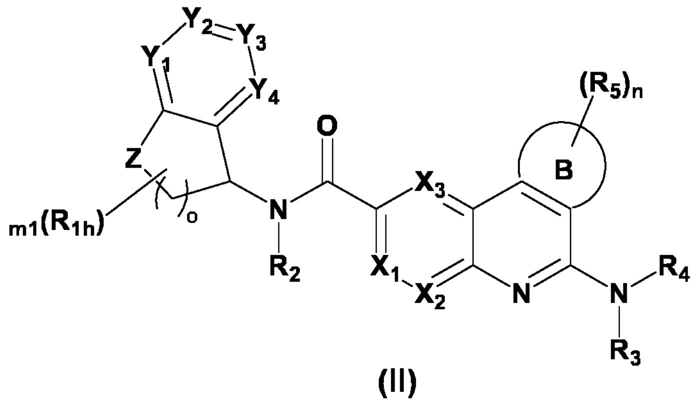

(II)

,

wherein, $X_1$ is $CR_6$ or N; $X_2$ is $CR_7$ or N; $X_3$ is $CR_8$ or N;

$Y_1$ is $CR_{1a}$ or N; $Y_2$ is $CR_{1b}$ or N; $Y_3$ is $CR_{1c}$ or N; $Y_4$ is $CR_{1d}$ or N;
Z is $-C(R_{1e}R_{1f})-$, $-C(R_{1e}R_{1f})O-$, $-N(R_{1g})-$, -O- or -S-;
ring B is $C_{4-6}$ cycloalkyl, 4-6 membered heterocyclyl, $C_{6-8}$ aryl or 5-8 membered heteroaryl;
$R_{1a}$, $R_{11}$, $R_{1c}$ and $R_{1d}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and - $N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, - $C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_{1e}$ and $R_{1r}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and - $N(R_{12})-C(O)R_{11}$, or, $R_{1e}$ and $R_{11}$, together with a carbon atom directly attached thereto, form a $C_{3-6}$ cycloalkyl or 4-6 membered heterocyclyl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, - $C(O)NR_{12}R_{13}$ and $-N(R_{12})-C(O)R_{11}$;
$R_{1g}$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, - $S(O)_rR_9$, $-O-R_{10}$, $-C(O)OR_{10}$, $-C(O)SR_{19}$, $-C(O)R_{11}$ and $-C(O)NR_{12}R_{13}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
each $R_{1h}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_2$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-OR_{10}$ and $-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, - $C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_3$ and $R_4$ are each independently selected from the group consisting of hydrogen, deuterium, hydroxy, $C_{1-4}$ alkyl, $C_{3-6}$

cycloalkyl and 3-6 membered heterocyclyl, or, $R_3$ and $R_4$, together with a nitrogen atom directly attached thereto, form a 4-6 membered heterocyclyl or 5-8 membered heteroaryl, and above groups are independently optionally further substituted by one or more substituents selected from the group consisting of deuterium, halogen, cyano, hydroxy, =O, =S, $C_{1-4}$ alkyl, $C_{1-4}$ haloalkyl, $C_{1-4}$ deuterioalkyl, $C_{2-4}$ alkenyl, $C_{2-4}$ alkynyl, $C_{1-4}$ alkoxy, $C_{3-6}$ cycloalkyl, $C_{3-6}$ cycloalkoxy, 3-6 membered heterocyclyl, 3-6 membered heterocyclyloxy and $-NR_{12}R_{13}$;

each $R_5$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

$R_6$, $R_7$ and $R_8$ are each independently selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{19}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

o is 0, 1, 2 or 3;

m1 is 0, 1 or 2;

wherein, $R_9$, $R_{10}$, $R_{11}$, $R_{12}$, $R_{13}$, n and r are defined as in the compound of formula (I).

As a further preferred embodiment, in the compound of formula (I), the stereoisomer or pharmaceutically acceptable salt thereof, ring B, together with a moiety to which it is directly attached thereto, forms the following structure:

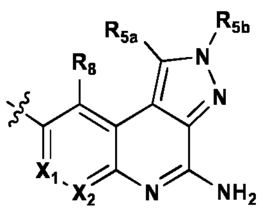
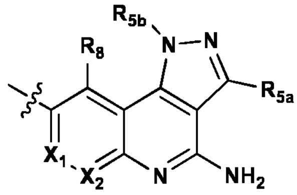
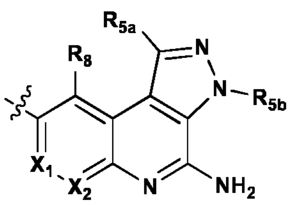
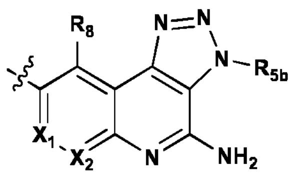

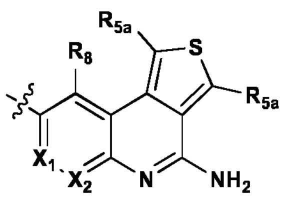
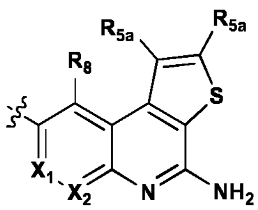
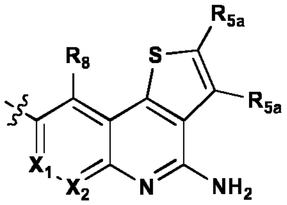
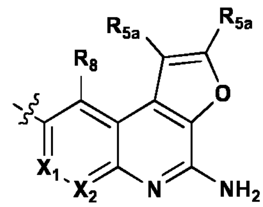

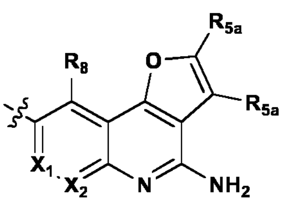
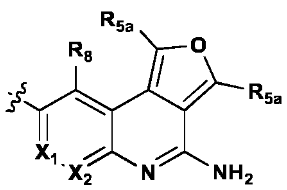
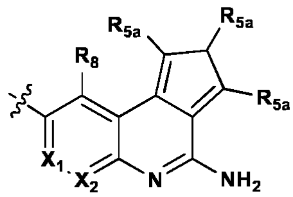
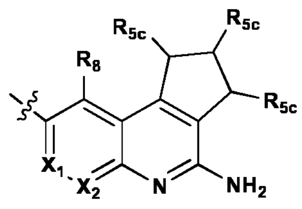

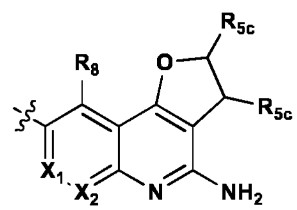
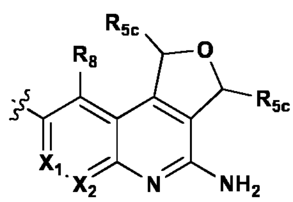
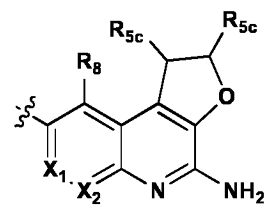
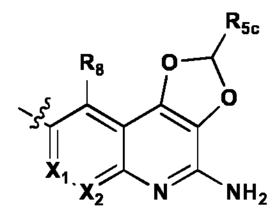

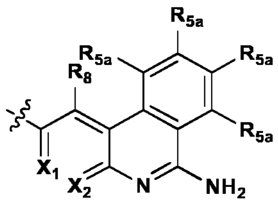 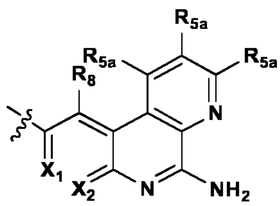 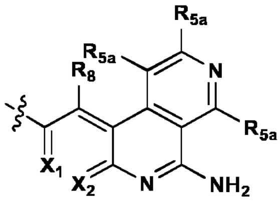 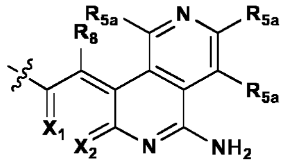

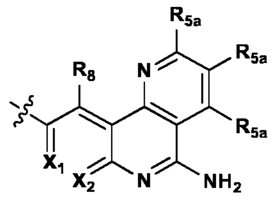 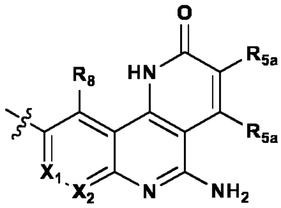 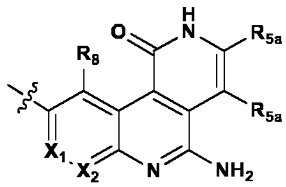 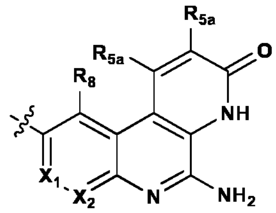

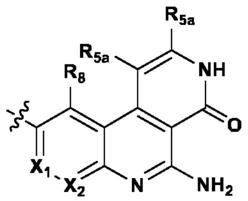 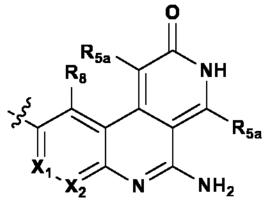 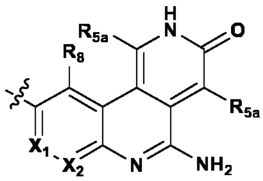 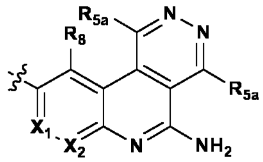

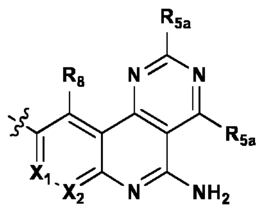 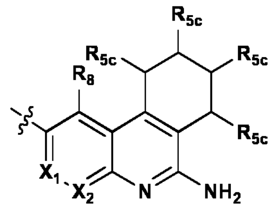 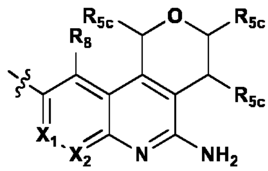 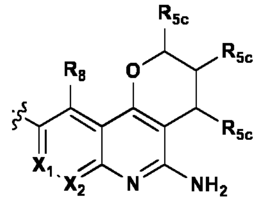

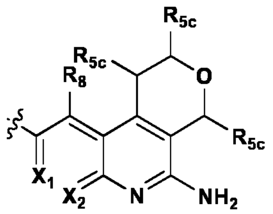 or 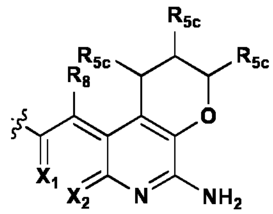 ;

wherein, each $R_{5a}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, - $C(O)NR_{12}R_{13}$ and $-N(R_{12})-C(O)R_{11}$;

each $R_{5b}$ is independently selected from the group consisting of hydrogen, deuterium, $C_{1-4}$ alkyl, $C_{3-6}$ cycloalkyl, 3-6 membered heterocyclyl and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{19}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of

deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, - C(O) $OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

$X_1$ is $CR_6$ or N, and each $R_6$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

$X_2$ is $CR_7$ or N, and each $R_7$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

each $R_8$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

wherein, $R_9$, $R_{10}$, $R_{11}$, $R_{12}$, $R_{13}$ and r are defined as in the compound of formula (II).

As a preferred embodiment, in the compound of formula (I), the stereoisomer or pharmaceutically acceptable salt thereof, the compound of formula (I) is a compound of formula (III):

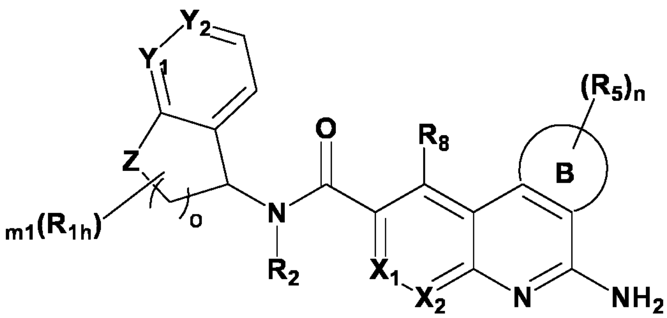

**(III)**

,

wherein, $X_1$ is $CR_6$ or N; $X_2$ is $CR_7$ or N;

$Y_1$ is $CR_{1a}$ or N; $Y_2$ is $CR_{1b}$ or N;

Z is $-C(R_{1e}R_{1f})-$, $-C(R_{1e}R_{1f})O-$, $-N(R_{1g})-$ or -O-;

ring B, together with a moiety to which it is directly attached thereto, forms the following structure:

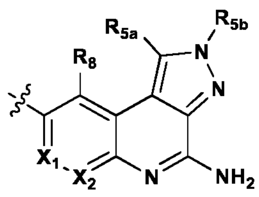
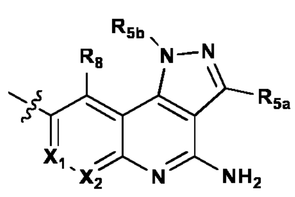
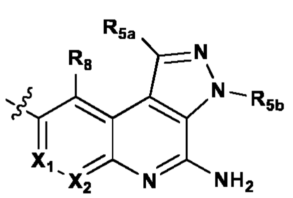
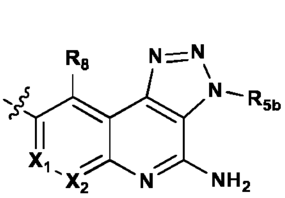
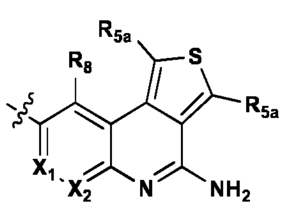
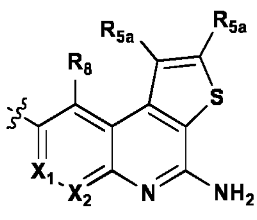
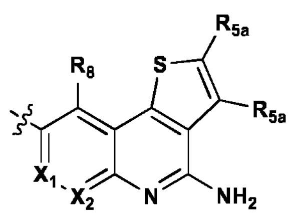
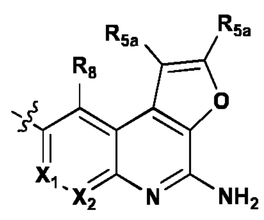
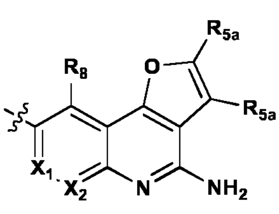
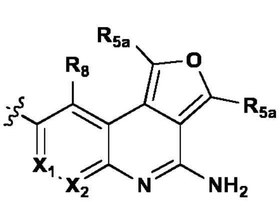
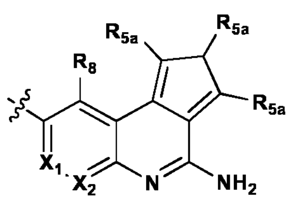
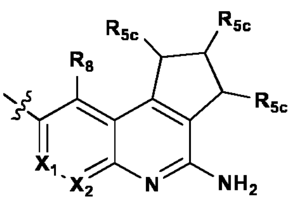

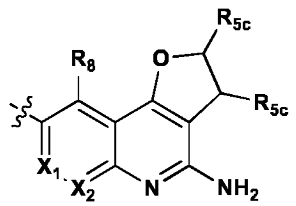
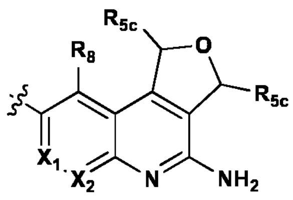
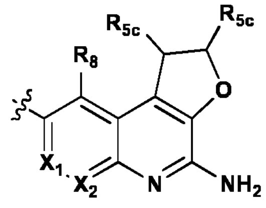
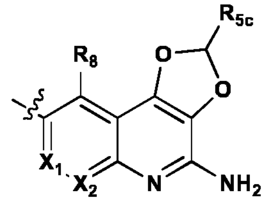
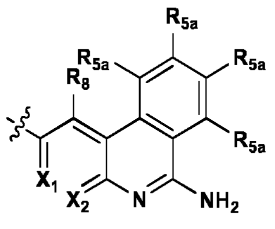
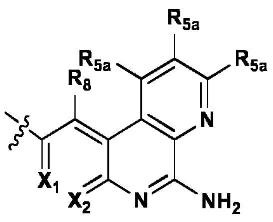
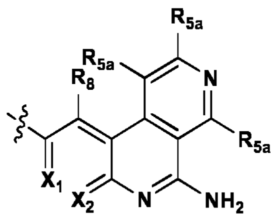
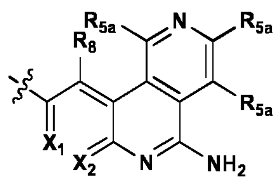
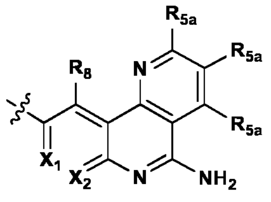
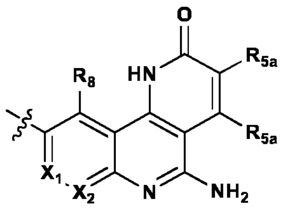
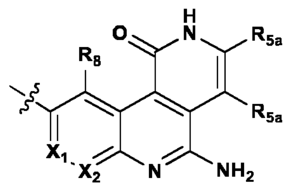
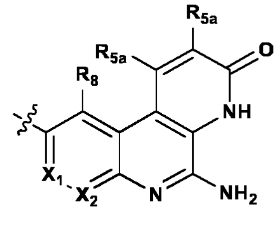
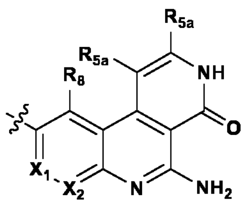
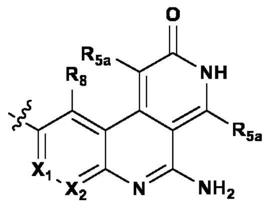
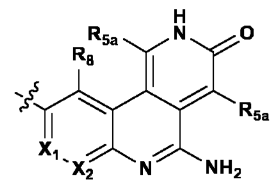
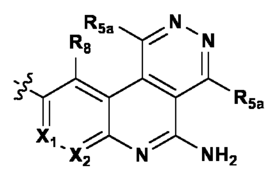
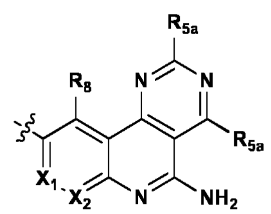
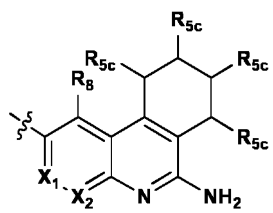
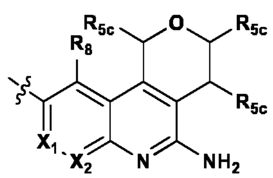
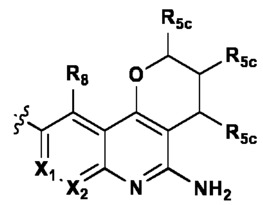
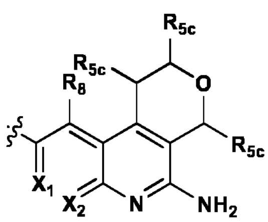 or 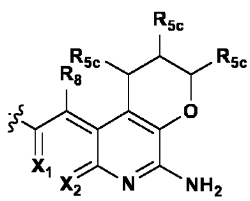 ;

$R_{1a}$ and $R_{1b}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and - $N(R_{12})-C(O)R_{11}$;

$R_{1e}$ and $R_{1f}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$

and -O-$R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, -C(O)$R_{11}$, -O-C(O)$R_{11}$, -$NR_{12}R_{13}$, -C(O)$NR_{12}R_{13}$ and - N($R_{12}$)-C(O)$R_{11}$;

$R_{1g}$ is selected from the group consisting of hydrogen, deuterium, $C_{1-4}$ alkyl, $C_{3-6}$ cycloalkyl, 3-6 membered heterocyclyl, hydroxy, $C_{1-4}$ alkoxy and -C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, -C(O)$R_{11}$, -O-C(O)$R_{11}$, -$NR_{12}R_{13}$, -C(O)$NR_{12}R_{13}$ and - N($R_{12}$)-C(O)$R_{11}$;

each $R_{1h}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$SF_5$ and -O-$R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, - $S(O)_rR_9$, -O-$R_{10}$, -C(O)$OR_{10}$, -C(O)$R_{11}$, -O-C(O)$R_{11}$, -$NR_{12}R_{13}$, -C(O)$NR_{12}R_{13}$ and -N($R_{12}$)-C(O)$R_{11}$;

$R_2$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$OR_{10}$ and -C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, - C(O)$R_{11}$, -O-C(O)$R_{11}$, -$NR_{12}R_{13}$, -C(O)$NR_{12}R_{13}$ and -N($R_{12}$)-C(O)$R_{11}$;

each $R_{5a}$ is independently selected from the group consisting of hydrogen, deuterium, halogen, cyano, $C_{1-4}$ alkyl, $C_{3-6}$ cycloalkyl, 3-6 membered heterocyclyl, -$SF_5$ and -O-$R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, -C(O)$R_{11}$, -O-C(O)$R_{11}$, - $NR_{12}R_{13}$, -C(O)$NR_{12}R_{13}$ and -N($R_{12}$)-C(O)$R_{11}$;

each $R_{5b}$ is independently selected from the group consisting of hydrogen, deuterium, $C_{1-4}$ alkyl, $C_{3-6}$ cycloalkyl, 3-6 membered heterocyclyl and -O-$R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, -C(O)$R_{11}$, -O-C(O)$R_{11}$, -$NR_{12}R_{13}$, -C(O)$NR_{12}R_{13}$ and - N($R_{12}$)-C(O)$R_{11}$;

each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, -$SF_5$ and -O-$R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$SF_5$, -$S(O)_rR_9$, -O-$R_{10}$, -C(O)$OR_{10}$, - C(O)$R_{11}$, -O-C(O)$R_{11}$, -$NR_{12}R_{13}$, -C(O)$NR_{12}R_{13}$ and -N($R_{12}$)-C(O)$R_{11}$;

$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$SF_5$ and -O-$R_{10}$;

$R_7$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$SF_5$ and -O-$R_{10}$;

$R_8$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$SF_5$ and -O-$R_{10}$;

o is 1 or 2;

m1 is 0, 1 or 2;

wherein, $R_9$, $R_{10}$, $R_{11}$, $R_{12}$, $R_{13}$, n and r are defined as in the compound of formula (I).

As a further preferred embodiment, in the compound of formula (I), the stereoisomer or pharmaceutically acceptable salt thereof, $Y_1$ is CH or N; $Y_2$ is $CR_{1b}$;

$R_{1b}$ is selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, -$SF_5$ and -O-$R_{10}$, and above groups are independently

optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
wherein, $R_{10}$ is defined as in the compound of formula (III).

As a further preferred embodiment, in the compound of formula (I), the stereoisomer or pharmaceutically acceptable salt thereof, $R_{1e}$ and $R_{1f}$ are each independently selected from the group consisting of hydrogen, deuterium, halogen, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;

$R_{1g}$ is selected from the group consisting of hydrogen, deuterium, $C_{1-4}$ alkyl, $C_{3-6}$ cycloalkyl, 3-6 membered heterocyclyl, hydroxy, methoxy and acetyl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
each $R_{1h}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
wherein, $R_{10}$ is defined as in the compound of formula (III).

As a further preferred embodiment, in the compound of formula (I), the stereoisomer or pharmaceutically acceptable salt thereof, each $X_1$ is independently $CR_6$ or N; each $X_2$ is independently CH;

each $R_6$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;
each $R_8$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;
wherein, $R_{10}$ is defined as in the compound of formula (III).

As a further preferred embodiment, in the compound of formula (I), the stereoisomer or pharmaceutically acceptable salt thereof, each $R_{5a}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;

each $R_{5b}$ is independently selected from the group consisting of hydrogen, deuterium, $C_{1-4}$ alkyl, $C_{3-6}$ cycloalkyl, 3-6 membered heterocyclyl and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
wherein, $R_{10}$ is defined as in the compound of formula (III).

As a further preferred embodiment, in the compound of formula (I), the stereoisomer or pharmaceutically acceptable salt thereof, $R_2$ is selected from the group consisting of hydrogen, deuterium, $C_{1-4}$ alkyl, $C_{3-6}$ cycloalkyl, 3-6 membered heterocyclyl, $C_{6-8}$ aryl and 5-8 membered heteroaryl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$

and $-N(R_{12})-C(O)R_{11}$;

wherein, $R_9$, $R_{10}$, $R_{11}$, $R_{12}$ and $R_{13}$ are defined as in the compound of formula (III).

As a further preferred embodiment, in the compound of formula (I), the stereoisomer or pharmaceutically acceptable salt thereof, $R_2$ is selected from the group consisting of hydrogen, deuterium, methyl, ethyl, n-propyl, isopropyl, n-butyl, isobutyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, sulfinyl, sulfonyl, methanesulfonyl, isopropylsulfonyl, hydroxy, methoxy, ethoxy, n-propoxy, isopropoxy, carboxyl, methoxycarbonyl, ethoxycarbonyl, n-propoxycarbonyl, isopropyloxycarbonyl, formyl, acetyl, n-propionyl, isopropionyl, formyloxy, acetoxy, n-propionyloxy, isopropionyloxy, amino, mono $C_{1-4}$ alkylamino and di $C_{1-4}$ alkylamino.

As a further preferred embodiment, in the compound of formula (I), the stereoisomer or pharmaceutically acceptable salt thereof,

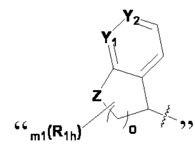

is selected from the following structure:

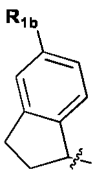 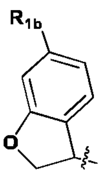 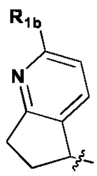 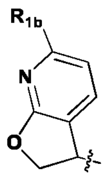 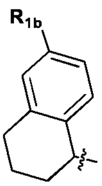 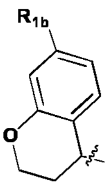 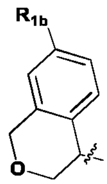 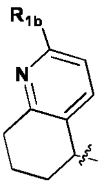 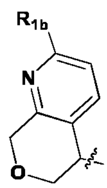 or 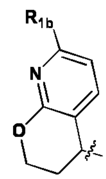,

wherein, each $R_{1b}$ is independently selected from the group consisting of hydrogen, deuterium, fluorine, chlorine, bromine, cyano, methyl, trifluoromethyl, difluoromethyl, trideuteriomethyl, dideuteriomethyl, trifluoromethoxy, difluoromethoxy, trideuteriomethoxy, dideuteriomethoxy, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy, cyclobutoxy, phenyl and methyl-substituted pyrazolyl.

As a further preferred embodiment, in the compound of formula (I), the stereoisomer or pharmaceutically acceptable salt thereof,

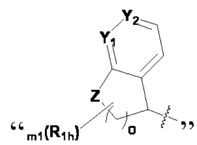

is selected from the following structure:

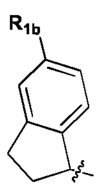 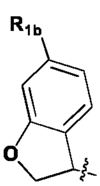 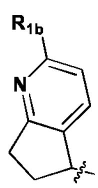 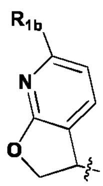 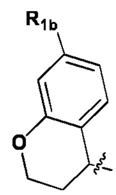 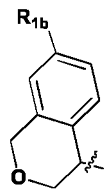 or 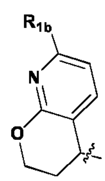,

wherein, each $R_{1b}$ is independently selected from the group consisting of hydrogen, deuterium, fluorine, chlorine, bromine, cyano, methyl, trifluoromethyl, difluoromethyl, trideuteriomethyl, dideuteriomethyl, trifluoromethoxy, difluoromethoxy, trideuteriomethoxy, dideuteriomethoxy, cyclopropyl and methyl-substituted pyrazolyl.

As a further preferred embodiment, in the compound of formula (I), the stereoisomer or pharmaceutically

acceptable salt thereof, the compound of formula (I) is a compound of formula (IVa):

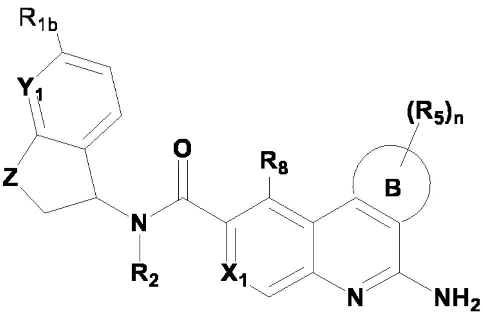

(IVa)

,

wherein,

“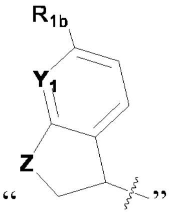”

is selected from the following structure:

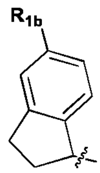

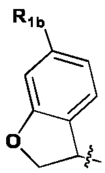

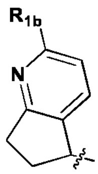

or

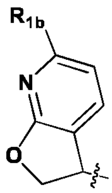

,

each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl, and methyl-substituted pyrazolyl;

ring B, together with a moiety

“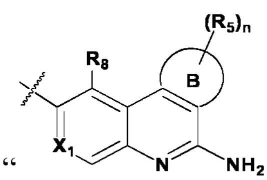”

to which it is directly attached thereto, is selected from the following structure:

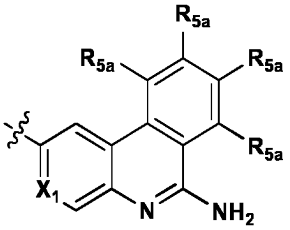 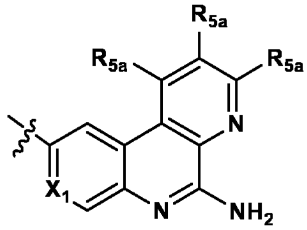 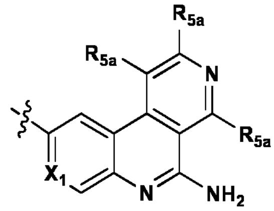 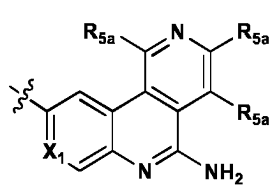

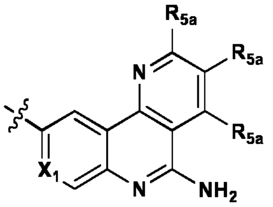 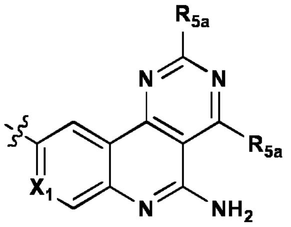 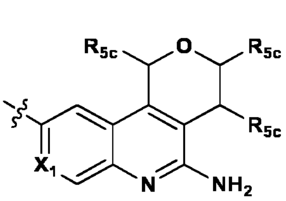 or 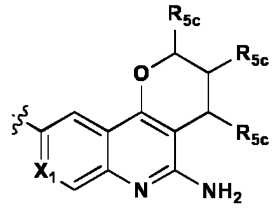 ;

each $X_1$ is independently $CR_6$ or N;
each $R_6$ is independently selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;
each $R_{5a}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
each $R_{5c}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
$R_2$ is selected from the group consisting of methyl, ethyl, n-propyl, isopropyl, n-butyl, isobutyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, hydroxy and methoxy; provided that when $R_2$ is methyl,

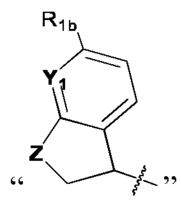

is not

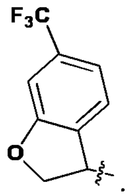

As a further preferred embodiment, in the compound of formula (I), the stereoisomer or pharmaceutically acceptable salt thereof, each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl and methyl-substituted pyrazolyl;

$R_6$ is selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;

$R_2$ is selected from the group consisting of cyclopropyl-substituted methyl, cyclobutyl-substituted methyl, thiazolyl-substituted methyl, pyrimidinyl-substituted methyl, ethyl, cyclopropyl-substituted ethyl, cyclobutyl-substituted ethyl, 2,2,2-trifluoroethyl, methoxyethyl, thiazolyl-substituted ethyl, pyrimidinyl-substituted ethyl, methoxy-substituted isopropyl, cyclopropyl, methyl-substituted pyrazolyl, thiazolyl, isothiazolyl, thiadiazolyl and pyrimidinyl.

As a further preferred embodiment, in the compound of formula (I), the stereoisomer or pharmaceutically acceptable salt thereof, the compound of formula (I) is a compound of formula (Va):

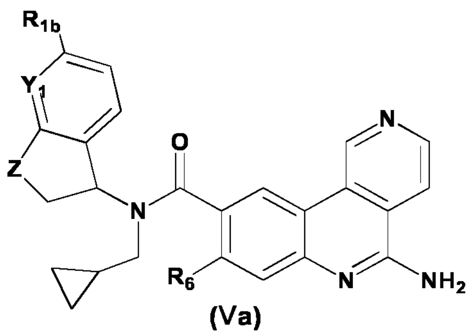

**(Va)**

wherein,

“ 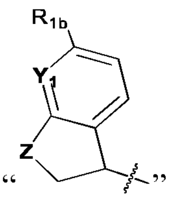 ”

is selected from the following structure:

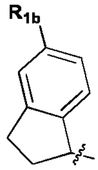 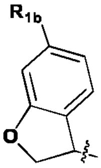 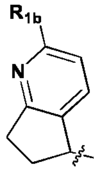

or

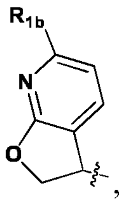

each $R_{1b}$ is independently selected from the group consisting of hydrogen, deuterium, fluorine, chlorine, methyl, trifluoromethyl, trideuteriomethyl, trifluoromethoxy, trideuteriomethoxy and cyclopropyl;
$R_6$ is selected from the group consisting of hydrogen, deuterium, fluorine, chlorine, methyl, trifluoromethyl, trideuteriomethyl and cyclopropyl.

As a further preferred embodiment, in the compound of formula (I), the stereoisomer or pharmaceutically acceptable salt thereof, the compound of formula (I) is a compound of formula (IVb):

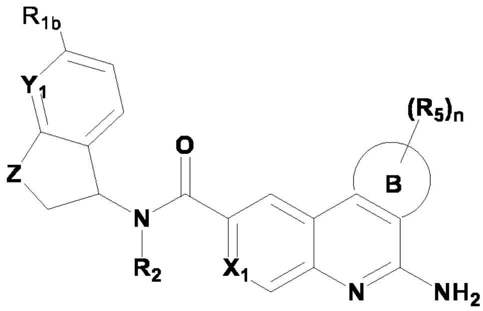

**(IVb)**

wherein,

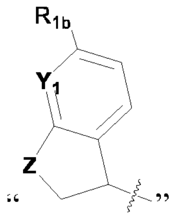

is selected from the following structure:

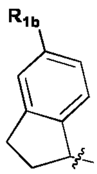
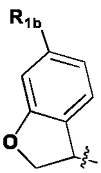
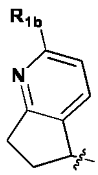

or

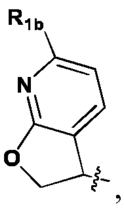

, each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl, and methyl-substituted

pyrazolyl;

ring B, together with a moiety

“ 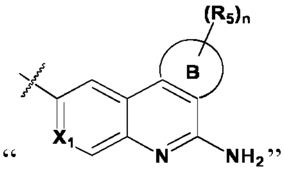 ”

to which it is directly attached thereto, is selected from the following structure:

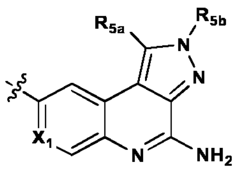 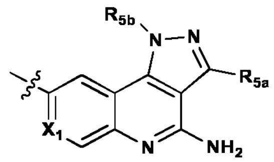 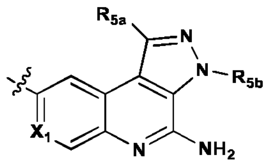

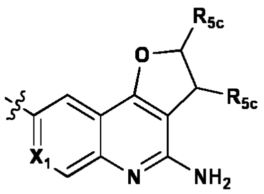 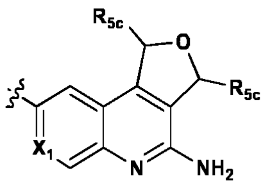 or 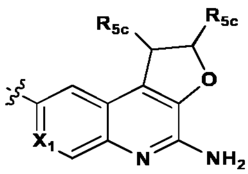 ;

each $X_1$ is independently $CR_6$ or N;
each $R_6$ is independently selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;
each $R_{5a}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
each $R_{5b}$ is independently selected from the group consisting of hydrogen, deuterium, methyl, ethyl, n-propyl, isopropyl, n-butyl, isobutyl, cyclopropyl, cyclobutyl, oxacyclobutyl, azacyclobutyl and hydroxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
each $R_{5c}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
wherein, $R_2$ is defined as in the compound of formula (III).

As a further preferred embodiment, in the compound of formula (I), the stereoisomer or pharmaceutically acceptable salt thereof, ring B, together with a moiety

“ 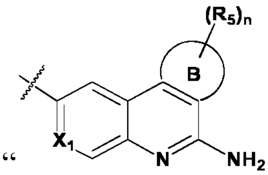 ” 1

:ure:

to which it is directly attached thereto, is selected from the following structure:

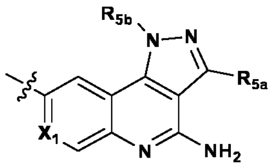 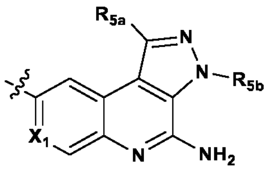 or 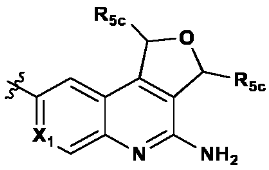 ;

each $X_1$ is independently $CR_6$; each $R_6$ is independently selected from the group consisting of hydrogen, deuterium, fluorine, chlorine, bromine, cyano, methyl, trifluoromethyl, trideuteriomethyl and cyclopropyl;
each $R_{5a}$ is independently selected from the group consisting of hydrogen, deuterium, fluorine, chlorine, bromine, cyano, methyl, trifluoromethyl, trideuteriomethyl and cyclopropyl;
each $R_{5b}$ is independently selected from the group consisting of hydrogen, deuterium, methyl, trifluoromethyl, trideuteriomethyl and cyclopropyl;
each $R_{5c}$ is independently selected from the group consisting of hydrogen, deuterium, fluorine, chlorine, bromine, cyano, methyl, trifluoromethyl, trideuteriomethyl and cyclopropyl.

As a further preferred embodiment, in the compound of formula (I), the stereoisomer or pharmaceutically acceptable salt thereof, $R_2$ is selected from the group consisting of substituted methyl, substituted ethyl, substituted or unsubstituted n-propyl, substituted isopropyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl,

and the substituents of the substituted methyl, substituted ethyl, substituted n-propyl and substituted isopropyl are each independently one or more substituents selected from the group consisting of fluorine, chlorine, bromine, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, $-SF_5$, hydroxy and methoxy,
and the bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl are each independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, hydroxy and methoxy.

As a further preferred embodiment, in the compound of formula (I), the stereoisomer or pharmaceutically acceptable salt thereof, $R_2$ is selected from the group consisting of substituted methyl, substituted ethyl, substituted or unsubstituted n-propyl, substituted isopropyl, phenyl, pyrazolyl, imidazolyl, triazolyl, oxazolyl, thiazolyl, isothiazolyl, thiadiazolyl and pyrimidinyl,

and the substituents of the substituted methyl, substituted ethyl, substituted n-propyl and substituted isopropyl are each independently one or more substituents selected from the group consisting of fluorine, chlorine, bromine, pyrazolyl, imidazolyl, triazolyl, oxazolyl, thiazolyl, isothiazolyl, thiadiazolyl, pyrimidinyl and methoxy,
and the phenyl, pyrazolyl, imidazolyl, triazolyl, oxazolyl, thiazolyl, isothiazolyl, thiadiazolyl and pyrimidinyl are each independently optionally further substituted by one or more substituents selected from the group consisting of deuterium, fluorine, chlorine, bromine, cyano, methyl, ethyl, trifluoromethyl, difluoromethyl, trideuteriomethyl, dideuteriomethyl, cyclopropyl, $-SF_5$, hydroxy and methoxy.

As a further preferred embodiment, in the compound of formula (I), the stereoisomer or pharmaceutically acceptable salt thereof, $R_2$ is selected from the group consisting of trifluoromethyl, pyrazolyl-substituted methyl,

imidazolyl-substituted methyl, triazolyl-substituted methyl, oxazolyl-substituted methyl, thiazolyl-substituted methyl, isothiazolyl-substituted methyl, thiadiazolyl-substituted methyl, pyrimidinyl-substituted methyl, fluorine-substituted ethyl, methoxyethyl, pyrazolyl-substituted ethyl, imidazolyl-substituted ethyl, triazolyl-substituted ethyl, oxazolyl-substituted ethyl, thiazolyl-substituted ethyl, isothiazolyl-substituted ethyl, thiadiazolyl-substituted ethyl, pyrimidinyl-substituted ethyl, methoxy-substituted isopropyl, pyrazolyl, methyl-substituted pyrazolyl, imidazolyl, methyl-substituted imidazolyl, triazolyl, methyl-substituted triazolyl, oxazolyl, methyl-substituted oxazolyl, thiazolyl, methyl-substituted thiazolyl, isothiazolyl, methyl-substituted isothiazolyl, thiadiazolyl, methyl-substituted thiadiazolyl, pyrimidinyl and methyl-substituted pyrimidinyl.

As a further preferred embodiment, in the compound of formula (I), the stereoisomer or pharmaceutically acceptable salt thereof, $R_2$ is selected from the group consisting of thiazolyl-substituted methyl, pyrimidinyl-substituted methyl, 2,2,2-trifluoroethyl, methoxyethyl, thiazolyl-substituted ethyl, pyrimidinyl-substituted ethyl, methoxy-substituted isopropyl, methyl-substituted pyrazolyl, thiazolyl, isothiazolyl, thiadiazolyl and pyrimidinyl.

As a further preferred embodiment, in the compound of formula (I), the stereoisomer or pharmaceutically acceptable salt thereof, $R_2$ is selected from the following structure:

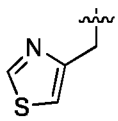 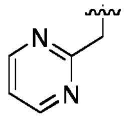 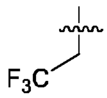 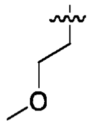 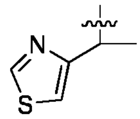 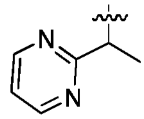 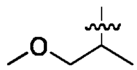

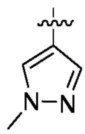 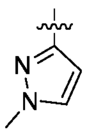 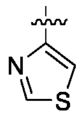 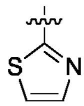 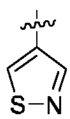 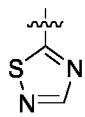 or 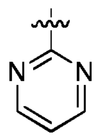 .

As a further preferred embodiment, in the compound of formula (I), the stereoisomer or pharmaceutically acceptable salt thereof, the compound of formula (I) is a compound of formula (Vb1), formula (Vb2), formula (Vb3) or formula (Vb4):

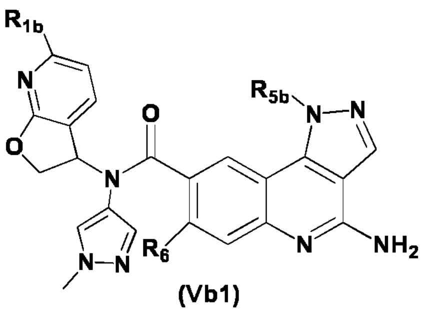
**(Vb1)**

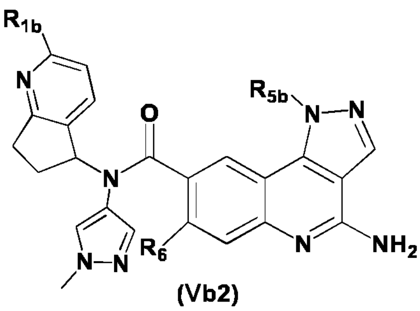
**(Vb2)**

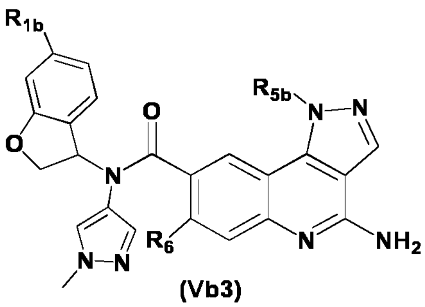
**(Vb3)**

or

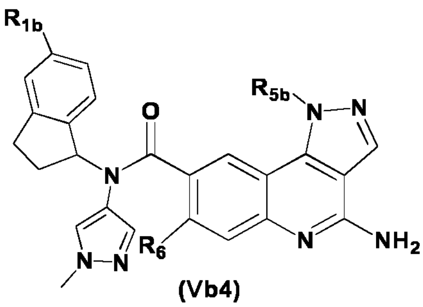
**(Vb4)**

,

each $R_{1b}$ is independently selected from the group consisting of hydrogen, deuterium, methyl, trifluoromethyl, trideuteriomethyl, trifluoromethoxy, trideuteriomethoxy and cyclopropyl;
each $R_6$ is independently selected from the group consisting of hydrogen, deuterium, fluorine, chlorine, bromine, cyano and methyl;
each $R_{5b}$ is independently selected from the group consisting of hydrogen, deuterium, methyl, trifluoromethyl,

trideuteriomethyl and cyclopropyl.

As the most preferred embodiment, the compound of formula (I), the stereoisomer or pharmaceutically acceptable salt thereof includes, but is not limited to, the following compounds:

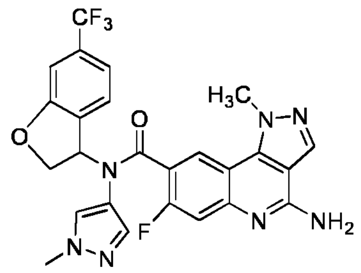
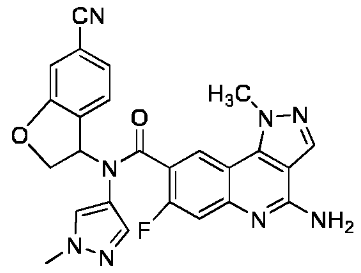
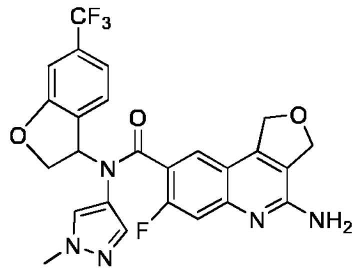
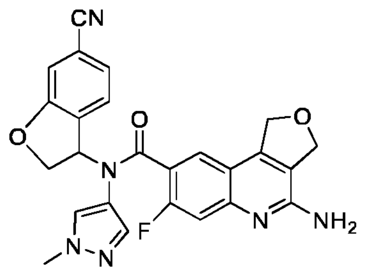
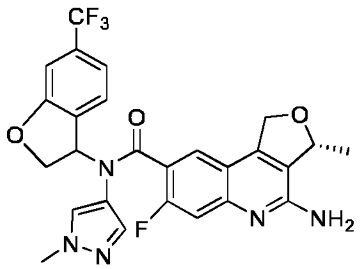
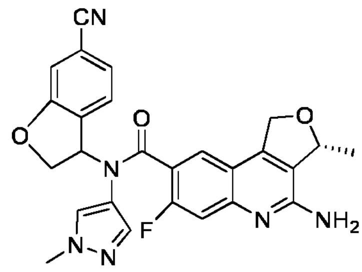
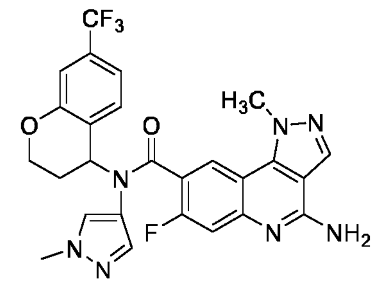
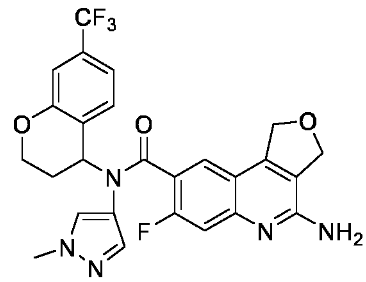
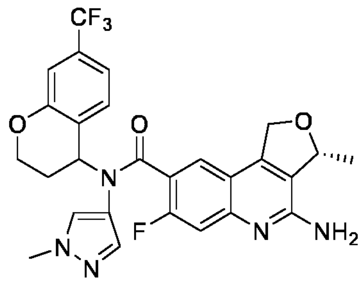
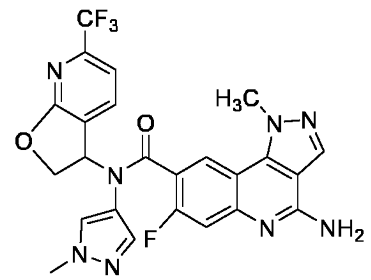
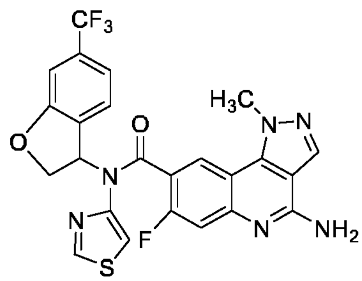
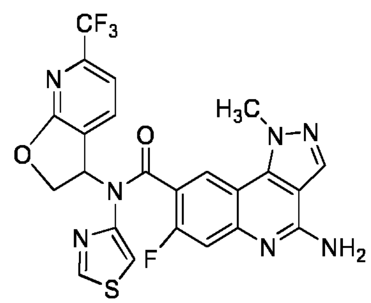
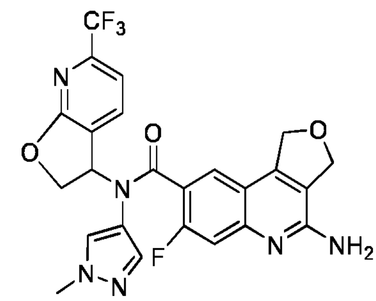
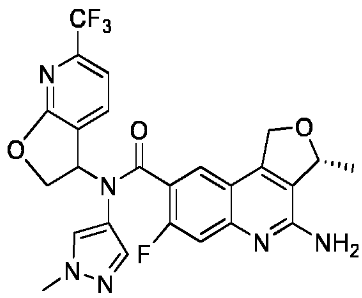

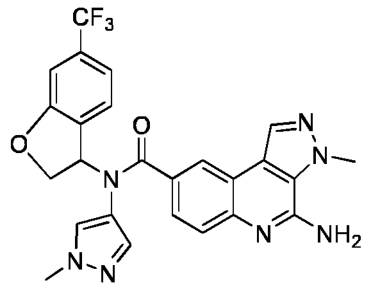
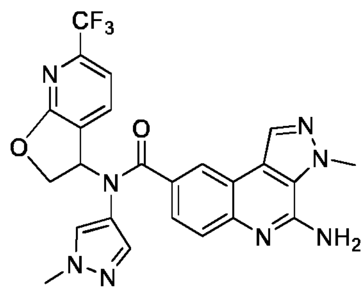
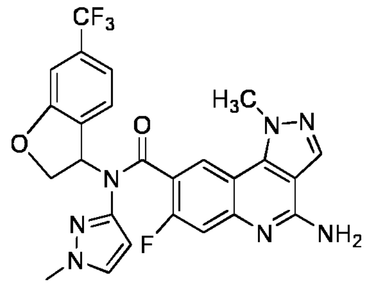

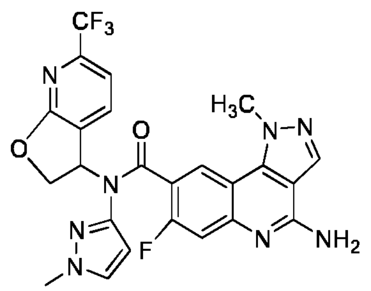
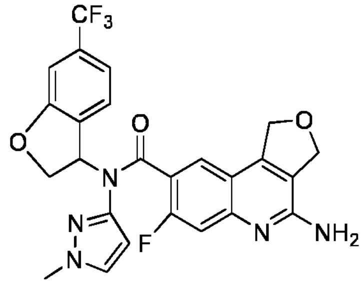
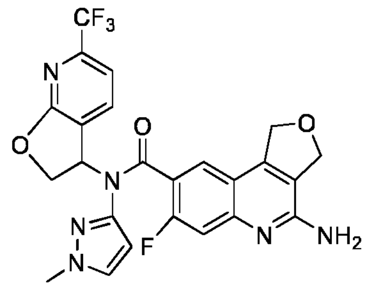

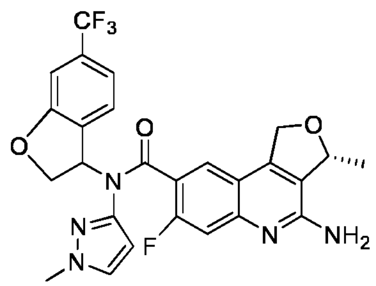
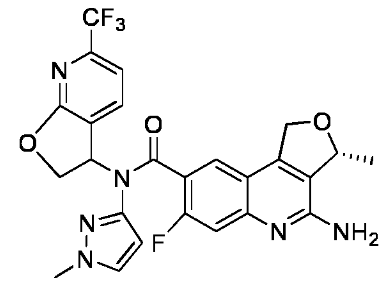
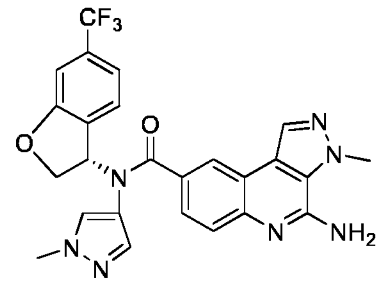

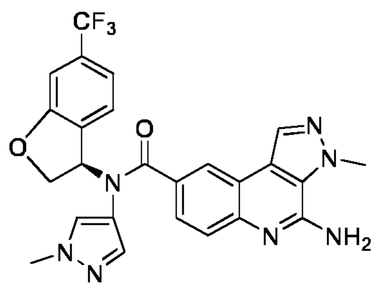
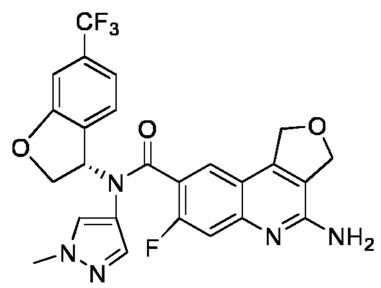

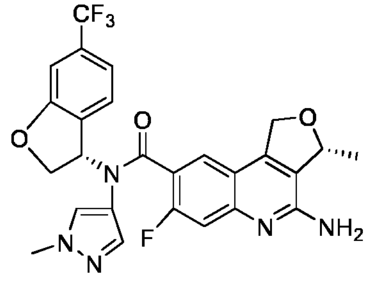
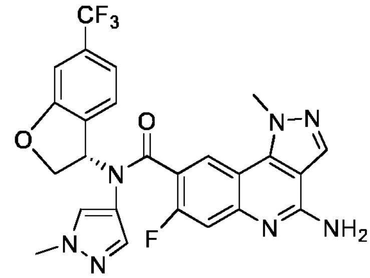
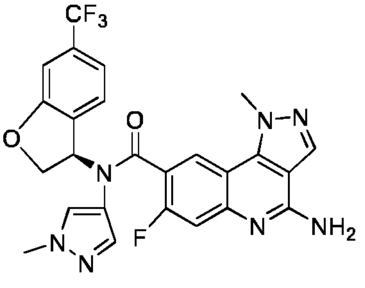

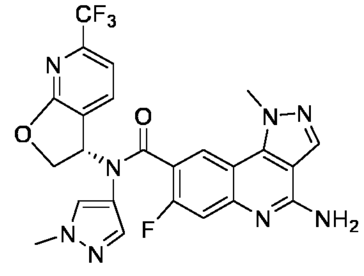
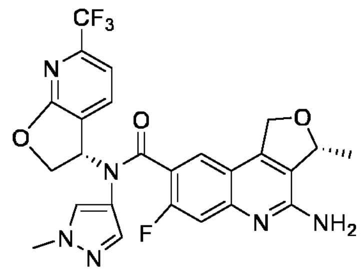
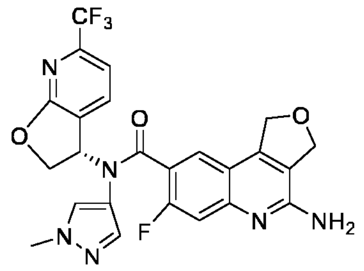
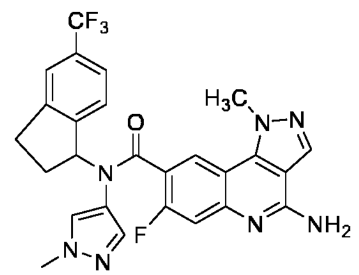
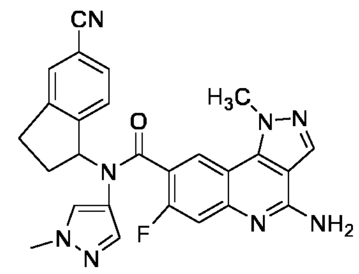
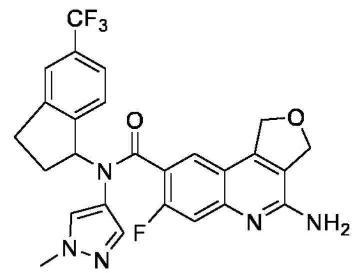
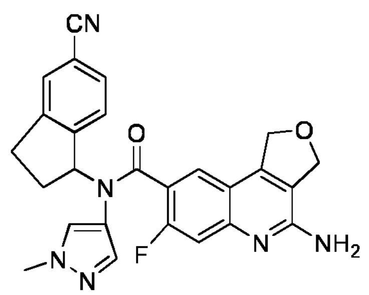
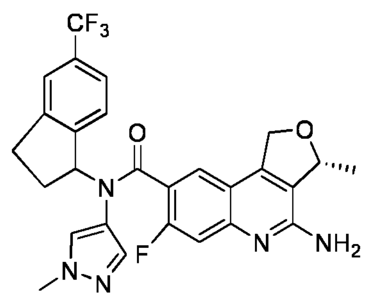
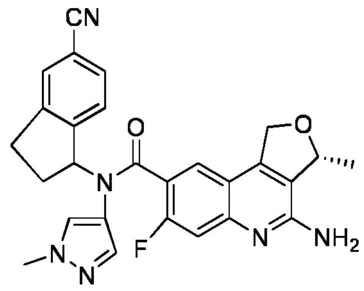
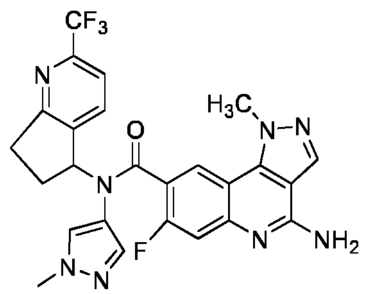
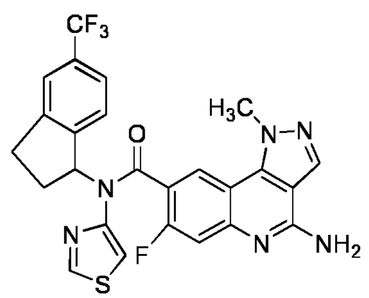
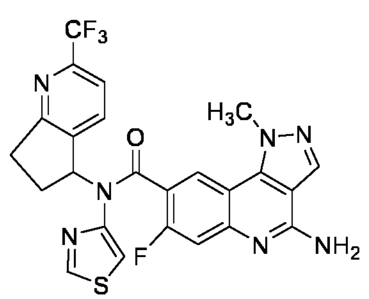
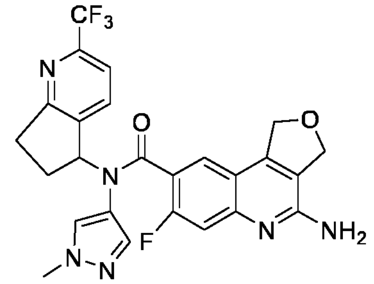
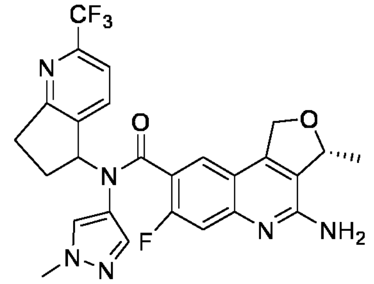

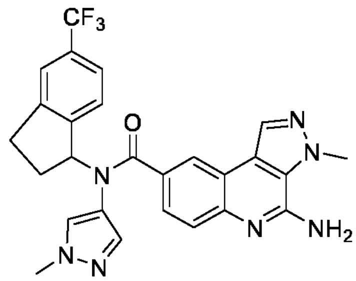

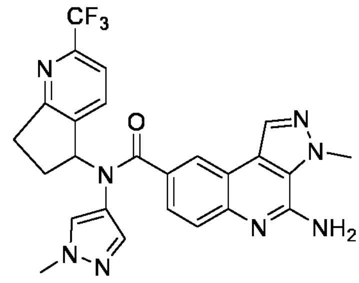

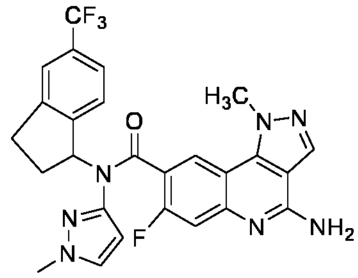

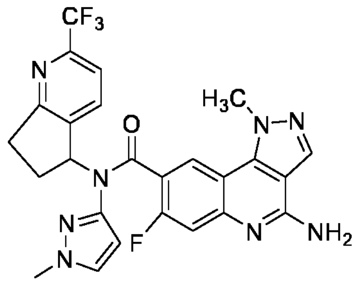

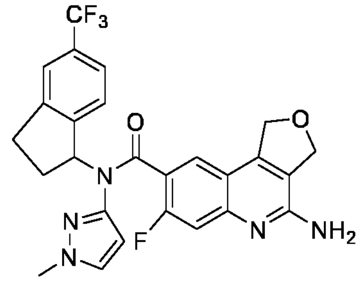

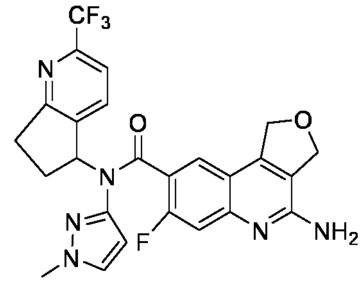

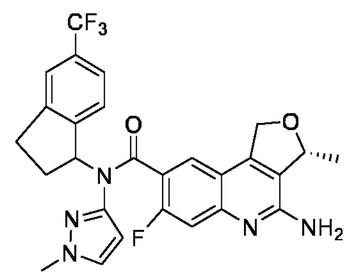

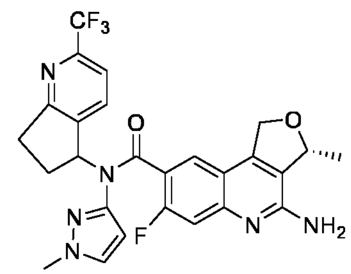

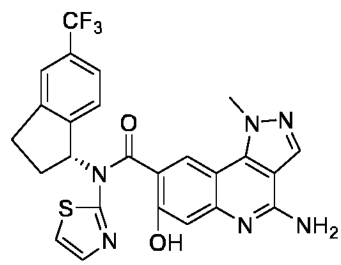

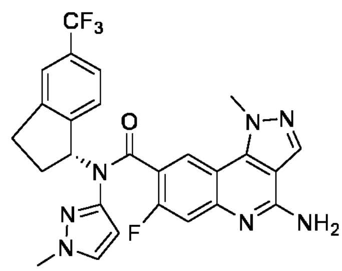

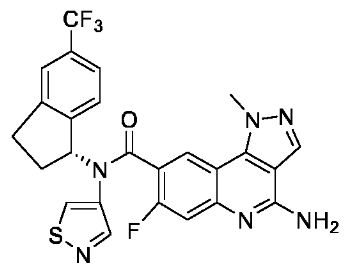

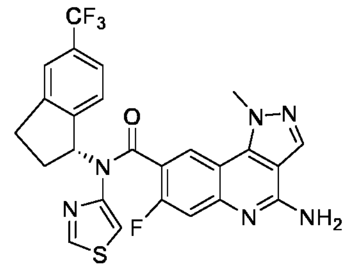

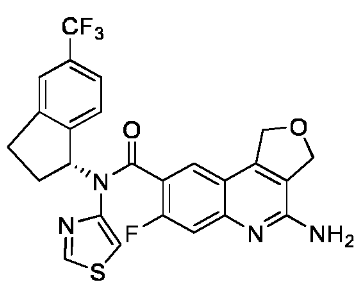

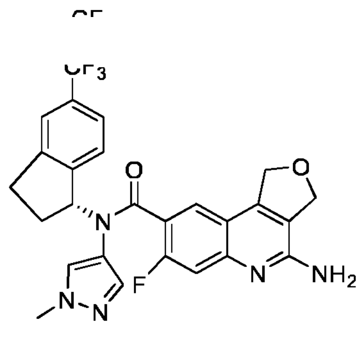

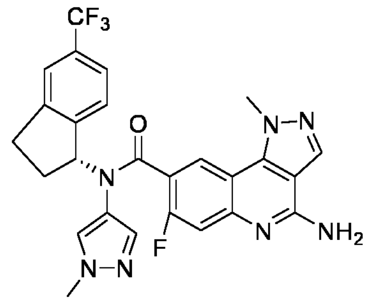

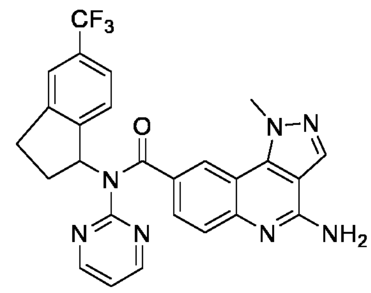

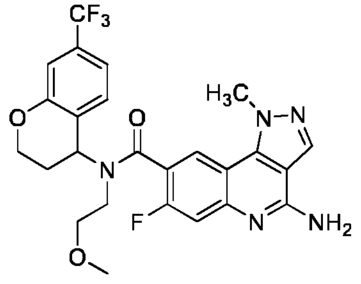

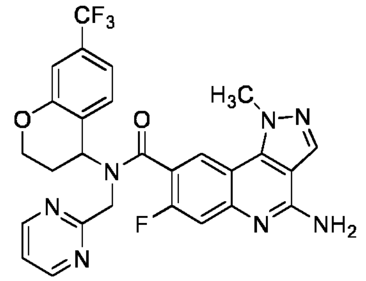

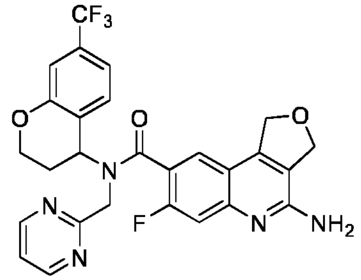

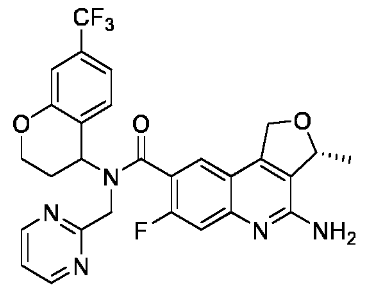

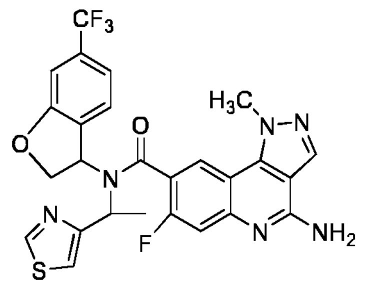

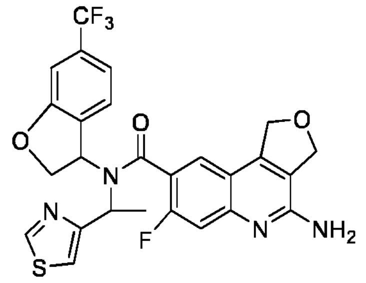

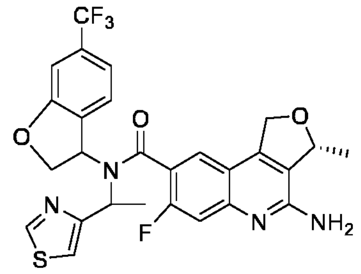

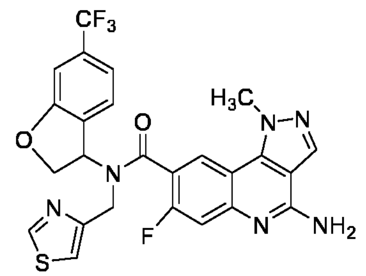

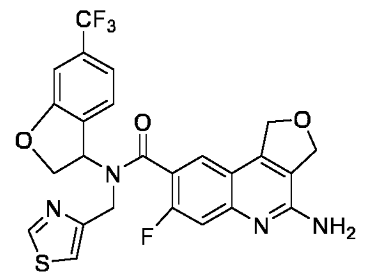

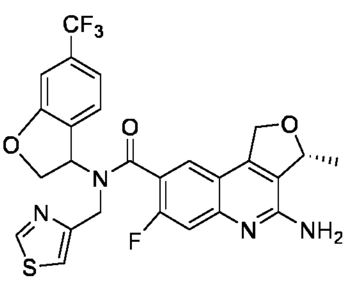

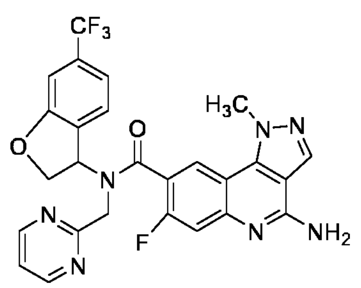

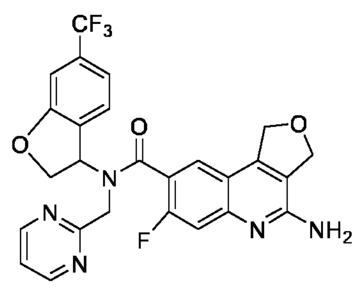

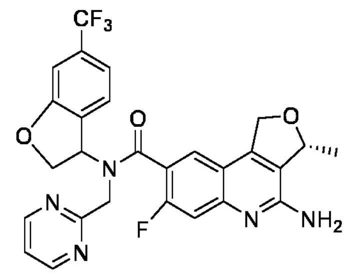

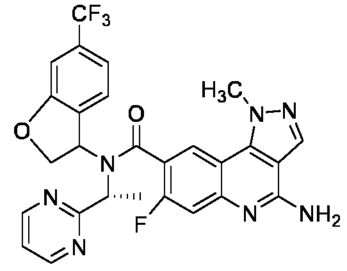

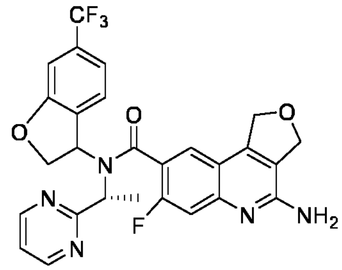

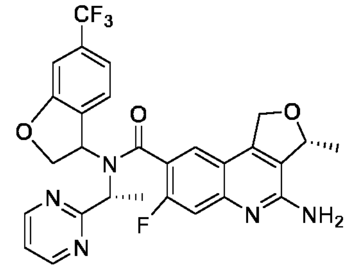

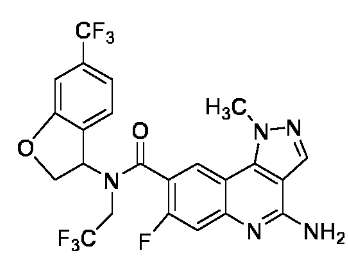

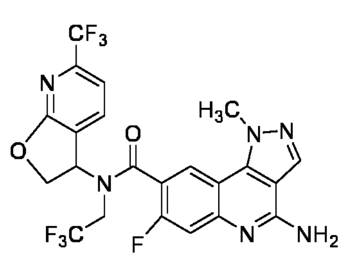

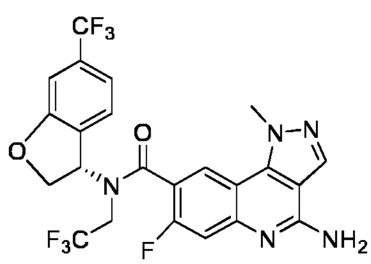

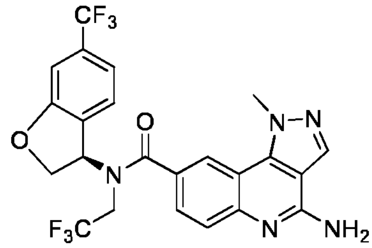

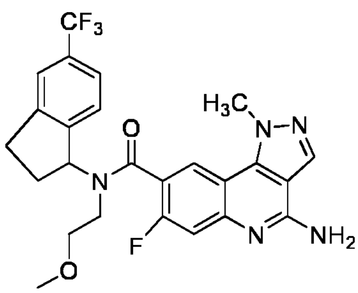

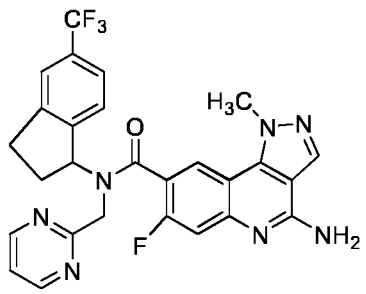

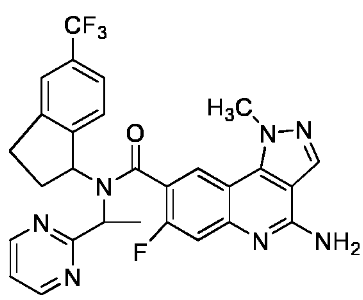

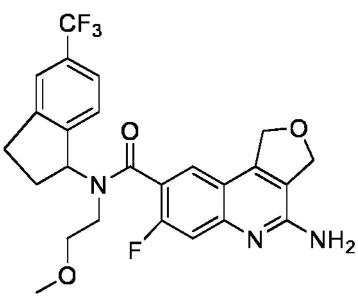

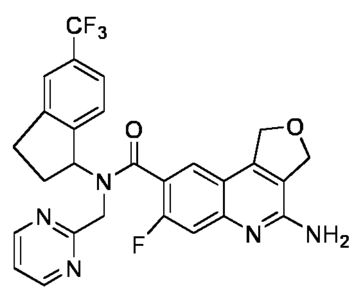

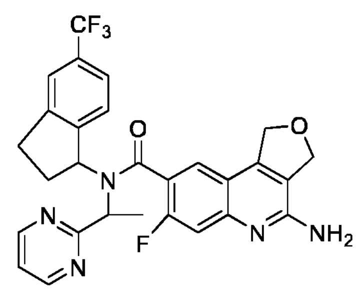

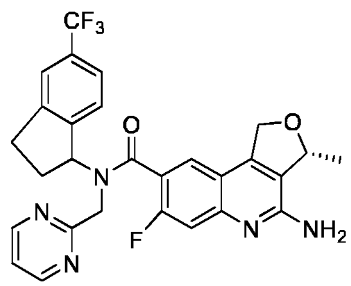

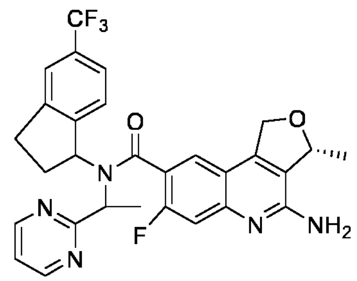

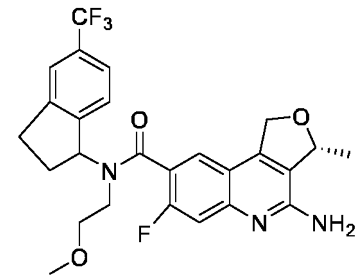

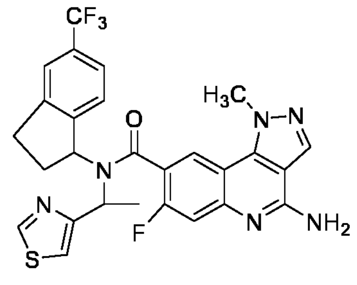

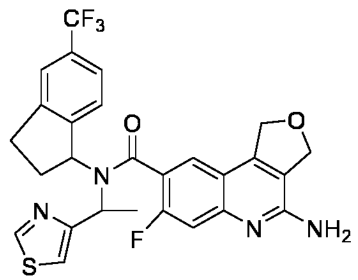

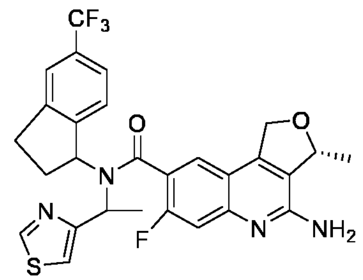

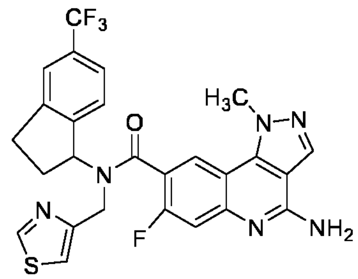

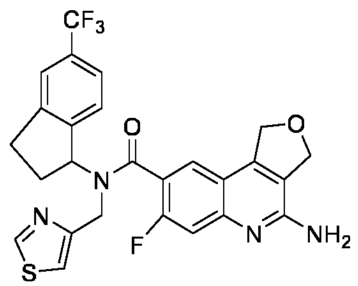

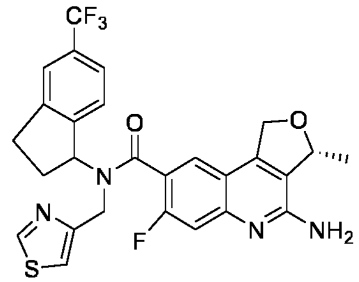

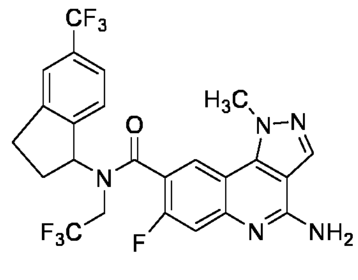

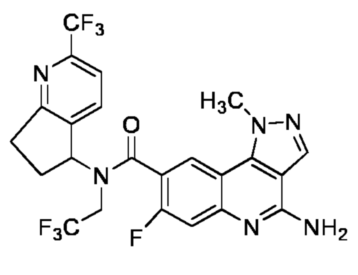

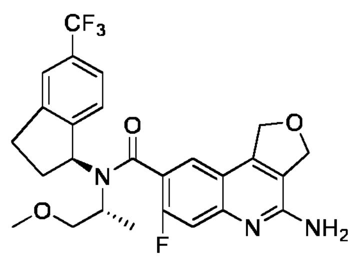

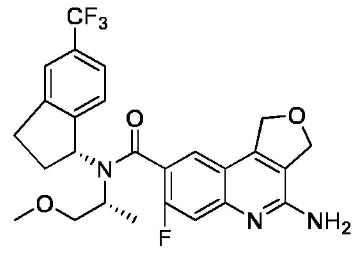

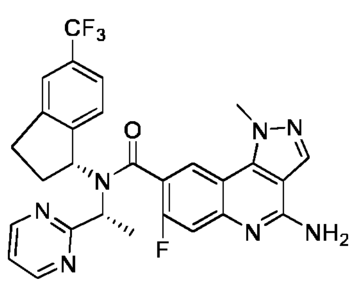

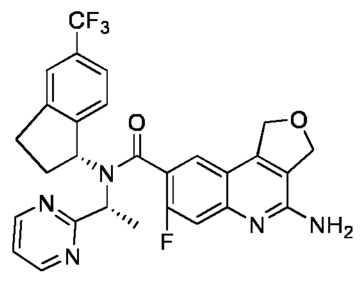

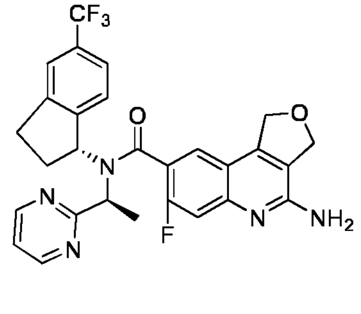

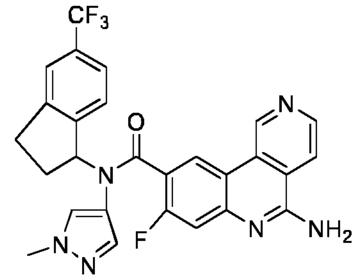

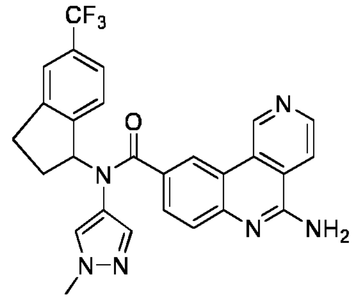

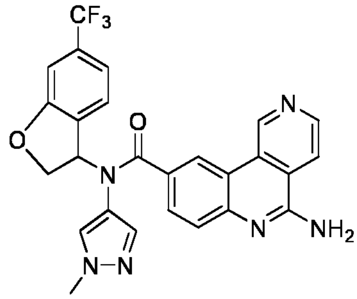

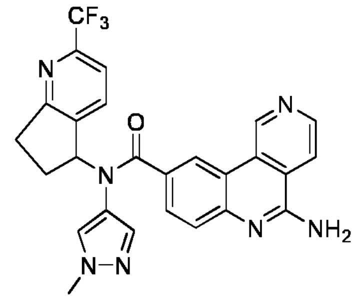

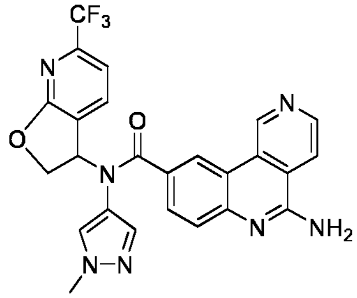

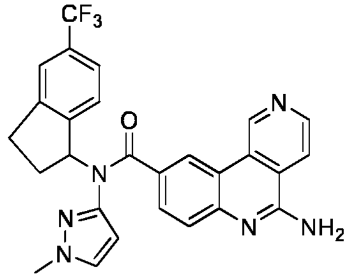

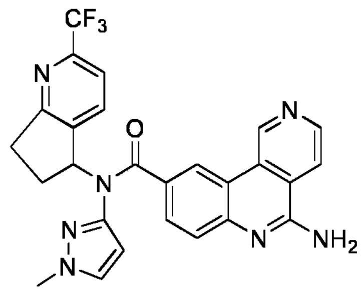

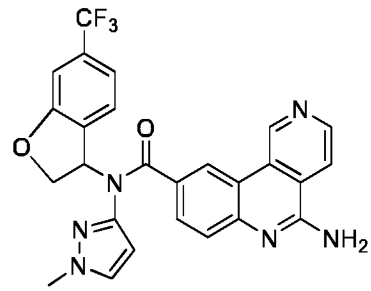

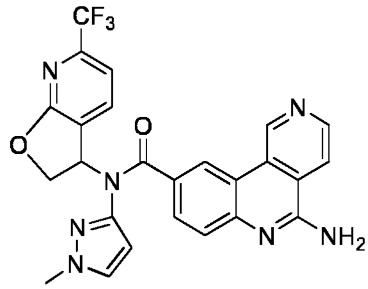

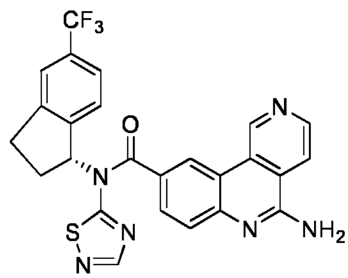

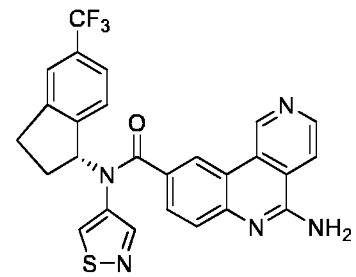

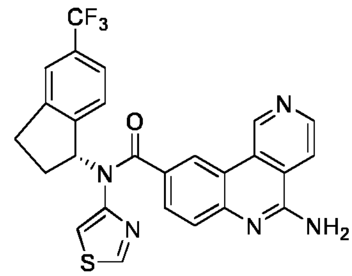

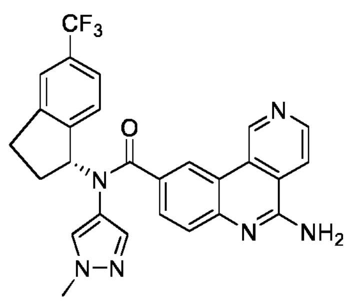

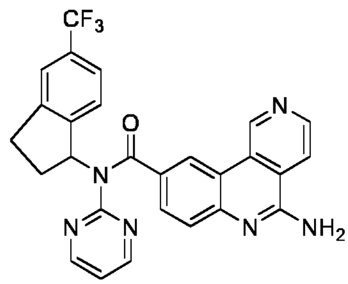

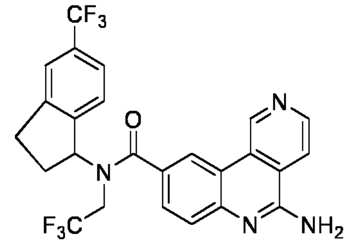

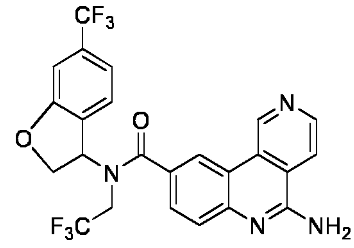

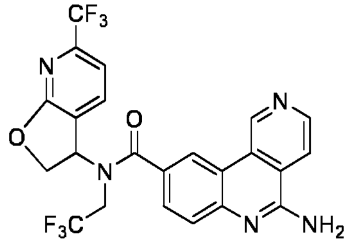

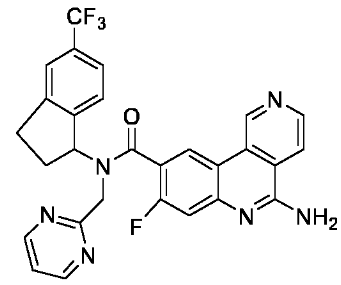

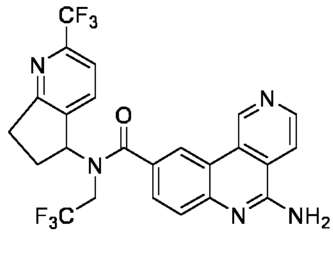

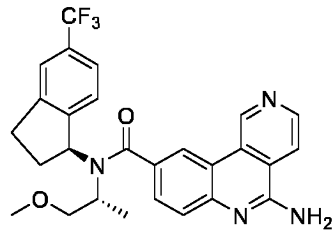

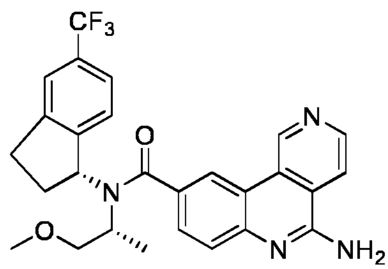

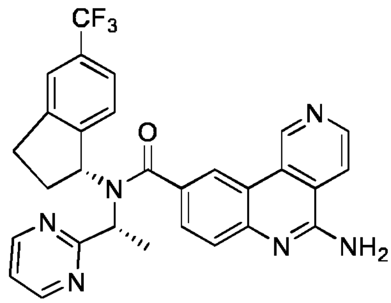

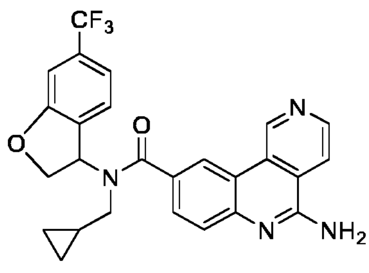

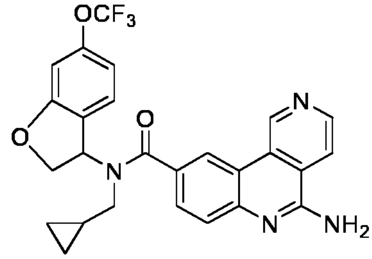

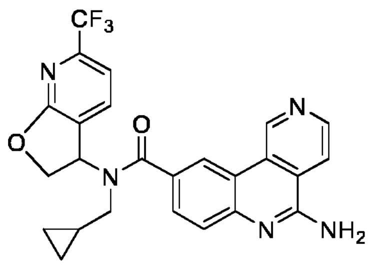

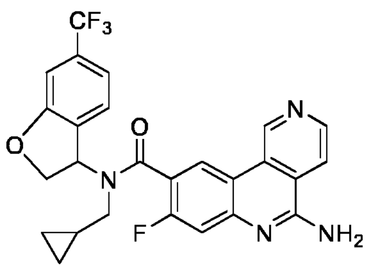

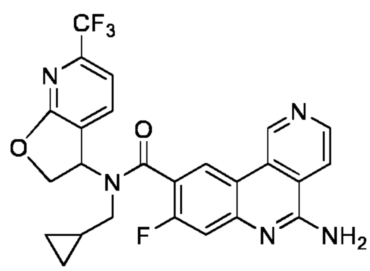

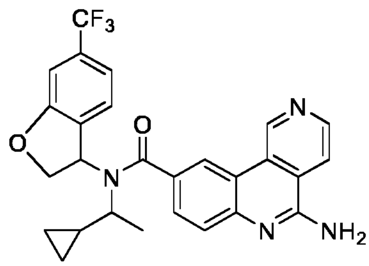

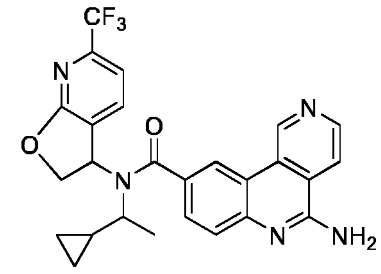

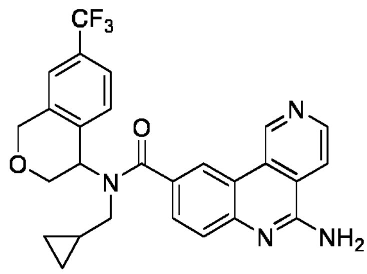

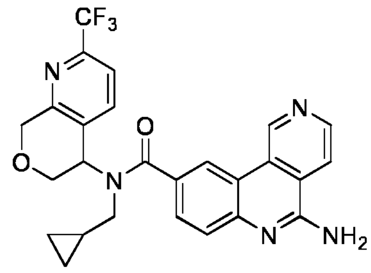

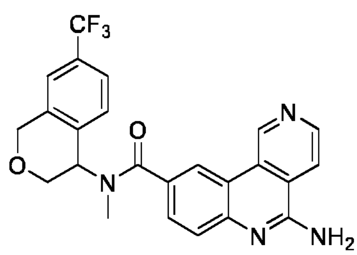

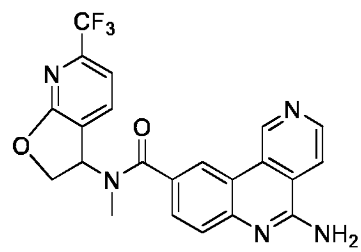

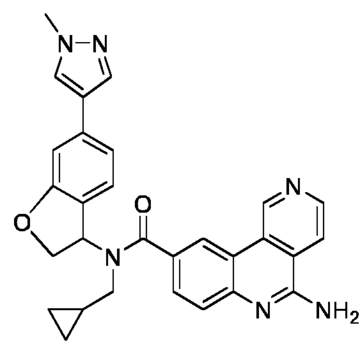

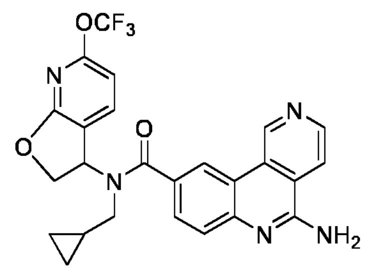

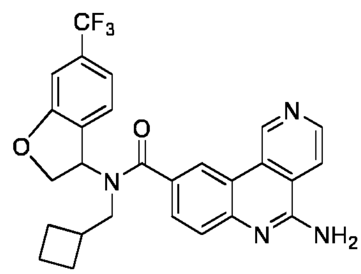

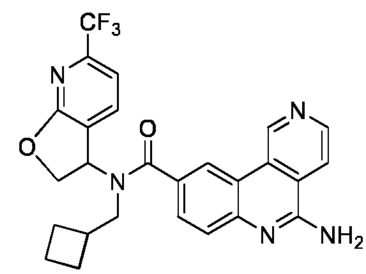

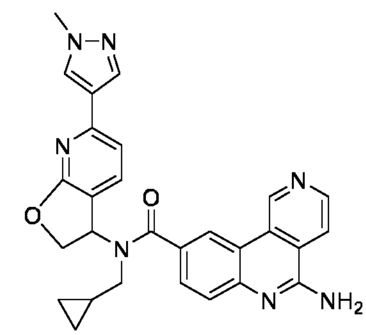

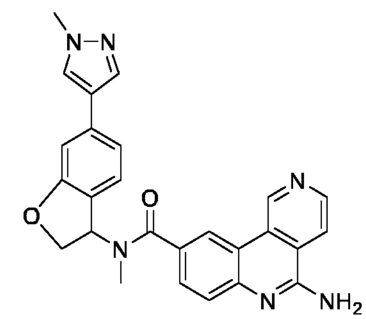

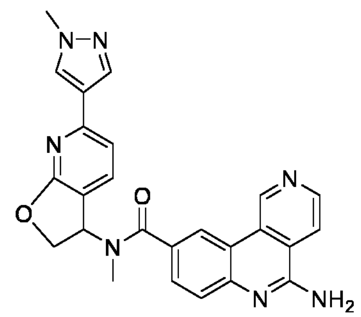

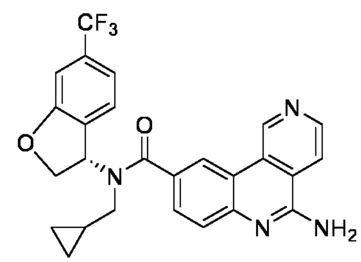

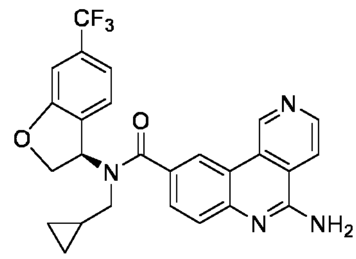

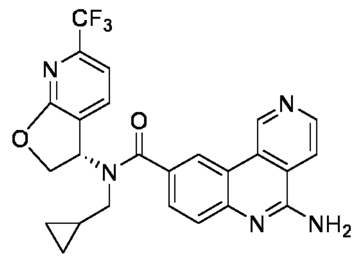

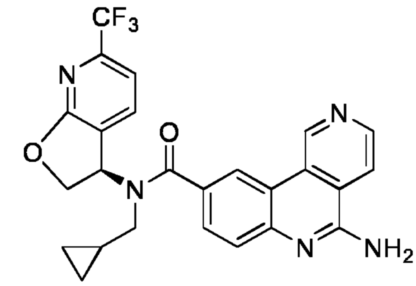

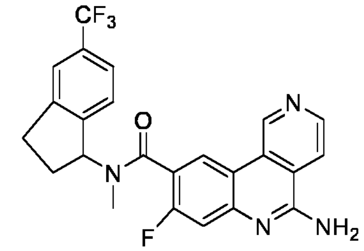

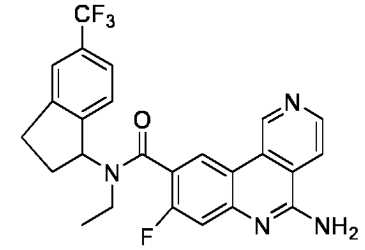

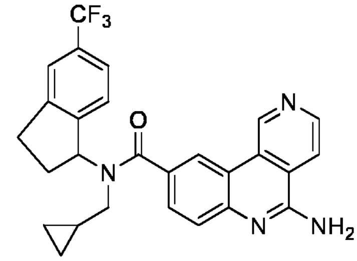

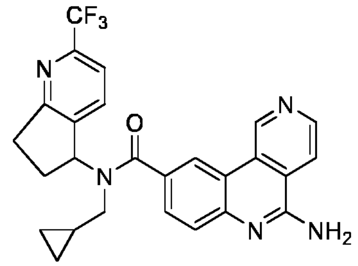

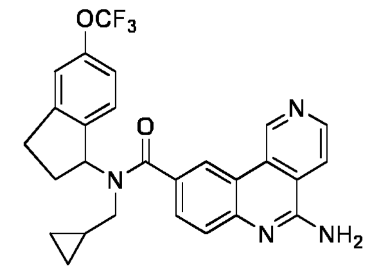

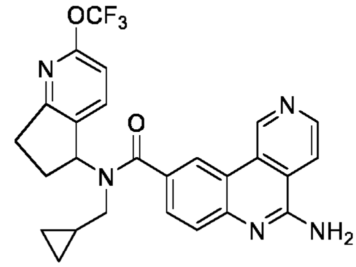

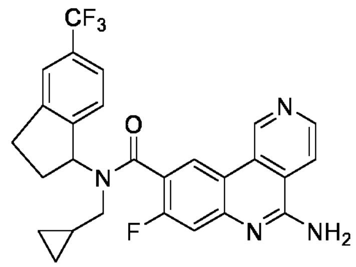

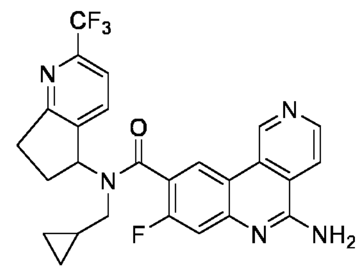

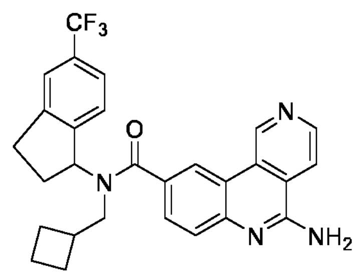

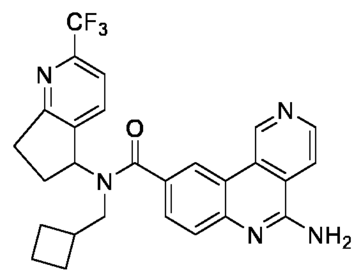

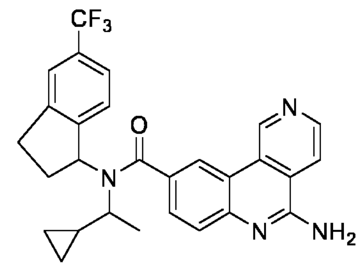

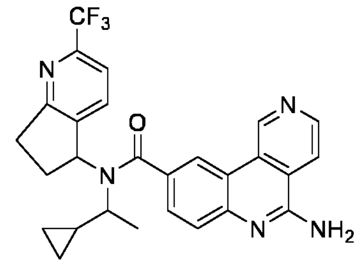

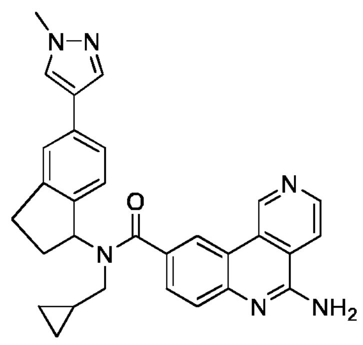
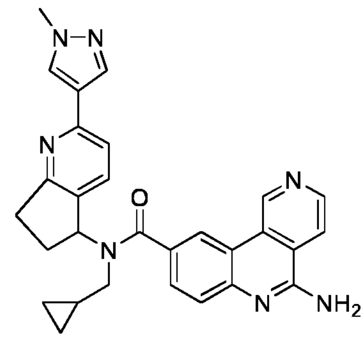
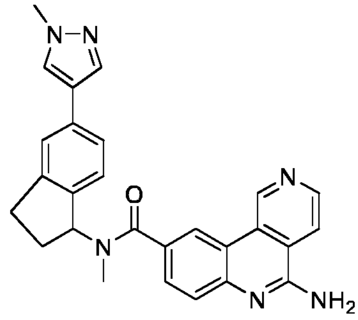

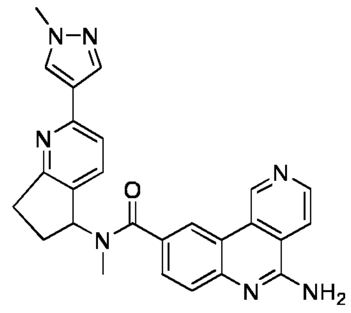
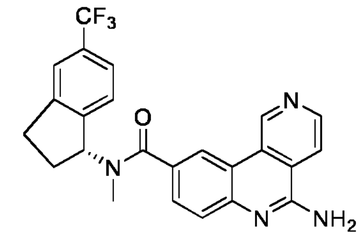
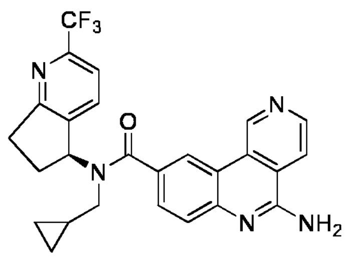

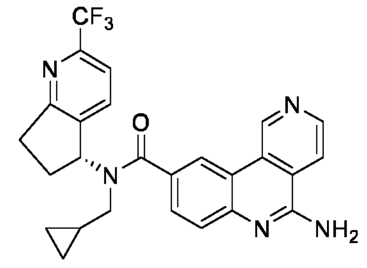
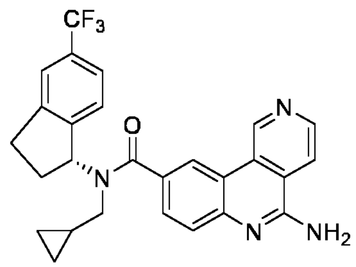
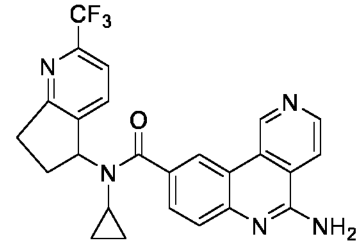

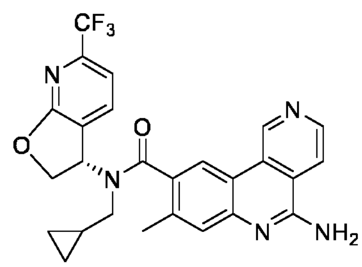
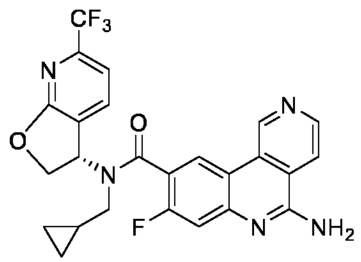
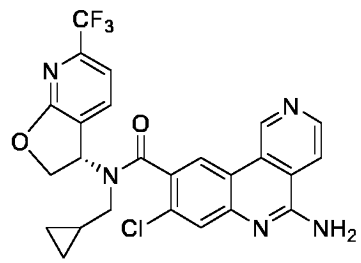

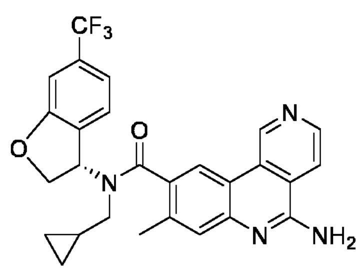
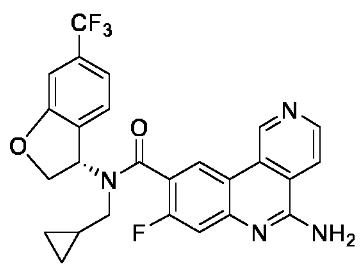
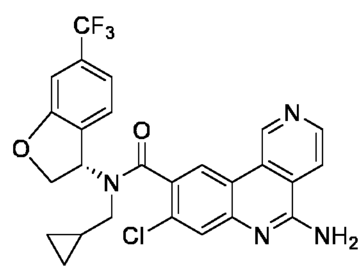

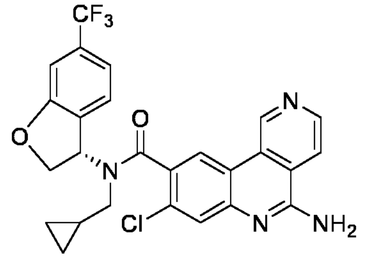

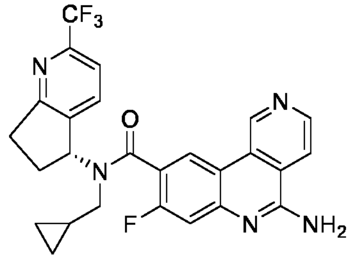

and

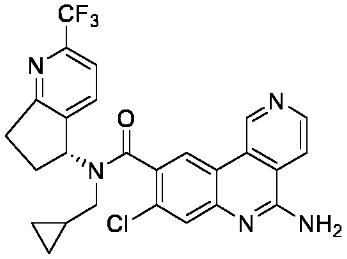

.

The second aspect of the present invention provides a process for preparing the compound of formula (I), the stereoisomer or pharmaceutically acceptable salt thereof, comprising the following step:

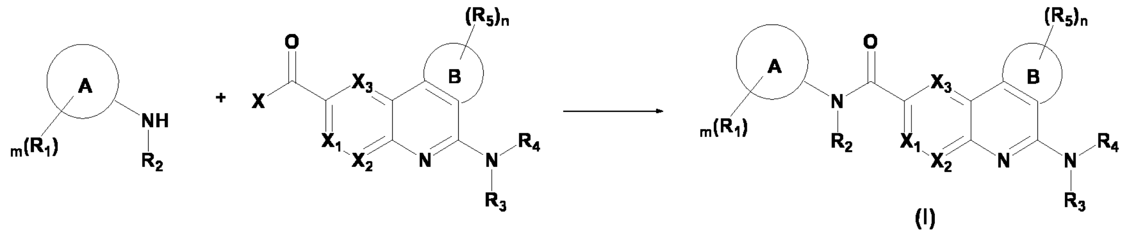

(I)

wherein, X is fluorine, chlorine, bromine or hydroxy, and ring A, ring B, $X_1$, $X_2$, $X_3$, $R_1$, $R_2$, $R_3$, $R_4$, $R_5$, m, and n are defined as in the compound of formula (I).

The third aspect of the present invention provides a pharmaceutical composition comprising the compound of formula (I), the stereoisomer or pharmaceutically acceptable salt thereof, and a pharmaceutically acceptable carrier.

The fourth aspect of the present invention provides a use of the compound of formula (I), the stereoisomer or pharmaceutically acceptable salt thereof in the preparation of a medicament for treating MATP-associated cancer or tumor.

As a preferred embodiment, the tumor or cancer is selected from the group consisting of endometrial carcinoma, granulosa-theca cell tumor, Sertoli-Leydig cell tumor, germinomas, malignant teratoma, squamous cell carcinoma, intraepithelial cancer, adenocarcinoma, fibrosarcoma, melanoma, clear cell carcinoma, squamous cell carcinoma, botryoid sarcoma, fallopian tube cancer, adenocarcinoma, nephroblastoma, lymphoma, leukemia, bladder cancer, squamous cell carcinoma, transitional cell carcinoma, adenocarcinoma, prostate cancer, seminoma, teratoma, embryonal carcinoma, teratoma, choriocarcinoma, sarcoma, mesenchymal cell carcinoma, fibroma, fibroadenoma, adenomatoid tumor, lipoma; liver cancer, cholangiocarcinoma, hepatoblastoma, hemangiosarcoma, hepatocellular adenoma, hemangioma, gallbladder cancer, ampullary carcinoma, cholangiocarcinoma, malignant melanoma, basal cell carcinoma, squamous cell carcinoma, Kaposi's sarcoma, moles, dysplastic nevus, lipomyoma, hemangioma, acute and chronic myeloid leukemia, acute lymphoblastic leukemia, chronic lymphoblastic leukemia, myeloproliferative disorder, multiple myeloma, myelodysplastic syndrome, Hodgkin's disease, non-Hodgkin's lymphoma, osteosarcoma, fibrosarcoma, malignant fibrous histiocytoma, chondrosarcoma, Ewing's sarcoma, malignant lymphoma, multiple myeloma, malignant giant cell tumor chordoma, osteochondroma, benign chondroma, chondroblastoma, chondromyxoid fibroma, osteoid osteoma, giant cell tumor, angiosarcoma, fibrosarcoma, rhabdomyosarcoma, liposarcoma, myxoma, rhabdomyoma, fibroma, lipomyoma and teratoma, bronchial carcinoma, alveolar carcinoma, bronchial adenoma, sarcoma, lymphoma, chondromatoid hamartoma, mesothelioma squamous cell carcinoma, adenocarcinoma, leiomyosarcoma, lymphoma, gastric cancer, lymphoma, leiomyosarcoma, ductal adenocarcinoma, insulinoma, glucagonoma, gastrinoma, carcinoid tumor, serpentine tumor, adenocarcinoma, lymphoma, carcinoid tumor, Kaposis sarcoma, leiomyoma, hemangioma, lipomyoma, neurofibroma, fibroma, colorectal adenocarcinoma, tubular adenoma, villous adenoma, hamartoma, leiomyoma, skull tumor, hemangioma, granuloma, xanthoma, osteitis deformans, meningioma, meningeal sarcoma, gliomatosis, astrocytoma, medulloblastoma, glioma, ependymoma, germ cell tumor, glioblastoma multiforme, oligodendroglioma, neurilemmoma, retinoblastoma, congenital tumor, spinal cord neurofibroma, meningioma, glioma or sarcoma.

As a more preferred embodiment, the cancer or tumor is selected from the group consisting of breast cancer, pancreatic cancer, skin cancer, bladder cancer, liver cancer and head and neck cancer.

The present invention also relates to the compound of formula (I), the stereoisomer or pharmaceutically acceptable salt thereof, for use as a PRMT5 inhibitor drug.

The present invention also relates to a use of the compound of formula (I), the stereoisomer or pharmaceutically acceptable salt thereof in the preparation of a medicament for treating and/or preventing PRMT5-mediated diseases.

The present invention also relates to a method for treating and/or preventing PRMT5-mediated diseases,

comprising administering to a patient in need a therapeutically effective amount of the compound of formula (I), the stereoisomer or pharmaceutically acceptable salt thereof.

## DETAILED DESCRIPTION OF EMBODIMENTS

After an extensive and intensive research, the inventors of the present invention develop a PRMT5 inhibitor with the structure of formula (I) for the first time. The series of compounds of the present invention can be widely applied in the preparation of drugs for treating and/or preventing PRMT5-mediated diseases, and are expected to be developed into a new generation of PRMT5 inhibitors. The present invention is achieved on this basis.

Detailed description: unless otherwise stated or specified, the following terms used in the specification and claims have the following meanings.

"Alkyl" refers to linear or branched saturated aliphatic alkyl groups, preferably including a linear or branched alkyl having 1 to 10 or 1 to 6 carbon atoms or 1 to 4 carbon atoms, including but not limited to methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof, etc. "$C_{1-10}$ alkyl" means a linear or branched alkyl containing 1 to 10 carbon atoms, "$C_{1-4}$ alkyl" means a linear or branched alkyl containing 1 to 4 carbon atoms, "$C_{0-8}$ alkyl" means a linear or branched alkyl containing 0 to 8 carbon atoms, and "$C_{0-4}$ alkyl" means a linear or branched alkyl containing 0 to 4 carbon atoms.

Alkyl can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkylS$(O)_rR_9$, -$C_{0-8}$ alkyl-O-$R_{10}$, -$C_{0-8}$ alkyl-C(O)O$R_{10}$, -$C_{0-8}$ alkyl-C(O)S$R_{10}$, -$C_{0-8}$ alkyl-S-C(O)$R_{11}$, - $C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-N$R_{12}R_{13}$, -$C_{0-8}$ alkyl-C(O)N$R_{12}R_{13}$ and -$C_{0-8}$ alkyl-N($R_{12}$)-C(O) $R_{11}$.

"Cycloalkyl" or "carbocyclic ring" refers to monocyclic or polycyclic hydrocarbon substituents that are saturated or partially unsaturated, wherein the partially unsaturated cyclic hydrocarbon refers to a cyclic hydrocarbon that may contain one or more (preferably 1, 2 or 3) double bonds, but none of the rings have a fully conjugated π-electron system. Cycloalkyl may be monocyclic cycloalkyl or polycyclic cycloalkyl, preferably including a cycloalkyl containing 3 to 12 or 3 to 8 or 3 to 6 carbon atoms. For example, "$C_{3-12}$ cycloalkyl" means a cycloalkyl having 3 to 12 carton atoms, "$C_{4-8}$ cycloalkyl" means a cycloalkyl having 4 to 8 carton atoms, "$C_{3-8}$ cycloalkyl" means a cycloalkyl having 3 to 8 carton atoms, and "$C_{3-6}$ cycloalkyl" means a cycloalkyl having 3 to 6 carton atoms, wherein:

monocyclic cycloalkyl includes but is not limited to cyclopropyl, cyclobutyl, cyclopentyl, cyclopentenyl, cyclohexyl, cyclohexenyl, cyclohexadienyl, cycloheptyl, cycloheptarienyl, cyclooctyl, etc.

Polycyclic cycloalkyl includes spirocycloalkyl, fused cycloalkyl and bridged cycloalkyl. "Spirocycloalkyl" refers to a polycyclic group in which a carton atom (called spiro-atom) is shared among monocyclic rings, wherein these rings may contain one or more (preferably 1, 2 or 3) double bonds, but none of them have a fully conjugated π-electron system. According to the number of the spiro-atoms shared among the rings, the spirocycloalkyl may be monospirocycloalkyl, bispirocycloalkyl or polyspirocycloalkyl, including but not limited to:

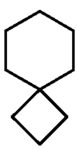 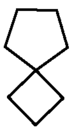 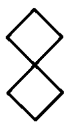 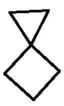 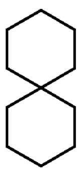 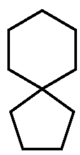 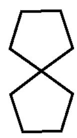 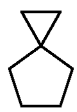.

"Fused cycloalkyl" refers to an all-carbon polycyclic group in which each ring shares a pair of adjacent carbon atoms with other rings in the system, wherein one or more of the rings may contain one or more (preferably 1, 2 or 3) double bonds, but none of them have a fully conjugated π-electron system. According to the number of formed rings, the fused cycloalkyl may be bicyclic, tricyclic, tetracyclic or polycyclic, including but not limited to:

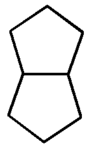 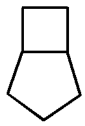 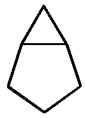 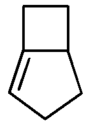 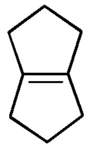

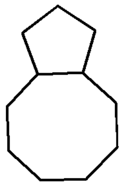 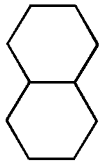 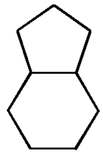 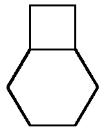 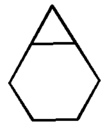 .

"Bridged cycloalkyl" refers to an all-carbon polycyclic group in which any two rings share two carbon atoms that are not directly connected to each other, wherein these rings may contain one or more (preferably 1, 2 or 3) double bonds, but none of them have a fully conjugated $\pi$-electron system. According to the number of formed rings, the bridged cycloalkyl may be bicyclic, tricyclic, tetracyclic or polycyclic, including but not limited to:

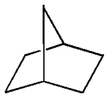 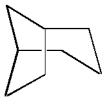 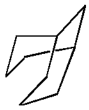 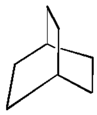 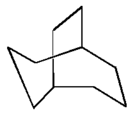 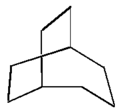 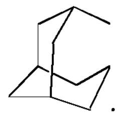 .

The cycloalkyl ring may be fused to an aryl, heteroaryl or heterocycloalkyl ring, wherein the ring attached to the parent structure is cycloalkyl, which includes, but is not limited to, indenyl, tetrahydronaphthyl, benzocycloheptyl, etc.

"Cycloalkyl" or "carbocyclic ring" can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, $-C_{0-8}$ alkyl-$SF_5$, $-C_{0-8}$ alkyl-O-$S(O)_2R_9$, $-C_{0-8}$ alkyl-$S(O)_rR_9$, $-C_{0-8}$ alkyl-O-$R_{10}$, $-C_{0-8}$ alkyl-$C(O)OR_{10}$, $-C_{0-8}$ alkyl-$C(O)SR_{10}$, $-C_{0-8}$ alkyl-S-$C(O)R_{11}$, $-C_{0-8}$ alkyl-$C(O)R_{11}$, $-C_{0-8}$ alkyl-O-$C(O)R_{11}$, $-C_{0-8}$ alkyl-$P(O)(R_{11})_2$, $-C_{0-8}$ alkyl-$NR_{12}R_{13}$, $-C_{0-8}$ alkyl-$C(O)NR_{12}R_{13}$, and $-C_{0-8}$ alkyl-$N(R_{12})$-$C(O)R_{11}$.

"Heterocyclyl" or "heterocycle" refers to a monocyclic or polycyclic hydrocarbon substituent that is saturated or partially unsaturated, wherein the partially unsaturated cyclic hydrocarbon refers to a cyclic hydrocarbon that may contain one or more (preferably 1, 2 or 3) double bonds, but none of the rings have a fully conjugated $\pi$-electron system, wherein one or more (preferably 1, 2, 3 or 4) of the rings atoms in heterocyclyl are heteroatoms selected from N, O, N•O or $S(O)_r$ (wherein r is an integer of 0, 1 or 2), excluding ring portions of -O-O-, -O-S- or -S-S-, and the remaining ring atoms are carton atoms. It preferrably includes heterocyclyl containing 3 to 12 or 3 to 8 or 3 to 6 ring atoms. For example, "3-6 membered heterocyclyl" means a heterocyclyl containing 3 to 6 ring atoms, "3-8 membered heterocyclyl" means a heterocyclyl containing 3 to 8 ring atoms, "4-8 membered heterocyclyl" means a heterocyclyl containing 4 to 8 ring atoms, "4-10 membered heterocyclyl" means a heterocyclyl containing 4 to 10 ring atoms, "5-8 membered heterocyclyl" means a heterocyclyl containing 5 to 8 ring atoms, and "3-12 membered heterocyclyl" means a heterocyclyl containing 3 to 12 ring atoms.

Monocyclic heterocyclyl includes but is not limited to pyrrolidinyl, piperidinyl, piperazinyl, morpholinyl, thiomorpholinyl, homopiperazinyl, oxacyclobutyl, tetrahydrofuranyl, etc.

Polycyclic heterocyclyl includes spiroheterocyclyl, fused heterocyclyl, and bridged heterocyclyl. "Spiroheterocyclyl" refers to a polycyclic heterocyclyl group in which an atom (called spiro-atom) is shared among monocyclic rings, wherein one or more (preferably 1, 2, 3 or 4) of the ring atoms are heteroatoms selected from N, O, N•O, or $S(O)_r$ (wherein r is an integer of 0, 1 or 2), and the remaining ring atoms are carbon atoms. These rings may contain one or more (preferably 1, 2 or 3) double bonds, but none of them have a fully conjugated $\pi$-electronic system. According to the number of sprio-atoms shared among the rings, spiroheterocyclyl may be monospiroheterocyclyl, bispiroheterocyclyl or polyspiroheterocyclyl. Spiroheterocyclyl includes but is not limited to:

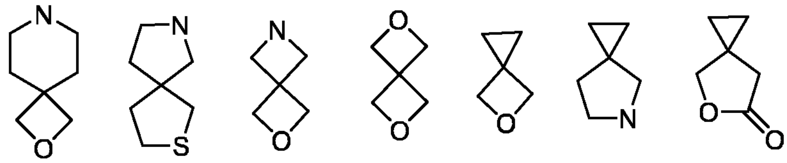

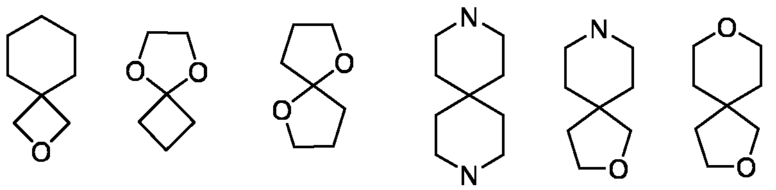
.

"Fused heterocyclyl" refers to a polycyclic heterocyclyl in which each ring shares a pair of adjacent atoms with the other rings in the system, wherein one or more (preferably 1, 2, 3 or 4) of the rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N•O, or $S(O)_r$ (wherein r is an integer of 0, 1 or 2), and the remaining ring atoms are carbon atoms. According to the number of formed rings, the fused heterocyclyl may be bicyclic, tricyclic, tetracyclic or polycyclic, including but not limited to:

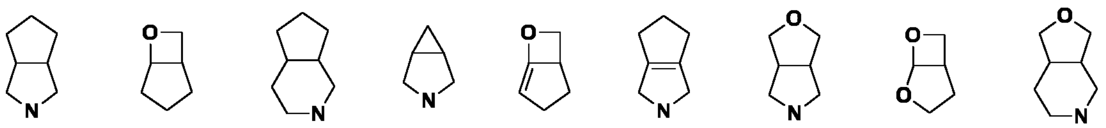

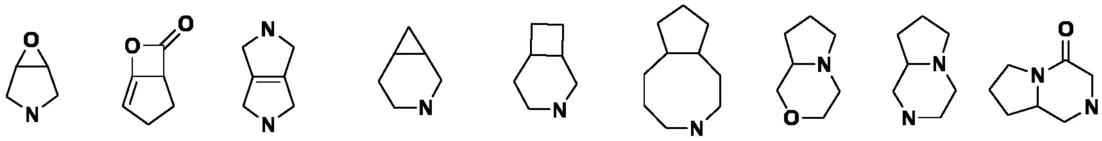

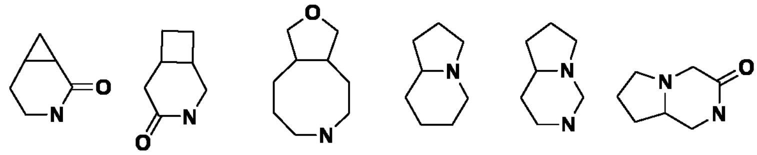

"Bridged heterocyclyl" refers to a polycyclic heterocyclyl in which any two rings share two carton atoms that are not directly attached to each other, wherein these rings may contain one or more (preferably 1, 2, or 3) double bonds, but none of them have a fully conjugated π-electron system, wherein one or more (preferably 1, 2, 3, or 4) of the ring atoms are heteroatoms selected from N, O, N•O, or $S(O)_r$ (wherein r is an integer of 0, 1 or 2), and the remaining ring atoms are carbon atoms. According to the number of formed rings, the bridged heterocyclyl may be bicyclic, tricyclic, tetracyclic or polycyclic, including but not limited to:

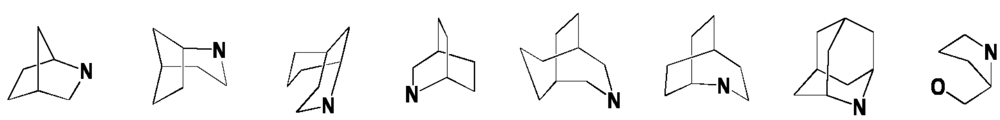
.

The heterocyclyl ring may be fused to an aryl, heteroaryl or cycloalkyl ring, wherein the ring attached to the parent structure is heterocyclyl, which includes but is not limited to:

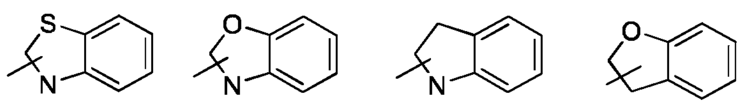

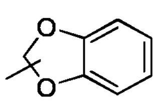 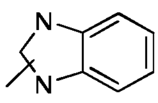 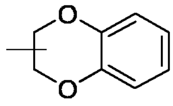 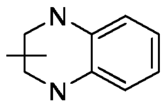.

"Heterocyclyl" or "heterocycle" can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Aryl" or "aromatic ring" means an all-carbon monocyclic or fused-polycyclic group (i.e., rings that share a pair of adjacent carbon atoms) and a polycyclic group having a conjugated $\pi$-electron system (i.e., rings with adjacent pairs of carbon atoms), preferably including all-carbon aryl containing 6-10 or 6-8 carbon atoms. For example, "$C_{6-10}$ aryl" means an all-carbon aryl containing 6-10 carbon atoms, including but not limited to phenyl and naphthyl, and "$C_{6-8}$ aryl" means an all-carbon aryl containing 6-8 carbon atoms. The aryl ring may be fused to an heteroaryl, heterocyclyl or cycloalkyl ring, wherein the ring attached to the parent structure is aryl ring, including but not limited to:

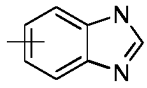 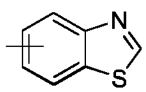 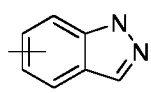 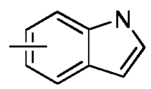 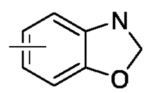

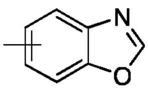 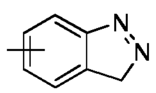 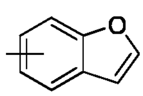 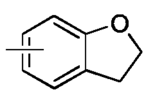 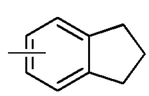

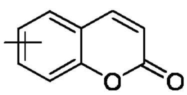 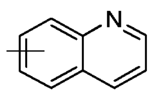 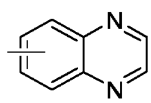 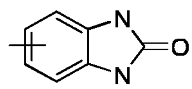 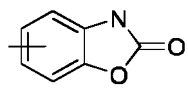.

"Aryl" or "aromatic ring" can be substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, - $C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Heteroaryl" or "heteroaromatic ring" refers to a heteroaromatic system containing one or more (preferably 1, 2, 3 or 4) of the heteroatoms, wherein the heteroatoms include heteroatoms selected from N, O, N•O, and S(O)r (wherein r is an integer of 0, 1 or 2), preferably including a heteroaromatic system containing 5-10 or 5-8 or 5-6 ring atoms. For example, "5-8 membered heteroaryl" means a heteroaromatic system containing 5-8 ring atoms, and "5-10 membered heteroaryl" means a heteroaromatic system containing 5-10 ring atoms, including but not limited to furyl, thiophenyl, pyridyl, pyrrolyl, N-alkylpyrrolyl, pyrimidinyl, pyrazinyl, imidazolyl, tetrazolyl, etc. The heteroaryl ring may be fused to an aryl, heterocyclyl or cycloalkyl ring, wherein the ring attached to the parent structure is heteroaryl ring, including but not limited to:

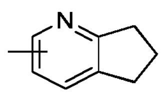 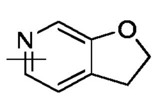 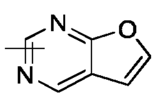 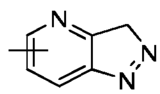 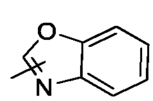

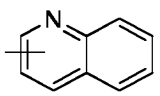 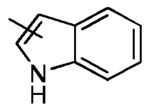 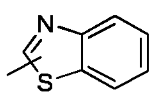 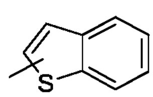 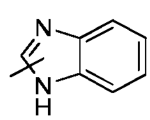.

"Heteroaryl" or "heteroaromatic ring" can be optionally substituted or unsubstituted, and when it is substituted, the substituent is preferably one or more (preferably 1, 2, 3, or 4) of the groups independently selected from the group

consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Alkenyl" refers to an alkyl defined as above consisting of at least two carbon atoms and at least one carbon-carbon double bond, preferably including a linear or branched alkenyl containing 2-10 or 2-4 carbon atoms. For example, "$C_{2-10}$ alkenyl" means a linear or branched alkenyl containing 2-10 carbon atoms, and "$C_{2-4}$ alkenyl" means a linear or branched alkenyl containing 2-4 carbon atoms. The alkenyl includes, but is not limited to, vinyl, 1-propenyl, 2-propenyl, 1-,2-, or 3-butenyl, etc.

"Alkenyl" can be substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, - $C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Alkynyl" refers to an alkyl defined as above consisting of at least two carbon atoms and at least one carbon-carbon triple bond, preferably including a linear or branched alkynyl containing 2-10 or 2-4 carbon atoms. For example, "$C_{2-10}$ alkynyl" means a linear or branched alkynyl containing 2-10 carbon atoms, and "$C_{2-4}$ alkynyl" means a linear or branched alkynyl containing 2-4 carbon atoms. The alkynyl includes, but is not limited to, ethynyl, 1-propynyl, 2-propynyl, 1-,2-, or 3-butynyl, etc.

"Alkynyl" can be substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, - $C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Alkoxy" refers to an -O-alkyl group, wherein the alkyl is defined as above. For example, "$C_{1-10}$ alkoxy" means an alkyloxy containing 1-10 carbon atoms, "$C_{1-4}$ alkoxy" means an alkyloxy containing 1-4 carbon atoms, and "$C_{1-2}$ alkoxy" means an alkyloxy containing 1-2 carbon atoms, including but not limited to, methoxy, ethoxy, propoxy, butoxy, etc.

"Alkoxy" can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, - $C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O) $R_{11}$.

"Cycloalkoxy" or "cycloalkyloxy" refers to an -O-cycloalkyl group, wherein cycloalkyl is defined as above. For example, "$C_{3-12}$ cycloalkoxy" means a cycloalkyloxy containing 3-12 carbon atoms, and "$C_{3-6}$ cycloalkoxy" means a cycloalkyloxy containing 3-6 carbon atoms, including but not limited to cyclopropoxy, cyclobutoxy, cyclopentoxy, cyclohexoxy, etc.

"Cycloalkoxy" or "cycloalkyloxy" can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"Heterocycloxy" or "heterocyclyloxy" refers to an -O-heterocyclyl group, wherein the heterocyclyl is defined as above, including but not limited to, azetidyloxy, oxetanyloxy, azetidyloxy, nitrogen, oxetanyloxy, etc.

"Heterocycloxy" or "heterocyclyloxy" can be optionally substituted or unsubstituted, and when it is substituted, the substituent is preferably one or more (preferably 1, 2, 3, or 4) of the groups independently selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$, and -$C_{0-8}$ alkyl-N($R_{12}$)-C(O)$R_{11}$.

"$C_{1-10}$ alkanoyl" refers to a monovalent atomic group which is obtained after a hydroxy is removed from the $C_{1-10}$

alkyl acid, and is also generally referred to as "$C_{0-9}$ alkyl-C(O)-". For example, "$C_1$ alkyl-C(O)-" refers to acetyl; "$C_2$ alkyl-C(O)-" refers to propanoyl; "$C_3$ alkyl-C(O)-" refers to butanoyl or isobutanoyl.

"-$C_{0-8}$ alkyl-O-$S(O)_2R_9$" refers to the oxygen atom in -O-$S(O)_2R_9$ attached to $C_{0-8}$ alkyl, wherein the $C_{0-8}$ alkyl is defined as above.

"-$C_{0-8}$ alkyl-$S(O)_rR_9$" refers to the sulfur atom in -$S(O)_rR_9$ attached to $C_{0-8}$ alkyl, wherein the $C_{0-8}$ alkyl is defined as above.

"-$C_{0-8}$ alkyl-O-$R_{10}$" refers to the oxygen atom in -O-$R_{10}$ attached to $C_{0-8}$ alkyl, wherein the $C_{0-8}$ alkyl is defined as above.

"-$C_{0-8}$ alkyl-C(O)$OR_{10}$" refers to the carbonyl in -C(O)$OR_{10}$ attached to $C_{0-8}$ alkyl, wherein the $C_{0-8}$ alkyl is defined as above.

"-$C_{0-8}$ alkyl-C(O)$SR_{10}$" refers to the carbonyl in -C(O)$SR_{10}$ attached to $C_{0-8}$ alkyl, wherein the $C_{0-8}$ alkyl is defined as above.

"-$C_{0-8}$ alkyl-S-C(O)$R_{11}$" refers to the sulfur atom in -S-C(O)$R_{11}$ attached to $C_{0-8}$ alkyl, wherein the $C_{0-8}$ alkyl is defined as above.

"-$C_{0-8}$ alkyl-C(O)$R_{11}$" refers to the carbonyl in -C(O)$R_{11}$ attached to $C_{0-8}$ alkyl, wherein the $C_{0-8}$ alkyl is defined as above.

"-$C_{0-8}$ alkyl-O-C(O)$R_{11}$" refers to the oxygen atom in -O-C(O)$R_{11}$ attached to $C_{0-8}$ alkyl, wherein the $C_{0-8}$ alkyl is defined as above.

"-$C_{0-8}$ alkyl-P(O)$(R_{11})_2$" refers to the phosphorus atom in -P(O)$(R_{11})_2$ attached to $C_{0-8}$ alkyl, wherein the $C_{0-8}$ alkyl is defined as above.

"-$C_{0-8}$ alkyl-$NR_{12}R_{13}$" refers to the nitrogen atom in -$NR_{12}R_{13}$ attached to $C_{0-8}$ alkyl, wherein the $C_{0-8}$ alkyl is defined as above.

"-$C_{0-8}$ alkyl-C(O)$NR_{12}R_{13}$" refers to the carbonyl in -C(O)$NR_{12}R_{13}$ attached to $C_{0-8}$ alkyl, wherein the $C_{0-8}$ alkyl is defined as above.

"-$C_{0-8}$ alkyl-N($R_{12}$)-C(O)$R_{11}$" refers to the nitrogen atom in -N($R_{12}$)-C(O)$R_{11}$ attached to $C_{0-8}$ alkyl, wherein the $C_{0-8}$ alkyl is defined as above.

"$C_{1-10}$ haloalkyl" refers to an alkyl having 1-10 carbon atoms in which hydrogens on the alkyl are optionally substituted by a fluorine, chlorine, bromine and iodine atom, including but not limited to difluoromethyl, dichloromethyl, dibromomethyl, trifluoromethyl, trichloromethyl, tribromomethyl, etc.

"$C_{1-10}$ haloalkoxy" refers to an alkoxy having 1-10 carbon atoms in which hydrogens on the alkyl are optionally substituted by a fluorine, chlorine, bromine, and iodine atom, including but not limited to difluoromethoxy, dichloromethoxy, dibromomethoxy, trifluoromethoxy, trichloromethoxy, tribromomethoxy, etc.

"$C_{1-10}$ deuterioalkyl" refers to an alkyl having 1-10 carbon atoms in which hydrogens on the alkyl are optionally substituted by a deuterium atom, including but not limited to monodeuterium, dideuterium, trideuterium, etc.

"Halogen" refers to fluorine, chlorine, bromine or iodine; "EA" refers to ethanol; "PE" refers to petroleum ether; "EtOAc" refers to ethyl acetate; "MeOH" refers to methanol, "DCM" refers to dichloromethane; "DMSO" refers to dimethyl sulfoxide.

The term "optional" or "optionally" means that the event or circumstance subsequently described may, but not necessarily, occur, and that the description includes instances where the event or circumstance occurs or dose not occur, i.e., both substituted or unsubstituted instances. For example, "heterocyclyl group optionally substituted by alkyl" means that alkyl may be, but not necessarily, present, and that the description includes instances where the heterocyclyl group is and is not substituted by alkyl.

The term "substituted" means that one or more "hydrogen atoms" in a group are each independently substituted by a corresponding number of substituents. It goes without saying that a substituent is only in its possible chemical position and consistent with the valence bond theory in chemistry, and those skilled in the art will be able to determine (by experiments or theories) possible or impossible substitution without undue efforts. For example, it may be unstable when an amino or hydroxy having a free hydrogen is bound to a carbon atom having an unsaturated bond (such as olefin).

"Stereoisomer" refers to isomers produced by different spatial arrangements of atoms in the molecules. They can be divided into cis-trans isomers and enantiomers and can also be divided into two categories: enantiomers and diastereomers. A stereoisomer produced by the rotation of a single bond is referred to as a conformational stereo-isomer, sometimes also referred to as a rotamer. A stereoisomer produced by bond length, bond angle, double bonds in the molecule, ring and other reasons is referred to as a configuration stereo-isomer, which can be divided into two categories. An isomer produced by the inability of a double bond or a single bond of ring carbon atoms to rotate freely becomes a geometric isomer, also known as a cis-trans isomer, which can be divided into Z and E configurations. For example: cis-2-butene and trans-2-butene are a pair of geometric isomers. Stereoisomers with different optical properties produced by the absence of anti-axial symmetry in the molecules are called optical isomers, which can be divided into R and S configurations. "Stereoisomers" as described in the present invention, if not specifically indicated, are to be understood to include one or more of the enantiomers, configurational isomers, and conformational isomers described above.

"Pharmacologically acceptable salt" in the present invention refers to pharmaceutically acceptable acid addition salts, including inorganic and organic acid salts, which may be prepared by methods known in the art.

"Pharmaceutical composition" refers to a mixture containing one or more of the compounds described herein or a physiologically/pharmaceutically acceptable salt or pro-drug thereof, and other chemical components, for example physiologically/pharmaceutically acceptable carriers and excipients. The purpose of the pharmaceutical composition is to promote the administration to an organism, which facilitates the absorption of the active ingredient, thereby exerting biological activities.

**The present invention is further explained in detail below with reference to examples, which are not intended to limit the present invention, and the present invention is not merely limited to the contents of the examples.**

The compound structure of the present invention is determined by nuclear magnetic resonance (NMR) or/and liquid chromatography-mass spectrometry (LC-MS). The NMR chemical shift (δ) is given in parts per million (ppm). The NMR determination is conducted by using a Bruker AVANCE-400/500 nuclear magnetic resonance apparatus, with hexadeuterodimethyl sulfoxide (DMSO-$d_6$), tetradeuteromethanol ($CD_3OD$) and deuterated chloroform ($CDCl_3$) as determination solvents, and tetramethylsilane (TMS) as internal standard.

The LC-MS determination is conducted by using an Agilent 6120 mass spectrometer. The HPLC determination is conducted by using an Agilent 1200 DAD high pressure liquid chromatography (Sunfire C18 150 × 4.6 mm chromatographic column) and a Waters 2695-2996 high pressure liquid chromatography (Gimini C18 150 × 4.6 mm chromatographic column).

Yantai Yellow Sea HSGF254 or Qingdao GF254 silica gel plate is adopted as a thin layer chromatography (TLC) silica gel plate. The specification adopted by the TLC is 0.15-0.20 mm, and the specification adopted by the thin layer chromatography for the separation of products is 0.4-0.5 mm. The Yantai Yellow Sea silica gel of 200-300 mesh is generally utilized as a carrier in column chromatography.

Starting materials in the examples of the present invention are known and commercially available, or may be synthesized by using or according to methods known in the art.

Unless otherwise stated, all reactions of the present invention are carried out under a dry nitrogen or argon atmosphere with continuous magnetic stirring, wherein the solvent is a dry solvent and the reaction temperature is in degree centigrade (°C).

**I. Preparation of Intermediates**

**Preparation of Intermediate 1: 4-amino-7-fluoro-1-methyl-1H-pyrazolo[4,3-c]quinolin-8-carboxylic acid**

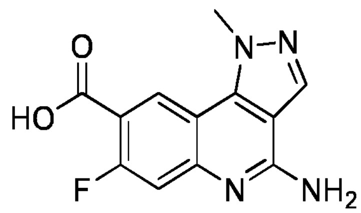

**Step 1: Synthesis of methyl 4-amino-5-bromo-2-fluorobenzoate**

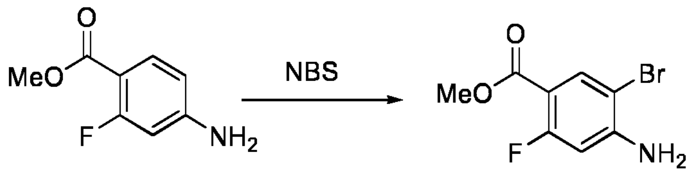


Methyl 4-amino-2-fluorobenzoate (20.8 g, 123.0 mmol) was dissolved in chloroform (500 mL), N-bromosuccinimide (21.89 g, 123.0 mmol) was added at 0°C, and the mixture was stirred and reacted at 20°C for 16 hours. The reaction solution was concentrated by rotary evaporation, and the crude product was separated by using a normal phase column to obtain methyl 4-amino-5-bromo-2-fluorobenzoate (27.9 g, 112.5 mmol, 91%). MS m/z (ESI): 247.9, 250.0 $[M+H]^+$.

$^1H$ NMR (400 MHz, *$CDCl_3$*) δ 8.04 (d, *J* = 7.2 Hz, 1H), 6.46 (d, *J* = 12.2 Hz, 1H), 4.62 (s, 2H), 3.88 (s, 3H).

**Step 2: Synthesis of methyl 4-amino-2-fluoro-5-(4,4,5,5-tetramethyl-1,3,2-dioxaborolan-2-yl)benzoate**

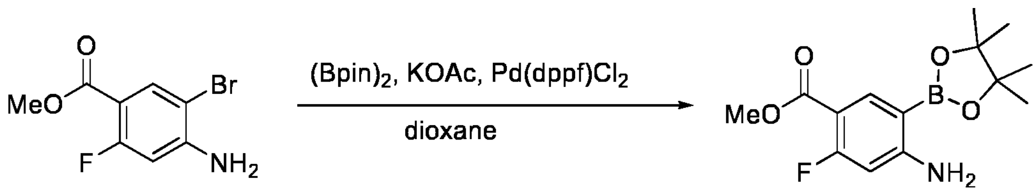


Methyl 4-amino-5-bromo-2-fluorobenzoate (27.9 g, 112.5 mmol) and bis(pinacolato)diboron (35 mL, 135 mmol) were dissolved in dioxane (300 mL), and potassium acetate (33.12 g, 337.4 mmol) and 1,1'-bis(diphenylphosphino) ferrocenepalladium(II) dichloride (8.23 g, 11.2 mmol) were added. The mixture was stirred and reacted at 100°C for 18 hours. The reaction solution was filtered and spin-dried. The crude product was diluted with water, and it was extracted with ethyl acetate three times. The organic phase was washed with saturated brine, dried over anhydrous sodium sulfate and filtered, and the filtrate was concentrated by rotary evaporation. The crude product was separated by using a normal phase column to obtain methyl 4-amino-2-fluoro-5-(4,4,5,5-tetramethyl-1,3,2-dioxaborolan-2-yl)benzoate (25.2 g, 85.4 mmol, 76%). MS m/z (ESI): 296.2 $[M+H]^+$.

$^1H$ NMR (400 MHz, $CDCl_3$) δ 8.25 (d, *J* = 9.2 Hz, 1H), 6.24 (d, *J* = 13.2 Hz, 1H), 5.29 (s, 2H), 3.86 (s, 3H), 1.34 (s, 12H).

**Step 3: Synthesis of methyl 4-amino-7-fluoro-1-methyl-1H-pyrazolo[4,3-c]quinolin-8-carboxylate**

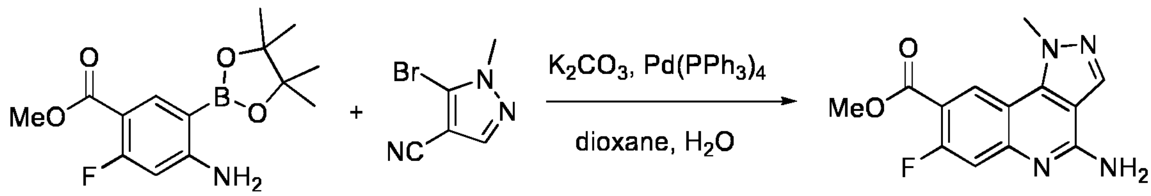


5-bromo-1-methyl-1H-pyrazol-4-carbonitrile (9.1 g, 48.9 mmol), methyl 4-amino-2-fluoro-5-(4,4,5,5-tetramethyl-1,3,2-dioxaborolan-2-yl)benzoate (14.4 g, 48.9 mmol), potassium carbonate (27.04 g, 195.7 mmol) and tetrakis(triphenylphosphine)palladium (7.35 g, 6.4 mmol) were added to 1,4-dioxane (100 mL) and water (100 mL), and the mixture was reacted under nitrogen protection at 100°C for 16 hours. The reaction solution was evaporated under reduced pressure to remove the organic solvent, and it was filtered. The filter cake was washed with ethanol (50 mL) and dried to obtain methyl 4-amino-7-fluoro-1-methyl-1H-pyrazolo[4,3-c]quinolin-8-carboxylate (13.28 g, 48.4 mmol, 99%). MS m/z (ESI): 275.1 $[M+H]^+$.

**Step 4: Synthesis of 4-amino-7-fluoro-1-methyl-1H-pyrazolo[4,3-c]quinolin-8-carboxylic acid**

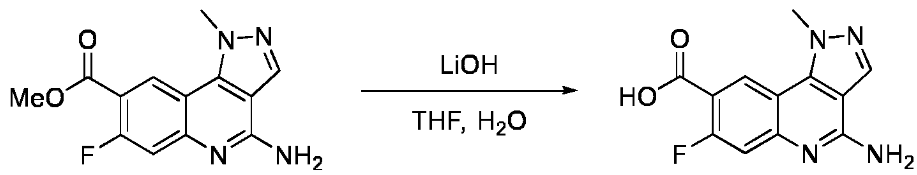


Methyl 4-amino-7-fluoro-1-methyl-1H-pyrazolo[4,3-c]quinolin-8-carboxylate (13.28 g, 48.4 mmol) was added to tetrahydrofuran (100 mL) and water (100 mL), then lithium hydroxide monohydrate (4.06 g, 96.8 mmol) was added, and the mixture was reacted at 50°C for 16 hours. After the reaction was completed, the organic phase was spin-dried, diluted with water and extracted with ethyl acetate (200 mL*6). The pH of the aqueous phase was adjusted to 2 with saturated

potassium hydrogen sulfate, large amounts of solids were precipitated and filtered, and the filter cake was washed with water (100 mL) and acetonitrile (100 mL) and dried to obtain 4-amino-7-fluoro-1-methyl-1H-pyrazolo[4,3-c]quinolin-8-carboxylic acid (6.7 g, 25.7 mmol, 53%). MS m/z (ESI): 261.1 $[M+H]^+$.

$^1H$ NMR (400 MHz, DMSO-$d_6$) δ 8.73 (d, *J* = 8.0 Hz, 1H), 8.27 (s, 1H), 7.54 (s, 2H), 7.27 (d, *J* = 13.2 Hz, 1H), 4.38 (s, 3H).

**Intermediates 2-5 can be prepared by selecting the corresponding raw materials with reference to all or part of the synthesis methods of intermediate 1**:

| Intermediate No. | Structural Formula | Chemical Name | MS m/z $[M+H]^+$ |
|---|---|---|---|
| 2 | 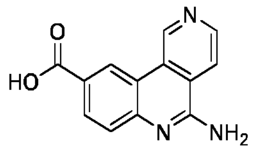 | 5-aminobenzo[c] [2,6]naphthyridin -9-carboxylic acid | **240.1** |
| 3 | 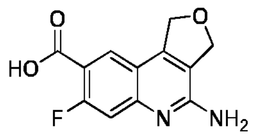 | 4-amino-7-fluoro-1,3-dihydrofuro[3,4-c]quinolin-8-carboxylic acid | **249.1** |
| 4 | 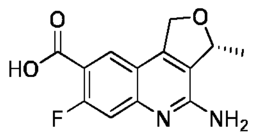 | (R)-4-amino-7-fluoro-3-methyl-1,3-dihydrofuro[3,4-c] quinolin-8-carboxylic acid | **263.1** |
| 5 | 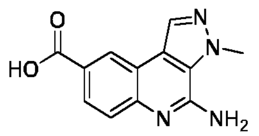 | 4-amino-3-methyl-3H-pyrazolo[3,4-c]quinolin-8-carboxylic acid | **243.1** |

**Preparation of Intermediate 6: 6-(trifluoromethyl)benzofuran-3(2H)-one**

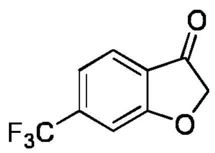

**Step 1: Synthesis of methyl 2-(2-methoxy-2-oxoethoxy)-4-(trifluoromethyl)benzoate**

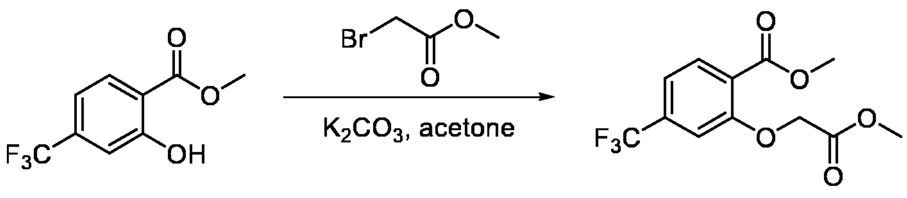


Methyl 2-hydroxy-4-(trifluoromethyl)benzoate (15.0 g, 68.1 mmol) was dissolved in acetone (200 mL), potassium

carbonate (12.24 g, 88.6 mmol) was added, and methyl bromoacetate (8.42 mL, 88.6 mmol) was added dropwise to the above reaction solution, then it was warmed to 55°C and reacted for 18 hours. After the reaction was complete, the reaction solution was cooled to room temperature, the solid was removed by filtration, and the filter cake was washed with ethyl acetate. The filtrate was washed with saturated brine, then it was dried and concentrated to obtain methyl 2-(2-methoxy-2-oxoethoxy)-4-(trifluoromethyl)benzoate (22.0 g, 64.0 mmol, 94%). MS m/z (ESI): 293.1 $[M+H]^+$.

**Step 2: Synthesis of 2-(carboxymethoxy)-4-(trifluoromethyl)benzoic acid**

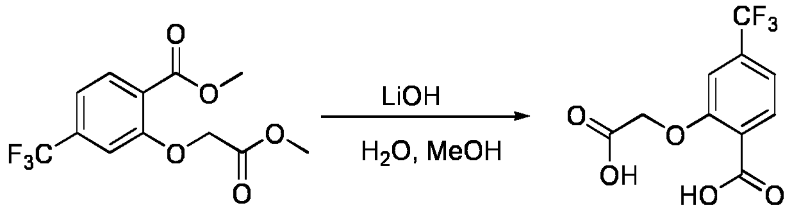


Methyl 2-(2-methoxy-2-oxoethoxy)-4-(trifluoromethyl)benzoate (74.0 g, 253.2 mmol) was dissolved in a mixed solvent of methanol (500 mL) and water (250 mL), lithium hydroxide monohydrate (31.91 g, 759.7 mmol) was added, and the mixture was reacted at room temperature for 18 hours. After TLC confirmed that the reaction was complete, the pH of the reaction solution was acidified to 2 with 1 N hydrochloric acid, it was concentrated to remove methanol, the aqueous phase was filtered, and the solid was washed with water. The filter cake was dried to obtain 2-(carboxymethoxy)-4-(trifluoromethyl)benzoic acid (61 g, 230.9 mmol, 91%). MS m/z (ESI): 265.0 $[M+H]^+$.

**Step 3: Synthesis of 6-(trifluoromethyl)benzofuran-3(2H)-one**

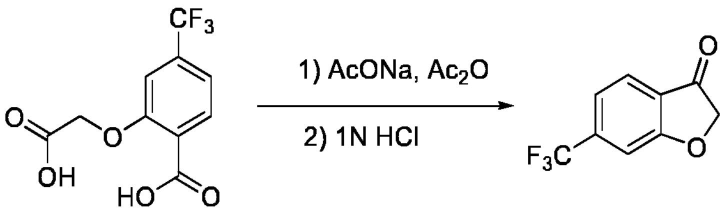


A mixture of 2-(carboxymethoxy)-4-(trifluoromethyl)benzoic acid (61 g, 230.9 mmol), acetic anhydride (377 mL, 4.02 mol), sodium acetate (28.41 g, 346.4 mmol) and acetic acid (110 mL) was heated to 150°C and reacted for 18 hours. After the reaction was completed, the reaction solution was cooled to room temperature, diluted with ethyl acetate, washed with water and saturated brine, dried over anhydrous sodium sulfate and concentrated. After concentration, it was separated by using silica gel column chromatography to obtain an intermediate (42 g). The intermediate was dissolved in methanol (300 mL), 1N hydrochloric acid (300 mL) was added, and the mixture was warmed to 100°C and reacted for 6 hours. After LCMS confirmed that the reaction was complete, the reaction solution was cooled to room temperature, water was added, and it was extracted with ethyl acetate. The organic phase was extracted, washed with saturated brine, dried over anhydrous sodium sulfate and concentrated. After concentration, it was slurried with methyl tert-butyl ether (MTBE) to obtain 6-(trifluoromethyl)benzofuran-3(2H)-one (17.0 g, 84.1 mmol, 36%).

$^1H$ NMR (400 MHz, *$CDCl_3$*) δ 7.80 (d, *J*= 8.0 Hz, 1H), 7.43 (s, 1H), 7.35 (dd, *J* = 8.1, 1.4 Hz, 1H), 4.72 (s, 2H).

**Preparation of Intermediate** 7: **2-(trifluoromethyl)-6,7-dihydro-5H-cyclopenta[b]pyridin-5-one**

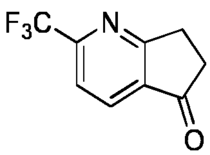

**Step 1: Synthesis of methyl 2-bromo-6-(trifluoromethyl) nicotinate**

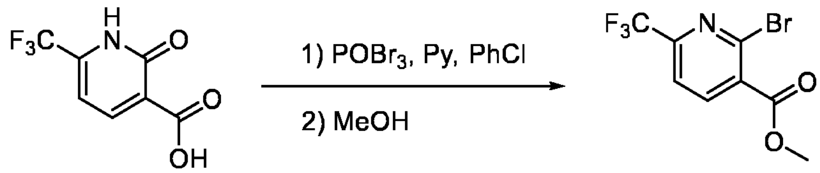


2-oxo-6-(trifluoromethyl)-1,2-dihydropyridin-3-carboxylic acid (5.0 g, 24.14 mmol) and pyridine (1.95 mL, 24.14 mmol) were dissolved in chlorobenzene (50 mL), phosphorus oxybromide (4.91 mL, 48.28 mmol) was slowly added in batches at room temperature, and the mixture was warmed to 120°C, stirred and reacted for 16 hours. After the reaction was completed, the reaction solution was concentrated in vacuum. The concentrate was cooled to 0°C in an ice bath, dry methanol (20 mL) pre-cooled on dry ice was slowly added dropwise, and the mixture was stirred at room temperature for 1 hour. The reaction solution was concentrated in vacuum to obtain a brown, oil-like substance. The oil-like substance was diluted with water, the pH of the solution was adjusted to 8 with saturated sodium bicarbonate solution, and it was extracted with ethyl acetate three times (50 mL*3). The organic phase was washed with saturated brine, dried over anhydrous sodium sulfate, filtered and concentrated. The concentrate was separated by using silica gel column chromatography to obtain methyl 2-bromo-6-(trifluoromethyl) nicotinate (5.7 g, 83%). MS m/z (ESI): 283.9, 285.9 $[M+H]^+$.

$^1H$ NMR (400 MHz, $CDCl_3$) $\delta$ 8.21 (d, $J$ = 8.0 Hz, 1H), 7.72 (d, $J$ = 8.0 Hz, 1H), 4.00 (s, 3H).

**Step 2: Synthesis of methyl (E)-2-(3-methoxy-3-oxoprop-1-en-1-yl)-6-(trifluoromethyl) nicotinate**

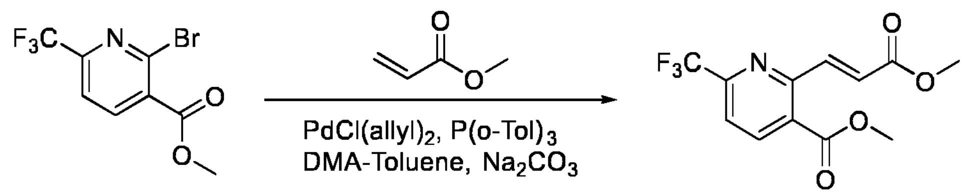


To a solution of methyl 2-bromo-6-(trifluoromethyl)nicotinate (5.7 g, 20.07 mmol) and methyl acrylate (4.52 mL, 50.17 mmol) in toluene (100 mL) and N,N-dimethylformamide (15 mL) were added allylpalladium(II) chloride dimer (0.37 g, 1.00 mmol), tri(o-methylphenyl)phosphine (0.61 g, 2.01 mmol) and sodium carbonate (6.38 g, 60.20 mmol), followed by stirring at 120°C for 16 hours under nitrogen atmosphere. The reaction solution was filtered, and the filter cake was washed with ethyl acetate three times (30 mL*3). The organic phases were combined and then concentrated in vacuum, and the concentrate was separated by using silica gel column chromatography to obtain methyl (E)-2-(3-methoxy-3-oxoprop-1-en-1-yl)-6-(trifluoromethyl) nicotinate (5.1 g, 88%). MS m/z (ESI): 290.0 $[M+H]^+$.

$^1H$ NMR (400 MHz, *$CDCl_3$*) $\delta$ 8.46 (d, $J$ = 15.2 Hz, 1H), 8.40 (d, $J$ = 8.0 Hz, 1H), 7.70 (d, $J$ = 8.4 Hz, 1H), 7.26 (d, $J$ = 2.8 Hz, 1H), 4.01 (s, 3H), 3.84 (s, 3H).

**Step 3: Synthesis of methyl 2-(3-methoxy-3-oxopropyl)-6-(trifluoromethyl) nicotinate**

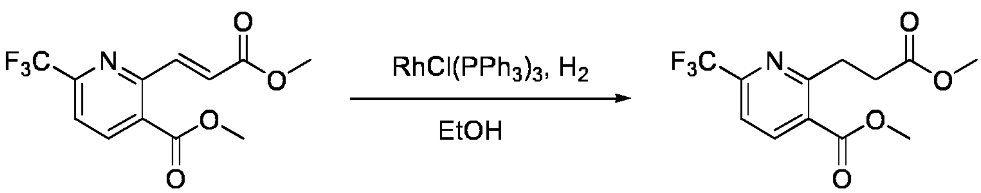


A solution of methyl (E)-2-(3-methoxy-3-oxoprop-1-en-1-yl)-6-(trifluoromethyl)nicotinate (5.1 g, 17.63 mmol) and triphenylphosphine rhodium chloride (1.31 g, 1.41 mmol) in ethanol (50 mL) was stirred under hydrogen atmosphere (15 Psi) at room temperature for 48 hours. The reaction solution was filtered, the filtrate was concentrated in vacuum, and

the crude product was separated by using silica gel column chromatography to obtain methyl 2-(3-methoxy-3-oxopropyl)-6-(trifluoromethyl) nicotinater (4.9 g, 95%). MS m/z (ESI): 292.1 [M+H]+.

$^1$H NMR (400 MHz, *CDCl$_3$*) δ 8.34 (d, *J* = 8.0 Hz, 1H), 7.58 (d, *J* = 8.0 Hz, 1H), 3.96 (s, 3H), 3.67 (s, 3H), 3.57 (t, *J* = 7.2 Hz, 2H), 2.85 (t, *J* = 7.2 Hz, 2H).

**Step 4: Synthesis of 2-(trifluoromethyl)-6,7-dihydro-5H-cyclopenta[b]pyridin-5-one**

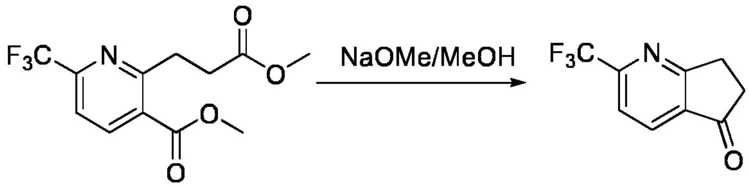


To a solution of methyl 2-(3-methoxy-3-oxopropyl)-6-(trifluoromethyl) nicotinate (4.9 g, 16.82 mmol) in methanol (60 mL) was added sodium methoxide (1.36 g, 25.23 mmol) in batches, and the reaction solution was stirred overnight at 80°C. After the reaction was completed, the reaction solution was cooled to room temperature and concentrated to obtain a brown solid. The brown solid was dispersed in 12 M concentrated hydrochloric acid (60 mL), and the mixture was stirred at 80°C for 3 hours. The reaction solution was cooled to room temperature, the pH of the reaction solution was adjusted to 6 with 1 M sodium hydroxide solution, and it was extracted with dichloromethane (100 mL*2). The organic phase was washed with saturated brine, dried over anhydrous sodium sulfate and filtered, and the filtrate was concentrated to obtain a brown solid. The brown solid was separated by using silica gel column chromatography to obtain 2-(trifluoromethyl)-6,7-dihydro-5H-cyclopenta[b]pyridin-5-one (2.07 g, 43%) MS m/z (ESI): 201.9 [M+H]+.

$^1$H NMR (400 MHz, *CDCl$_3$*) δ 8.21 (d, *J* = 8.0 Hz, 1H), 7.73 (d, *J* = 8.0 Hz, 1H), 3.42-3.36 (m, 2H), 2.92-2.85 (m, 2H).

**Preparation of Intermediate 8: 6-(trifluoromethyl)furo[2,3-b]pyridin-3(2H)-one**

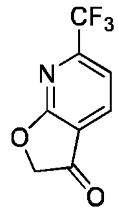

**Step 1: Synthesis of ethyl 2-chloro-6-(trifluoromethyl) nicotinate**

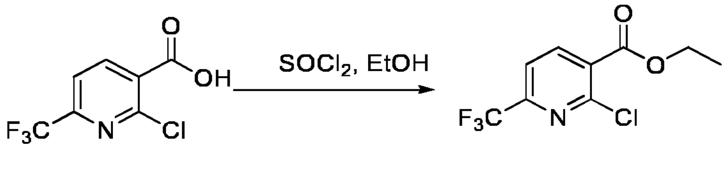


Thionyl chloride (3.62 mL, 49.9 mmol) was added dropwise at room temperature to a nitrogen protected solution of 2-chloro-6-(trifluoromethyl)nicotinic acid (7.5 g, 33.25 mmol) in ethanol (150 mL). The reaction solution was then heated to 70°C, stirred and reacted for 14 hours. After the reaction was completed, the reaction solution was concentrated to remove ethanol, the residue was dissolved in ethyl acetate (200 mL), and the organic phase was washed with water (100 mL*3) and saturated brine, dried over anhydrous sodium sulfate, filtered and concentrated. The concentrate was separated by using column chromatography [85% ethyl acetate - petroleum ether system] to obtain ethyl 2-chloro-6-(trifluoromethyl) nicotinate (7.72 g, 30.44 mmol, 92%). MS m/z (ESI): 254.0 [M+H]+.

$^1$H NMR (400 MHz, $CDCl_3$) δ 8.30 (d, *J* = 8.0 Hz, 1H), 7.70 (d, *J* = 8.0 Hz, 1H), 4.46 (d, *J* = 7.2 Hz, 2H), 1.43 (d, *J* =

7.2 Hz, 3H).

**Step 2: Synthesis of ethyl 3-hydroxy-6-(trifluoromethyl)furo[2,3-b]pyridin-2-carboxylate**

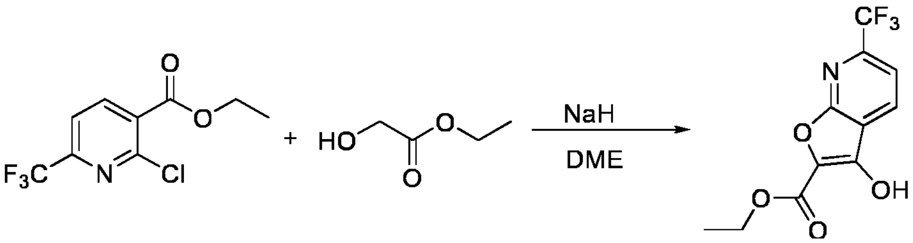


Sodium hydride (15.22 g, 380.5 mmol, 60% purity) was added in batches at 0°C to a solution of ethyl glycolate (36.7 mL, 380.5 mmol) in ethylene glycol dimethyl ether (530 mL). The reaction solution was warmed to room temperature and stirred for 30 minutes. Ethyl 2-chloro-6-(trifluoromethyl) nicotinate (38.6 g, 152.2 mmol) was then dissolved in ethylene glycol dimethyl ether (530 mL) and added dropwise to the above reaction solution, and the reaction solution was reacted at 35°C for 2 hours. After the reaction was over, the reaction solution was diluted with ethyl acetate and quenched with saturated sodium bicarbonate solution. The organic phase was extracted, washed with saturated brine, dried over anhydrous sodium sulfate and concentrated. After concentration, it was slurried with acetonitrile (300 mL) to obtain ethyl 3-hydroxy-6-(trifluoromethyl)furo[2,3-b]pyridin-2-carboxylate (29.5 g, 107.2 mmol, 70%).

$^1H$ NMR (400 MHz, DMSO-$d_6$) δ 8.24 (d, *J*= 7.6 Hz, 1H), 7.59 (d, *J*= 7.6 Hz, 1H), 4.17 (q, *J* = 7.2 Hz,2H), 1.26 (t, *J* = 7.2 Hz, 3H).

**Step 3: Synthesis of 6-(trifluoromethyl)furo[2,3-b]pyridin-3(2H)-one**

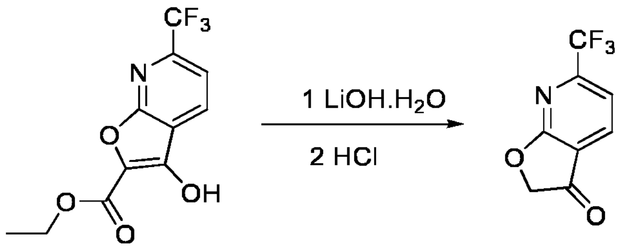


Ethyl 3-hydroxy-6-(trifluoromethyl)furo[2,3-b]pyridin-2-carboxylate (2.83 g, 10.28 mmol) was dissolved in a mixed solvent of tetrahydrofuran (32.0 mL) and water (8.0 mL), and lithium hydroxide monohydrate (1.73 g, 41.1 mmol) was added. The reaction solution was warmed to 40°C for 2 days. After the reaction was over, the reaction solution was cooled to room temperature and filtered, and the filter cake was washed with tetrahydrofuran. The filter cake was then dissolved in a mixed solvent of ethyl acetate and water, and the pH of the system was acidified to 6-7 with 1 M hydrochloric acid in an ice water bath. The solution was separated, the aqueous phase was extracted with ethyl acetate, the organic phase was combined, dried over anhydrous sodium sulfate and filtered, and the filtrate was evaporated and concentrated to obtain 6-(trifluoromethyl)furo[2,3-b]pyridin-3(2H)-one (1.43 g, 7.04 mmol, 68%). MS m/z (ESI): 204.0 $[M+H]^+$.

$^1H$ NMR (400 MHz, *$CDCl_3$*) δ 8.23 (d, *J* = 7.6 Hz, 1H), 7.52 (d, *J* = 7.6 Hz, 1H), 4.85 (s, 2H).

**Preparation of Intermediate 9: (S)-N-(1-methyl-1H-pyrazol-4-yl)-6-(trifluoromethyl)-2,3-dihydrofuro[2,3-b]pyridin-3-amine**

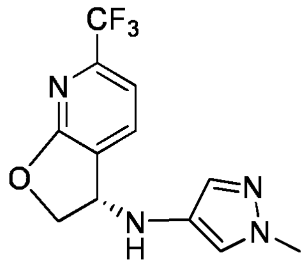

**Step 1: Synthesis of (R)-6-(trifluoromethyl)-2,3-dihydrofuro[2,3-b]pyridin-3-phenol**

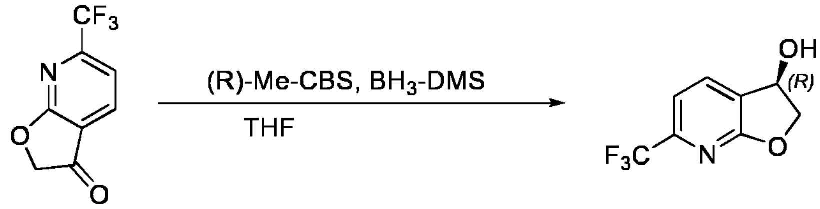


(R)-Me-CBS (5.94 g, 21.416 mmol) was dissolved in tetrahydrofuran (1100 mL), and the mixture was cooled to 0°C. Borane dimethyl sulfide complex (53.5 mL, 107.1 mmol, 2 M in THF) was then added to the above solution, and it was stirred for 1 hour. The reaction system was cooled to -70°C, 6-(trifluoromethyl)furo[2,3-b]pyridin-3(2H)-one (14.5 g, 71.4 mmol) was dissolved in tetrahydrofuran (140 mL), and it was added dropwise to the reaction system, stirred at a constant temperature for 1 hour, then it was warmed to -10°C and stirred for 2 hours. After the reaction was confirmed to be completed, it was quenched with methanol and stirred at room temperature for 30 minutes. 70 mL of aqueous ammonia was added, the supernatant was taken after stirring, and it was concentrated. The residue was separated by using silica gel column chromatography (dichloromethane/ethyl acetate = 10/1) to obtain (R)-6-(trifluoromethyl)-2,3-dihydrofuro[2,3-b] pyridin-3-phenol (12.0 g, 58.5 mmol, 82%). MS m/z (ESI): 205.9 $[M+H]^+$.

**Step 2: Synthesis of (S)-3-azido-6-(trifluoromethyl)-2,3-dihydrofuro[2,3-b]pyridin**

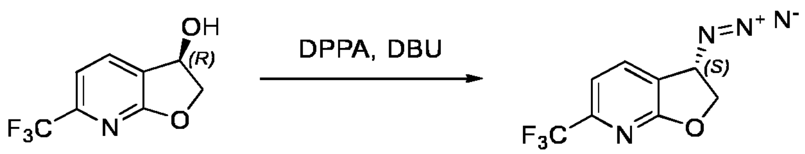


(R)-6-(trifluoromethyl)-2,3-dihydrofuro[2,3-b]pyridin-3-phenol (12 g, 58.5 mmol) was dissolved in tetrahydrofuran (100 mL), the mixture was cooled to 0°C, and diphenylphosphate azide (15.2 mL, 70.2 mmol) and DBU (10.5 mL, 70.2 mmol) were added. It was warmed to room temperature and reacted for 18 hours. After the reaction was over, the reaction solution was diluted with water and extracted with ethyl acetate, and the organic phase was dried and concentrated. It was separated by using silica gel column chromatography to obtain (S)-3-azido-6-(trifluoromethyl)-2,3-dihydrofuro[2,3-b] pyridin (12.5 g, 54.3 mmol, 93%). MS m/z (ESI): 231.0 $[M+H]^+$.

**Step 3: Synthesis of (S)-6-(trifluoromethyl)-2,3-dihydrofuro[2,3-b]pyridin-3-amine hydrochloride**

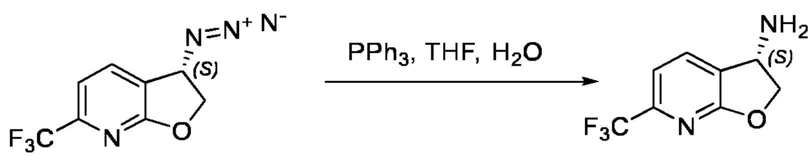

(S)-3-azido-6-(trifluoromethyl)-2,3-dihydrofuro[2,3-b]pyridin (12.5 g, 54.3 mmol) was dissolved in tetrahydrofuran (200 mL) and water (6 mL), triphenylphosphine (42.74 g, 162.9 mmol) was added, and the mixture was warmed to 50°C and reacted for 18 hours. After LCMS confirmed that the reaction was complete, 0.5 M of hydrochloric acid was added, and the mixture was extracted with ethyl acetate three times. The solution was separated, and the obtained aqueous phase was concentrated to obtain (S)-6-(trifluoromethyl)-2,3-dihydrofuro[2,3-b]pyridin-3-amine hydrochloride (11.0 g, 45.7 mmol, 84%). MS m/z (ESI): 205.1 [M+H]+.

**Step 4: Synthesis of (S)-N-(1-methyl-1H-pyrazol-4-yl)-6-(trifluoromethyl)-2,3-dihydrofuro[2,3-b]pyridin-3-amine**

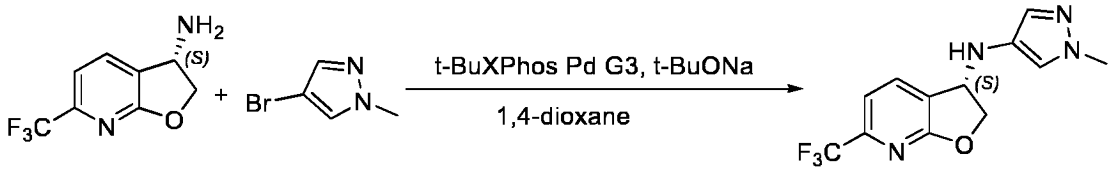


(S)-6-(trifluoromethyl)-2,3-dihydrofuro[2,3-b]pyridin-3-amine hydrochloride (5.5 g, 22.9 mmol) was dissolved in dioxane (500 mL), and 4-bromo-1-methyl-1H-pyrazol (4.23 g, 26.3 mmol), methanesulfonic acid (2-di-tert-butylphosphino-2',4',6'-triisopropyl-1,1'-biphenyl)(2'-amino-1,1'-biphenyl-2-yl)palladium(II) (5.45 g, 6.86 mmol) and sodium tert-butoxide were added (6.59 g, 68.58 mmol). After the nitrogen substitution was carried out 3 times, the mixture was warmed to 95°C and reacted for 2 hours. The reaction solution was cooled to room temperature, water was added, and it was extracted with ethyl acetate. After the solution was separated and the obtained organic phase was dried and concentrated, it was separated by using silica gel column chromatography (PE/EA=1/10) and C18 reversed phase column (Water (10mM $NH_4HCO_3$)-ACN: 21%-51%) to obtain (S)-N-(1-methyl-1H-pyrazol-4-yl)-6-(trifluoromethyl)-2,3-dihydrofuro[2,3-b]pyridin-3-amine (2.4 g, 8.44 mmol, 37%). MS m/z (ESI): 285.1 [M+H]+.

$^1$H NMR (400 MHz, DMSO-$d_6$) δ 7.88 (d, *J* = 7.4 Hz, 1H), 7.41 (d, *J* = 7.4 Hz, 1H), 7.22 (d, *J* = 1.0 Hz, 1H), 7.02 (d, *J* = 1.0 Hz, 1H), 5.19 (d, *J* = 8.9 Hz, 1H), 5.02 (td, *J* = 8.5, 4.5 Hz, 1H), 4.84 (dd, *J* = 9.6, 8.1 Hz, 1H), 4.38 (dd, *J* = 9.6, 4.6 Hz, 1H), 3.71 (s, 3H).

**Intermediates 10-19 can be prepared by selecting the corresponding raw materials with reference to all or part of the synthesis methods of intermediate 9:**

| Intermediate No. | Structural Formula | Chemical Name | MS m/z [M+H]+ |
|---|---|---|---|
| **10** | 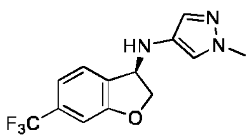 | (R)-1-methyl-N-(6-(trifluoromethyl)-2,3-dihydrobenzofuran-3-yl)-1H-pyrazol-4-amine | **284.1** |
| **11** | 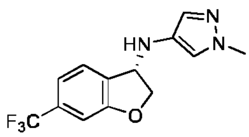 | (S)-1-methyl-N-(6-(trifluoromethyl)-2,3-dihydrobenzofuran-3-yl)-1H-pyrazol-4-amine | **284.1** |
| **12** | 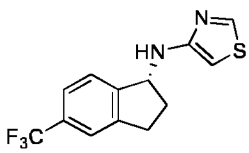 | (R)-N-(5-(trifluoromethyl)-2,3-dihydro-1H-inden-1-yl) thiazol-4-amine | **285.1** |

(continued)

| Intermediate No. | Structural Formula | Chemical Name | MS m/z [M+H]+ |
|---|---|---|---|
| 13 | 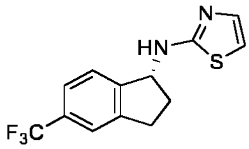 | (R)-N-(5-(trifluoromethyl)-2,3-dihydro-1H-inden-1-yl) thiazol-2-amine | 285.1 |
| 14 | 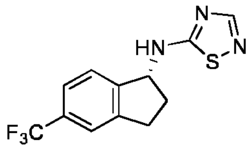 | (R)-N-(5-(trifluoromethyl)-2,3-dihydro-1H-inden-1-yl)-1,2,4-thiadiazole-5-amine | 286.1 |
| 15 | 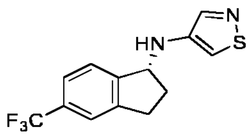 | (R)-N-(5-(trifluoromethyl)-2,3-dihydro-1H-inden-1-yl) isothiazol-4-amine | 285.1 |
| 16 | 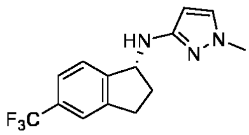 | (R)-1-methyl-N-(5-(trifluoromethyl)-2,3-dihydro-1H-inden-1-yl)-1H-pyrazol-3-amine | 282.1 |
| 17 | 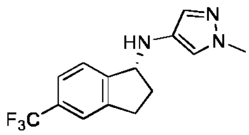 | (R)-1-methyl-N-(5-(trifluoromethyl)-2,3-dihydro-1H-inden-1-yl)-1H-pyrazol-4-amine | 282.1 |
| 18 | 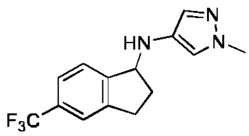 | 1-methyl-N-(5-(trifluoromethyl)-2,3-dihydro-1H-inden-1-yl)-1H-pyrazol-4-amine | 282.1 |
| 19 | 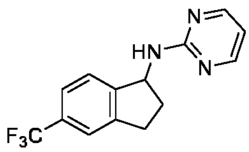 | N-(5-(trifluoromethyl)-2,3-dihydro-1H-inden-1-yl)pyrimidin-2-amine | 280.1 |

**Preparation of Intermediate 20: (R)-N-(cyclopropylmethyl)-5-(trifluoromethyl)-2,3-dihydro-1H-inden-1-amine**

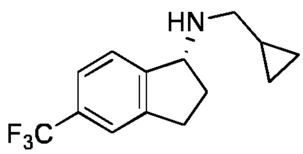

**Step 1: Synthesis of (R)-N-(cyclopropylmethyl)-5-(trifluoromethyl)-2,3-dihydro-1H-inden-1-amine**

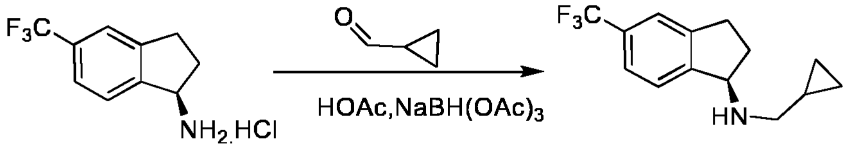


(R)-5-(trifluoromethyl)-2,3-dihydro-1H-inden-1-amine hydrochloride (200 mg, 0.84 mmol) was dissolved in a mixed solution of dichloromethane (3 mL) and ethanol (3 mL), potassium acetate (82 mg, 0.84 mmol), cyclopropanecarboxaldehyde (59 mg, 0.84 mmol), acetic acid (5 mg, 0.084 mmol) and sodium triacetoxyborohydride (355 mg, 1.68 mmol) were added, and the mixture was stirred overnight at room temperature. The reaction solution was diluted with dichloromethane and saturated sodium bicarbonate solution, the solution was separated, and the organic phase was concentrated by rotary evaporation. The crude product was separated by using column chromatography (methanol/dichloromethane=1/19) to obtain (R)-N-(cyclopropylmethyl)-5-(trifluoromethyl)-2,3-dihydro-1H-inden-1-amine (108 mg, 48%). MS m/z (ESI): 256.1 $[M+H]^+$.

**Intermediates 21-22 can be prepared by selecting the corresponding raw materials with reference to all or part of the synthesis methods of intermediate 20:**

| Intermediate No. | Structural Formula | Chemical Name | MS m/z $[M+H]^+$ |
|---|---|---|---|
| 21 | 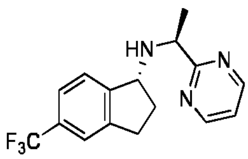 | (R)-N-((S)-1-(pyrimidin-2-yl)ethyl)-5-(trifluoromethyl)-2,3-dihydro-1H-inden-1-amine | 308.1 |
| 22 | 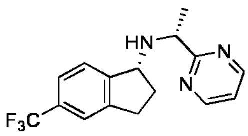 | (R)-N-((R)-1-(pyrimidin-2-yl)ethyl)-5-(trifluoromethyl)-2,3-dihydro-1H-inden-1-amine | 308.1 |

**Preparation of Intermediates 23, 24 and 25: N-(cyclopropylmethyl)-2-(trifluoromethyl)-6,7-dihydro-5H-cyclopenta[b]pyridin-5-amine**

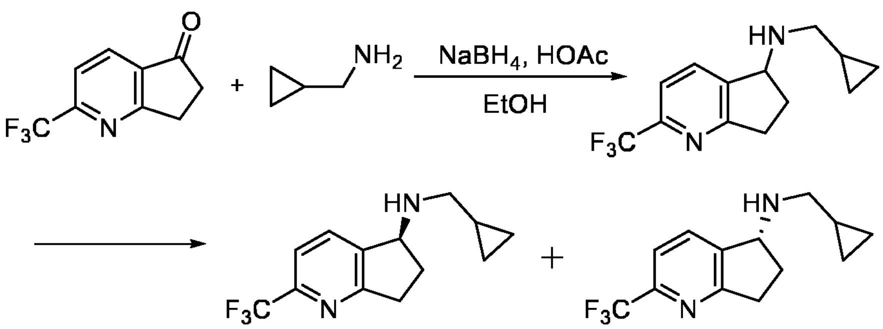


2-(trifluoromethyl)-6,7-dihydro-5H-cyclopenta[b]pyridin-5-one (1.35 g, 6.71 mmol) was dissolved in ethanol (20 mL), and a mixture of cyclopropylmethylamine (1.75 mL, 20.13 mmol) and acetic acid (0.24 mL, 1.34 mmol) was added, and it was stirred overnight at 40°C. The reaction solution was cooled in an ice bath, sodium borohydride (0.45 g, 13.42 mmol) was added, and the reaction solution was stirred and reacted at room temperature for 1 hour. The reaction solution was quenched with water and extracted with ethyl acetate, the organic phase was concentrated by rotary evaporation, and the crude product was separated by using column chromatography (methanol/dichloromethane = 1/10) to obtain (N-(cyclopropylmethyl)-2-(trifluoromethyl)-6,7-dihydro-5H-cyclopenta[b]pyridin-5-amine (850 mg, 49%). MS m/z (ESI):

257.1 $[M+H]^+$.

$^1H$ NMR (400 MHz, *$CDCl_3$*) δ 7.79 (d, *J* = 7.8 Hz, 1H), 7.50 (d, *J* = 7.8 Hz, 1H), 4.35 (t, *J* = 7.0 Hz, 1H), 3.21-3.13 (m, 1H), 3.04-2.95 (m, 1H), 2.70-2.50 (m, 3H), 1.94-1.87 (m, 1H), 1.07-0.95 (m, 1H), 0.55-0.47 (m, 2H), 0.22-0.10 (m, 2H).

(N-(cyclopropylmethyl)-2-(trifluoromethyl)-6,7-dihydro-5H-cyclopenta[b]pyridin-5-amine (700 mg, 2.73 mmol) was separated by using a chiral preparative column to obtain (S)-N-(cyclopropylmethyl)-2-(trifluoromethyl)-6,7-dihydro-5H-cyclopenta[b]pyridin-5-amine **(24)** (310 mg, 44%) and (R)-N-(cyclopropylmethyl)-2-(trifluoromethyl)-6,7-dihydro-5H-cyclopenta[b]pyridin-5-amine **(25)** (305 mg, 43%).

**Intermediates 26-38 can be prepared by selecting the corresponding raw materials with reference to all or part of the synthesis methods of intermediates 23, 24 and 25:**

| Intermediate No. | Structural Formula | Chemical Name | MS m/z $[M+H]^+$ |
|---|---|---|---|
| **26** | 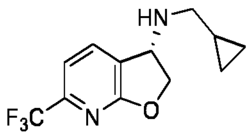 | (S)-N-(cyclopropylmethyl)-6-(trifluoromethyl)-2,3-dihydrofuro[2,3-b]pyridin-3-amine | **259.1** |
| **27** | 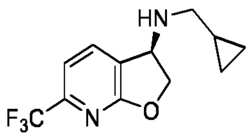 | (R)-N-(cyclopropylmethyl)-6-(trifluoromethyl)-2,3-dihydrofuro[2,3-b]pyridin-3-amine | **259.1** |
| **28** | 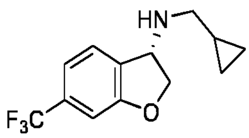 | (S)-N-(cyclopropylmethyl)-6-(trifluoromethyl)-2,3-dihydrobenzofuran-3-amine | **258.1** |
| **29** | 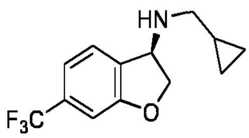 | (R)-N-(cyclopropylmethyl)-6-(trifluoromethyl)-2,3-dihydrobenzofuran-3-amine | **258.1** |
| **30** | 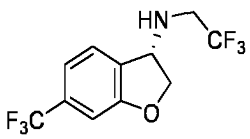 | (S)-N-(2,2,2-trifluoroethyl)-6-(trifluoromethyl)-2,3-dihydrobenzofuran-3-amine | **286.1** |
| **31** | 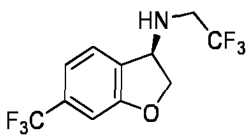 | (R)-N-(2,2,2-trifluoroethyl)-6-(trifluoromethyl)-2,3-dihydrobenzofuran-3-amine | **286.1** |
| **32** | 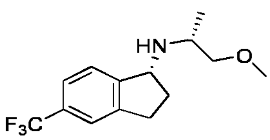 | (R)-N-((R)-1-methoxypropan-2-yl)-5-(trifluoromethyl)-2,3-dihydro-1H-inden-1-amine | **274.1** |
| **33** | 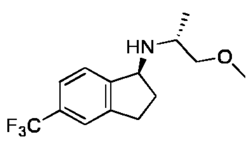 | (S)-N-((R)-1-methoxypropan-2-yl)-5-(trifluoromethyl)-2,3-dihydro-1H-inden-1-amine | **274.1** |

(continued)

| Intermediate No. | Structural Formula | Chemical Name | MS m/z [M+H]+ |
|---|---|---|---|
| **34** | 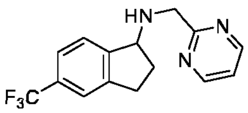 | N-(pyrimidin-2-ylmethyl)-5-(trifluoromethyl)-2,3-dihydro-1H-inden-1-amine | **294.1** |
| **35** | 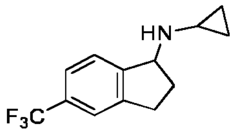 | N-cyclopropyl-5-(trifluoromethyl)-2,3-dihydro-1H-inden-1-amine | **242.1** |
| **36** | 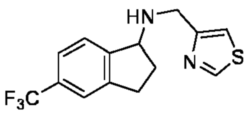 | N-(thiazol-4-ylmethyl)-5-(trifluoromethyl)-2,3-dihydro-1H-inden-1-amine | **299.1** |
| **37** | 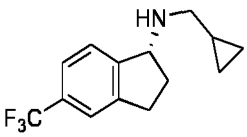 | (R)-N-(cyclopropylmethyl)-5-(trifluoromethyl)-2,3-dihydro-1H-inden-1-amine | **256.1** |
| **38** | 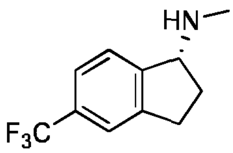 | (R)-N-methyl-5-(trifluoromethyl)-2,3-dihydro-1H-inden-1-amine | **216.1** |

**I. Preparation of Examples**

**Example 1: Preparation of (S)-5-amino-N-(cyclopropylmethyl)-N-(2-(trifluoromethyl)-6,7-dihydro-5H-cyclopenta[b] pyridin-5-yl)benzo[c] [2,6] naphthyridin-9-carboxamide**

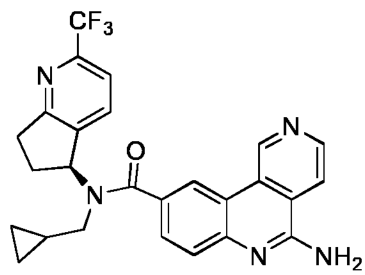

**Step 1: Synthesis of N-ethyl-5-(trifluoromethyl)-2,3-dihydro-1H-inden-1-amine**

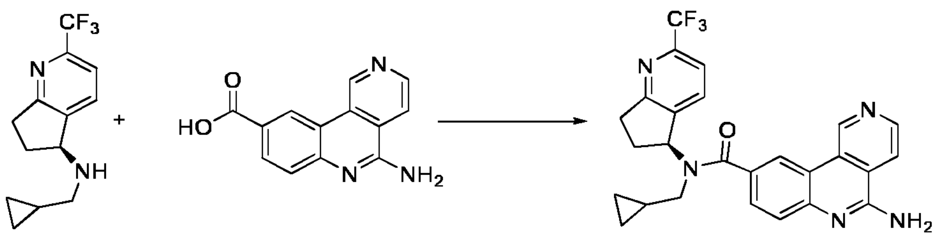

5-aminobenzo[c][2,6]naphthyridin-9-carboxylic acid (67 mg, 0.28 mmol) was dissolved in N,N-dimethylacetamide (3 mL), 2-chloro-1-methylpyridinium iodide (72 mg, 0.28 mmol) and N,N-diisopropylethylamine (121 mg, 0.94 mmol) were added, and the mixture was stirred and reacted at 40°C for 1 hour. (S)-N-(cyclopropylmethyl)-2-(trifluoromethyl)-6,7-dihydro-5H-cyclopenta[b]pyridin-5-amine (60 mg, 0.23 mmol) was added to the reaction solution, and the mixture was stirred and reacted overnight at 40°C. The reaction solution was directly separated by using reverse-phase column chromatography (40% acetonitrile - 10 mM aqueous ammonium bicarbonate solution system) and freeze-dried to obtain (S)-5-amino-N-(cyclopropylmethyl)-N-(2-(trifluoromethyl)-6,7-dihydro-5H-cyclopenta[b]pyridin-5-yl)benzo[c][2,6] naphthyridin-9-carboxamide (37 mg, yield: 33%). MS m/z (ESI): 478.2 $[M+H]^+$.

$^1$H NMR (400 MHz, MeOH-$d_4$) δ 10.00 (s, 1H), 8.80 (d, *J* = 5.6 Hz, 1H), 8.71 (s, 1H), 8.17 (d, *J*= 5.6 Hz, 1H), 8.09 (d, *J* = 7.9 Hz, 1H), 7.70-7.67 (m, 3H), 5.63-5.56 (m, 1H), 3.54-3.35 (m, 2H), 2.87-2.52 (m, 4H), 1.16-1.01 (m, 1H), 0.55-0.45 (m, 2H), 0.14-0.01 (m, 2H).

**Example 2: Preparation of (S)-4-amino-7-fluoro-1-methyl-N-(1-methyl-1H-pyrazol-4-yl)-N-(6-(trifluoromethyl)-2,3-dihydrofuro[2,3-b]pyridin-3-yl)-1H-pyrazolo[4,3-c]quinolin-8-carboxamide**

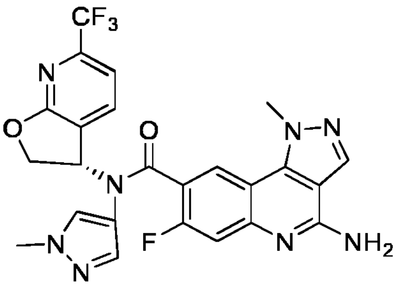

**Step 1: Synthesis of 4-amino-7-fluoro-1-methyl-1H-pyrazolo[4,3-c]quinolin-8-carbonyl chloride hydrochloride**

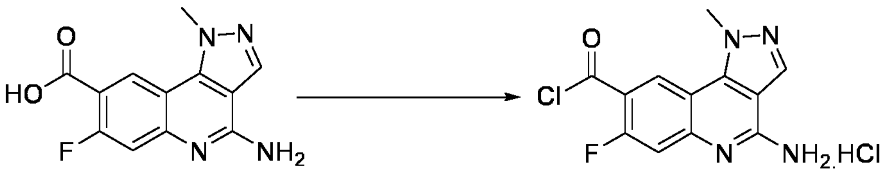

4-amino-7-fluoro-1-methyl-1H-pyrazolo[4,3-c]quinolin-8-carboxylic acid (100 mg, 0.38 mmol) was put into dichloromethane (10 mL), hydrochloric acid in 1,4-dioxane (0.5 mL, 2.0 mmol, 4 mol/L) was added, the mixture was stirred at room temperature for half an hour, and the reaction solution was concentrated to dryness under reduced pressure by rotary evaporation. Thionyl chloride (1.0 mL, 13.79 mmol) was added to the residue, and the mixture was stirred and reacted overnight at 70°C. The reaction solution was concentrated under reduced pressure by rotary evaporation, dry dichloromethane (5 mL) was added to the residue, and the mixture was concentrated by rotary evaporation to obtain 4-amino-7-fluoro-1-methyl-1H-pyrazolo[4,3-c]quinolin-8-carbonyl chloride hydrochloride (118 mg, 99% yield). MS m/z (ESI): 275.2 $[M+H]^+$.

**Step 2: Synthesis of (S)-4-amino-7-fluoro-1-methyl-N-(1-methyl-1H-pyrazol-4-yl)-N-(6-(trifluoromethyl)-2,3-dihydrofuro[2,3-b]pyridin-3-yl)-1H-pyrazolo[4,3-c]quinolin-8-carboxamide**

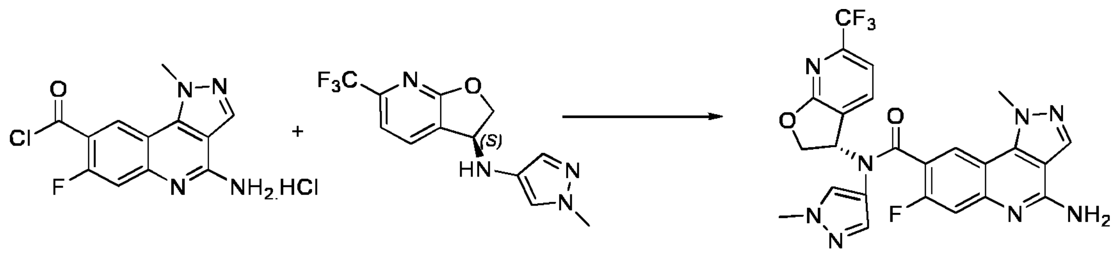

4-amino-7-fluoro-1-methyl-1H-pyrazolo[4,3-c]quinolin-8-carbonyl chloride hydrochloride (119 mg, 0.38 mmol) was put into dichloroethane (6 mL), pyridine (0.26 mL, 3.20 mmol) and (S)-N-(1-methyl-1H-pyrazol-4-yl)-6-(trifluoromethyl)-2,3-dihydrofuro[2,3-b]pyridin-3-amine (96 mg, 0.34 mmol) were added, and the mixture was stirred and reacted at 70°C for 4 hours. After the reaction was over, saturated sodium bicarbonate solution and dichloromethane were added, the solution was filtered and separated, the aqueous phase was extracted with dichloromethane, and the organic phase was concentrated by rotary evaporation. The crude product was separated by using silica gel column chromatography [5% methanol - dichloromethane system] to obtain (S)-4-amino-7-fluoro-1-methyl-N-(1-methyl-1H-pyrazol-4-yl)-N-(6-(trifluoromethyl)-2,3-dihydrofuro[2,3-b]pyridin-3-yl)-1H-pyrazolo[4,3-c]quinolin-8-carboxamide (140 mg, yield: 82%). MS m/z (ESI): 527.2 $[M+H]^+$.

$^1H$ NMR (400 MHz, MeOH-$d_4$) δ 8.26 (d, $J$ = 7.1 Hz, 1H), 8.18 (s, 1H), 8.14 (d, $J$ = 7.5 Hz, 1H), 7.48-7.42 (m, 2H), 7.15-7.05 (m, 2H), 6.67 (d, $J$ = 8.3 Hz, 1H), 4.99 (t, $J$ = 9.7 Hz, 1H), 4.85 (dd, $J$ = 10.8, 3.8 Hz, 1H), 4.41 (s, 3H), 3.57 (s, 3H).

**Examples 6-238 can be prepared by selecting the corresponding raw materials with reference to all or part of the synthesis methods of Example 1 or Example 2:**

| Example No. | Structural Formula | Chemical Name | $[M+H]^+$ |
|---|---|---|---|
| 6 | 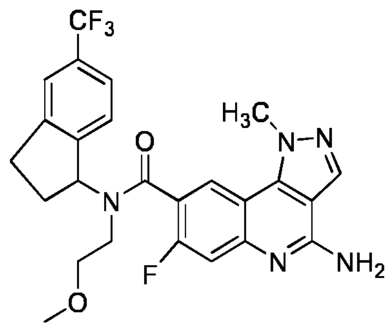 | 4-amino-7-fluoro-N-(2-methoxyethyl)-1-methyl-N-(5-(trifluoromethyl)-2,3-dihydro-1H-inden-1-yl)-1H-pyrazolin[4,3-c]quinolin-8-carboxamide | **502.2** |
| 7 | 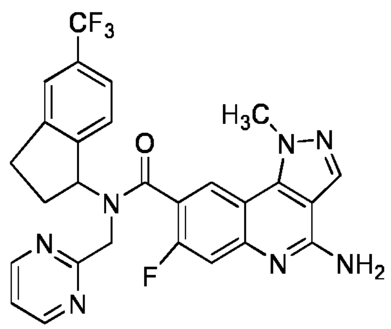 | 4-amino-7-fluoro-1-methyl-N-(pyrimidin-2-ylmethyl)-N-(5-(trifluoromethyl)-2,3-dihydro-1H-inden-1-yl)-1H-pyrazol[4,3-c]quinolin-8-carboxamide | **536.2** |

(continued)

| Example No. | Structural Formula | Chemical Name | [M+H]+ |
|---|---|---|---|
| **8** | 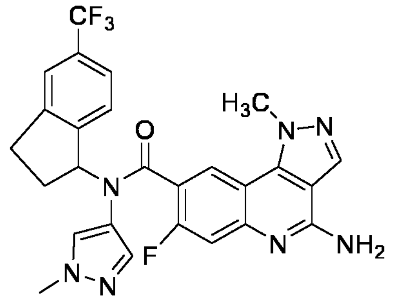 | 4-amino-7-fluoro-1-methyl-N-(1-methyl-1H-pyrazol-4-yl)-N-(5-(trifluoromethyl)-2,3-dihydro-1H-inden-1-yl)-1H-pyrazolo[4,3-c]quinolin-8-carboxamide | **524.2** |
| **10** | 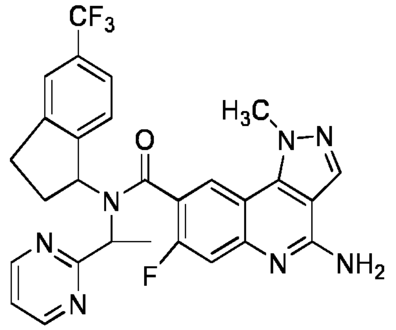 | 4-amino-7-fluoro-1-methyl-N-(1-(pyrimidin-2-yl)ethyl)-N-(5-(trifluoromethyl)-2,3-dihydro-1H-inden-1-yl)-1H-pyrazolo[4,3-c]quinolin-8-carboxamide | **550.2** |
| **16** | 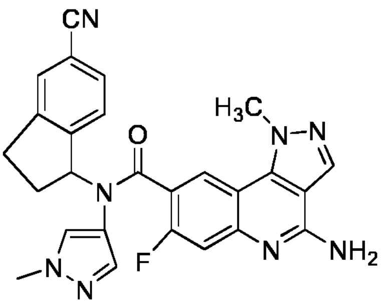 | 4-amino-N-(5-cyano-2,3-dihydro-1H-inden-1-yl)-7-fluoro-1-methyl-N-(1-methyl-1H-pyrazol-4-yl)-1H-pyrazolo[4,3-c]quinolin-8-carboxamide | **481.2** |
| **21** | 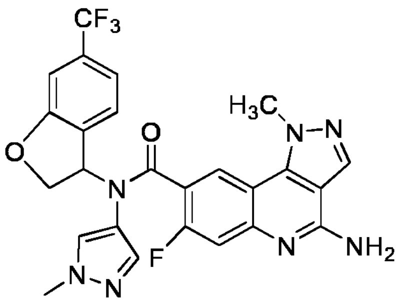 | 4-amino-7-fluoro-1-methyl-N-(1-methyl-1H-pyrazol-4-yl)-N-(6-(trifluoromethyl)-2,3-dihydrobenzofuran-3-yl)-1H-pyrazolo[4,3-c]quinolin-8-carboxamide | **526.2** |
| **23** | 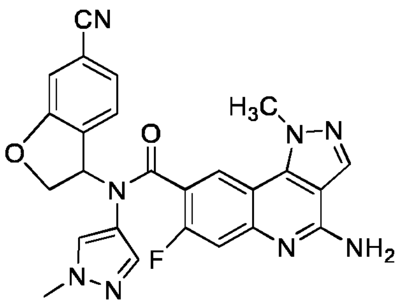 | 4-amino-N-(6-cyano-2,3-dihydrobenzofuran-3-yl)-7-fluoro-1-methyl-N-(1-methyl-1H-pyrazol-4-yl)-1H-pyrazolo[4,3-c]quinolin-8-carboxamide | **483.2** |

(continued)

| Example No. | Structural Formula | Chemical Name | [M+H]+ |
|---|---|---|---|
| **28** | 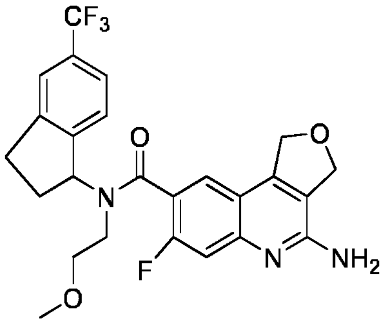 | 4-amino-7-fluoro-N-(2-methoxyethyl)-N-(5-(trifluoromethyl)-2,3-dihydro-1H-inden-1-yl)-1,3-dihydrofuran[3,4-c]quinolin-8-carboxamide | **490.2** |
| **29** | 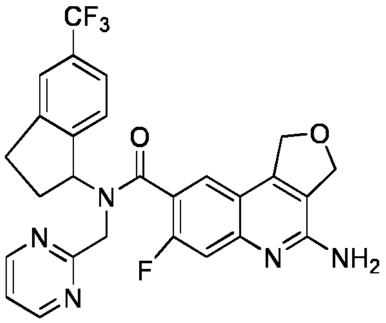 | 4-amino-7-fluoro-N-(pyrimidin-2-ylmethyl)-N-(5-(trifluoromethyl)-2,3-dihydro-1H-inden-1-yl)-1,3-dihydrofuran[3,4-c]quinolin-8-carboxamide | **524.2** |
| **30** | 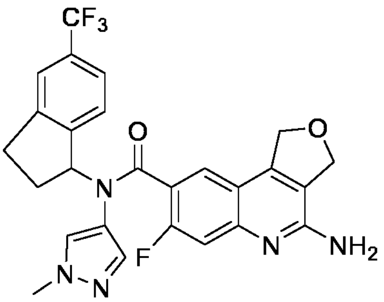 | 4-amino-7-fluoro-N-(1-methyl-1H-pyrazol-4-yl)-N-(5-(trifluoromethyl)-2,3-dihydro-1H-inden-1-yl)-1,3-dihydrofuran[3,4-c]quinolin-8-carboxamide | **512.2** |
| **32** | 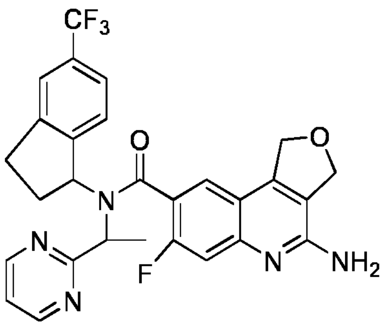 | 4-amino-7-fluoro-N-(1-(pyrimidin-2-yl)ethyl)-N-(5-(trifluoromethyl)-2,3-dihydro-1H-inden-1-yl)-1,3-dihydrofuro[3,4-c]quinolin-8-carboxamide | **538.2** |
| **38** | 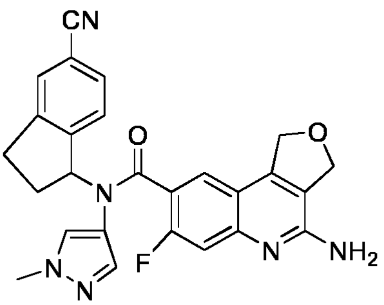 | 4-amino-N-(5-cyano-2,3-dihydro-1H-inden-1-yl)-7-fluoro-N-(1-methyl-1H-pyrazol-4-yl)-1,3-dihydrofuro[3,4-c]quinolin-8-carboxamide | **469.2** |

(continued)

| Example No. | Structural Formula | Chemical Name | [M+H]+ |
|---|---|---|---|
| **43** | 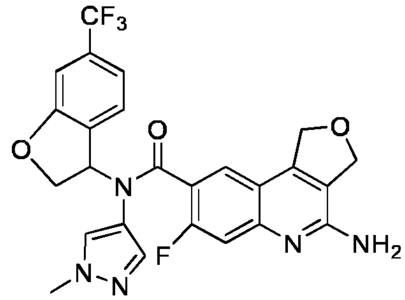 | 4-amino-7-fluoro-N-(1-methyl-1H-pyrazol-4-yl)-N-(6-(trifluoromethyl)-2,3-dihydrobenzofuran-3-yl)-1,3-dihydrofuran[3,4-c]quinolin-8-carboxamide | **514.2** |
| **45** | 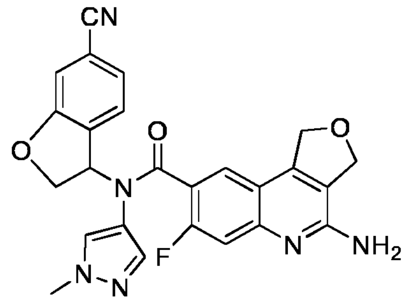 | 4-amino-N-(6-cyano-2,3-dihydrobenzofuran-3-yl)-7-fluoro-N-(1-methyl-1H-pyrazol-4-yl)-1,3-dihydrofuro[3,4-c]quinolin-8-carboxamide | **471.2** |
| **47** | 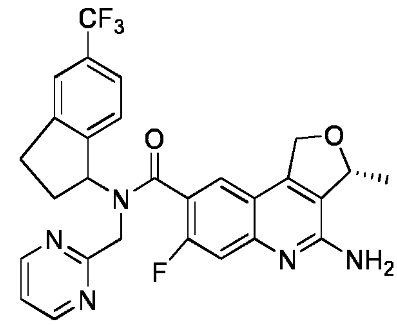 | (3R)-4-amino-7-fluoro-3-methyl-N-(pyrimidin-2-ylmethyl)-N-(5-(trifluoromethyl)-2,3-dihydro-1H-inden-1-yl)-1,3-dihydrofuran[3,4-c]quinolin-8-carboxamide | **538.2** |
| **48** | 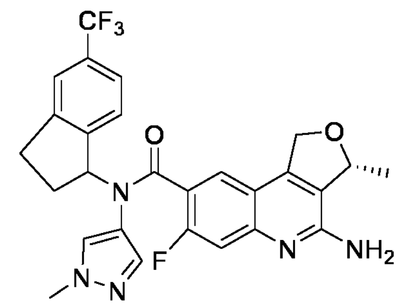 | (3R)-4-amino-7-fluoro-3-methyl-N-(1-methyl-1H-pyrazol-4-yl)-N-(5-(trifluoromethyl)-2,3-dihydro-1H-inden-1-yl)-1,3-dihydrofuro[3,4-c]quinolin-8-carboxamide | **526.2** |
| **50** | 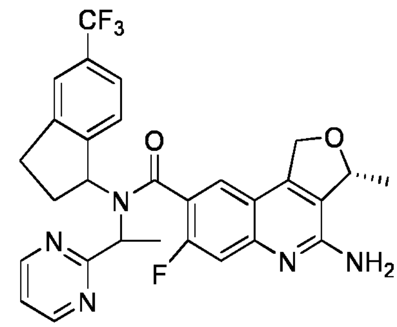 | (3R)-4-amino-7-fluoro-3-methyl-N-(1-(pyrimidin-2-yl)ethyl)-N-(5-(trifluoromethyl)-2,3-dihydro-1H-inden-1-yl)-1,3-dihydrofuran[3,4-c]quinolin-8-carboxamide | **552.2** |

(continued)

| Example No. | Structural Formula | Chemical Name | [M+H]+ |
|---|---|---|---|
| 56 | 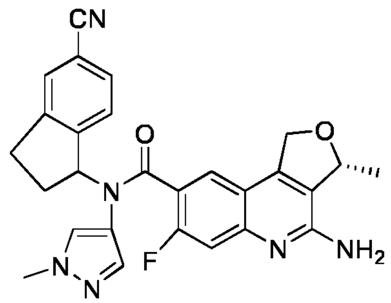 | (3R)-4-amino-N-(5-cyano-2,3-dihydro-1H-inden-1-yl)-7-fluoro-3-methyl-N-(1-methyl-1H-pyrazol-4-yl)-1,3-dihydrofuro[3,4-c]quinolin-8-carboxamide | 483.2 |
| 61 | 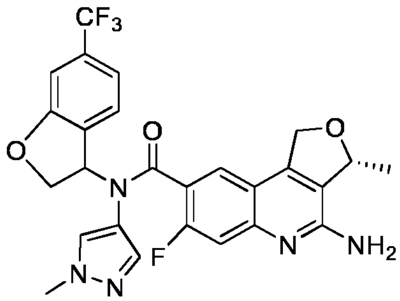 | (3R)-4-amino-7-fluoro-3-methyl-N-(1-methyl-1H-pyrazol-4-yl)-N-(6-(trifluoromethyl)-2,3-dihydrobenzofuran-3-yl)-1,3-dihydrofuran[3,4-c]quinolin-8-carboxamide | 528.2 |
| 63 | 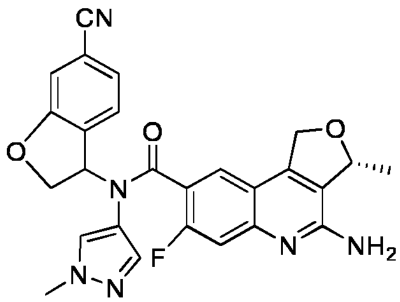 | (3R)-4-amino-N-(6-cyano-2,3-dihydrobenzofuran-3-yl)-7-fluoro-3-methyl-N-(1-methyl-1H-pyrazol-4-yl)-1,3-dihydrofuran[3,4-c]quinolin-8-carboxamide | 485.2 |
| 67 | 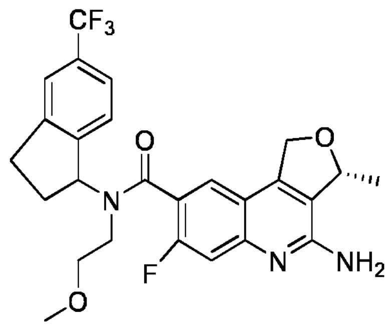 | (3R)-4-amino-7-fluoro-N-(2-methoxyethyl)-3-methyl-N-(5-(trifluoromethyl)-2,3-dihydro-1H-inden-1-yl)-1,3-dihydrofuran[3,4-c]quinolin-8-carboxamide | 504.2 |
| 68 | 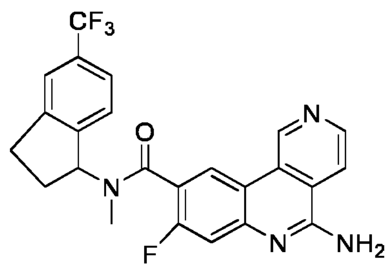 | 5-amino-8-fluoro-N-methyl-N-(5-(trifluoromethyl)-2,3-dihydro-1H-inden-1-yl)benzo[c][2,6]naphthyridin-9-carboxamide | 455.1 |

(continued)

| Example No. | Structural Formula | Chemical Name | [M+H]+ |
|---|---|---|---|
| 69 | 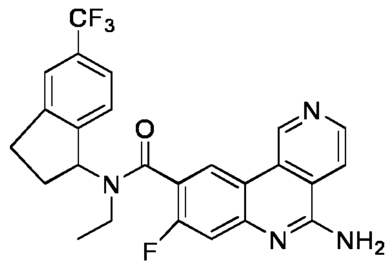 | 5-amino-N-ethyl-8-fluoro-N-(5-(trifluoromethyl)-2,3-dihydro-1H-inden-1-yl)benzo[c][2,6]naphthyridin-9-carboxamide | 469.2 |
| 70 | 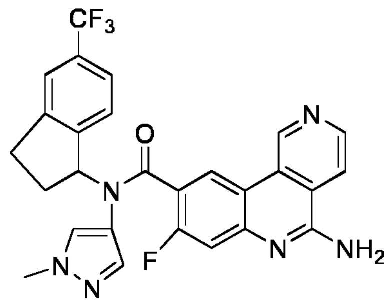 | 5-amino-8-fluoro-N-(1-methyl-1H-pyrazol-4-yl)-N-(5-(trifluoromethyl)-2,3-dihydro-1H-inden-1-yl)benzo[c][2,6]naphthyridin-9-carboxamide | 521.2 |
| 71 | 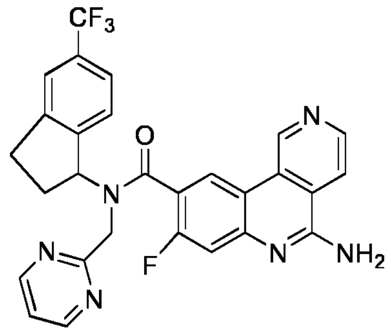 | 5-amino-8-fluoro-N-(pyrimidin-2-ylmethyl)-N-(5-(trifluoromethyl)-2,3-dihydro-1H-inden-1-yl)benzo[c][2,6]naphthyridin-9-carboxamide | 533.2 |
| 76 | 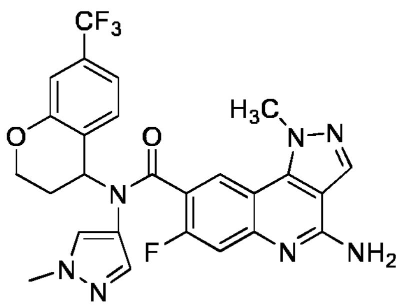 | 4-amino-7-fluoro-1-methyl-N-(1-methyl-1H-pyrazol-4-yl)-N-(7-(trifluoromethyl)chroman-4-yl)-1H-pyrazolo[4,3-c]quinolin-8-carboxamide | 540.2 |
| 78 | 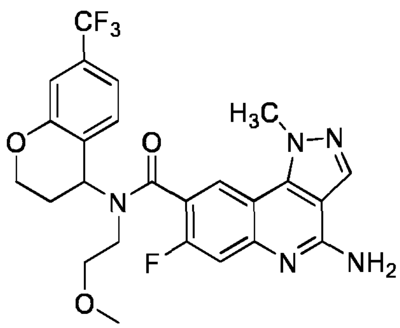 | 4-amino-7-fluoro-N-(2-methoxyethyl)-1-methyl-N-(7-(trifluoromethyl)chroman-4-yl)-1H-pyrazolo[4,3-c]quinolin-8-carboxamide | 518.2 |

(continued)

| Example No. | Structural Formula | Chemical Name | [M+H]+ |
|---|---|---|---|
| **79** | 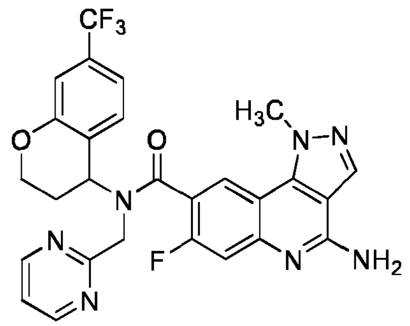 | 4-amino-7-fluoro-1-methyl-N-(pyrimidin-2-ylmethyl)-N-(7-(trifluoromethyl)chroman-4-yl)-1H-pyrazolo[4,3-c]quinolin-8-carboxamide | **552.2** |
| **83** | 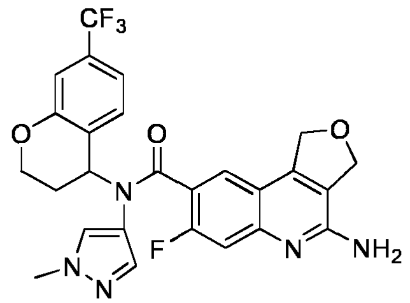 | 4-amino-7-fluoro-N-(1-methyl-1H-pyrazol-4-yl)-N-(7-(trifluoromethyl)chroman-4-yl)-1,3-dihydrofuro[3,4-c]quinolin-8-carboxamide | **528.2** |
| **84** | 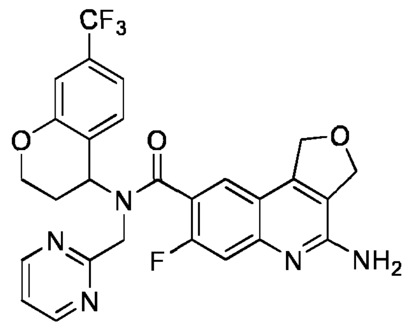 | 4-amino-7-fluoro-N-(pyrimidin-2-ylmethyl)-N-(7-(trifluoromethyl)chroman-4-yl)-1,3-dihydrofuro[3,4-c]quinolin-8-carboxamide | **540.2** |
| **86** | 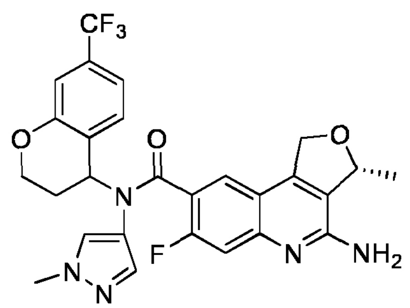 | (3R)-4-amino-7-fluoro-3-methyl-N-(1-methyl-1H-pyrazol-4-yl)-N-(7-(trifluoromethyl) chroman-4-yl)-1,3-dihydrofuro[3,4-c]quinolin-8-carboxamide | **542.2** |
| **87** | 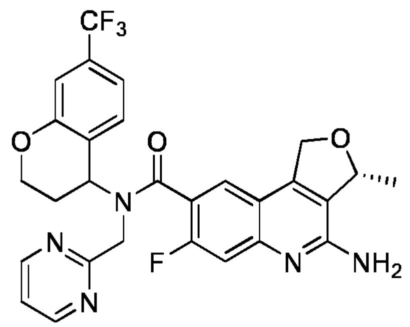 | (3R)-4-amino-7-fluoro-3-methyl-N-(pyrimidin-2-ylmethyl)-N-(7-(trifluoromethyl)chroman-4-yl)-1,3-dihydrofuro[3,4-c]quinolin-8-carboxamide | **554.2** |

(continued)

| Example No. | Structural Formula | Chemical Name | [M+H]+ |
|---|---|---|---|
| **89** | 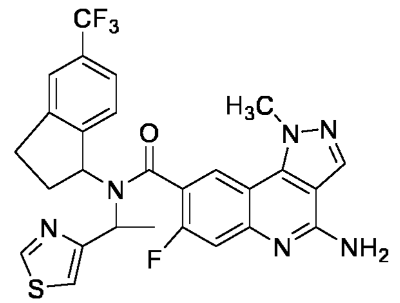 | 4-amino-7-fluoro-1-methyl-N-(1-(thiazol-4-yl) ethyl)-N-(5-(trifluoromethyl)-2,3-dihydro-1H-inden-1-yl)-1H-pyrazolo[4,3-c]quinolin-8-carboxamide | **555.2** |
| **90** | 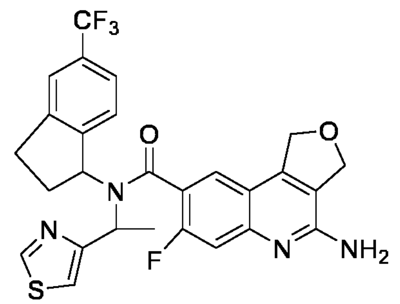 | 4-amino-7-fluoro-N-(1-(thiazol-4-yl) ethyl)-N-(5-(trifluoromethyl)-2,3-dihydro-1H-inden-1-yl)-1,3-dihydrofuro[3,4-c]quinolin-8-carboxamide | **543.1** |
| **91** | 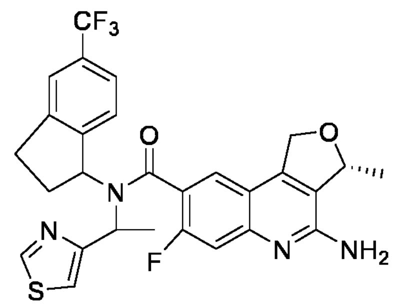 | (3R)-4-amino-7-fluoro-3-methyl-N-(1-(thiazol-4-yl)ethyl)-N-(5-(trifluoromethyl)-2,3-dihydro-1H-inden-1-yl)-1,3-dihydrofuro[3,4-c]quinolin-8-carboxamide | **557.2** |
| **92** | 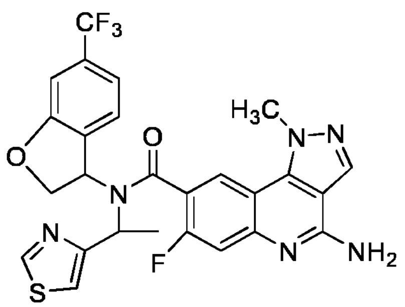 | 4-amino-7-fluoro-1-methyl-N-(1-(thiazol-4-yl) ethyl)-N-(6-(trifluoromethyl)-2,3-dihydrobenzofuran-3-yl)-1H-pyrazolo[4,3-c]quinolin-8-carboxamide | **557.1** |
| **93** | 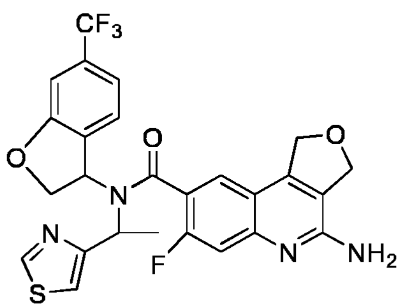 | 4-amino-7-fluoro-N-(1-(thiazol-4-yl) ethyl)-N-(6-(trifluoromethyl)-2,3-dihydrobenzofuran-3-yl)-1,3-dihydrofuro[3,4-c]quinolin-8-carboxamide | **545.1** |

(continued)

| Example No. | Structural Formula | Chemical Name | [M+H]+ |
|---|---|---|---|
| 94 | 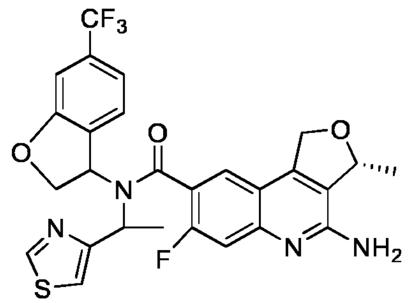 | (3R)-4-amino-7-fluoro-3-methyl-N-(1-(thiazol-4-yl)ethyl)-N-(6-(trifluoromethyl)-2,3-dihydrobenzofuran-3-yl)-1,3-dihydrofuro[3,4-c]quinolin-8-carboxamide | 559.1 |
| 95 | 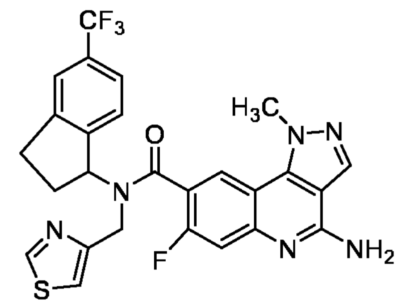 | 4-amino-7-fluoro-1-methyl-N-(thiazol-4-ylmethyl)-N-(5-(trifluoromethyl)-2,3-dihydro-1H-inden-1-yl)-1H-pyrazolo[4,3-c]quinolin-8-carboxamide | 541.1 |
| 96 | 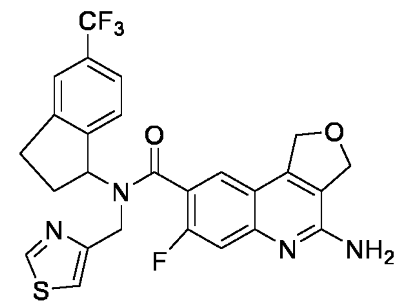 | 4-amino-7-fluoro-N-(thiazol-4-ylmethyl)-N-(5-(trifluoromethyl)-2,3-dihydro-1H-inden-1-yl)-1,3-dihydrofuro[3,4-c]quinolin-8-carboxamide | 529.1 |
| 97 | 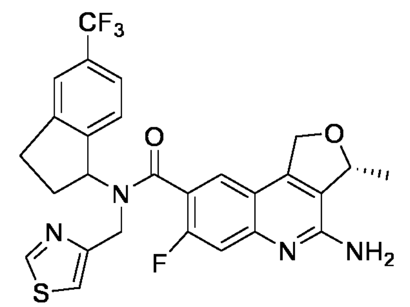 | (3R)-4-amino-7-fluoro-3-methyl-N-(thiazol-4-ylmethyl)-N-(5-(trifluoromethyl)-2,3-dihydro-1H-inden-1-yl)-1,3-dihydrofuro[3,4-c]quinolin-8-carboxamide | 543.1 |
| 98 | 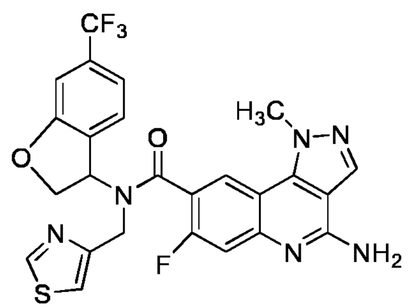 | 4-amino-7-fluoro-1-methyl-N-(thiazol-4-ylmethyl)-N-(6-(trifluoromethyl)-2,3-dihydrobenzofuran-3-yl)-1H-pyrazolo[4,3-c]quinolin-8-carboxamide | 543.1 |

(continued)

| Example No. | Structural Formula | Chemical Name | [M+H]+ |
|---|---|---|---|
| 99 | 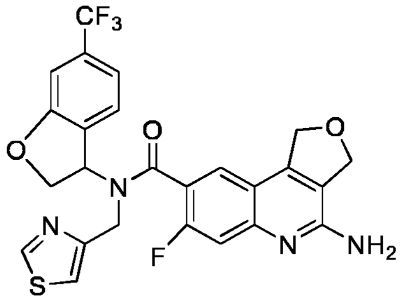 | 4-amino-7-fluoro-N-(thiazol-4-ylmethyl)-N-(6-(trifluoromethyl)-2,3-dihydrobenzofuran-3-yl)-1,3-dihydrofuro[3,4-c]quinolin-8-carboxamide | 531.1 |
| 100 | 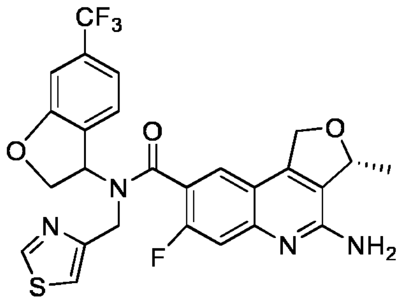 | (3R)-4-amino-7-fluoro-3-methyl-N-(thiazol-4-ylmethyl)-N-(6-(trifluoromethyl)-2,3-dihydrobenzofuran-3-yl)-1,3-dihydrofuro[3,4-c]quinolin-8-carboxamide | 545.1 |
| 101 | 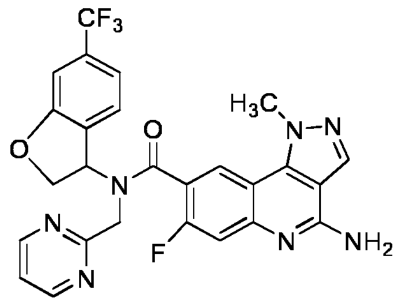 | 4-amino-7-fluoro-1-methyl-N-(pyrimidin-2-ylmethyl)-N-(6-(trifluoromethyl)-2,3-dihydrobenzofuran-3-yl)-1H-pyrazolo[4,3-c]quinolin-8-carboxamide | 538.2 |
| 102 | 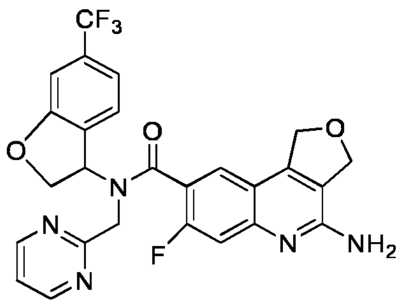 | 4-amino-7-fluoro-N-(pyrimidin-2-ylmethyl)-N-(6-(trifluoromethyl)-2,3-dihydrobenzofuran-3-yl)-1,3-dihydrofuro[3,4-c]quinolin-8-carboxamide | 526.1 |
| 103 | 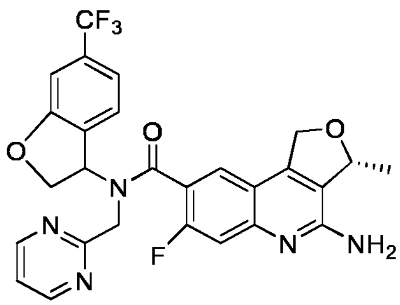 | (3R)-4-amino-7-fluoro-3-methyl-N-(pyrimidin-2-ylmethyl)-N-(6-(trifluoromethyl)-2,3-dihydrobenzofuran-3-yl)-1,3-dihydrofuro[3,4-c]quinolin-8-carboxamide | 540.2 |

(continued)

| Example No. | Structural Formula | Chemical Name | $[M+H]^+$ |
|---|---|---|---|
| 104 | 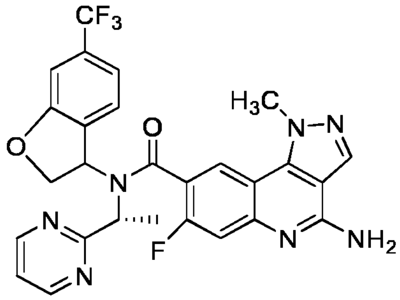 | 4-amino-7-fluoro-1-methyl-N-((R)-1-(pyrimidin-2-yl)ethyl)-N-(6-(trifluoromethyl)-2,3-dihydrobenzofuran-3-yl)-1H-pyrazolo[4,3-c]quinolin-8-carboxamide | 552.2 |
| 105 | 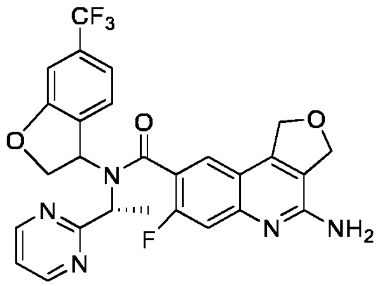 | 4-amino-7-fluoro-N-((R)-1-(pyrimidin-2-yl)ethyl)-N-(6-(trifluoromethyl)-2,3-dihydrobenzofuran-3-yl)-1,3-dihydrofuro[3,4-c]quinolin-8-carboxamide | 540.2 |
| 106 | 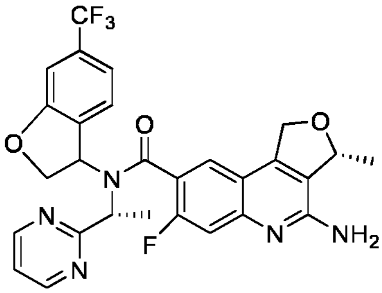 | (3R)-4-amino-7-fluoro-3-methyl-N-((R)-1-(pyrimidin-2-yl)ethyl)-N-(6-(trifluoromethyl)-2,3-dihydrobenzofuran-3-yl)-1,3-dihydrofuro[3,4-c]quinolin-8-carboxamide | 554.2 |
| 108 | 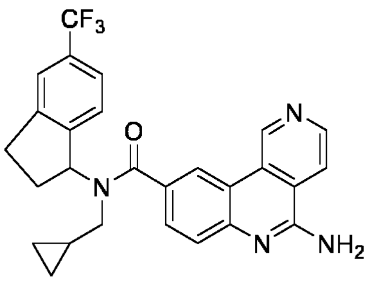 | 5-amino-N-(cyclopropylmethyl)-N-(5-(trifluoromethyl)-2,3-dihydro-1H-inden-1-yl)benzo[c][2,6]naphthyridin-9-carboxamide | 477.2 |
| 109 | 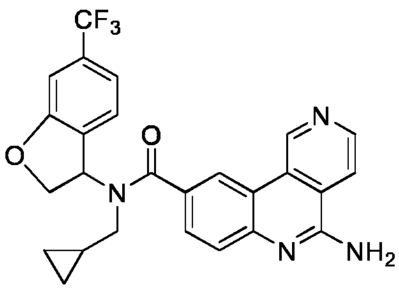 | 5-amino-N-(cyclopropylmethyl)-N-(6-(trifluoromethyl)-2,3-dihydrobenzofuran-3-yl)benzo[c][2,6]naphthyridin-9-carboxamide | 479.2 |

(continued)

| Example No. | Structural Formula | Chemical Name | [M+H]+ |
|---|---|---|---|
| **110** | 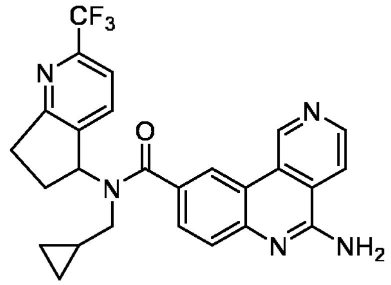 | 5-amino-N-(cyclopropylmethyl)-N-(2-(trifluoromethyl)-6,7-dihydro-5H-cyclopenta[b]pyridin-5-yl)benzo[c][2,6]naphthyridin-9-carboxamide | **478.2** |
| **111** | 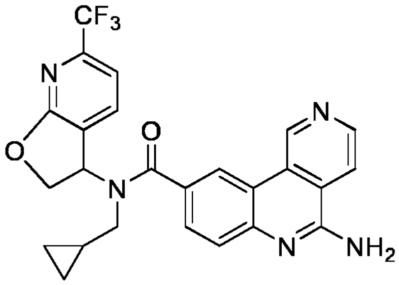 | 5-amino-N-(cyclopropylmethyl)-N-(6-(trifluoromethyl)-2,3-dihydrofuro[2,3-b]pyridin-3-yl)benzo[c][2,6]naphthyridin-9-carboxamide | **480.2** |
| **112** | 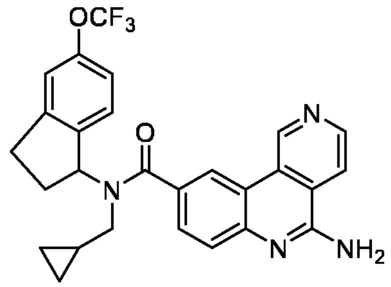 | 5-amino-N-(cyclopropylmethyl)-N-(5-(trifluoromethoxy)-2,3-dihydro-1H-inden-1-yl)benzo[c][2,6]naphthyridin-9-carboxamide | **493.2** |
| **113** | 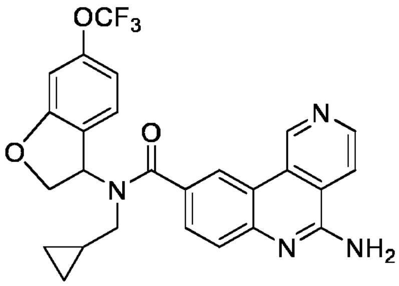 | 5-amino-N-(cyclopropylmethyl)-N-(6-(trifluoromethoxy)-2,3-dihydrobenzofuran-3-yl)benzo[c][2,6]naphthyridin-9-carboxamide | **495.2** |
| **114** | 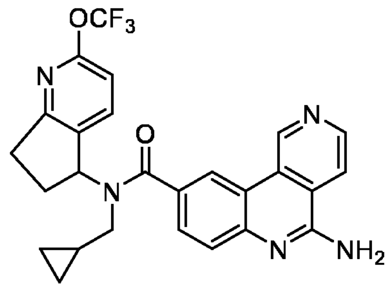 | 5-amino-N-(cyclopropylmethyl)-N-(2-(trifluoromethoxy)-6,7-dihydro-5H-cyclopenta[b]pyridin-5-yl)benzo[c][2,6]naphthyridin-9-carboxamide | **494.2** |

(continued)

| Example No. | Structural Formula | Chemical Name | [M+H]+ |
|---|---|---|---|
| **115** | 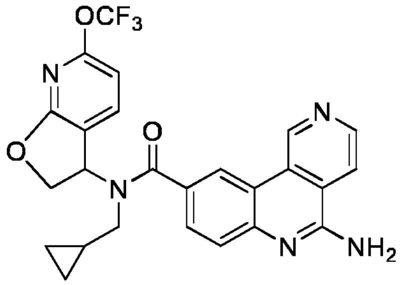 | 5-amino-N-(cyclopropylmethyl)-N-(6-(trifluoromethoxy)-2,3-dihydrofuro[2,3-b]pyridin-3-yl)benzo[c][2,6]naphthyridin-9-carboxamide | **496.2** |
| **116** | 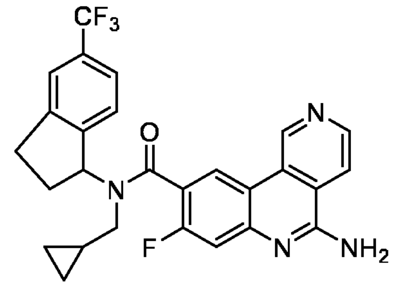 | 5-amino-N-(cyclopropylmethyl)-8-fluoro-N-(5-(trifluoromethyl)-2,3-dihydro-1H-inden-1-yl)benzo[c][2,6]naphthyridin-9-carboxamide | **495.2** |
| **117** | 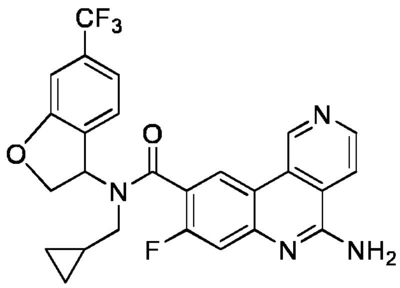 | 5-amino-N-(cyclopropylmethyl)-8-fluoro-N-(6-(trifluoromethyl)-2,3-dihydrobenzofuran-3-yl)benzo[c][2,6]naphthyridin-9-carboxamide | **497.2** |
| **118** | 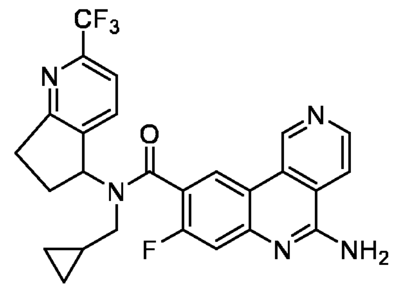 | 5-amino-N-(cyclopropylmethyl)-8-fluoro-N-(2-(trifluoromethyl)-6,7-dihydro-5H-cyclopenta[b]pyridin-5-yl)benzo[c][2,6]naphthyridin-9-carboxamide | **496.2** |
| **119** | 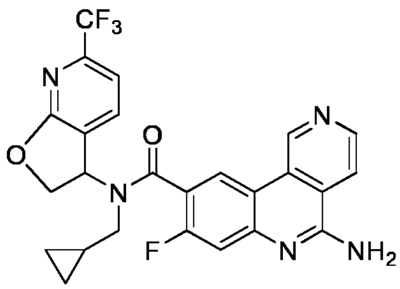 | 5-amino-N-(cyclopropylmethyl)-8-fluoro-N-(6-(trifluoromethyl)-2,3-dihydrofuro[2,3-b]pyridin-3-yl)benzo[c][2,6]naphthyridin-9-carboxamide | **498.1** |

(continued)

| Example No. | Structural Formula | Chemical Name | [M+H]+ |
|---|---|---|---|
| **120** | 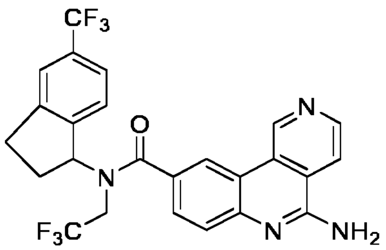 | 5-amino-N-(2,2,2-trifluoroethyl)-N-(5-(trifluoromethyl)-2,3-dihydro-1H-inden-1-yl)benzo[c][2,6]naphthyridin-9-carboxamide | **505.1** |
| **121** | 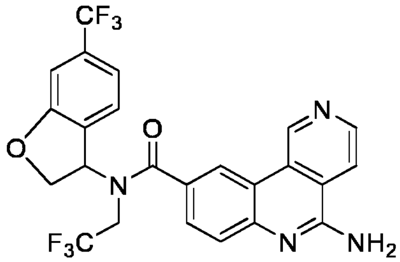 | 5-amino-N-(2,2,2-trifluoroethyl)-N-(6-(trifluoromethyl)-2,3-dihydrobenzofuran-3-yl)benzo[c][2,6]naphthyridin-9-carboxamide | **507.1** |
| **122** | 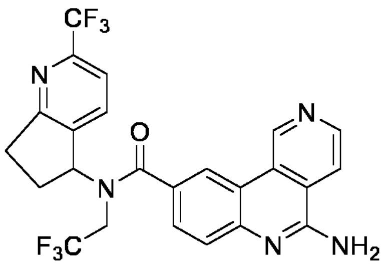 | 5-amino-N-(2,2,2-trifluoroethyl)-N-(2-(trifluoromethyl)-6,7-dihydro-5H-cyclopenta[b]pyridin-5-yl)benzo[c][2,6]naphthyridin-9-carboxamide | **506.1** |
| **123** | 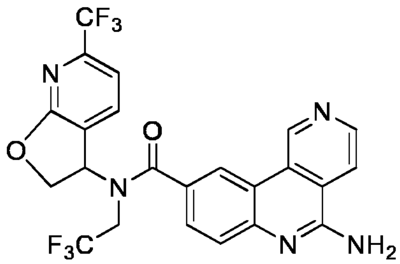 | 5-amino-N-(2,2,2-trifluoroethyl)-N-(6-(trifluoromethyl)-2,3-dihydrofuro[2,3-b]pyridin-3-yl)benzo[c][2,6]naphthyridin-9-carboxamide | **508.1** |
| **124** | 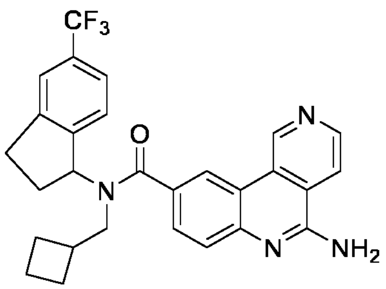 | 5-amino-N-(cyclobutylmethyl)-N-(5-(trifluoromethyl)-2,3-dihydro-1H-inden-1-yl)benzo[c][2,6]naphthyridin-9-carboxamide | **491.2** |

(continued)

| Example No. | Structural Formula | Chemical Name | $[M+H]^+$ |
|---|---|---|---|
| **125** | 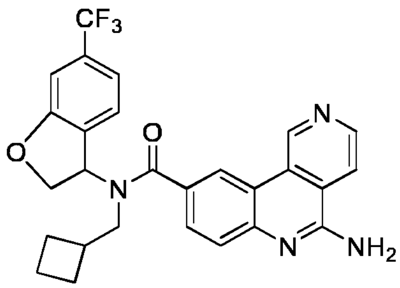 | 5-amino-N-(cyclobutylmethyl)-N-(6-(trifluoromethyl)-2,3-dihydrobenzofuran-3-yl)benzo[c][2,6]naphthyridin-9-carboxamide | **493.2** |
| **126** | 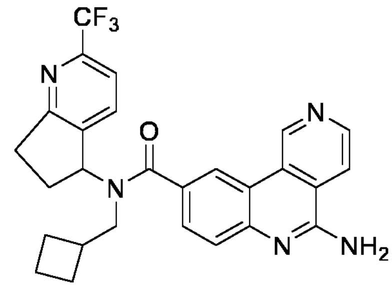 | 5-amino-N-(cyclobutylmethyl)-N-(2-(trifluoromethyl)-6,7-dihydro-5H-cyclopenta[b]pyridin-5-yl)benzo[c][2,6]naphthyridin-9-carboxamide | **492.2** |
| **127** | 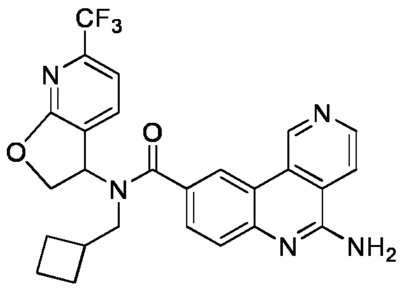 | 5-amino-N-(cyclobutylmethyl)-N-(6-(trifluoromethyl)-2,3-dihydrofuro[2,3-b]pyridin-3-yl)benzo[c][2,6]naphthyridin-9-carboxamide | **494.2** |
| **128** | 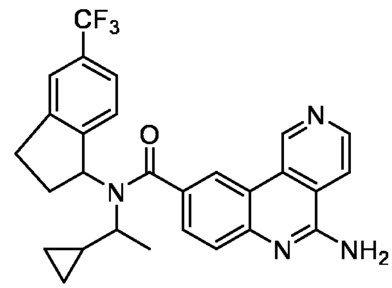 | 5-amino-N-(1-cyclopropylethyl)-N-(5-(trifluoromethyl)-2,3-dihydro-1H-inden-1-yl)benzo[c][2,6]naphthyridin-9-carboxamide | **491.2** |
| **129** | 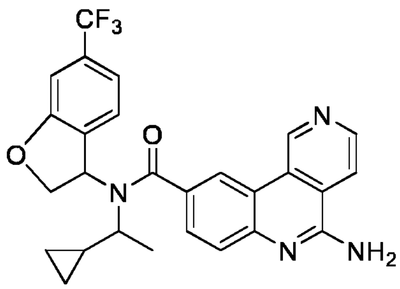 | 5-amino-N-(1-cyclopropylethyl)-N-(6-(trifluoromethyl)-2,3-dihydrobenzofuran-3-yl)benzo[c][2,6]naphthyridin-9-carboxamide | **493.2** |

(continued)

| Example No. | Structural Formula | Chemical Name | [M+H]+ |
|---|---|---|---|
| **130** | 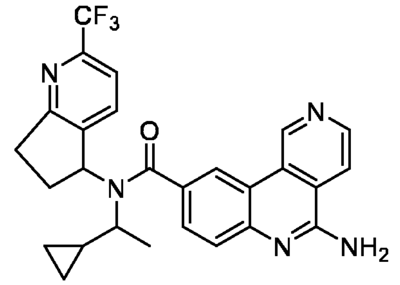 | 5-amino-N-(1-cyclopropylethyl)-N-(2-(trifluoromethyl)-6,7-dihydro-5H-cyclopenta[b]pyridin-5-yl)benzo[c][2,6]naphthyridin-9-carboxamide | **492.2** |
| **131** | 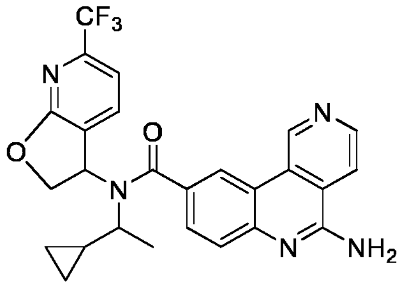 | 5-amino-N-(1-cyclopropylethyl)-N-(6-(trifluoromethyl)-2,3-dihydrofuro[2,3-b]pyridin-3-yl)benzo[c][2,6]naphthyridin-9-carboxamide | **494.2** |
| **132** | 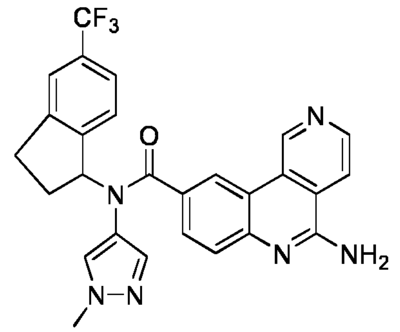 | 5-amino-N-(1-methyl-1H-pyrazol-4-yl)-N-(5-(trifluoromethyl)-2,3-dihydro-1H-inden-1-yl)benzo[c][2,6]naphthyridin-9-carboxamide | **503.2** |
| **133** | 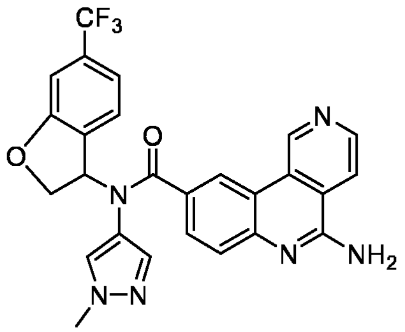 | 5-amino-N-(1-methyl-1H-pyrazol-4-yl)-N-(6-(trifluoromethyl)-2,3-dihydrobenzofuran-3-yl)benzo[c][2,6]naphthyridin-9-carboxamide | **505.2** |
| **134** | 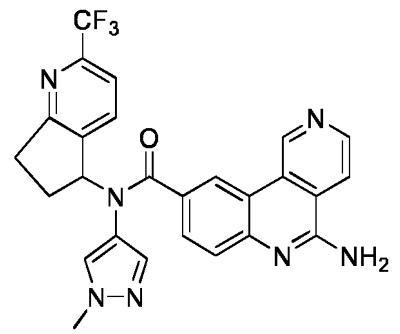 | 5-amino-N-(1-methyl-1H-pyrazol-4-yl)-N-(2-(trifluoromethyl)-6,7-dihydro-5H-cyclopenta[b]pyridin-5-yl)benzo[c][2,6]naphthyridin-9-carboxamide | **504.2** |

(continued)

| Example No. | Structural Formula | Chemical Name | [M+H]+ |
|---|---|---|---|
| **135** | 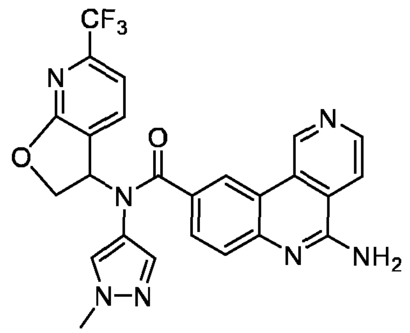 | 5-amino-N-(1-methyl-1H-pyrazol-4-yl)-N-(6-(trifluoromethyl)-2,3-dihydrofuro[2,3-b]pyridin-3-yl) benzo[c][2,6]naphthyridin-9-carboxamide | **506.2** |
| **136** | 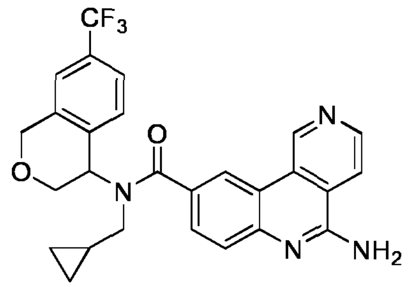 | 5-amino-N-(cyclopropylmethyl)-N-(7-(trifluoromethyl)isochroman-4-yl)benzo[c][2,6]naphthyridin-9-carboxamide | **493.2** |
| **137** | 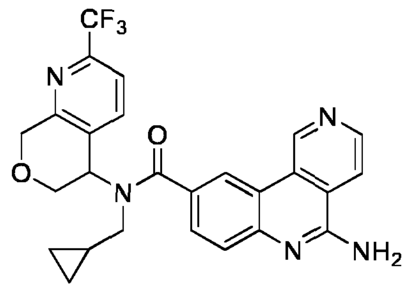 | 5-amino-N-(cyclopropylmethyl)-N-(2-(trifluoromethyl)-5,8-dihydro-6H-pyrano[3,4-b]pyridin-5-yl)benzo[c][2,6]naphthyridin-9-carboxamide | **494.2** |
| **138** | 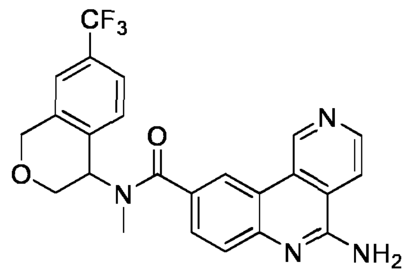 | 5-amino-N-methyl-N-(7-(trifluoromethyl)isochroman-4-yl)benzo[c][2,6]naphthyridin-9-carboxamide | **453.1** |
| **139** | 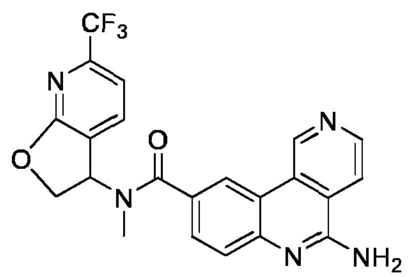 | 5-amino-N-methyl-N-(6-(trifluoromethyl)-2,3-dihydrofuro[2,3-b]pyridin-3-yl)benzo[c][2,6] naphthyridin-9-carboxamide | **440.1** |

(continued)

| Example No. | Structural Formula | Chemical Name | [M+H]+ |
|---|---|---|---|
| **140** | 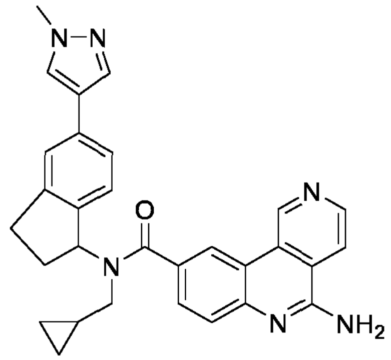 | 5-amino-N-(cyclopropylmethyl)-N-(5-(1-methyl-1H-pyrazol-4-yl)-2,3-dihydro-1H-inden-1-yl)benzo[c][2,6]naphthyridin-9-carboxamide | **489.2** |
| **141** | 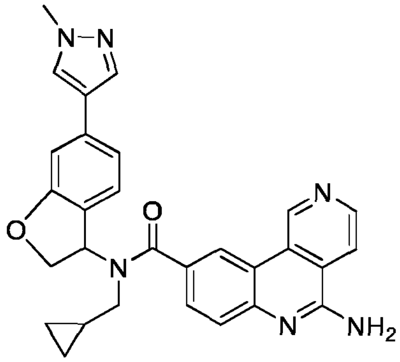 | 5-amino-N-(cyclopropylmethyl)-N-(6-(1-methyl-1H-pyrazol-4-yl)-2,3-dihydrobenzofuran-3-yl)benzo[c][2,6]naphthyridin-9-carboxamide | **491.2** |
| **142** | 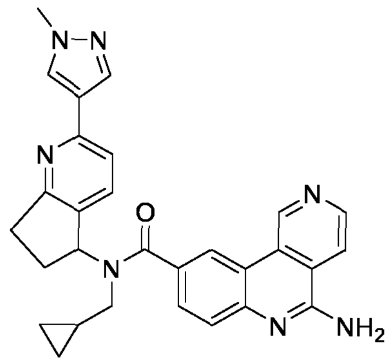 | 5-amino-N-(cyclopropylmethyl)-N-(2-(1-methyl-1H-pyrazol-4-yl)-6,7-dihydro-5H-cyclopenta[b]pyridin-5-yl)benzo[c][2,6]naphthyridin-9-carboxamide | **490.2** |
| **143** | 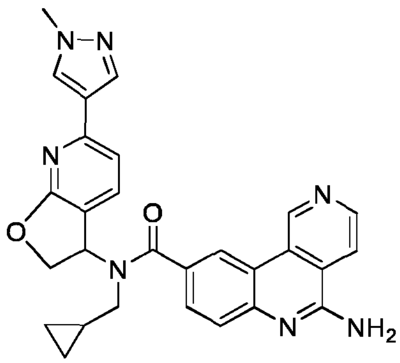 | 5-amino-N-(cyclopropylmethyl)-N-(6-(1-methyl-1H-pyrazol-4-yl)-2,3-dihydrofuro[2,3-b]pyridin-3-yl)benzo[c][2,6]naphthyridin-9-carboxamide | **492.2** |

(continued)

| Example No. | Structural Formula | Chemical Name | [M+H]+ |
|---|---|---|---|
| **144** | 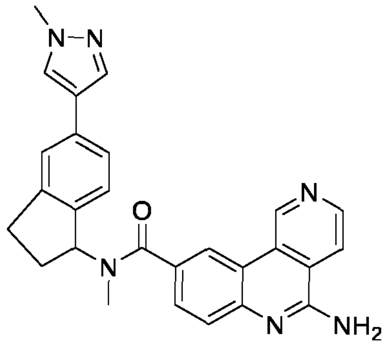 | 5-amino-N-methyl-N-(5-(1-methyl-1H-pyrazol-4-yl)-2,3-dihydro-1H-inden-1-yl)benzo[c][2,6]naphthyridin-9-carboxamide | **449.2** |
| **145** | 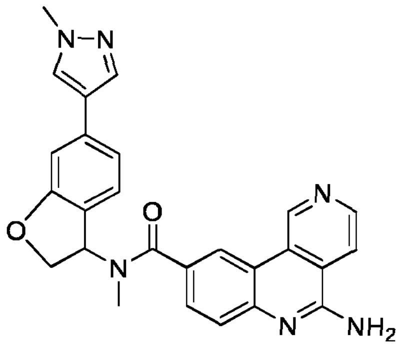 | 5-amino-N-methyl-N-(6-(1-methyl-1H-pyrazol-4-yl)-2,3-dihydrobenzofuran-3-yl)benzo[c][2,6]naphthyridin-9-carboxamide | **451.2** |
| **146** | 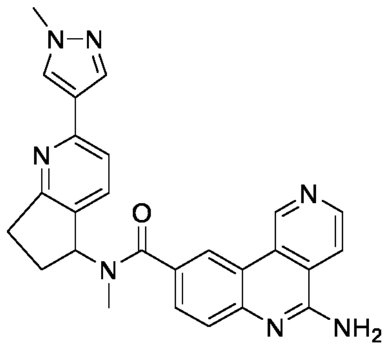 | 5-amino-N-methyl-N-(2-(1-methyl-1H-pyrazol-4-yl)-6,7-dihydro-5H-cyclopenta[b]pyridin-5-yl)benzo[c][2,6]naphthyridin-9-carboxamide | **450.2** |
| **147** | 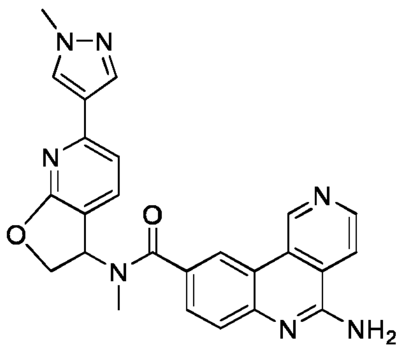 | 5-amino-N-methyl-N-(6-(1-methyl-1H-pyrazol-4-yl)-2,3-dihydrofuro[2,3-b]pyridin-3-yl)benzo[c][2,6]naphthyridin-9-carboxamide | **452.2** |

(continued)

| Example No. | Structural Formula | Chemical Name | [M+H]+ |
|---|---|---|---|
| 150 | 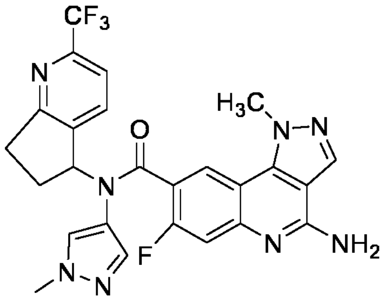 | 4-amino-7-fluoro-1-methyl-N-(1-methyl-1H-pyrazol-4-yl)-N-(2-(trifluoromethyl)-6,7-dihydro-5H-cyclopenta[b]pyridin-5-yl)-1H-pyrazolo[4,3-c]quinolin-8-carboxamide | 525.2 |
| 151 | 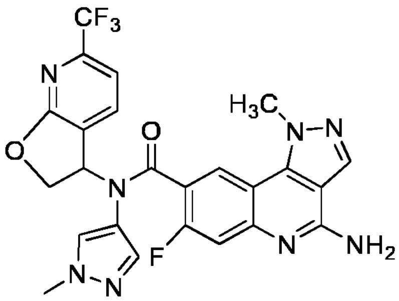 | 4-amino-7-fluoro-1-methyl-N-(1-methyl-1H-pyrazol-4-yl)-N-(6-(trifluoromethyl)-2,3-dihydrofuro[2,3-b]pyridin-3-yl)-1H-pyrazolo[4,3-c]quinolin-8-carboxamide | 527.1 |
| 152 | 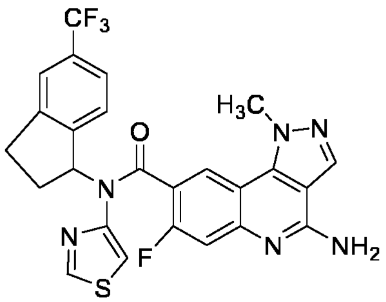 | 4-amino-7-fluoro-1-methyl-N-(thiazol-4-yl)-N-(5-(trifluoromethyl)-2,3-dihydro-1H-inden-1-yl)-1H-pyrazolo[4,3-c]quinolin-8-carboxamide | 527.1 |
| 153 | 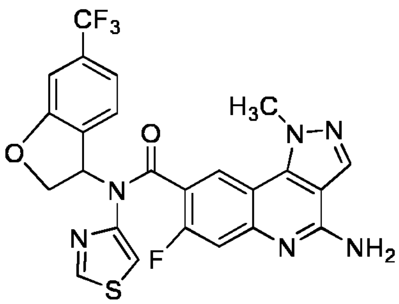 | 4-amino-7-fluoro-1-methyl-N-(thiazol-4-yl)-N-(6-(trifluoromethyl)-2,3-dihydrobenzofuran-3-yl)-1H-pyrazolo[4,3-c]quinolin-8-carboxamide | 529.1 |
| 154 | 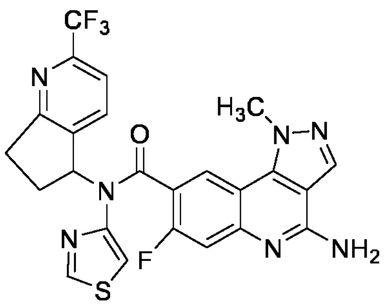 | 4-amino-7-fluoro-1-methyl-N-(thiazol-4-yl)-N-(2-(trifluoromethyl)-6,7-dihydro-5H-cyclopenta[b]pyridin-5-yl)-1H-pyrazolo[4,3-c]quinolin-8-carboxamide | 528.1 |

(continued)

| Example No. | Structural Formula | Chemical Name | [M+H]+ |
|---|---|---|---|
| 155 | 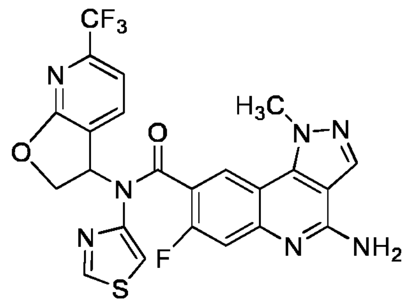 | 4-amino-7-fluoro-1-methyl-N-(thiazol-4-yl)-N-(6-(trifluoromethyl)-2,3-dihydrofuro[2,3-b]pyridin-3-yl)-1H-pyrazolo[4,3-c]quinolin-8-carboxamide | 530.1 |
| 156 | 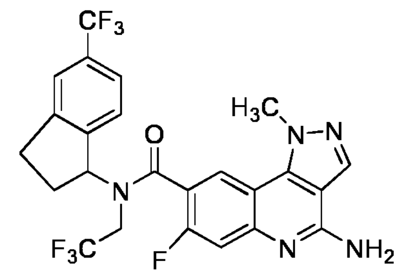 | 4-amino-7-fluoro-1-methyl-N-(2,2,2-trifluoroethyl)-N-(5-(trifluoromethyl)-2,3-dihydro-1H-inden-1-yl)-1H-pyrazolo[4,3-c]quinolin-8-carboxamide | 526.1 |
| 157 | 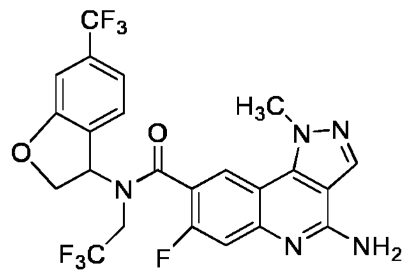 | 4-amino-7-fluoro-1-methyl-N-(2,2,2-trifluoroethyl)-N-(6-(trifluoromethyl)-2,3-dihydrobenzofuran-3-yl)-1H-pyrazolo[4,3-c]quinolin-8-carboxamide | 528.1 |
| 158 | 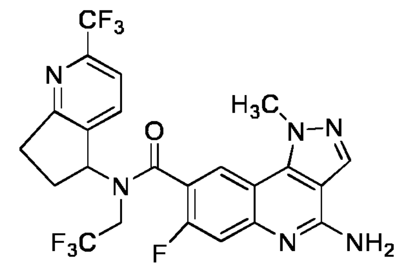 | 4-amino-7-fluoro-1-methyl-N-(2,2,2-trifluoroethyl)-N-(2-(trifluoromethyl)-6,7-dihydro-5H-cyclopenta[b]pyridin-5-yl)-1H-pyrazolo[4,3-c]quinolin-8-carboxamide | 527.1 |
| 159 | 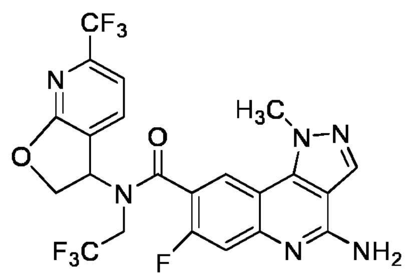 | 4-amino-7-fluoro-1-methyl-N-(2,2,2-trifluoroethyl)-N-(6-(trifluoromethyl)-2,3-dihydrofuro[2,3-b]pyridin-3-yl)-1H-pyrazolo[4,3-c]quinolin-8-carboxamide | 529.1 |

(continued)

| Example No. | Structural Formula | Chemical Name | [M+H]+ |
|---|---|---|---|
| 160 | 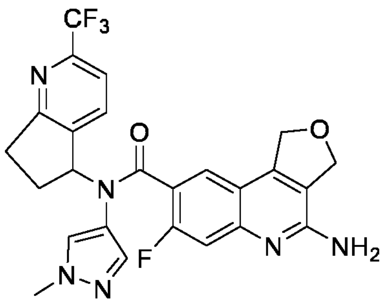 | 4-amino-7-fluoro-N-(1-methyl-1H-pyrazol-4-yl)-N-(2-(trifluoromethyl)-6,7-dihydro-5H-cyclopenta[b]pyridin-5-yl)-1,3-dihydrofuro[3,4-c]quinolin-8-carboxamide | 513.2 |
| 161 | 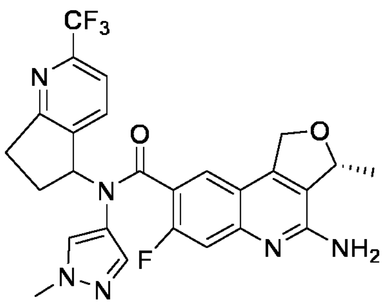 | (3R)-4-amino-7-fluoro-3-methyl-N-(1-methyl-1H-pyrazol-4-yl)-N-(2-(trifluoromethyl)-6,7-dihydro-5H-cyclopenta[b]pyridin-5-yl)-1,3-dihydrofuro[3,4-c]quinolin-8-carboxamide | 527.2 |
| 162 | 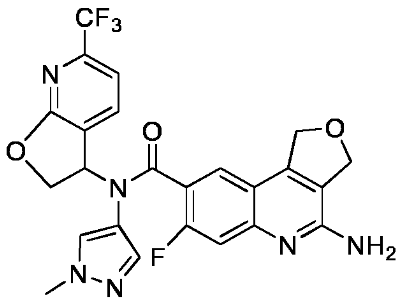 | 4-amino-7-fluoro-N-(1-methyl-1H-pyrazol-4-yl)-N-(6-(trifluoromethyl)-2,3-dihydrofuro[2,3-b]pyridin-3-yl)-1,3-dihydrofuro[3,4-c]quinolin-8-carboxamide | 515.1 |
| 163 | 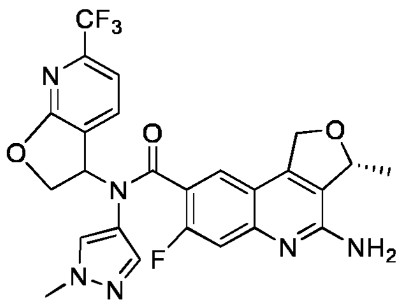 | (3R)-4-amino-7-fluoro-3-methyl-N-(1-methyl-1H-pyrazol-4-yl)-N-(6-(trifluoromethyl)-2,3-dihydrofuro[2,3-b]pyridin-3-yl)-1,3-dihydrofuro[3,4-c]quinolin-8-carboxamide | 529.2 |
| 164 | 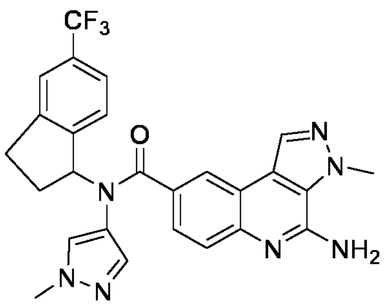 | 4-amino-3-methyl-N-(1-methyl-1H-pyrazol-4-yl)-N-(5-(trifluoromethyl)-2,3-dihydro-1H-inden-1-yl)-3H-pyrazolo[3,4-c]quinolin-8-carboxamide | 506.2 |

(continued)

| Example No. | Structural Formula | Chemical Name | [M+H]+ |
|---|---|---|---|
| **165** | 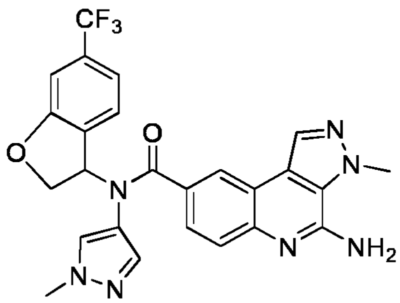 | 4-amino-3-methyl-N-(1-methyl-1H-pyrazol-4-yl)-N-(6-(trifluoromethyl)-2,3-dihydrobenzofuran-3-yl)-3H-pyrazolo[3,4-c]quinolin-8-carboxamide | **508.2** |
| **166** | 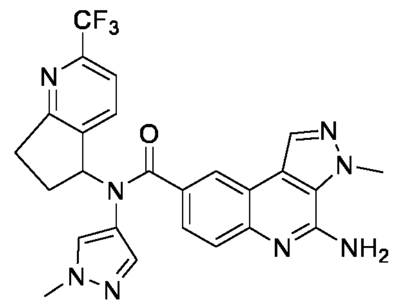 | 4-amino-3-methyl-N-(1-methyl-1H-pyrazol-4-yl)-N-(2-(trifluoromethyl)-6,7-dihydro-5H-cyclopenta[b]pyridin-5-yl)-3H-pyrazolo[3,4-c]quinolin-8-carboxamide | **507.2** |
| **167** | 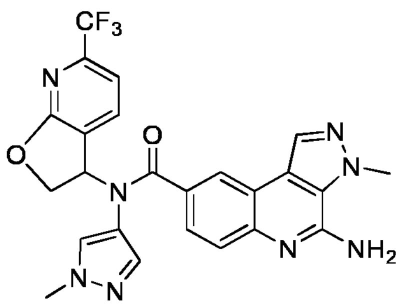 | 4-amino-3-methyl-N-(1-methyl-1H-pyrazol-4-yl)-N-(6-(trifluoromethyl)-2,3-dihydrofuro[2,3-b]pyridin-3-yl)-3H-pyrazolo[3,4-c]quinolin-8-carboxamide | **509.2** |
| **168** | 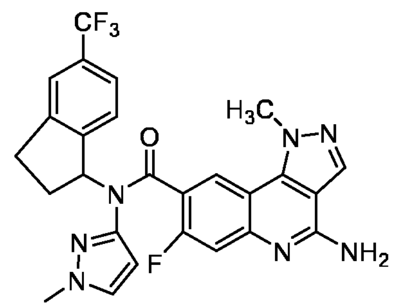 | 4-amino-7-fluoro-1-methyl-N-(1-methyl-1H-pyrazol-3-yl)-N-(5-(trifluoromethyl)-2,3-dihydro-1H-inden-1-yl)-1H-pyrazolo[4,3-c]quinolin-8-carboxamide | **524.2** |
| **169** | 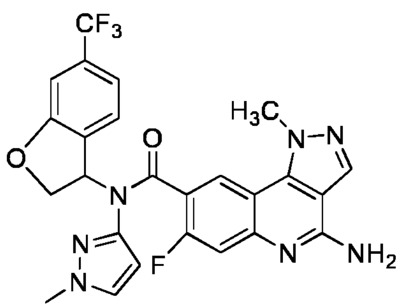 | 4-amino-7-fluoro-1-methyl-N-(1-methyl-1H-pyrazol-3-yl)-N-(6-(trifluoromethyl)-2,3-dihydrobenzofuran-3-yl)-1H-pyrazolo[4,3-c]quinolin-8-carboxamide | **526.2** |

(continued)

| Example No. | Structural Formula | Chemical Name | [M+H]+ |
|---|---|---|---|
| 170 | 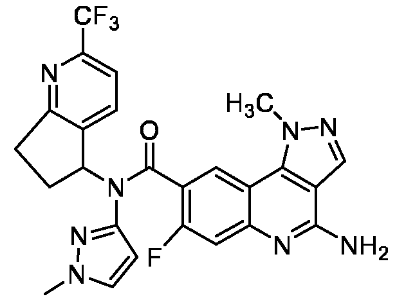 | 4-amino-7-fluoro-1-methyl-N-(1-methyl-1H-pyrazol-3-yl)-N-(2-(trifluoromethyl)-6,7-dihydro-5H-cyclopenta[b]pyridin-5-yl)-1H-pyrazolo[4,3-c]quinolin-8-carboxamide | 525.2 |
| 171 | 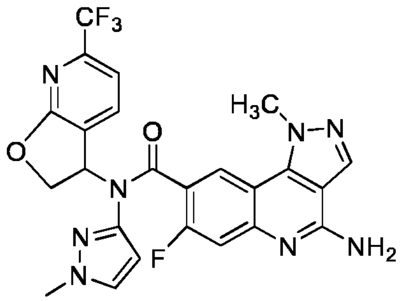 | 4-amino-7-fluoro-1-methyl-N-(1-methyl-1H-pyrazol-3-yl)-N-(6-(trifluoromethyl)-2,3-dihydrofuro[2,3-b]pyridin-3-yl)-1H-pyrazolo[4,3-c]quinolin-8-carboxamide | 527.2 |
| 172 | 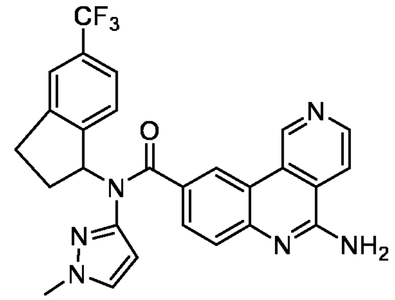 | 5-amino-N-(1-methyl-1H-pyrazol-3-yl)-N-(5-(trifluoromethyl)-2,3-dihydro-1H-inden-1-yl)benzo[c][2,6]naphthyridin-9-carboxamide | 503.2 |
| 173 | 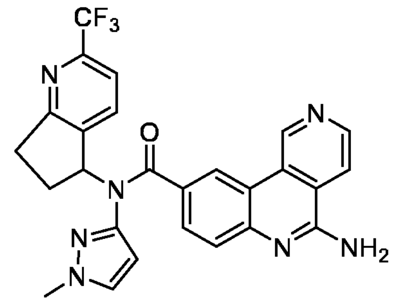 | 5-amino-N-(1-methyl-1H-pyrazol-3-yl)-N-(2-(trifluoromethyl)-6,7-dihydro-5H-cyclopenta[b]pyridin-5-yl)benzo[c][2,6]naphthyridin-9-carboxamide | 504.2 |
| 174 | 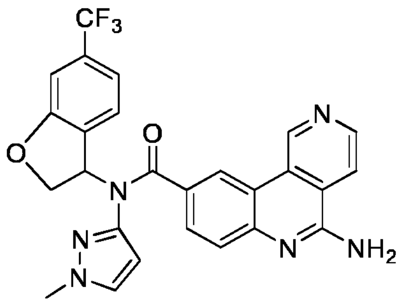 | 5-amino-N-(1-methyl-1H-pyrazol-3-yl)-N-(6-(trifluoromethyl)-2,3-dihydrobenzofuran-3-yl)benzo[c][2,6]naphthyridin-9-carboxamide | 505.2 |

(continued)

| Example No. | Structural Formula | Chemical Name | [M+H]+ |
|---|---|---|---|
| **175** | 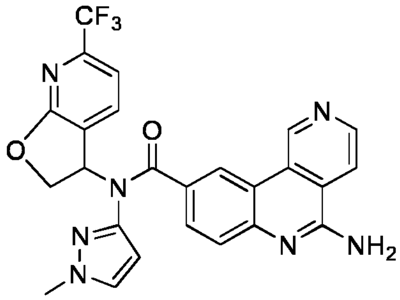 | 5-amino-N-(1-methyl-1H-pyrazol-3-yl)-N-(6-(trifluoromethyl)-2,3-dihydrofuro[2,3-b]pyridin-3-yl)benzo[c][2,6]naphthyridin-9-carboxamide | **506.2** |
| **176** | 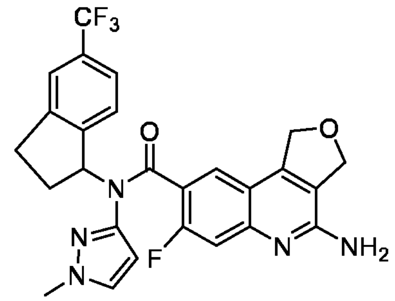 | 4-amino-7-fluoro-N-(1-methyl-1H-pyrazol-3-yl)-N-(5-(trifluoromethyl)-2,3-dihydro-1H-inden-1-yl)-1,3-dihydrofuro[3,4-c]quinolin-8-carboxamide | **512.2** |
| **177** | 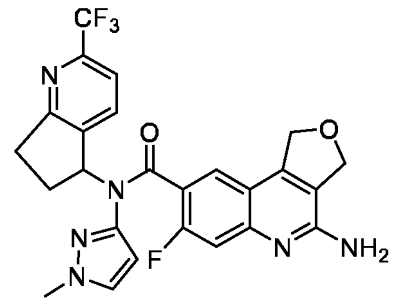 | 4-amino-7-fluoro-N-(1-methyl-1H-pyrazol-3-yl)-N-(2-(trifluoromethyl)-6,7-dihydro-5H-cyclopenta[b]pyridin-5-yl)-1,3-dihydrofuro[3,4-c]quinolin-8-carboxamide | **513.2** |
| **178** | 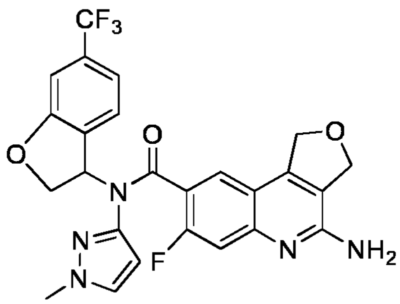 | 4-amino-7-fluoro-N-(1-methyl-1H-pyrazol-3-yl)-N-(6-(trifluoromethyl)-2,3-dihydrobenzofuran-3-yl)-1,3-dihydrofuro[3,4-c]quinolin-8-carboxamide | **514.1** |
| **179** | 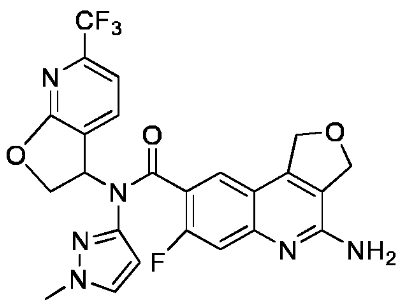 | 4-amino-7-fluoro-N-(1-methyl-1H-pyrazol-3-yl)-N-(6-(trifluoromethyl)-2,3-dihydrofuro[2,3-b]pyridin-3-yl)-1,3-dihydrofuro[3,4-c]quinolin-8-carboxamide | **515.1** |

(continued)

| Example No. | Structural Formula | Chemical Name | [M+H]+ |
|---|---|---|---|
| 180 | 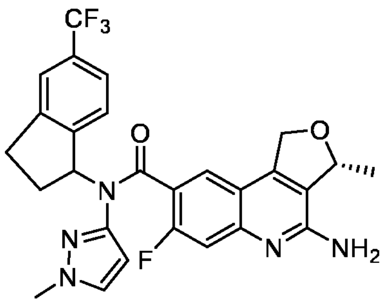 | (3R)-4-amino-7-fluoro-3-methyl-N-(1-methyl-1H-pyrazol-3-yl)-N-(5-(trifluoromethyl)-2,3-dihydro-1H-inden-1-yl)-1,3-dihydrofuro[3,4-c]quinolin-8-carboxamide | 526.2 |
| 181 | 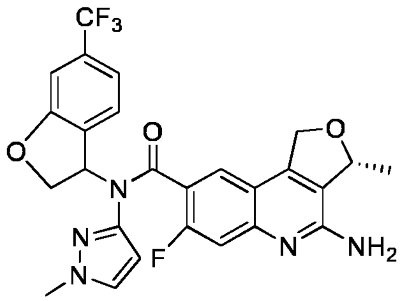 | (3R)-4-amino-7-fluoro-3-methyl-N-(1-methyl-1H-pyrazol-3-yl)-N-(6-(trifluoromethyl)-2,3-dihydrobenzofuran-3-yl)-1,3-dihydrofuro[3,4-c]quinolin-8-carboxamide | 528.2 |
| 182 | 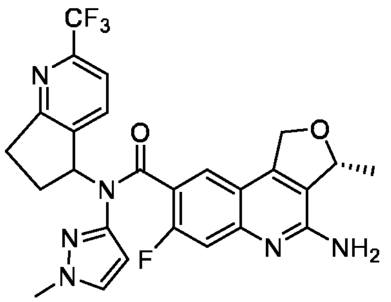 | (3R)-4-amino-7-fluoro-3-methyl-N-(1-methyl-1H-pyrazol-3-yl)-N-(2-(trifluoromethyl)-6,7-dihydro-5H-cyclopenta[b]pyridin-5-yl)-1,3-dihydrofuro[3,4-c]quinolin-8-carboxamide | 527.2 |
| 183 | 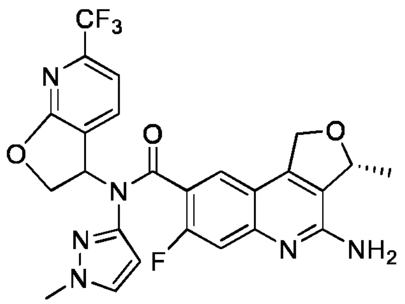 | (3R)-4-amino-7-fluoro-3-methyl-N-(1-methyl-1H-pyrazol-3-yl)-N-(6-(trifluoromethyl)-2,3-dihydrofuro[2,3-b]pyridin-3-yl)-1,3-dihydrofuro[3,4-c]quinolin-8-carboxamide | 529.2 |
| 184 | 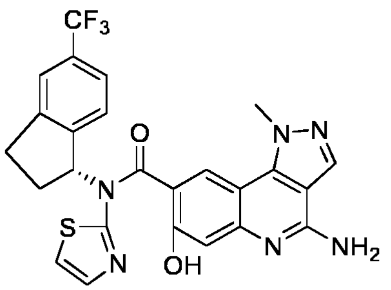 | (R)-4-amino-7-hydroxy-1-methyl-N-(thiazol-2-yl)-N-(5-(trifluoromethyl)-2,3-dihydro-1H-inden-1-yl)-1H-pyrazolo[4,3-c]quinolin-8-carboxamide | 525.2 |

(continued)

| Example No. | Structural Formula | Chemical Name | [M+H]+ |
|---|---|---|---|
| 185 | 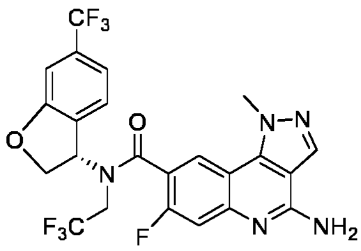 | (S)-4-amino-7-fluoro-1-methyl-N-(2,2,2-trifluoroethyl)-N-(6-(trifluoromethyl)-2,3-dihydrobenzofuran-3-yl)-1H-pyrazolo[4,3-c]quinolin-8-carboxamide | 528.2 |
| 186 | 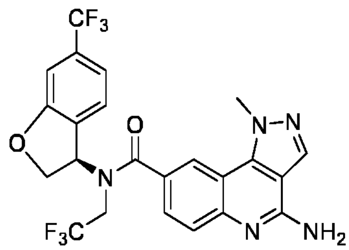 | (R)-4-amino-7-fluoro-1-methyl-N-(2,2,2-trifluoroethyl)-N-(6-(trifluoromethyl)-2,3-dihydrobenzofuran-3-yl)-1H-pyrazolo[4,3-c]quinolin-8-carboxamide | 528.2 |
| 187 | 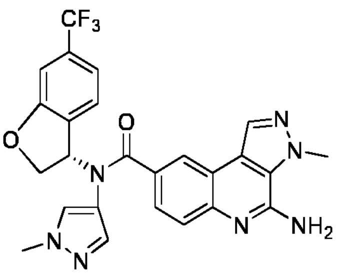 | (S)-4-amino-3-methyl-N-(1-methyl-1H-pyrazol-4-yl)-N-(6-(trifluoromethyl)-2,3-dihydrobenzofuran-3-yl)-3H-pyrazolo[3,4-c]quinolin-8-carboxamide | 508.2 |
| 188 | 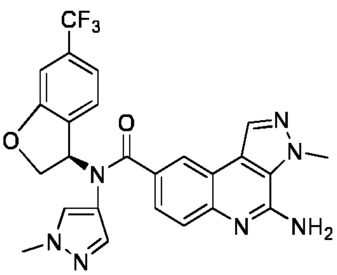 | (R)-4-amino-3-methyl-N-(1-methyl-1H-pyrazol-4-yl)-N-(6-(trifluoromethyl)-2,3-dihydrobenzofuran-3-yl)-3H-pyrazolo[3,4-c]quinolin-8-carboxamide | 508.2 |
| 191 | 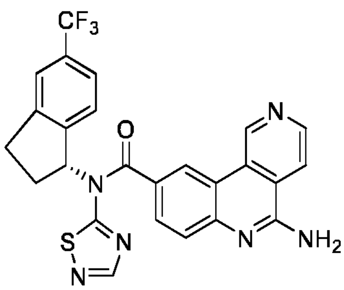 | (R)-5-amino-N-(1,2,4-thiadiazol-5-yl)-N-(5-(trifluoromethyl)-2,3-dihydro-1H-inden-1-yl)benzo[c][2,6]naphthyridin-9-carboxamide | 507.2 |

(continued)

| Example No. | Structural Formula | Chemical Name | [M+H]+ |
|---|---|---|---|
| 192 | 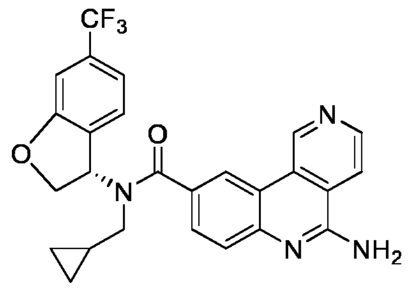 | (S)-5-amino-N-(cyclopropylmethyl)-N-(6-(trifluoromethyl)-2,3-dihydrobenzofuran-3-yl)benzo[c][2,6]naphthyridin-9-carboxamide | 479.2 |
| 193 | 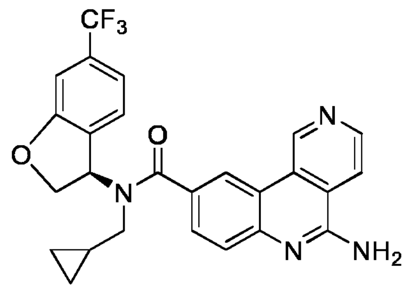 | (R)-5-amino-N-(cyclopropylmethyl)-N-(6-(trifluoromethyl)-2,3-dihydrobenzofuran-3-yl)benzo[c][2,6]naphthyridin-9-carboxamide | 479.2 |
| 195 | 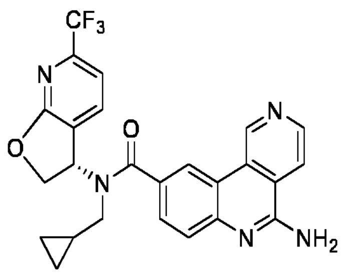 | (S)-5-amino-N-(cyclopropylmethyl)-N-(6-(trifluoromethyl)-2,3-dihydrofuro[2,3-b]pyridin-3-yl) benzo[c][2,6]naphthyridin-9-carboxamide | 480.2 |
| 196 | 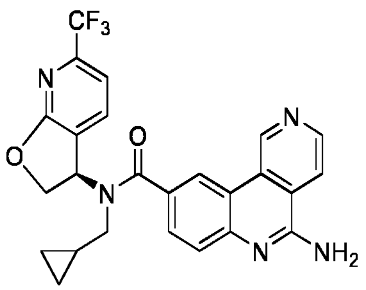 | (R)-5-amino-N-(cyclopropylmethyl)-N-(6-(trifluoromethyl)-2,3-dihydrofuro[2,3-b]pyridin-3-yl) benzo[c][2,6]naphthyridin-9-carboxamide | 480.2 |
| 197 | 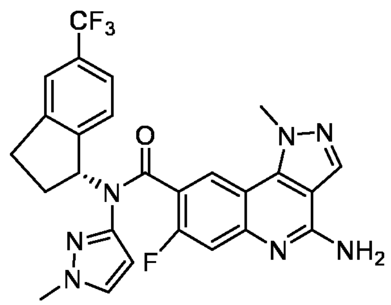 | (R)-4-amino-7-fluoro-1-methyl-N-(1-methyl-1H-pyrazol-3-yl)-N-(5-(trifluoromethyl)-2,3-dihydro-1H-inden-1-yl)-1H-pyrazolo[4,3-c]quinolin-8-carboxamide | 524.2 |

(continued)

| Example No. | Structural Formula | Chemical Name | [M+H]+ |
|---|---|---|---|
| 199 | 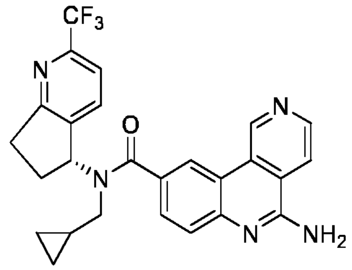 | (R)-5-amino-N-(cyclopropylmethyl)-N-(2-(trifluoromethyl)-6,7-dihydro-5H-cyclopenta[b]pyridin-5-yl)benzo[c][2,6]naphthyridin-9-carboxamide | 478.2 |
| 201 | 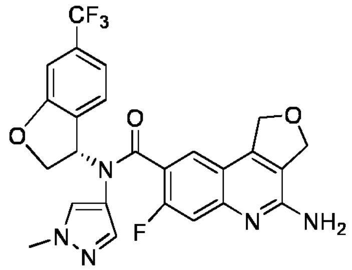 | (S)-4-amino-7-fluoro-N-(1-methyl-1H-pyrazol-4-yl)-N-(6-(trifluoromethyl)-2,3-dihydrobenzofuran-3-yl)-1,3-dihydrofuro[3,4-c]quinolin-8-carboxamide | 514.2 |
| 202 | 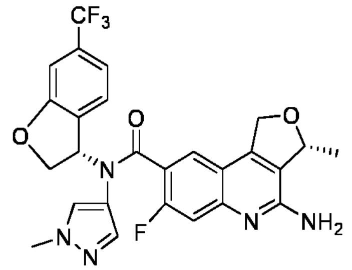 | (R)-4-amino-7-fluoro-3-methyl-N-(1-methyl-1H-pyrazol-4-yl)-N-((S)-6-(trifluoromethyl)-2,3-dihydrobenzofuran-3-yl)-1,3-dihydrofuro[3,4-c]quinolin-8-carboxamide | 528.2 |
| 203 | 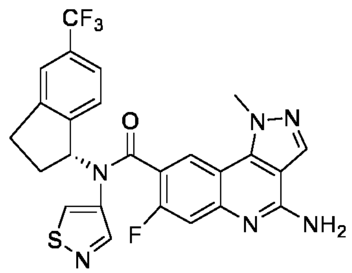 | (R)-4-amino-7-fluoro-N-(isothiazol-4-yl)-1-methyl-N-(5-(trifluoromethyl)-2,3-dihydro-1H-inden-1-yl)-1H-pyrazolo[4,3-c]quinolin-8-carboxamide | 527.2 |
| 204 | 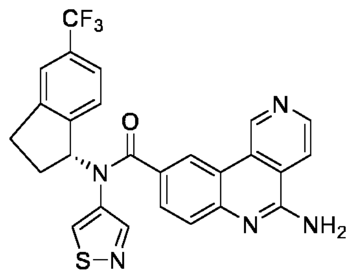 | (R)-5-amino-N-(isothiazol-4-yl)-N-(5-(trifluoromethyl)-2,3-dihydro-1H-inden-1-yl)benzo[c][2,6]naphthyridin-9-carboxamide | 506.2 |

(continued)

| Example No. | Structural Formula | Chemical Name | [M+H]+ |
|---|---|---|---|
| 205 | 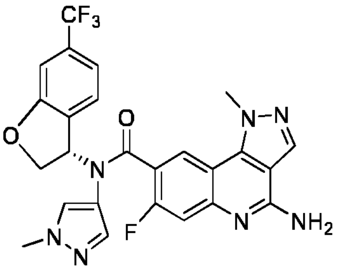 | (S)-4-amino-7-fluoro-1-methyl-N-(1-methyl-1H-pyrazol-4-yl)-N-(6-(trifluoromethyl)-2,3-dihydrobenzofuran-3-yl)-1H-pyrazolo[4,3-c]quinolin-8-carboxamide | 526.2 |
| 206 | 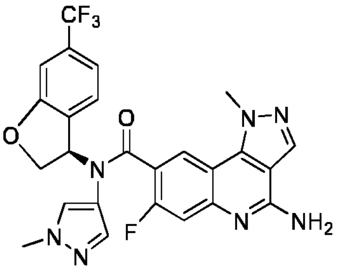 | (R)-4-amino-7-fluoro-1-methyl-N-(1-methyl-1H-pyrazol-4-yl)-N-(6-(trifluoromethyl)-2,3-dihydrobenzofuran-3-yl)-1H-pyrazolo[4,3-c]quinolin-8-carboxamide | 526.2 |
| 208 | 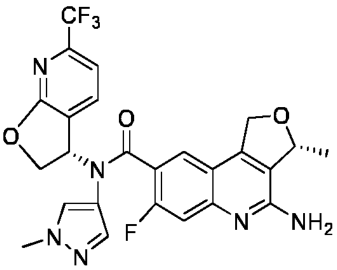 | (R)-4-amino-7-fluoro-3-methyl-N-(1-methyl-1H-pyrazol-4-yl)-N-((S)-6-(trifluoromethyl)-2,3-dihydrofuro[2,3-b]pyridin-3-yl)-1,3-dihydrofuro[3,4-c]quinolin-8-carboxamide | 529.2 |
| 209 | 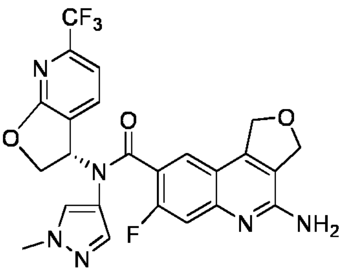 | (S)-4-amino-7-fluoro-N-(1-methyl-1H-pyrazol-4-yl)-N-(6-(trifluoromethyl)-2,3-dihydrofuro[2,3-b]pyridin-3-yl)-1,3-dihydrofuro[3,4-c]quinolin-8-carboxamide | 515.2 |
| 210 | 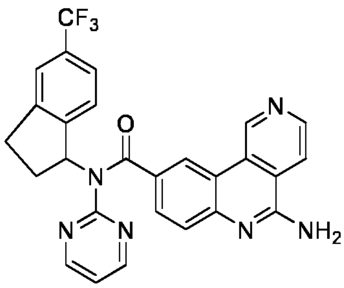 | 5-amino-N-(pyrimidin-2-yl)-N-(5-(trifluoromethyl)-2,3-dihydro-1H-inden-1-yl)benzo[c][2,6]naphthyridin-9-carboxamide | 501.3 |

(continued)

| Example No. | Structural Formula | Chemical Name | [M+H]+ |
|---|---|---|---|
| 211 | 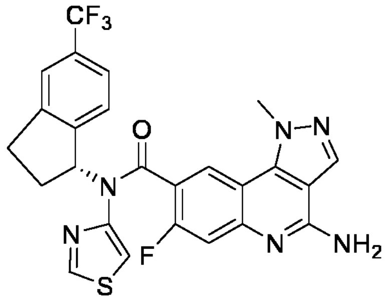 | (R)-4-amino-7-fluoro-1-methyl-N-(thiazol-4-yl)-N-(5-(trifluoromethyl)-2,3-dihydro-1H-inden-1-yl)-1H-pyrazolo[4,3-c]quinolin-8-carboxamide | 527.2 |
| 212 | 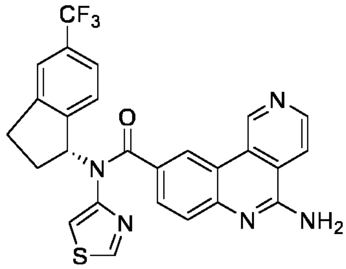 | (R)-5-amino-N-(thiazol-4-yl)-N-(5-(trifluoromethyl)-2,3-dihydro-1H-inden-1-yl)benzo[c][2,6]naphthyridin-9-carboxamide | 506.2 |
| 213 | 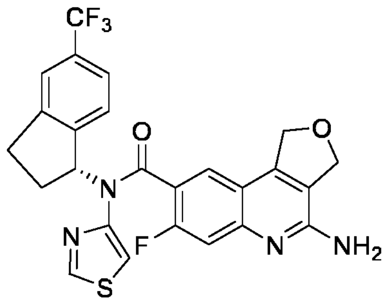 | (R)-4-amino-7-fluoro-N-(thiazol-4-yl)-N-(5-(trifluoromethyl)-2,3-dihydro-1H-inden-1-yl)-1,3-dihydrofuro[3,4-c]quinolin-8-carboxamide | 515.2 |
| 214 | 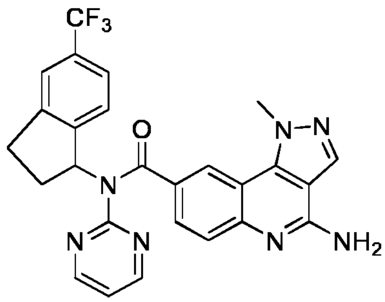 | 4-amino-1-methyl-N-(pyrimidin-2-yl)-N-(5-(trifluoromethyl)-2,3-dihydro-1H-inden-1-yl)-1H-pyrazolo[4,3-c]quinolin-8-carboxamide | 504.2 |
| 215 | 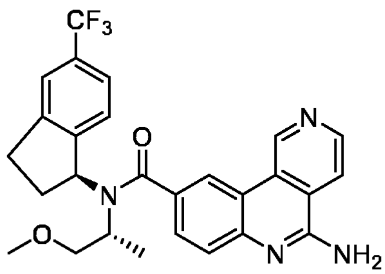 | 5-amino-N-((R)-1-methoxypropan-2-yl)-N-((S)-5-(trifluoromethyl)-2,3-dihydro-1H-inden-1-yl)benzo[c][2,6]naphthyridin-9-carboxamide | 495.2 |

(continued)

| Example No. | Structural Formula | Chemical Name | [M+H]+ |
|---|---|---|---|
| **216** | 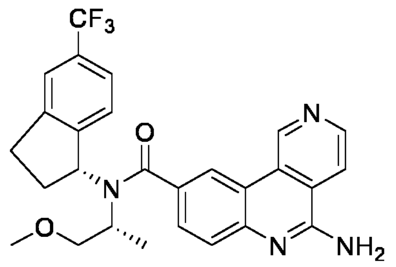 | 5-amino-N-((R)-1-methoxypropan-2-yl)-N-((R)-5-(trifluoromethyl)-2,3-dihydro-1H-inden-1-yl)benzo[c][2,6]naphthyridin-9-carboxamide | **495.2** |
| **217** | 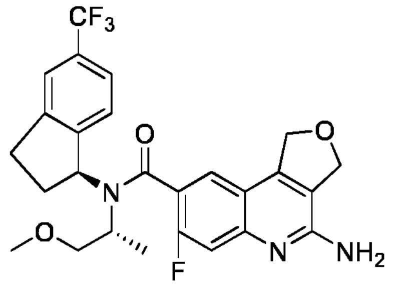 | 4-amino-7-fluoro-N-((R)-1-methoxypropan-2-yl)-N-((S)-5-(trifluoromethyl)-2,3-dihydro-1H-inden-1-yl)-1,3-dihydrofuro[3,4-c]quinolin-8-carboxamide | **504.2** |
| **218** | 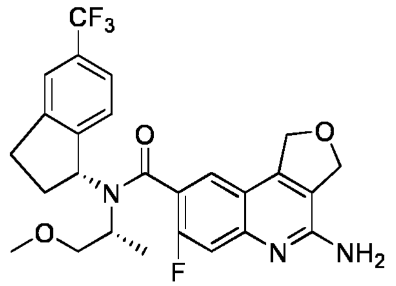 | 4-amino-7-fluoro-N-((R)-1-methoxypropan-2-yl)-N-((R)-5-(trifluoromethyl)-2,3-dihydro-1H-inden-1-yl)-1,3-dihydrofuro[3,4-c]quinolin-8-carboxamide | **504.2** |
| **219** | 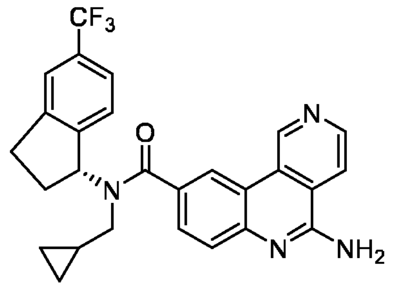 | (R)-5-amino-N-(cyclopropylmethyl)-N-(5-(trifluoromethyl)-2,3-dihydro-1H-inden-1-yl)benzo[c][2,6]naphthyridin-9-carboxamide | **477.2** |
| **220** | 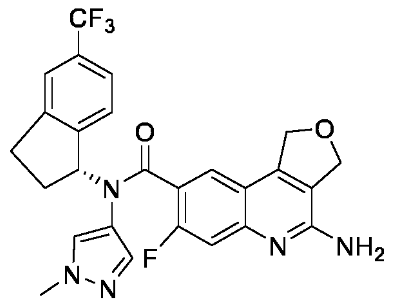 | (R)-4-amino-7-fluoro-N-(1-methyl-1H-pyrazol-4-yl)-N-(5-(trifluoromethyl)-2,3-dihydro-1H-inden-1-yl)-1,3-dihydrofuro[3,4-c]quinolin-8-carboxamide | **512.2** |

(continued)

| Example No. | Structural Formula | Chemical Name | [M+H]+ |
|---|---|---|---|
| 221 | 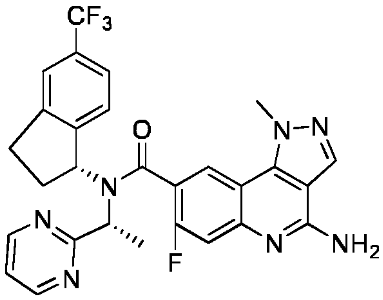 | 4-amino-7-fluoro-1-methyl-N-((R)-1-(pyrimidin-2-yl)ethyl)-N-((R)-5-(trifluoromethyl)-2,3-dihydro-1H-inden-1-yl)-1H-pyrazolo[4,3-c]quinolin-8-carboxamide | 550.2 |
| 222 | 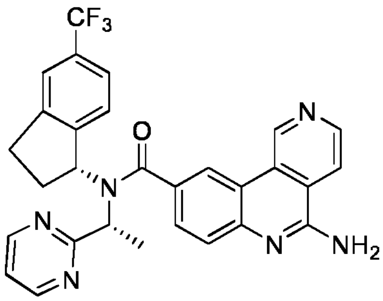 | 5-amino-N-((R)-1-(pyrimidin-2-yl) ethyl)-N-((R)-5-(trifluoromethyl)-2,3-dihydro-1H-inden-1-yl)benzo[c][2,6]naphthyridin-9-carboxamide | 529.2 |
| 223 | 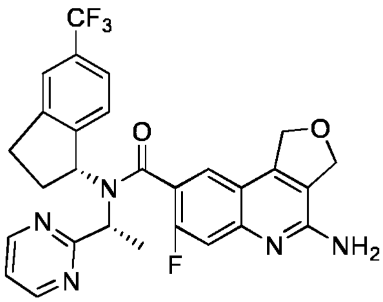 | 4-amino-7-fluoro-N-((R)-1-(pyrimidin-2-yl) ethyl)-N-((R)-5-(trifluoromethyl)-2,3-dihydro-1H-inden-1-yl)-1,3-dihydrofuro[3,4-c]quinolin-8-carboxamide | 538.2 |
| 224 | 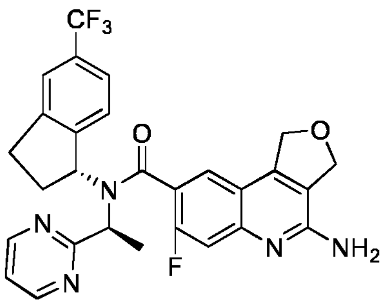 | 4-amino-7-fluoro-N-((S)-1-(pyrimidin-2-yl) ethyl)-N-((R)-5-(trifluoromethyl)-2,3-dihydro-1H-inden-1-yl)-1,3-dihydrofuro[3,4-c]quinolin-8-carboxamide | 538.2 |
| 225 | 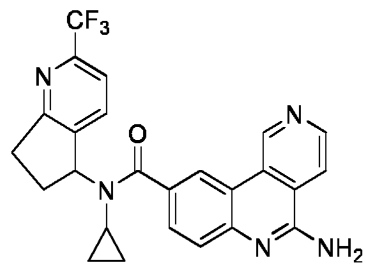 | 5-amino-N-cyclopropyl-N-(2-(trifluoromethyl)-6,7-dihydro-5H-cyclopenta[b]pyridin-5-yl)benzo[c][2,6]naphthyridin-9-carboxamide | 464.2 |

(continued)

| Example No. | Structural Formula | Chemical Name | $[M+H]^+$ |
|---|---|---|---|
| 226 | 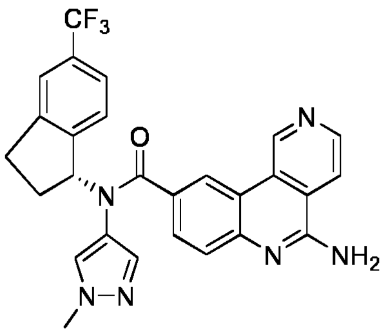 | (R)-5-amino-N-(1-methyl-1H-pyrazol-4-yl)-N-(5-(trifluoromethyl)-2,3-dihydro-1H-inden-1-yl)benzo[c][2,6]naphthyridin-9-carboxamide | 503.2 |
| 227 | 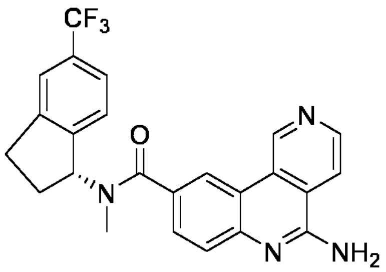 | (R)-5-amino-N-methyl-N-(5-(trifluoromethyl)-2,3-dihydro-1H-inden-1-yl)benzo[c][2,6]naphthyridin-9-carboxamide | 437.2 |
| 229 | 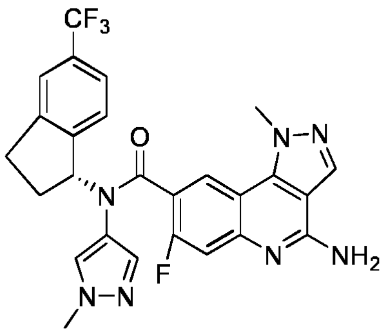 | (R)-4-amino-7-fluoro-1-methyl-N-(1-methyl-1H-pyrazol-4-yl)-N-(5-(trifluoromethyl)-2,3-dihydro-1H-inden-1-yl)-1H-pyrazolo[4,3-c]quinolin-8-carboxamide | 524.2 |
| 230 | 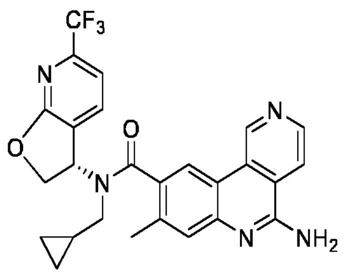 | (S)-5-amino-N-(cyclopropylmethyl)-8-methyl-N-(6-(trifluoromethyl)-2,3-dihydrofuro[2,3-b]pyridin-3-yl)benzo[c][2,6]naphthyridin-9-carboxamide | 494.2 |
| 231 | 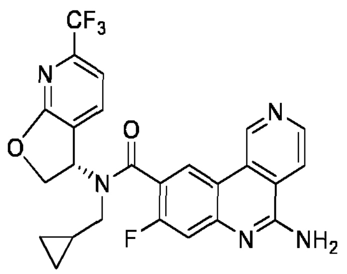 | (S)-5-amino-N-(cyclopropylmethyl)-8-fluoro-N-(6-(trifluoromethyl)-2,3-dihydrofuro[2,3-b]pyridin-3-yl)benzo[c][2,6]naphthyridin-9-carboxamide | 498.2 |

(continued)

| Example No. | Structural Formula | Chemical Name | [M+H]+ |
|---|---|---|---|
| **232** | 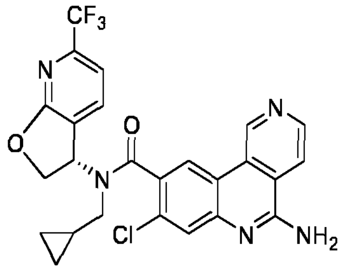 | (S)-5-amino-8-chloro-N-(cyclopropylmethyl)-N-(6-(trifluoromethyl)-2,3-dihydrofuro[2,3-b]pyridin-3-yl)benzo[c][2,6]naphthyridin-9-carboxamide | **514.2** |
| **233** | 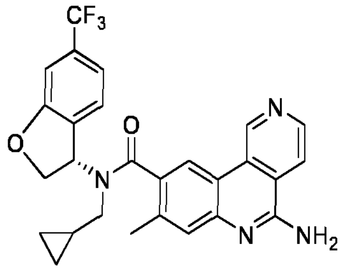 | (S)-5-amino-N-(cyclopropylmethyl)-8-methyl-N-(6-(trifluoromethyl)-2,3-dihydrobenzofuran-3-yl)benzo[c][2,6]naphthyridin-9-carboxamide | **493.2** |
| **234** | 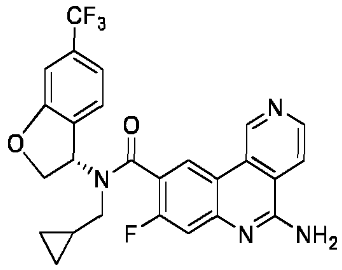 | (S)-5-amino-N-(cyclopropylmethyl)-8-fluoro-N-(6-(trifluoromethyl)-2,3-dihydrobenzofuran-3-yl)benzo[c][2,6]naphthyridin-9-carboxamide | **497.2** |
| **235** | 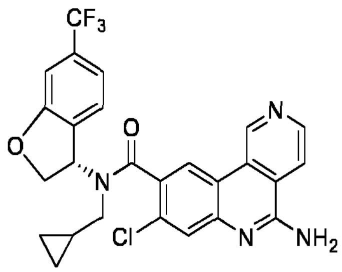 | (S)-5-amino-8-chloro-N-(cyclopropylmethyl)-N-(6-(trifluoromethyl)-2,3-dihydrobenzofuran-3-yl)benzo[c][2,6]naphthyridin-9-carboxamide | **513.2** |
| **236** | 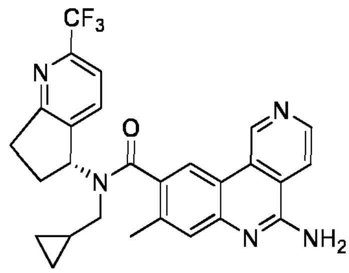 | (R)-5-amino-N-(cyclopropylmethyl)-8-methyl-N-(2-(trifluoromethyl)-6,7-dihydro-5H-cyclopenta[b]pyridin-5-yl)benzo[c][2,6]naphthyridin-9-carboxamide | **492.2** |

(continued)

| Example No. | Structural Formula | Chemical Name | [M+H]+ |
|---|---|---|---|
| 237 | 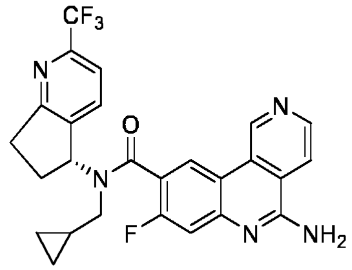 | (R)-5-amino-N-(cyclopropylmethyl)-8-fluoro-N-(2-(trifluoromethyl)-6,7-dihydro-5H-cyclopenta[b]pyridin-5-yl)benzo[c][2,6]naphthyridin-9-carboxamide | 496.2 |
| 238 | 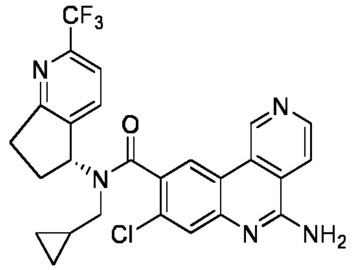 | (R)-5-amino-8-chloro-N-(cyclopropylmethyl)-N-(2-(trifluoromethyl)-6,7-dihydro-5H-cyclopenta[b]pyridin-5-yl)benzo[c][2,6]naphthyridin-9-carboxamide | 512.2 |

$^1$HNMR data for the compounds prepared in the above examples are as follows:

| Example No. | $^1$H NMR |
|---|---|
| 7 | $^1$H NMR (400 MHz, DMSO-$d_6$) δ 8.78 (d, *J* = 4.9 Hz, 1H), 8.66 (d, *J* = 5.0 Hz, 1H), 8.38(d, *J* = 8.0 Hz, 0.48H), 8.23 (d, *J* = 21.1 Hz, 1H), 8.11(d, *J* = 8.0 Hz, 0.52H), 7.83-7.44 (m, 3H), 7.43-7.17 (m, 4H), 6.17 (t, *J* = 8.5 Hz, 0.48H), 5.52 (t, *J* = 8.5 Hz, 0.52H), 4.84-4.19 (m, 5H), 3.01-2.81 (m, 1H), 2.80-2.69 (m, 1H), 2.46-2.35 (m, 1H), 2.25-2.05 (m, 1H). |
| 8 | $^1$H NMR (400 MHz, DMSO-$d_6$) δ 8.23 (s, 1H), 8.16 (d, *J* = 7.3 Hz, 1H), 7.76-7.61 (m, 2H), 7.56 (s, 1H), 7.42 (s, 1H), 7.22 (s, 2H), 7.09 (d, *J* = 11.7 Hz, 1H), 6.99 (s, 1H), 6.51-6.39 (m, 1H), 4.38 (s, 3H), 3.48 (s, 3H), 3.00-2.68 (m, 3H), 2.14-1.98 (m, 1H). |
| 21 | $^1$H NMR (400 MHz, DMSO-$d_6$) δ 8.22 (s, 1H), 8.15 (d, *J* = 7.3 Hz, 1H), 7.72 (d,*J* = 7.8 Hz, 1H), 7.40 (s, 1H), 7.33 (d, *J* = 7.8 Hz, 1H), 7.24 (s, 2H), 7.10 (s, 1H), 7.06 (s, 1H), 6.90 (s, 1H), 6.63 (s, 1H), 4.90 (t, *J* = 9.7 Hz, 1H), 4.68 (dd, *J* = 10.4, 4.0 Hz, 1H), 4.36 (s, 3H), 3.47 (s, 3H). |
| 95 | $^1$H NMR (400 MHz, MeOH-$d_4$) δ 9.02-8.87 (m, 1H), 8.69 (s, 1H), 8.51-8.44 (m, 1H), 7.69-7.56 (m, 1H), 7.52 (s, 1H), 7.50-7.37 (m, 2H), 7.37-7.19 (m, 1H), 4.68 (d, *J* = 16.4 Hz, 1H), 4.52 (d, *J* = 9.0 Hz, 4H), 3.15-2.91 (m, 2H), 2.90-2.67 (m, 1H), 2.49 (s, 1H), 2.39-2.26 (m, 1H). |
| 108 | $^1$H NMR (400 MHz, MeOH-$d_4$) δ 10.14 (s, 1H), 8.99 (d, *J* = 5.6 Hz, 1H), 8.87 (s, 1H), 8.38 (d, *J* = 5.6 Hz, 1H), 7.89 (s, 1H), 7.82 (d, *J* = 8.4 Hz, 1H), 7.62 (s, 1H), 7.55 (d, *J* = 8.1 Hz, 2H), 5.9-5.35 (br s, 1H), 3.39 (dd, *J* = 14.5, 6.5 Hz, 1H), 3.20-3.00 (m, 2H), 2.92-2.32(m, 3H), 1.24-0.82 (m, 1H), 0.45 (br s, 2H), 0.3-0.04 (m, 1H), -0.10 (br s, 2H). |
| 109 | $^1$H NMR (400 MHz, DMSO-$d_6$) δ 10.13 (s, 1H), 8.86 (d, *J* = 5.5 Hz, 1H), 8.75 (d, *J* = 1.8 Hz, 1H), 8.24 (d, *J* = 5.6 Hz, 1H), 7.74 (d, *J* = 7.8 Hz, 1H), 7.68-7.57 (m, 2H), 7.53 (s, 2H), 7.30 (d, *J* = 7.7 Hz, 1H), 7.20 (s, 1H), 5.91 (s, 1H), 4.91-4.83 (m, 2H), 3.27-3.07 (m, 2H), 0.90-0.76 (m, 1H), 0.42-0.22 (m, 2H), 0.05-0.26 (m, 2H). |
| 110 | $^1$H NMR (400 MHz, MeOH-$d_4$) δ 10.01 (s, 1H), 8.81 (d, *J* = 5.6 Hz, 1H), 8.72 (s, 1H), 8.18 (d, *J* = 5.6 Hz, 1H), 8.10 (d, *J* = 8.0 Hz, 1H), 7.73-7.65 (m, 3H), 5.66-5.56 (m, 1H), 3.49-3.35 (m, 2H), 3.13-2.60 (m, 4H), 1.15-0.93 (m, 1H), 0.54-0.48 (m, 2H), 0.19-0.01 (m, 2H). |

(continued)

| Example No. | $^1$H NMR |
|---|---|
| **111** | $^1$H NMR (400 MHz, *CDCl$_3$*) δ 9.89 (s, 1H), 8.86 (d, *J* = 5.5 Hz, 1H), 8.56 (d, *J* = 1.8 Hz, 1H), 7.89 (d, *J* = 7.5 Hz, 1H), 7.75 (d, *J* = 5.5 Hz, 1H), 7.73 (d, *J* = 8.4 Hz, 1H), 7.64 (dd, *J* = 8.4, 1.8 Hz, 1H), 7.35 (d, *J* = 7.5 Hz, 1H), 6.10 (br s, 1H), 6.00 (br s, 2H), 5.06-4.94 (m, 1H), 4.92 (dd, *J* = 10.3, 5.0 Hz, 1H), 3.35 (dd, *J* = 15.0, 6.9 Hz, 1H), 3.20 (dd, *J* = 15.0, 6.5 Hz, 1H), 0.78 (br s, 1H), 0.52-0.34(m, 2H), -0.08 (br s, 1H), -0.11-0.20 (m, 1H). |
| **132** | $^1$H NMR (400 MHz, DMSO-*d$_6$*) δ 9.96 (s, 1H), 8.83 (d, *J* = 5.5 Hz, 1H), 8.63 (s, 1H), 8.20 (d, *J* = 5.5 Hz, 1H), 7.83 (d, *J* = 7.9 Hz, 1H), 7.65-7.38 (m, 7H), 7.09 (s, 1H), 6.39 (s, 1H), 3.49 (s, 3H), 2.98-2.81 (m, 2H), 2.79-2.68 (m, 1H), 2.18-2.03 (m, 1H). |
| **133** | $^1$H NMR (400 MHz, DMSO-*d$_6$*) δ 9.85 (s, 1H), 8.76 (d, *J* = 5.5 Hz, 1H), 8.55 (d, *J* = 1.9 Hz, 1H), 8.13 (d, *J*= 5.6 Hz, 1H), 7.75 (d, *J* = 7.8 Hz, 1H), 7.52 (dd, *J* = 8.5, 1.8 Hz, 1H), 7.46-7.40 (m, 3H), 7.34 (d, *J*= 8.5 Hz, 1H), 7.25 (dd, *J* = 8.0, 1.6 Hz, 1H), 7.01 (d, *J* = 1.5 Hz, 1H), 6.97 (s, 1H), 6.49 (d, *J* = 8.9 Hz, 1H), 4.85 (dd, *J* = 10.4, 9.0 Hz, 1H), 4.67 (dd, *J* = 10.5, 4.1 Hz, 1H), 3.40 (s, 3H). |
| **152** | $^1$H NMR (400 MHz, DMSO-*d$_6$*) δ 8.80 (d, *J* = 2.2 Hz, 1H), 8.22 (s, 1H), 8.11 (d, *J* = 7.3 Hz, 1H), 7.69 *(d, J* = 8.0 Hz, 1H), 7.59 (d, *J*= 8.0 Hz, 1H), 7.56 (s, 1H), 7.34-7.20 (m, 3H), 7.12 (d, *J* = 11.9 Hz, 1H), 6.35 (s, 1H), 4.33 (s, 3H), 2.97-2.86 (m, 1H), 2.84-2.81 (m, 1H), 2.32-2.27 (m, 2H). |
| **160** | $^1$H NMR (400 MHz, DMSO-*d$_6$*) δ 7.71-7.60 (m, 2H), 7.58-7.53 (m, 2H), 7.40 (s, 1H), 7.07 (d, *J* = 11.8 Hz, 1H), 6.92 (s, 1H), 6.76 (s, 2H), 6.40 (t, *J* = 7.9 Hz, 1H), 5.29 (s, 2H), 4.96 (s, 2H), 3.49 (s, 3H), 2.95-2.80 (m, 2H), 2.76-2.65 (m, 1H), 2.07-1.98 (m, 1H). |
| **165** | $^1$H NMR (400 MHz, DMSO-*d$_6$*) δ 8.44 (s, 1H), 8.13 (s, 1H), 7.77 (d, *J* = 7.8 Hz, 1H), 7.51-7.25 (m, 4H), 7.08-6.94 (m, 4H), 6.58-6.47 (m, 1H), 4.89 (t, *J* = 9.7 Hz, 1H), 4.69 (dd, *J* = 10.3, 4.0 Hz, 1H), 4.33 (s, 3H), 3.49 (s, 3H). |
| **168** | $^1$H NMR (400 MHz, DMSO-*d$_6$*) δ 8.15 (s, 1H), 8.03 (d, *J* = 7.3 Hz, 1H), 7.63 (d,*J* = 7.9 Hz, 1H), 7.53 (d, *J* = 8.0 Hz, 1H), 7.48 (s, 1H), 7.22 (s, 1H), 7.18 (s, 2H), 7.08 (d, *J*= 11.9 Hz, 1H), 6.27 (br s, 1H), 5.62 (s, 1H), 4.25 (s, 3H), 3.45 (s, 3H), 2.87-2.71 (m, 2H), 2.51-2.45 (m, 1H), 2.28-2.13 (m, 1H). |
| **184** | $^1$H NMR (400 MHz, DMSO-*d$_6$*) δ 8.78 (s, 1H), 8.18 (s, 1H), 7.68-7.55 (m, 1H), 7.52 (s, 1H), 7.50 (s, 2H), 7.29 (s, 2H), 7.08 (d, *J* = 3.7 Hz, 1H), 6.96 (s, 1H), 6.59 (d, *J* = 3.6 Hz, 1H), 6.15 (s, 1H), 4.40-4.30 (m, 3H), 2.84-2.75 (m, 2H), 2.05-1.88 (m, 2H). |
| **185** | $^1$H NMR (400 MHz, DMSO-*d$_6$*) δ 8.26 (s, 1H), 8.18 (br s, 1H), 7.58 (d, *J*= 7.8 Hz, 1H), 7.34 (s, 2H), 7.32-7.24 (m, 2H), 7.10 (br s, 1H), 5.82-5.69 (m, 1H), 4.81 (br s, 1H), 4.77-4.69 (m, 1H), 4.60-4.10 (m, 5H). |
| **186** | $^1$H NMR (400 MHz, DMSO-*d$_6$*) δ 8.26 (s, 1H), 8.17 (br s, 1H), 7.58 (d, *J*= 7.9 Hz, 1H), 7.34 (s, 2H), 7.32-7.24 (m, 2H), 7.10 (br s, 1H), 5.82-5.68 (m, 1H), 4.81 (br s, 1H), 4.78-4.65 (m, 1H), 4.60-4.10 (m, 5H). |
| **187** | $^1$H NMR (400 MHz, DMSO-*d$_6$*) δ 8.44 (s, 1H), 8.13 (s, 1H), 7.77 (d, *J* = 7.8 Hz, 1H), 7.45 (s, 1H), 7.42-7.35 (m, 2H), 7.31 (d, *J* = 7.8 Hz, 1H), 7.08 (s, 1H), 7.03-6.85 (m, 3H), 6.58-6.47 (m, 1H), 4.89 (t, *J* = 9.7 Hz, 1H), 4.69 (dd, *J* = 10.3, 4.0 Hz, 1H), 4.33 (s, 3H), 3.49 (s, 3H). |
| **188** | $^1$H NMR (400 MHz, DMSO-*d$_6$*) δ 8.44 (s, 1H), 8.13 (s, 1H), 7.77 (d, *J* = 7.8 Hz, 1H), 7.45 (s, 1H), 7.42-7.35 (m, 2H), 7.31 (d, *J* = 7.8 Hz, 1H), 7.08 (s, 1H), 7.03-6.85 (m, 3H), 6.58-6.47 (m, 1H), 4.89 (t, *J* = 9.7 Hz, 1H), 4.69 (dd, *J* = 10.3, 4.0 Hz, 1H), 4.33 (s, 3H), 3.49 (s, 3H). |
| **191** | $^1$H NMR (400 MHz, DMSO-*d$_6$*) δ 10.04 (s, 1H), 9.09 (s, 1H), 8.87 (d, *J* = 5.5 Hz, 1H), 8.39 (s, 1H), 8.26 (d, *J* = 5.5 Hz, 1H), 7.95 (d, *J* = 8.4 Hz, 1H), 7.70 (s, 2H), 7.68 (s, 1H), 7.57 (s, 1H), 7.51 (d, *J* = 8.0 Hz, 1H), 7.45 (d, *J* = 8.1 Hz, 1H), 6.14 (s, 1H), 3.05-2.75 (m, 4H). |
| **192** | $^1$H NMR (400 MHz, DMSO-*d$_6$*) δ 10.13 (s, 1H), 8.75 (s, 1H), 8.75 (d, *J* = 1.8 Hz, 1H), 8.24 (d, *J* = 5.5 Hz, 1H), 7.75 (d, *J* = 7.8 Hz, 1H), 7.69-7.51 (m, 4H), 7.30 (d, *J* = 7.8 Hz, 1H), 7.20 (s, 1H), 6.01-5.78 (m, 1H), 4.95-4.72 (m, 2H), 3.28-3.08 (m, 2H), 0.94-0.78 (m, 1H), 0.43-0.23 (m, 2H), -0.01 - -0.26 (m, 2H). |

(continued)

| Example No. | $^1H$ NMR |
|---|---|
| **193** | $^1H$ NMR (400 MHz, DMSO-$d_6$) δ 10.13 (s, 1H), 8.75 (s, 1H), 8.75 (d, *J* = 1.8 Hz, 1H), 8.24 (d, *J* = 5.5 Hz, 1H), 7.75 (d, *J* = 7.8 Hz, 1H), 7.69-7.51 (m, 4H), 7.30 (d, *J* = 7.8 Hz, 1H), 7.20 (s, 1H), 6.01-5.78 (m, 1H), 4.95-4.72 (m, 2H), 3.28-3.08 (m, 2H), 0.94-0.78 (m, 1H), 0.43-0.23 (m, 2H), -0.01 - -0.26 (m, 2H). |
| **195** | $^1H$ NMR (400 MHz, $CDCl_3$) δ 9.89 (s, 1H), 8.86 (d, *J* = 5.5 Hz, 1H), 8.56 (d, *J* = 1.8 Hz, 1H), 7.89 (d, *J* = 7.5 Hz, 1H), 7.75 (d, *J* = 5.5 Hz, 1H), 7.73 (d, *J* = 8.4 Hz, 1H), 7.64 (dd, *J* = 8.4, 1.8 Hz, 1H), 7.35 (d, *J* = 7.5 Hz, 1H), 6.10 (s, 1H), 5.05-4.94 (m, 1H), 4.94-4.86 (m, 1H), 3.35 (dd, *J* = 15.0, 6.9 Hz, 1H), 3.20 (dd, *J* = 15.0, 6.5 Hz, 1H), 0.78 (s, 1H), 0.52-0.32 (m, 2H), -0.08 (s, 1H), -0.10 - -0.22 (m, 1H). |
| **196** | $^1H$ NMR (400 MHz, DMSO-$d_6$) δ 10.11 (s, 1H), 8.85 (d, *J* = 5.5 Hz, 1H), 8.73 (s, 1H), 8.24 (d, *J* = 5.6 Hz, 1H), 8.15 (d, *J* = 7.5 Hz, 1H), 7.65-7.56 (m, 2H), 7.53 (s, 2H), 7.45 (d, *J* = 7.5 Hz, 1H), 5.82-5.70 (m, 1H), 5.04-4.89 (m, 1H), 4.89-4.78 (m, 1H), 3.47-3.38 (m, 2H), 1.03-0.92 (m, 1H), 0.50-0.32 (m, 2H), 0.06-0.01 (m, 2H). |
| **197** | $^1H$ NMR (400 MHz, DMSO-$d_6$) δ 8.15 (s, 1H), 8.03 (d, *J* = 7.3 Hz, 1H), 7.63 (d, *J* = 7.9 Hz, 1H), 7.53 (d, *J* = 8.0 Hz, 1H), 7.48 (s, 1H), 7.22 (s, 1H), 7.18 (s, 2H), 7.08 (d, *J* = 11.9 Hz, 1H), 6.27 (s, 1H), 5.62 (s, 1H), 4.25 (s, 3H), 3.45 (s, 3H), 2.87-2.71 (m, 2H), 2.51-2.45 (m, 1H), 2.29-2.12 (m, 1H). |
| **199** | $^1H$ NMR (400 MHz, MeOH-$d_4$) δ 10.01 (s, 1H), 8.81 (d, *J* = 5.6 Hz, 1H), 8.72 (s, 1H), 8.18 (d, *J* = 5.7 Hz, 1H), 8.10 (d, *J* = 7.9 Hz, 1H), 7.82-7.54 (m, 3H), 5.68-5.50 (m, 1H), 3.52-3.40 (m, 2H), 2.97-2.51 (m, 4H), 1.16-1.06 (m, 1H), 0.64-0.37 (m, 2H), 0.25-0.01 (m, 2H). |
| **201** | $^1H$ NMR (400 MHz, MeOH-$d_4$) δ 7.83 (d, *J* = 6.6 Hz, 1H), 7.69 (d, *J* = 7.9 Hz, 1H), 7.38-7.21 (m, 3H), 6.96 (s, 1H), 6.92 (s, 1H), 6.68-6.62 (m, 1H), 5.47-5.41 (m, 2H), 5.15-5.09 (m, 2H), 4.87-4.81 (m, 1H), 4.74-4.68 (m, 1H), 3.53 (s, 3H). |
| **202** | $^1H$ NMR (400 MHz, MeOH-$d_4$) δ 7.84 (d, *J* = 6.6 Hz, 1H), 7.69 (d, *J*= 7.8 Hz, 1H), 7.42-7.25 (m, 3H), 6.97 (s, 1H), 6.92 (s, 1H), 6.66 (dd, *J* = 8.5, 3.2 Hz, 1H), 5.58-5.36 (m, 3H), 4.87-4.82 (m, 1H), 4.71 (dd, *J* = 10.9, 3.3 Hz, 1H), 3.54 (s, 3H), 1.49 (d, *J* = 6.2 Hz, 3H). |
| **203** | $^1H$ NMR (400 MHz, DMSO-$d_6$) δ 8.61 (s, 1H), 8.26 (br s, 1H),8.23 (s, 1H), 8.15 (s, 1H), 7.74 (d, *J* = 7.9 Hz, 1H), 7.67 (d, *J* = 8.0 Hz, 1H), 7.59 (s, 1H), 7.28 (s, 1H), 7.04 (d, *J* = 11.7 Hz, 1H), 6.49 (s, 1H), 5.37-5.30 (m, 1H), 4.40 (s, 3H), 2.97-2.84 (m, 2H), 2.15-2.05 (m, 2H). |
| **204** | $^1H$ NMR (400 MHz, MeOH-$d_4$) δ 9.83 (s, 1H), 8.78 (d, *J* = 5.6 Hz, 1H), 8.62 (s, 1H), 8.47 (s, 1H), 8.13 (d, *J* = 5.7 Hz, 1H), 8.09 (s, 1H), 7.84 (d, *J* = 7.9 Hz, 1H), 7.66-7.58 (m, 2H), 7.51-7.45 (m, 2H), 6.56 (s, 1H), 2.98-2.87 (m, 1H), 2.75-2.60 (m, 2H), 2.35-2.24 (m, 1H). |
| **205** | $^1H$ NMR (400 MHz, DMSO-$d_6$) δ 8.22 (s, 1H), 8.15 (d, *J* = 7.3 Hz, 1H), 7.72 (d, *J* = 7.8 Hz, 1H), 7.40 (s, 1H), 7.33 (d, *J* = 7.8 Hz, 1H), 7.24 (s, 2H),7.14-7.03 (m, 2H), 6.90 (s, 1H), 6.63 (s, 1H), 4.90 (t, *J* = 9.7 Hz, 1H), 4.68 (dd, *J* = 10.4, 4.0 Hz, 1H), 4.36 (s, 3H), 3.47 (s, 3H). |
| **206** | $^1H$ NMR (400 MHz, DMSO-$d_6$) δ 8.22 (s, 1H), 8.15 (d, *J* = 7.3 Hz, 1H), 7.72 (d, *J* = 7.8 Hz, 1H), 7.40 (s, 1H), 7.33 (d, *J* = 7.8 Hz, 1H), 7.24 (s, 2H), 7.14-7.03 (m, 2H), 6.90 (s, 1H), 6.63 (s, 1H), 4.90 (t, *J* = 9.7 Hz, 1H), 4.68 (dd, *J* = 10.4, 4.0 Hz, 1H), 4.36 (s, 3H), 3.47 (s, 3H). |
| **208** | $^1H$ NMR (400 MHz, MeOH-$d_4$) δ 8.12 (d, *J* = 7.5 Hz, 1H), 7.58 (d, *J* = 7.3 Hz, 1H), 7.45-7.40 (m, 2H), 7.07 (d, *J* = 11.7 Hz, 1H), 7.03 (s, 1H), 6.67-6.59 (m, 1H), 5.49-5.41 (m, 1H), 5.41-5.33 (m, 1H), 5.31-5.22 (m, 1H), 5.02-4.92 (m, 1H), 4.80 (dd, *J* = 10.8, 3.7 Hz, 1H), 3.57 (s, 3H), 1.46 (d, *J*= 6.3 Hz, 3H). |
| **209** | $^1H$ NMR (400 MHz, MeOH-$d_4$) δ 8.12 (d, *J* = 7.4 Hz, 1H), 7.84 (d, *J* = 6.7 Hz, 1H), 7.44 (d, *J* = 7.7 Hz, 2H), 7.33 (d, *J* = 10.0 Hz, 1H), 7.04 (s, 1H), 6.62 (dd, *J* = 8.6, 3.5 Hz, 1H), 5.44 (t, *J* = 3.8 Hz, 2H), 5.13 (t, *J* = 3.6 Hz, 2H), 5.00-4.94 (m, 1H), 4.81 (dd, *J* = 10.8, 3.5 Hz, 1H), 3.57 (s, 3H). |
| **210** | $^1H$ NMR (400 MHz, DMSO-$d_6$) δ 9.82 (s, 1H), 8.83 (d, *J* = 5.4 Hz, 1H), 8.61 (s, 1H), 8.54 (d, *J* = 4.8 Hz, 2H), 8.20 (d, *J* = 5.6 Hz, 1H), 7.60 (d, *J* = 16.1 Hz, 3H), 7.52-7.32 (m, 4H), 7.13 (t, *J* = 4.8 Hz, 1H), 6.47 (t, *J* = 8.3 Hz, 1H), 3.06-3.00 (m, 1H), 3.26-3.17(m, 1H), 2.63 (dd, *J*= 10.8, 6.1 Hz, 2H). |

(continued)

| Example No. | $^1H$ NMR |
|---|---|
| 211 | $^1H$ NMR (400 MHz, DMSO-$d_6$) δ 8.80 (d, J = 2.2 Hz, 1H), 8.22 (s, 1H), 8.11 (d, J = 7.3 Hz, 1H), 7.69 (d, J = 8.0 Hz, 1H), 7.59 (d, J = 8.0 Hz, 1H), 7.56 (s, 1H), 7.37 -7.17 (m, 3H), 7.12 (d, J = 11.9 Hz, 1H), 6.35 (s, 1H), 4.33 (s, 3H), 2.98-2.86 (m, 1H), 2.86-2.79 (m, 1H), 2.35-2.25 (m, 2H). |
| 212 | $^1H$ NMR (400 MHz, DMSO-$d_6$) δ 9.82 (s, 1H), 8.85 (d, J = 2.0 Hz, 1H), 8.82 (d, J = 5.5 Hz, 1H), 8.56 (d, J = 1.9 Hz, 1H), 8.20 (d, J = 5.6 Hz, 1H), 7.76 (d, J = 7.9 Hz, 1H), 7.58 (d, J = 7.9 Hz, 1H), 7.55 (s, 1H), 7.54-7.48 (m, 3H), 7.37 (d, J = 8.5 Hz, 1H), 7.30 (d, J = 2.2 Hz, 1H), 6.39-6.32 (m, 1H), 2.95-2.85 (m, 1H), 2.85-2.75 (m, 1H), 2.36-2.27 (m, 2H). |
| 213 | $^1H$ NMR (400 MHz, DMSO-$d_6$) δ 8.76 (d, J = 2.1 Hz, 1H), 7.65 (d, J = 7.9 Hz, 1H), 7.58 (d, J = 8.1 Hz, 1H), 7.55 (s, 1H), 7.46 (d, J = 7.5 Hz, 1H), 7.20 (s, 1H), 7.07 (d, J = 11.9 Hz, 1H), 6.79 (s, 2H), 6.30 (s, 1H), 5.30-5.17 (m, 2H), 5.00-4.89 (m, 2H), 2.96-2.74 (m, 2H), 2.83-2.73 (m, 1H), 2.30-2.19 (m, 1H). |
| 214 | $^1H$ NMR (400 MHz, DMSO-$d_6$) δ 8.57-8.53 (m, 2H), 8.26-8.19 (m, 1H), 8.15 (s, 1H), 7.61 (s, 1H), 7.49-7.36 (m, 4H), 7.23 (s, 2H), 7.17-7.12 (m, 1H), 6.44 (t, J= 8.6 Hz, 1H), 4.17 (s, 3H), 3.26-3.17 (m, 1H), 3.07-2.96 (m, 1H), 2.69-2.55 (m, 2H). |
| 215 | $^1H$ NMR (400 MHz, MeOH-$d_4$) δ 9.97 (s, 1H), 8.80 (d, J = 5.7 Hz, 1H), 8.65 (s, 1H), 8.18 (d, J = 5.6 Hz, 1H), 7.75-7.62 (m, 2H), 7.60-7.42 (m, 3H), 5.16-5.06 (m, 1H), 4.31 (s, 1H), 3.67-3.55 (m, 1H), 3.39 (s, 3H), 3.15-3.11 (m, 1H), 2.92-2.60 (m, 3H), 2.56-2.42 (m, 1H), 1.43 (d, J = 6.8 Hz, 3H). |
| 216 | $^1H$ NMR (400 MHz, DMSO-$d_6$) δ 10.04 (s, 1H), 8.84 (d, J = 5.5 Hz, 1H), 8.70 (s, 1H), 8.23 (d, J = 5.6 Hz, 1H), 7.61-7.51 (m, 5H), 7.47 (s, 2H), 5.15-5.00 (m, 1H), 4.27-4.14 (m, 1H), 3.70 (t, J = 9.4 Hz, 1H), 3.47-3.39 (m, 1H), 3.35 (s, 3H), 3.23-3.12 (m, 1H), 3.06-2.82 (m, 2H), 2.45-2.34 (m, 1H), 1.24 (d, J = 6.9 Hz, 3H). |
| 217 | $^1H$ NMR (400 MHz, DMSO-$d_6$) δ 7.60-7.41 (m, 2H), 7.32-7.17 (m, 1H), 6.85-6.60 (m, 2H), 5.41-5.15 (m, 2H), 5.08-4.92 (m, 2H), 3.93-3.79 (m, 1H), 3.51-3.43 (m, 1H), 3.19 (s, 3H), 3.02-2.83 (m, 4H), 2.04-1.94 (m, 2H), 1.28 (d, J = 6.9 Hz, 3H). |
| 218 | $^1H$ NMR (400 MHz, MeOH-$d_4$) δ 7.75-7.37 (m, 4H), 7.31 (d, J = 11.6 Hz, 1H), 5.52-5.25 (m, 2H), 5.22-5.00 (m, 2H), 4.58 (s, 2H), 4.18-3.84 (m, 1H), 3.82-3.59 (m, 1H), 3.38 (s, 3H), 3.27-3.17 (m, 1H), 3.11-2.99 (m, 1H), 2.77-2.60 (m, 1H), 2.59-2.46 (m, 1H), 1.29 (d, J = 6.6 Hz, 3H). |
| 219 | $^1H$ NMR (400 MHz, MeOH-$d_4$) δ 10.14 (s, 1H), 8.99 (d, J = 5.6 Hz, 1H), 8.87 (s, 1H), 8.38 (d, J = 5.6 Hz, 1H), 7.89 (s, 1H), 7.82 (d, J = 8.4 Hz, 1H), 7.62 (s, 1H), 7.55 (d, J = 8.1 Hz, 2H), 5.95-5.35 (m, 1H), 3.39 (dd, J = 14.5, 6.5 Hz, 1H), 3.23-3.03 (m, 2H), 2.90-2.35 (m, 3H), 1.22-0.82 (m, 1H), 0.45 (s, 2H), 0.30 - -0.10 (m, 2H). |
| 220 | $^1H$ NMR (400 MHz, DMSO-$d_6$) δ 7.71-7.60 (m, 2H), 7.58-7.51 (m, 2H), 7.40 (s, 1H), 7.07 (d, J = 11.8 Hz, 1H), 6.92 (s, 1H), 6.76 (s, 2H), 6.40 (t, J = 7.9 Hz, 1H), 5.29 (s, 2H), 4.96 (s, 2H), 3.49 (s, 3H), 2.96-2.81 (m, 2H), 2.76-2.65 (m, 1H), 2.07-1.98 (m, 1H). |
| 221 | $^1H$ NMR (400 MHz, DMSO-$d_6$) δ 9.01-8.75 (m, 2H), 8.60-8.15 (m, 2H), 7.60-7.45 (m, 2H), 7.44-7.03 (m, 5H), 5.56-4.75 (m, 2H), 4.46-4.28 (m, 3H), 3.17-3.06 (m, 1H), 3.0-2.85 (m, 1H), 2.04-1.94 (m, 1H), 1.94-1.85 (m, 1H), 1.34-1.16 (m, 3H). |
| 222 | $^1H$ NMR (400 MHz, DMSO-$d_6$) δ 10.02 (s, 1H), 9.06-8.83 (m, 4H), 8.40 (s, 1H), 7.80 (s, 1H), 7.69 (s, 1H), 7.56 (s, 1H), 7.52 (s, 1H), 7.41 (s, 1H), 7.31 (s, 1H), 5.45 -4.90 (m, 1H), 3.24-3.07 (m, 2H), 3.05-2.80 (m, 2H), 2.75-2.63(m, 1H), 1.24 (s, 3H). |
| 223 | $^1H$ NMR (400 MHz, DMSO-$d_6$) δ 9.00-8.75 (m, 2H), 7.69-7.35 (m, 4H), 7.28-7.04 (m, 2H), 6.84-6.70 (m, 2H), 5.45-5.30 (m, 1H), 5.30-5.04 (m, 2H), 5.04-4.90 (m, 2H), 3.29-3.06 (m, 2H), 3.03-2.80 (m, 2H), 2.65-2.53 (m, 1H), 1.94-1.60 (m, 3H). |
| 224 | $^1H$ NMR (400 MHz, DMSO-$d_6$) δ 8.87-8.68 (m, 2H), 7.83-7.13 (m, 6H), 6.83-6.65 (m, 2H), 5.39-5.21 (m, 2H), 5.05 (s, 1H), 4.98-4.85 (m, 2H), 3.24-3.15 (m, 1H), 3.03-2.79 (m, 2H), 2.79-2.63 (m, 1H), 2.36-2.23 (m, 1H), 1.80-1.62 (m, 3H). |
| 225 | $^1H$ NMR (400 MHz, MeOH-$d_4$) δ 10.17 (s, 1H), 9.01 (d, J = 5.6 Hz, 1H), 8.97 (d, J = 1.7 Hz, 1H), 8.41 (d, J = 5.6 Hz, 1H), 8.09-7.95 (m, 2H), 7.80 (d, J = 8.5 Hz, 1H), 7.66 (d, J = 7.9 Hz, 1H), 5.77 (t, J = 8.3 Hz, 1H), 3.22-3.11 (m, 2H), 2.96-2.43 (m, 3H), 0.68-0.62 (m, 1H), 0.56-0.33 (m, 3H). |

(continued)

| Example No. | $^1$H NMR |
|---|---|
| **226** | $^1$H NMR (400 MHz, DMSO-$d_6$) δ 10.11 (s, 1H), 9.00 (d, *J* = 5.5 Hz, 1H), 8.77 (s, 1H), 8.50 (d, *J* = 5.6 Hz, 1H), 7.85 (d, *J* = 7.8 Hz, 1H), 7.75 (d, *J* = 8.6 Hz, 1H), 7.68-7.50 (m, 4H), 7.10 (s, 1H), 6.50-6.34 (m, 1H), 3.49 (s, 3H), 2.98-2.82 (m, 2H), 2.79-2.69 (m, 1H), 2.17-2.04 (m, 1H). |
| **227** | $^1$H NMR (400 MHz, MeOH-$d_4$) δ 10.07-9.94 (m, 1H), 8.83-8.76 (m, 2H), 8.17 (d, *J* = 5.7 Hz, 1H), 7.78 (dd, *J* = 8.4, 1.8 Hz, 1H), 7.71 (d, *J* = 8.5 Hz, 1H), 7.64-7.51 (m, 3H), 6.50-5.50 (m, 1H), 3.24-3.03 (m, 2H), 2.93-2.77 (m, 3H), 2.69-2.45 (m, 1H), 2.41-2.24 (m, 1H). |
| **229** | $^1$H NMR (400 MHz, DMSO-$d_6$) δ 8.26-8.08 (m, 2H), 7.73-7.60 (m, 2H), 7.57 (s, 1H), 7.42 (s, 1H), 7.22 (s, 2H), 7.09 (d, *J* = 11.7 Hz, 1H), 6.99 (s, 1H), 6.49-6.38 (m, 1H), 4.38 (s, 3H), 3.48 (s, 3H), 2.98-2.84 (m, 2H), 2.79-2.65 (m, 1H), 2.14-1.97 (m, 1H). |
| **230** | $^1$H NMR (400 MHz, MeOH-$d_4$) δ 10.08-9.80 (m, 1H), 8.80-8.57 (m, 1H), 8.57-8.32 (m, 1H), 8.25-7.85 (m, 2H), 7.58-7.41 (m, 1H), 7.39 (d, *J* = 7.5 Hz, 1H), 5.96-5.58 (m, 1H), 5.12-4.90 (m, 2H), 2.40 (s, 2H), 1.15-0.90 (m, 1H), 0.62-0.20 (m, 2H), 0.10 - -0.18 (m, 2H). |
| **231** | $^1$H NMR (400 MHz, MeOH-$d_4$) δ 9.84 (s, 1H), 8.71 (d, *J* = 5.6 Hz, 1H), 8.57 (d, *J* = 7.2 Hz, 1H), 8.08 (d, *J* = 5.6 Hz, 1H), 7.97 (d, *J* = 7.5 Hz, 1H), 7.34 (d, *J* = 7.5 Hz, 1H), 7.26 (d, *J* = 11.1 Hz, 1H), 5.84-5.66 (m, 1H), 5.05-4.85 (m, 2H), 3.37-3.25 (m, 2H), 1.05-0.85 (m, 1H), 0.57-0.30 (m, 2H), 0.10 - -0.25 (m, 2H). |
| **232** | $^1$H NMR (400 MHz, MeOH-$d_4$) δ 10.00-9.79 (m, 1H), 8.77-8.43 (m, 2H), 8.12-7.88 (m, 2H), 7.69-7.48 (m, 1H), 7.31 (dd, *J* = 15.7, 7.3 Hz, 1H), 5.86-5.50 (m, 1H), 5.10-4.82 (m, 2H), 3.19-2.46 (m, 2H), 1.06-0.86 (m, 1H), 0.55-0.15 (m, 2H), 0.10 - -0.40 (m, 2H). |
| **233** | $^1$H NMR (400 MHz, DMSO-$d_6$) δ 10.26-10.01 (m, 1H), 8.86-8.73 (m, 1H), 8.61-8.48 (m, 1H), 8.21 (d, *J* = 5.6 Hz, 1H), 7.96-7.14 (m, 6H), 6.21-5.47 (m, 1H), 5.06-4.54 (m, 2H), 3.17-2.92 (m, 2H), 2.36 (s, 3H), 1.09-0.69 (m, 1H), 0.53 - -0.18 (m, 4H). |
| **234** | $^1$H NMR (400 MHz, DMSO-$d_6$) δ 10.20-9.98 (m, 1H), 8.87-8.70 (m, 2H), 8.23 (d, *J* = 5.6 Hz, 1H), 7.74-7.61 (m, 3H), 7.39-7.26 (m, 2H), 7.25-7.10 (m, 1H), 6.07-5.59 (m, 1H), 5.01-4.58 (m, 2H), 3.24-3.01 (m, 2H), 1.03-0.77 (m, 1H), 0.5 - -0.29 (m, 4H). |
| **235** | $^1$H NMR (400 MHz, DMSO-$d_6$) δ 10.30-10.00 (m, 1H), 9.05-8.61 (m, 2H), 8.30-8.15 (m, 1H), 7.91-7.47 (m, 4H), 7.40-7.10 (m, 2H), 6.21-5.41 (m, 1H), 5.01-4.50 (m, 2H), 3.13-2.85 (m, 2H), 1.10-0.75 (m, 1H), 0.54 - -0.31 (m, 4H). |
| **236** | $^1$H NMR (400 MHz, DMSO-$d_6$) δ 10.16-10.06 (m, 1H), 8.85-8.75 (m, 1H), 8.74-8.48 (m, 1H), 8.44-7.84 (m, 2H), 7.73 (d, *J* = 7.9 Hz, 1H), 7.53-7.30 (m, 3H), 5.75 -5.19 (m, 1H), 3.27-2.95 (m, 4H), 2.90-2.58 (m, 2H), 2.36 (s, 3H), 1.18-0.81 (m, 1H), 0.46 - -0.07 (m, 4H). |
| **237** | $^1$H NMR (400 MHz, DMSO-$d_6$) δ 10.17-10.05 (m, 1H), 8.97-8.69 (m, 2H), 8.29-8.17 (m, 1H), 8.12-7.58 (m, 4H), 7.37-7.28 (m, 1H), 5.71-5.31 (m, 1H), 3.28-2.82 (m, 4H), 2.71-2.27 (m, 2H), 1.13-0.92 (m, 1H), 0.50 - -0.15 (m, 4H). |
| **238** | $^1$H NMR (400 MHz, DMSO-$d_6$) δ 10.22-10.10 (m, 1H), 8.98-8.66 (m, 2H), 8.39-7.96 (m, 2H), 7.79-7.57 (m, 4H), 5.66-5.21 (m, 1H), 3.24-2.80 (m, 4H), 2.71-2.29 (m, 1H), 2.38 (d, *J* = 9.2 Hz, 1H), 1.14-0.92 (m, 1H), 0.50 - -0.15 (m, 4H). |

## Biological Test Evaluation

### I. Human Colon Cancer HCT116 Cell Proliferation Inhibition Test

1. HCT 116 MTAP knockout and MTAP wild-type cells were plated in 96-well flat bottom plates, and cultured overnight with McCoy's 5A medium containing 10% fetal bovine serum + 1% penicillin-streptomycin at 37°C and 5% $CO_2$.
2. On the next day, the compound was dissolved with DMSO, diluted with DMSO and medium successively and transferred to the cell plate. The final concentration of the compound was 10 μM, the compound was diluted 4 times, with 9 concentration gradients, and DMSO control was added.

3. Cells that have not been treated with the compound were removed. CellTiter-Glo Luminescent Cell Viability Assay (Promega) was used to detect cell viability, the operation was performed with reference to the package insert of the kit, and then the cell plate was placed on EnVision Multilabel Reader to detect the cold light signal.
4. Meanwhile, the cell plate treated with the compound was cultured at 37°C and 5% $CO_2$ for 6 consecutive days.
5. Then, CellTiter-Glo was also used to detect cell viability.
6. Finally, the four-parameter dose-response curve module of GraphPad Prism v 9.2.0 software was used to plot the dose-response curve and calculate the $IC_{50}$ for proliferation inhibition (unit: nM). The test results are as follows:

**Table 1: Biology Test Results**

| Example No. | HCT116 wild-type cells $IC_{50}$ (nM) | HCT116 MTAP knockout cells $IC_{50}$ (nM) | Example No. | HCT116 wild-type cells $IC_{50}$ (nM) | HCT116 MTAP knockout cells $IC_{50}$ (nM) |
|---|---|---|---|---|---|
| **1** | NT | 792 | **205** | 64 | 0.8 |
| **2** | 30 | 1 | **206** | 8469 | 82 |
| **7** | NT | 57 | **208** | 130 | 4 |
| **8** | NT | 4 | **209** | 279 | 4 |
| **21** | 79 | 0.8 | **210** | 7400 | 63 |
| **95** | NT | 52 | **211** | NT | 4 |
| **108** | 1422 | 36 | **212** | NT | 16 |
| **109** | 824 | 15 | **213** | 70 | 3 |
| **110** | NT | 116 | **214** | NT | 325 |
| **111** | NT | 115 | **215** | NT | 6926 |
| **132** | 1954 | 44 | **216** | NT | 940 |
| **133** | 708 | 8 | **217** | NT | 2360 |
| **152** | NT | 4 | **218** | NT | 485 |
| **157** | NT | 139 | **219** | 1565 | 35 |
| **160** | 335 | 17 | **220** | 335 | 17 |
| **165** | 2514 | 35 | **221** | 1339 | 88 |
| **168** | 276 | 0.3 | **222** | NT | 217 |
| **184** | >10000 | >10000 | **223** | NT | 118 |
| **185** | 1261 | 28 | **224** | NT | 640 |
| **186** | >10000 | 506 | **225** | 9472 | 286 |
| **187** | 2445 | 50 | **226** | 1954 | 50 |
| **188** | >10000 | 2635 | **227** | 1068 | 85 |

| | | | | | |
|---|---|---|---|---|---|
| **191** | NT | 2900 | **229** | 61 | 3 |
| **192** | 79 | 3.6 | **230** | 145 | 13 |
| **193** | 9089 | 452 | **231** | 604 | 15 |
| **195** | 423.4 | 8.6 | **232** | 363 | 10 |
| **196** | NT | 1527 | **233** | 102 | 33 |
| **197** | 264 | 0.9 | **234** | 224 | 20 |
| **199** | NT | 134 | **235** | 176 | 21 |
| **201** | 78 | 2 | **236** | 227 | 9 |
| **202** | 36 | 2 | **237** | 788 | 20 |
| **203** | 104 | 9 | **238** | 264 | 14 |
| **204** | 293 | 6 | **Reference Compound 1** | 696 | 54 |
| **Reference Compound 2** | 702 | 25 | **Reference Compound 3** | 278 | 9 |
| Notes | 1. "NT" is an abbreviation of "Not Tested", which means that it has not been tested for the time being.<br>2. The reference compounds are as follows: | | | | |

| **Reference Compound 1** | **Reference Compound 2** | **Reference Compound 3** |
|---|---|---|
| Fall into the scope of WO2022169948A1 | WO2022169948A1-EX220 | WO2022115377A1-EX740 |
| 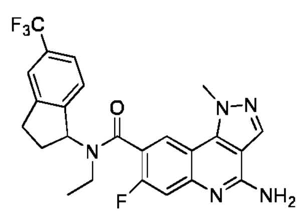 | 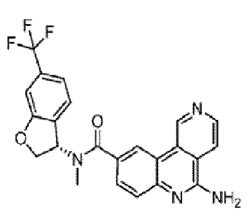 | 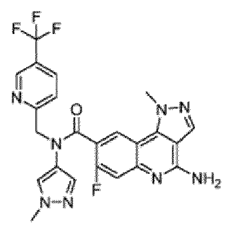 |

It can be seen from the biological activity data of the compounds of specific examples that the series of compounds of the present invention have a strong inhibitory effect on the proliferation of human colon cancer HCT116 MTAP knockout cells at the cellular level, and some compounds have a weak inhibitory effect on MTAP wild-type HCT116 cells and have high selectivity.

## II. Pharmacokinetic Assay in Mice

### 1. Study Purpose

The purpose of this trial is to study the pharmacokinetic behavior of some compounds of the present invention, and the administration method is: ICR mice were orally administered (PO) a single dose, at a dose of 10 mg/kg.

### 2. Test Protocol

#### 2.1 Test Drugs

The compounds used in this trial are from the compounds in the specific examples of the present invention.

#### 2.2 Test Animals

ICR mice male N=3 original source: Shanghai Sippr-Bk Lab Animal Co., Ltd.

#### 2.3 Drug Preparation and Administration

The compounds were weighed and dissolved in 20% PG + 10% Solutol HS15 + 70% pH 3 citrate buffer, respectively, shaken well and sonicated to prepare colorless clear solutions. 9 mice were administered orally after an overnight fast. The dose was 10 mg/kg.

#### 2.4 Sample Collection

1) About 90 μL blood was collected via the submandibular vein per time point, heparin sodium anticoagulant was added, the sample was placed on ice after collection, and plasma was centrifuged within 1 hour (centrifugation conditions: 8,000 rpm, 6 minutes, 2-8°C).
2) The blood collection time points were 1, 4, and 24 h. The samples were stored in a refrigerator at -20°C.
3) The plasma sample was 40 μL, 160 μL of ice-cold acetonitrile containing internal standard was added, vortexed for 3 minutes, and centrifuged at 11,000 rpm for 5 minutes.
4) 100 μL of the supernatant was taken and added to 100 μL of water, 5 μL of the sample was taken and injected into LC/MS/MS for analysis.

### 3. Blood-Brain Barrier Penetration Analysis

AUC or concentration in brain or plasma measured by using the above method was used to calculate the Kp. Kp refers to the relationship between drug concentrations in brain and blood and is used to evaluate the ability of a drug to penetrate the blood-brain barrier. Post-dose Kp was calculated by the following formula:

$$Kp = [\text{AUC (brain)/AUC (plasma)}],$$

or

$$Kp = [\text{drug concentration (brain)/drug concentration (plasma)}]$$

Table 2: Data of Kp of Compounds in Examples and Reference Compounds

| Compound No. | Kp (4 h after administration) |
|---|---|
| Example 192 | 0.4 |
| Example 219 | 0.5 |
| Example 233 | 0.4 |
| Example 234 | 0.7 |
| Example 235 | 0.7 |
| Reference Compound 2 | 0.1 |
| Notes: | 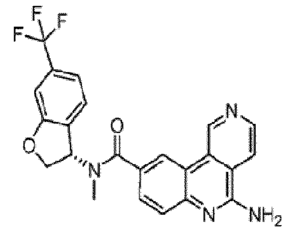 **Reference Compound 2** - WO2022169948A1-EX220 |

It can be seen from the above data that multiple compounds of the present invention have significantly higher Kp than Reference Compound 2 and have superior blood-brain barrier penetration properties when compared to the reference compounds of the prior art.

All documents mentioned in the present invention are incorporated by reference in this application, just as each document is cited separately as a reference. In addition, it should be understood that various modifications or changes may be made by those skilled in the art after reading the above disclosed content of the present invention, and these equivalent forms also fall within the scope defined by the claims appended hereto.

## Claims

**1.** A compound of formula (I), a stereoisomer or pharmaceutically acceptable salt thereof:

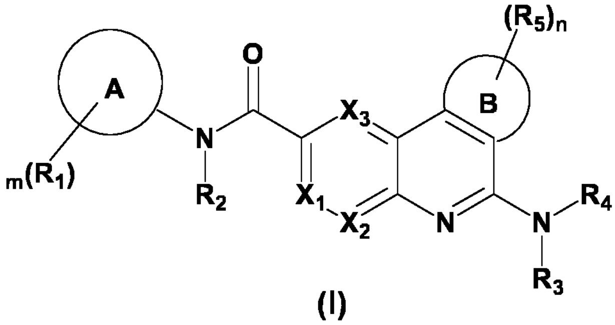

(I)

,

wherein, $X_1$ is $CR_6$ or N; $X_2$ is $CR_7$ or N; $X_3$ is $CR_8$ or N;

ring A is $C_{3-10}$ cycloalkyl, 4-10 membered heterocyclyl, $C_{6-10}$ aryl or 5-10 membered heteroaryl, and the $C_{3-10}$ cycloalkyl or 4-10 membered heterocyclyl is further fused to $C_{6-10}$ aryl or 5-10 membered heteroaryl, the $C_{6-10}$ aryl or 5-10 membered heteroaryl is further fused to $C_{3-10}$ cycloalkyl or 4-10 membered heterocyclyl;
ring B is $C_{4-12}$ cycloalkyl, 4-12 membered heterocyclyl, $C_{6-10}$ aryl or 5-10 membered heteroaryl;
each $R_1$ is independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, $-C_{0-8}$ alkyl-$SF_5$, $-C_{0-8}$ alkyl-O-$S(O)_2R_9$, $-C_{0-8}$ alkyl-$S(O)_rR_9$, $-C_{0-8}$ alkyl-O-$R_{10}$, $-C_{0-8}$ alkyl-C(O)$OR_{10}$, $-C_{0-8}$ alkyl-C(O)$SR_{10}$, $-C_{0-8}$ alkyl-S-C(O)$R_{11}$, $-C_{0-8}$ alkyl-C(O)$R_{11}$, $-C_{0-8}$ alkyl-O-C(O)$R_{11}$, $-C_{0-8}$ alkyl-P(O)$(R_{11})_2$, $-C_{0-8}$ alkyl-$NR_{12}R_{13}$, $-C_{0-8}$ alkyl-C(O)$NR_{12}R_{13}$ and $-C_{0-8}$ alkyl-N($R_{12}$)-C(O)$R_{11}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$ and -$C_{0-8}$ alkyl-N($R_{12}$)-C(O)$R_{11}$;

$R_2$ is selected from the group consisting of hydrogen, deuterium, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$S(O)_rR_9$, -O-$R_{10}$, -C(O)$OR_{10}$, -C(O)$R_{11}$ and -C(O)$NR_{12}R_{13}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$ and -$C_{0-8}$ alkyl-N($R_{12}$)-C(O)$R_{11}$;

$R_3$ and $R_4$ are each independently selected from the group consisting of hydrogen, deuterium, hydroxy, $C_{1-10}$ alkyl, $C_{2-10}$ alkenyl, $C_{2-10}$ alkynyl, $C_{3-12}$ cycloalkyl and 3-12 membered heterocyclyl, or, $R_3$ and $R_4$, together with a nitrogen atom directly attached thereto, form a 4-10 membered heterocyclyl or 5-10 membered heteroaryl, and above groups are independently optionally further substituted by one or more substituents selected from the group consisting of deuterium, halogen, cyano, hydroxy, =O, =S, $C_{1-10}$ alkyl, $C_{1-10}$ haloalkyl, $C_{1-10}$ deuterioalkyl, $C_{2-10}$ alkenyl, $C_{2-10}$ alkynyl, $C_{1-10}$ alkoxy, $C_{3-12}$ cycloalkyl, $C_{3-12}$ cycloalkoxy, 3-12 membered heterocyclyl, 3-12 membered heterocyclyloxy and -$NR_{12}R_{13}$;

each $R_5$ is independently selected from the group consisting of hydrogen, deuterium, halogen, cyano, nitro, azido, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$ and -$C_{0-8}$ alkyl-N($R_{12}$)-C(O)$R_{11}$, and above groups are independently optionally further substituted by one or more substituents selected from the group consisting of deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, =O, =S, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$ and -$C_{0-8}$ alkyl-N($R_{12}$)-C(O)$R_{11}$;

$R_6$, $R_7$ and $R_8$ are each independently selected from the group consisting of hydrogen, deuterium, halogen, cyano, nitro, azido, $C_{1-10}$ alkyl, $C_{1-10}$ haloalkyl, $C_{1-10}$ deuterioalkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$C_{0-8}$ alkyl-$SF_5$, -$C_{0-8}$ alkyl-O-$S(O)_2R_9$, -$C_{0-8}$ alkyl-$S(O)_rR_9$, -$C_{0-8}$ alkyl-O-$R_{10}$, -$C_{0-8}$ alkyl-C(O)$OR_{10}$, -$C_{0-8}$ alkyl-C(O)$SR_{10}$, -$C_{0-8}$ alkyl-S-C(O)$R_{11}$, -$C_{0-8}$ alkyl-C(O)$R_{11}$, -$C_{0-8}$ alkyl-O-C(O)$R_{11}$, -$C_{0-8}$ alkyl-P(O)$(R_{11})_2$, -$C_{0-8}$ alkyl-$NR_{12}R_{13}$, -$C_{0-8}$ alkyl-C(O)$NR_{12}R_{13}$ and -$C_{0-8}$ alkyl-N($R_{12}$)-C(O)$R_{11}$;

each $R_9$ is independently selected from the group consisting of hydrogen, deuterium, hydroxy, $C_{1-10}$ alkyl, $C_{2-10}$ alkenyl, $C_{3-12}$ cycloalkyl, 3-12 membered heterocyclyl, $C_{6-10}$ aryl, 5-10 membered heteroaryl and -$NR_{12}R_{13}$, and above groups are independently optionally further substituted by one or more substituents selected from the group consisting of deuterium, halogen, hydroxy, =O, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and -$NR_{12}R_{13}$;

each $R_{10}$ is independently selected from the group consisting of hydrogen, deuterium, $C_{1-10}$ alkyl, $C_{2-10}$ alkenyl, $C_{3-12}$ cycloalkyl, 3-12 membered heterocyclyl, $C_{6-10}$ aryl and 5-10 membered heteroaryl, and above groups are independently optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and -$NR_{12}R_{13}$;

each $R_{11}$ is independently selected from the group consisting of hydrogen, deuterium, hydroxy, $C_{1-10}$ alkyl, $C_{1-10}$ alkoxy, $C_{2-10}$ alkenyl, $C_{2-10}$ alkynyl, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and -$NR_{12}R_{13}$, and above groups are independently optionally further substituted by one or more substituents selected from the group consisting of deuterium, halogen, hydroxy, =O, cyano, $C_{1-10}$ alkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy and -$NR_{12}R_{13}$;

each $R_{12}$ and each $R_{13}$ are independently selected from the group consisting of hydrogen, deuterium, hydroxy,

$C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and $C_{1-10}$ alkanoyl, and above groups are independently optionally further substituted by one or more substituents selected from the group consisting of deuterium, halogen, hydroxy, =O, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono-$C_{1-10}$ alkylamino, di-$C_{1-10}$ alkylamino and $C_{1-10}$ alkanoyl, or,

$R_{12}$ and $R_{13}$, together with a nitrogen atom directly attached thereto, form a 4-10 membered heterocyclyl or 5-10 membered heteroaryl, and the 4-10 membered heterocyclyl or 5-10 membered heteroaryl is further optionally substituted by one or more substituents selected from the group consisting of deuterium, halogen, hydroxy, =O, $C_{1-10}$ alkyl, $C_{2-10}$ alkenyl, $C_{2-10}$ alkynyl, $C_{1-10}$ haloalkyl, $C_{1-10}$ deuterioalkyl, $C_{1-10}$ alkoxy, $C_{3-12}$ cycloalkyl, $C_{3-12}$ cycloalkoxy, 3-12 membered heterocyclyl, 3-12 membered heterocyclyloxy, $C_{6-10}$ aryl, $C_{6-10}$ aryloxy, 5-10 membered heteroaryl, 5-10 membered heteroaryloxy, amino, mono-$C_{1-10}$ alkylamino, di-$C_{1-10}$ alkylamino and $C_{1-10}$ alkanoyl;

each r is independently 0, 1 or 2;

m is 0, 1, 2, 3, 4, 5 or 6; and

n is 0, 1, 2, 3, 4 or 5.

**2.** The compound of formula (I), the stereoisomer or pharmaceutically acceptable salt thereof according to claim 1, wherein ring A is $C_{3-8}$ cycloalkyl, 4-8 membered heterocyclyl, $C_{6-8}$ aryl or 5-8 membered heteroaryl, and the $C_{3-8}$ cycloalkyl or 4-8 membered heterocyclyl is further fused to $C_{6-8}$ aryl or 5-8 membered heteroaryl, the $C_{6-8}$ aryl or 5-8 membered heteroaryl is further fused to $C_{3-8}$ cycloalkyl or 4-8 membered heterocyclyl;

ring B is $C_{4-6}$ cycloalkyl, 4-6 membered heterocyclyl, $C_{6-8}$ aryl or 5-8 membered heteroaryl;

each $R_1$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$C_{0-4}$ alkyl-$SF_5$, -$C_{0-4}$ alkyl-O-$S(O)_2R_9$, -$C_{0-4}$ alkyl-$S(O)_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, - $C_{0-4}$ alkyl-O-C(O)$R_{11}$, -$C_{0-4}$ alkyl-P(O)$(R_{11})_2$, -$C_{0-4}$ alkyl-$NR_{12}R_{13}$, -$C_{0-4}$ alkyl-C(O)$NR_{12}R_{13}$ and - $C_{0-4}$ alkyl-N($R_{12}$)-C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$C_{0-4}$ alkyl-$SF_5$, -$C_{0-4}$ alkyl-O-$S(O)_2R_9$, -$C_{0-4}$ alkyl-$S(O)_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)$(R_{11})_2$, -$C_{0-4}$ alkyl-$NR_{12}R_{13}$, -$C_{0-4}$ alkyl-C(O)$NR_{12}R_{13}$ and -$C_{0-4}$ alkyl-N($R_{12}$)-C(O)$R_{11}$;

$R_2$ is selected from the group consisting of hydrogen, deuterium, $C_{1-10}$ alkyl, $C_{2-10}$ alkenyl, $C_{2-10}$ alkynyl, $C_{3-12}$ cycloalkyl, 3-12 membered heterocyclyl, $C_{6-10}$ aryl, 5-10 membered heteroaryl, -$S(O)_rR_9$, -O-$R_{10}$, -C(O)O$R_{10}$, -C(O)$R_{11}$ and -C(O)$NR_{12}R_{13}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, -$C_{0-4}$ alkyl-$SF_5$, -$C_{0-4}$ alkyl-O-$S(O)_2R_9$, -$C_{0-4}$ alkyl-$S(O)_rR_9$, -$C_{0-4}$ alkyl-O-$R_{10}$, -$C_{0-4}$ alkyl-C(O)O$R_{10}$, - $C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)$(R_{11})_2$, -$C_{0-4}$ alkyl-$NR_{12}R_{13}$, -$C_{0-4}$ alkyl-C(O)$NR_{12}R_{13}$ and -$C_{0-4}$ alkyl-N($R_{12}$)-C(O)$R_{11}$;

$R_3$ and $R_4$ are each independently selected from the group consisting of hydrogen, deuterium, hydroxy, $C_{1-4}$ alkyl, $C_{2-4}$ alkenyl, $C_{2-4}$ alkynyl, $C_{3-6}$ cycloalkyl and 3-6 membered heterocyclyl, or, $R_3$ and $R_4$, together with a nitrogen atom directly attached thereto, form a 4-6 membered heterocyclyl or 5-8 membered heteroaryl, and above groups are independently optionally further substituted by one or more substituents selected from the group consisting of deuterium, halogen, cyano, hydroxy, =O, =S, $C_{1-4}$ alkyl, $C_{1-4}$ haloalkyl, $C_{1-4}$ deuterioalkyl, $C_{2-4}$ alkenyl, $C_{2-4}$ alkynyl, $C_{1-4}$ alkoxy, $C_{3-6}$ cycloalkyl, $C_{3-6}$ cycloalkoxy, 3-6 membered heterocyclyl, 3-6 membered heterocyclyloxy and -$NR_{12}R_{13}$;

each $R_5$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$C_{0-4}$ alkyl-$SF_5$, -$C_{0-4}$ alkyl-O-$S(O)_2R_9$, -$C_{0-4}$ alkyl-$S(O)_rR_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, - $C_{0-4}$ alkyl-O-C(O)$R_{11}$, -$C_{0-4}$ alkyl-P(O)$(R_{11})_2$, -$C_{0-4}$ alkyl-$NR_{12}R_{13}$, -$C_{0-4}$ alkyl-C(O)$NR_{12}R_{13}$ and - $C_{0-4}$ alkyl-N($R_{12}$)-C(O)$R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6

membered heterocyclyl, $C_{6-8}$ aryl, 5-8 membered heteroaryl, =O, =S, -$C_{0-4}$ alkyl-$SF_5$, -$C_{0-4}$ alkyl-O-S(O)$_2$$R_9$, -$C_{0-4}$ alkyl-S(O)$_r$$R_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)($R_{11}$)$_2$, -$C_{0-4}$ alkyl-N$R_{12}$$R_{13}$, -$C_{0-4}$ alkyl-C(O)N$R_{12}$$R_{13}$ and -$C_{0-4}$ alkyl-N($R_{12}$)-C(O)$R_{11}$;

$R_6$, $R_7$ and $R_8$ are each independently selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, -$C_{0-4}$ alkyl-$SF_5$, -$C_{0-4}$ alkyl-O-S(O)$_2$$R_9$, -$C_{0-4}$ alkyl-S(O)$_r$$R_9$, -$C_{0-4}$ alkyl-O-$R_{10}$, -$C_{0-4}$ alkyl-C(O)O$R_{10}$, -$C_{0-4}$ alkyl-C(O)S$R_{10}$, -$C_{0-4}$ alkyl-S-C(O)$R_{11}$, -$C_{0-4}$ alkyl-C(O)$R_{11}$, -$C_{0-4}$ alkyl-O-C(O)$R_{11}$, -$C_{0-4}$ alkyl-P(O)($R_{11}$)$_2$, -$C_{0-4}$ alkyl-N$R_{12}$$R_{13}$, -$C_{0-4}$ alkyl-C(O)N$R_{12}$$R_{13}$ and -$C_{0-4}$ alkyl-N($R_{12}$)-C(O)$R_{11}$;

wherein, $R_9$, $R_{10}$, $R_{11}$, $R_{12}$, $R_{13}$ and r are defined as in claim 1.

**3.** The compound of formula (I), the stereoisomer or pharmaceutically acceptable salt thereof according to claim 1, wherein each $R_9$ is independently selected from the group consisting of hydrogen, deuterium, hydroxy, $C_{1-4}$ alkyl, $C_{2-4}$ alkenyl, $C_{3-6}$ cycloalkyl, 3-6 membered heterocyclyl, $C_{6-8}$ aryl, 5-8 membered heteroaryl and -N$R_{12}$$R_{13}$, and above groups are independently optionally further substituted by one or more substituents selected from the group consisting of deuterium, halogen, hydroxy, =O, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}$$R_{13}$;

each $R_{10}$ is independently selected from the group consisting of hydrogen, deuterium, $C_{1-4}$ alkyl, $C_{2-4}$ alkenyl, $C_{3-6}$ cycloalkyl, 3-6 membered heterocyclyl, $C_{6-8}$ aryl and 5-8 membered heteroaryl, and above groups are independently optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}$$R_{13}$;

each $R_{11}$ is independently selected from the group consisting of hydrogen, deuterium, hydroxy, $C_{1-4}$ alkyl, $C_{1-4}$ alkoxy, $C_{2-4}$ alkenyl, $C_{2-4}$ alkynyl, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}$$R_{13}$, and above groups are independently optionally further substituted by one or more substituents selected from the group consisting of deuterium, halogen, hydroxy, =O, cyano, $C_{1-4}$ alkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy and -N$R_{12}$$R_{13}$;

each $R_{12}$ and each $R_{13}$ are independently selected from the group consisting of hydrogen, deuterium, hydroxy, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, sulfinyl, sulfonyl, methanesulfonyl, isopropylsulfonyl, cyclopropylsulfonyl, p-toluenesulfonyl, aminosulfonyl, dimethylaminosulfonyl and $C_{1-4}$ alkanoyl, and above groups are independently optionally further substituted by one or more substituents selected from the group consisting of deuterium, halogen, hydroxy, =O, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono-$C_{1-4}$ alkylamino, di-$C_{1-4}$ alkylamino and $C_{1-4}$ alkanoyl, or, $R_{12}$ and $R_{13}$, together with a nitrogen atom directly attached thereto, form a 4-6 membered heterocyclyl or 5-8 membered heteroaryl, and the 4-6 membered heterocyclyl or 5-8 membered heteroaryl is further optionally substituted by one or more substituents selected from the group consisting of deuterium, halogen, hydroxy, =O, $C_{1-4}$ alkyl, $C_{2-4}$ alkenyl, $C_{2-4}$ alkynyl, $C_{1-4}$ haloalkyl, $C_{1-4}$ deuterioalkyl, $C_{1-4}$ alkoxy, $C_{3-6}$ cycloalkyl, $C_{3-6}$ cycloalkoxy, 3-6 membered heterocyclyl, 3-6 membered heterocyclyloxy, $C_{6-8}$ aryl, $C_{6-8}$ aryloxy, 5-8 membered heteroaryl, 5-8 membered heteroaryloxy, amino, mono-$C_{1-4}$ alkylamino, di-$C_{1-4}$ alkylamino and $C_{1-4}$ alkanoyl.

**4.** The compound of formula (I), the stereoisomer or pharmaceutically acceptable salt thereof according to claim 1, wherein the compound of formula (I) is a compound of formula (II):

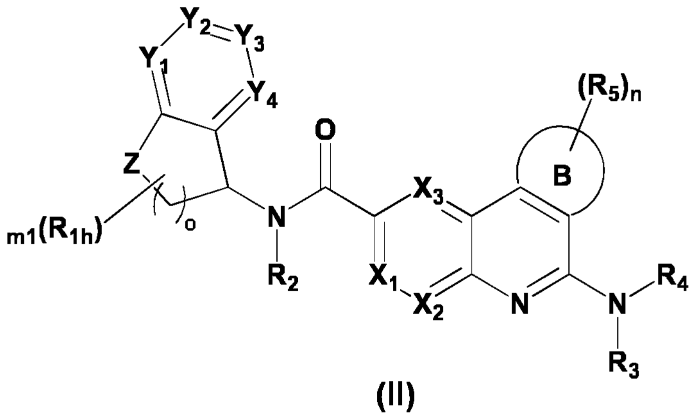

(II)

,

wherein, $X_1$ is $CR_6$ or N; $X_2$ is $CR_7$ or N; $X_3$ is $CR_8$ or N;

$Y_1$ is $CR_{1a}$ or N; $Y_2$ is $CR_{1b}$ or N; $Y_3$ is $CR_{1c}$ or N; $Y_4$ is $CR_{1d}$ or N;
Z is $-C(R_{1e}R_{1f})-$, $-C(R_{1e}R_{1f})O-$, $-N(R_{1g})-$, -O- or -S-;
ring B is $C_{4-6}$ cycloalkyl, 4-6 membered heterocyclyl, $C_{6-8}$ aryl or 5-8 membered heteroaryl;
$R_{1a}$, $R_{1b}$, $R_{1c}$ and $R_{1d}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_{1e}$ and $R_{1f}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, or, $R_{1e}$ and $R_{1f}$, together with a carbon atom directly attached thereto, form a $C_{3-6}$ cycloalkyl or 4-6 membered heterocyclyl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_{1g}$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-S(O)_rR_9$, $-O-R_{10}$, $-C(O)OR_{10}$, $-C(O)SR_{10}$, $-C(O)R_{11}$ and $-C(O)NR_{12}R_{13}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
each $R_{1h}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;
$R_2$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-OR_{10}$ and $-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl,

$C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, - $C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

$R_3$ and $R_4$ are each independently selected from the group consisting of hydrogen, deuterium, hydroxy, $C_{1-4}$ alkyl, $C_{3-6}$ cycloalkyl and 3-6 membered heterocyclyl, or, $R_3$ and $R_4$, together with a nitrogen atom directly attached thereto, form a 4-6 membered heterocyclyl or 5-8 membered heteroaryl, and above groups are independently optionally further substituted by one or more substituents selected from the group consisting of deuterium, halogen, cyano, hydroxy, =O, =S, $C_{1-4}$ alkyl, $C_{1-4}$ haloalkyl, $C_{1-4}$ deuterioalkyl, $C_{2-4}$ alkenyl, $C_{2-4}$ alkynyl, $C_{1-4}$ alkoxy, $C_{3-6}$ cycloalkyl, $C_{3-6}$ cycloalkoxy, 3-6 membered heterocyclyl, 3-6 membered heterocyclyloxy and $-NR_{12}R_{13}$;

each $R_5$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

$R_6$, $R_7$ and $R_8$ are each independently selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, - $NR_{12}R_{13}$, $-C(O)NR_{12}R_{13}$ and $-N(R_{12})-C(O)R_{11}$;

o is 0, 1, 2 or 3;

m1 is 0, 1 or 2;

wherein, $R_9$, $R_{10}$, $R_{11}$, $R_{12}$, $R_{13}$, n and r are defined as in claim 1.

**5.** The compound of formula (I), the stereoisomer or pharmaceutically acceptable salt thereof according to claim 4, wherein ring B, together with a moiety to which it is directly attached thereto, forms the following structure:

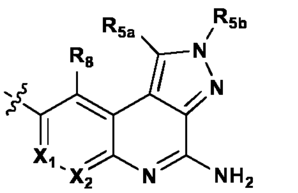
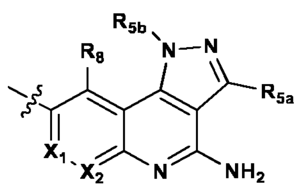
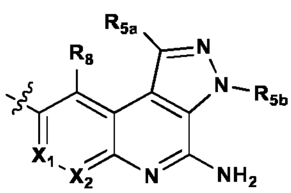
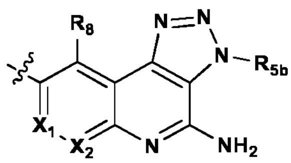

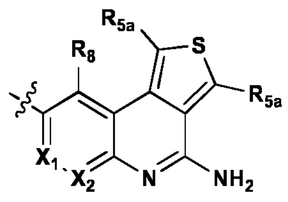
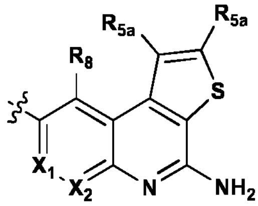
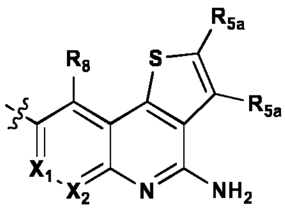
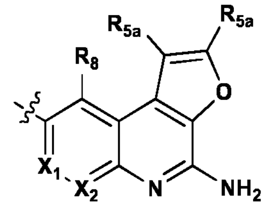

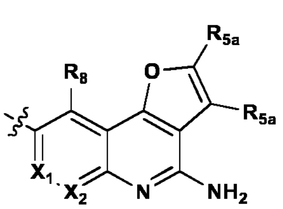
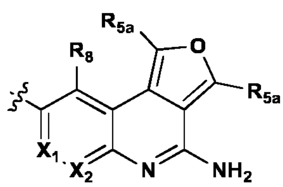
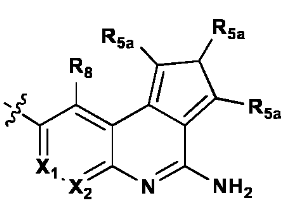
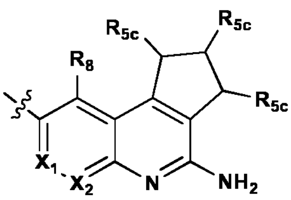

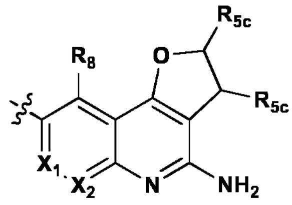
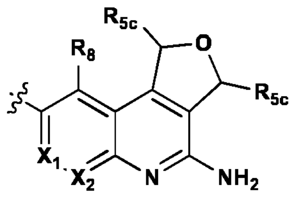
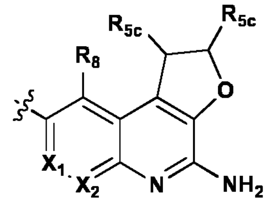
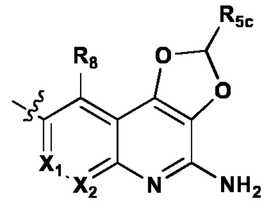

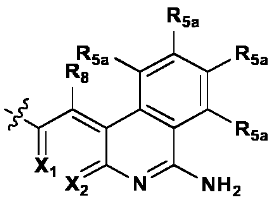
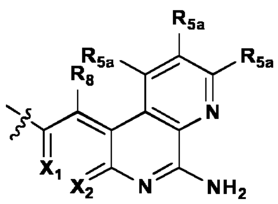
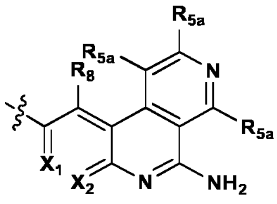
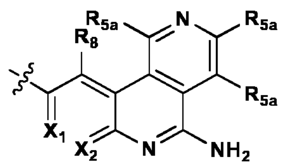

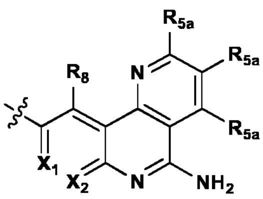
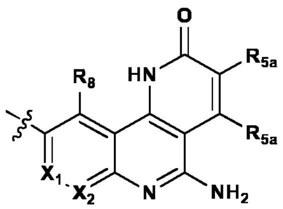
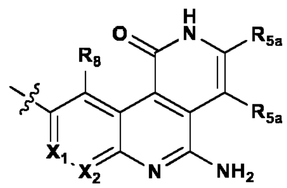
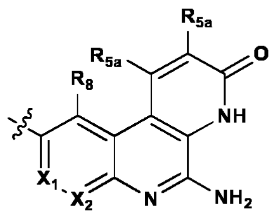

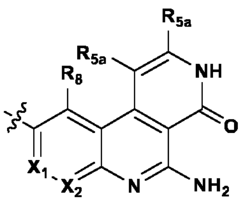
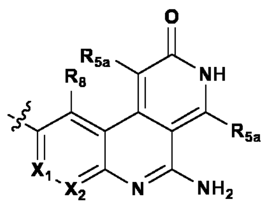
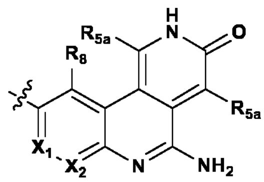
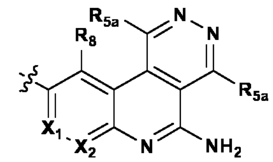

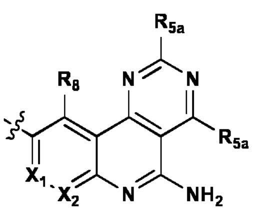
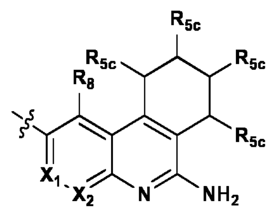
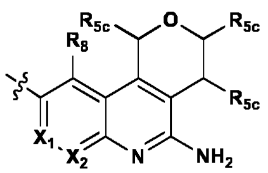
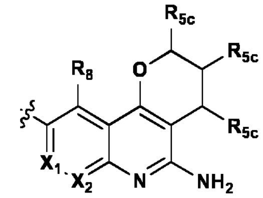

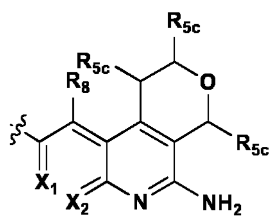
or
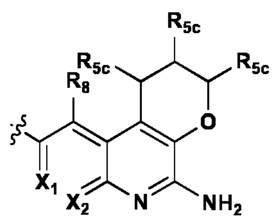
;

wherein, each $R_{5a}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

each $R_{5b}$ is independently selected from the group consisting of hydrogen, deuterium, $C_{1-4}$ alkyl, $C_{3-6}$ cycloalkyl, 3-6 membered heterocyclyl and $-O-R_{10}$, and above groups are independently optionally further substituted by

one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-O-S(O)_2R_9$, $-S(O)_rR_9$, $-O-R_{10}$, $-C(O)OR_{10}$, $-C(O)SR_{10}$, $-S-C(O)R_{11}$, $-C(O)R_{11}$, $-O-C(O)R_{11}$, $-P(O)(R_{11})_2$, $-NR_{12}R_{13}$, $-C(O)NR_{12}R_{13}$ and $-N(R_{12})-C(O)R_{11}$;

$X_1$ is $CR_6$ or N, and each $R_6$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

$X_2$ is $CR_7$ or N, and each $R_7$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

each $R_8$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;

wherein, $R_9$, $R_{10}$, $R_{11}$, $R_{12}$, $R_{13}$ and r are defined as in claim 4.

**6.** The compound of formula (I), the stereoisomer or pharmaceutically acceptable salt thereof according to claim 1, wherein the compound of formula (I) is a compound of formula (III):

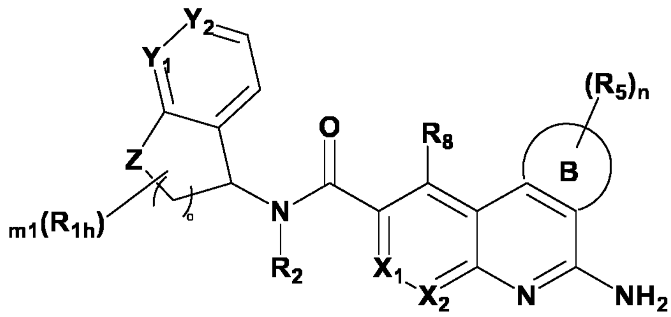

(III)

,

wherein, $X_1$ is $CR_6$ or N; $X_2$ is $CR_7$ or N;

$Y_1$ is $CR_{1a}$ or N; $Y_2$ is $CR_{1b}$ or N;

Z is $-C(R_{1e}R_{1f})-$, $-C(R_{1e}R_{1f})O-$, $-N(R_{1g})-$ or -O-;

ring B, together with a moiety to which it is directly attached thereto, forms the following structure:

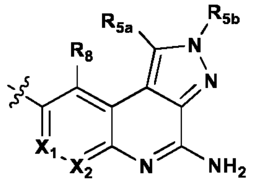
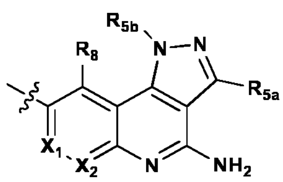
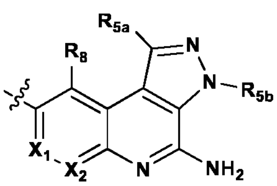
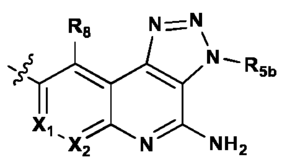

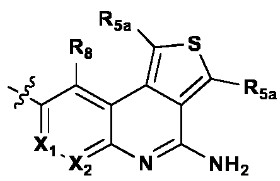
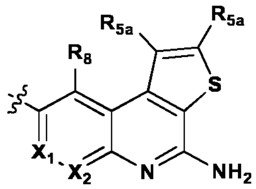
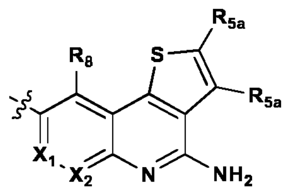
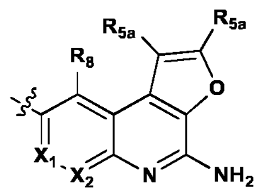

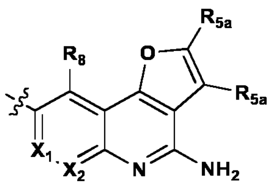
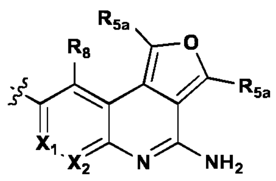
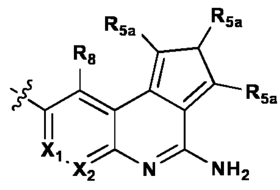
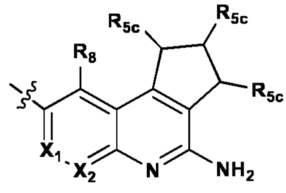
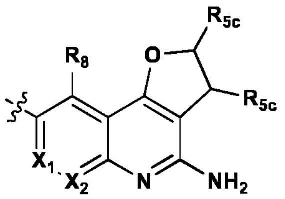
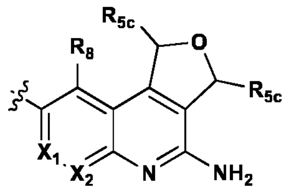
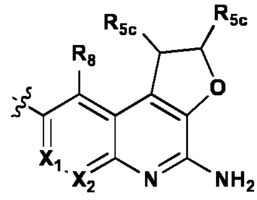
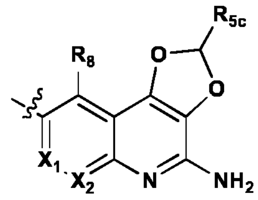
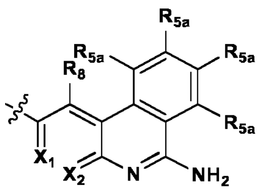
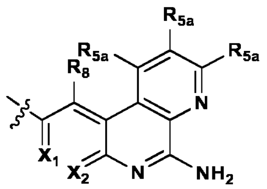
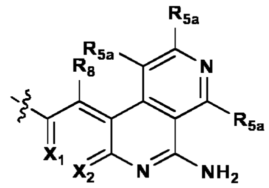
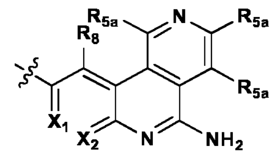
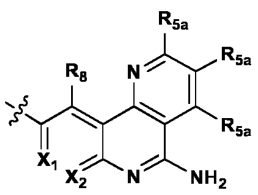
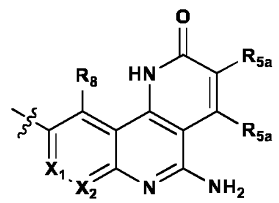
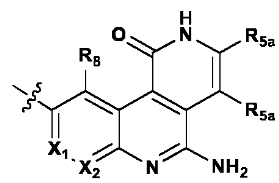
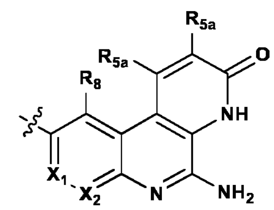
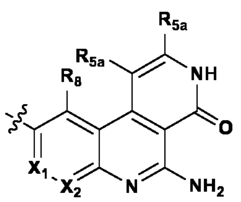
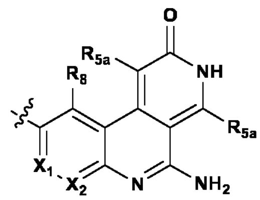
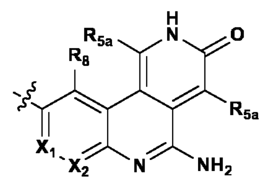
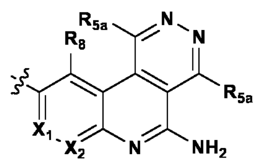
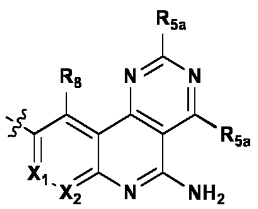
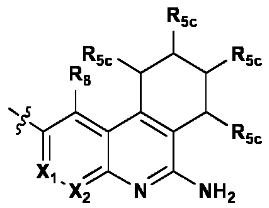
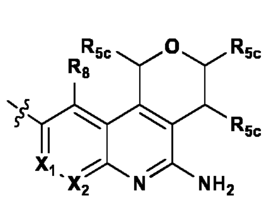
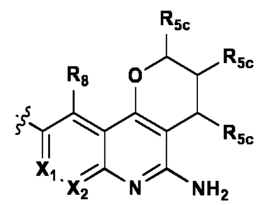
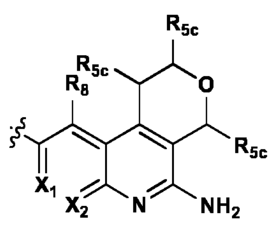
or
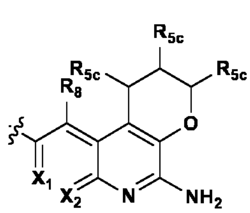
;

$R_{1a}$ and $R_{1b}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered

heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

$R_{1e}$ and $R_{1f}$ are each independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

$R_{1g}$ is selected from the group consisting of hydrogen, deuterium, $C_{1-4}$ alkyl, $C_{3-6}$ cycloalkyl, 3-6 membered heterocyclyl, hydroxy, $C_{1-4}$ alkoxy and $-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

each $R_{1h}$ is independently selected from the group consisting of hydrogen, deuterium, halogen, cyano, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

$R_2$ is selected from the group consisting of hydrogen, deuterium, $C_{1-4}$ alkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-OR_{10}$ and $-C(O)R_{11}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

each $R_{5a}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

each $R_{5b}$ is independently selected from the group consisting of hydrogen, deuterium, $C_{1-4}$ alkyl, $C_{3-6}$ cycloalkyl, 3-6 membered heterocyclyl and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

$R_6$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$;

$R_7$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$;

$R_8$ is selected from the group consisting of hydrogen, deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, $-SF_5$ and $-O-R_{10}$;

o is 1 or 2;

m1 is 0, 1 or 2;
wherein, $R_9$, $R_{10}$, $R_{11}$, $R_{12}$, $R_{13}$, n and r are defined as in claim 1.

**7.** The compound of formula (I), the stereoisomer or pharmaceutically acceptable salt thereof according to claim 6, wherein $Y_1$ is CH or N; $Y_2$ is $CR_{1b}$;

$R_{1b}$ is selected from the group consisting of hydrogen, deuterium, halogen, cyano, $C_{1-4}$ alkyl, $C_{3-6}$ cycloalkyl, 3-6 membered heterocyclyl, $C_{6-8}$ aryl, 5-8 membered heteroaryl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
wherein, $R_{10}$ is defined as in claim 6.

**8.** The compound of formula (I), the stereoisomer or pharmaceutically acceptable salt thereof according to claim 6, wherein $R_{1e}$ and $R_{1f}$ are each independently selected from the group consisting of hydrogen, deuterium, halogen, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;

$R_{1g}$ is selected from the group consisting of hydrogen, deuterium, $C_{1-4}$ alkyl, $C_{3-6}$ cycloalkyl, 3-6 membered heterocyclyl, hydroxy, methoxy and acetyl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
each $R_{1h}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
wherein, $R_{10}$ is defined as in claim 6.

**9.** The compound of formula (I), the stereoisomer or pharmaceutically acceptable salt thereof according to claim 6, wherein each $X_1$ is independently $CR_6$ or N; each $X_2$ is independently CH;

each $R_6$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;
each $R_8$ is independently selected from the group consisting of hydrogen, deuterium, halogen, cyano, $C_{1-4}$ alkyl, $C_{1-4}$ haloalkyl, $C_{1-4}$ deuterioalkyl, $C_{3-6}$ cycloalkyl, 3-6 membered heterocyclyl, $-SF_5$ and $-O-R_{10}$;
wherein, $R_{10}$ is defined as in claim 6.

**10.** The compound of formula (I), the stereoisomer or pharmaceutically acceptable salt thereof according to claim 6, wherein each $R_{5a}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;

each $R_{5b}$ is independently selected from the group consisting of hydrogen, deuterium, $C_{1-4}$ alkyl, $C_{3-6}$ cycloalkyl, 3-6 membered heterocyclyl and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;
each $R_{5c}$ is independently selected from the group consisting of hydrogen, deuterium, halogen, cyano, $C_{1-4}$ alkyl, $C_{3-6}$ cycloalkyl, 3-6 membered heterocyclyl, $-SF_5$ and $-O-R_{10}$, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S and $-SF_5$;

wherein, $R_{10}$ is defined as in claim 6.

**11.** The compound of formula (I), the stereoisomer or pharmaceutically acceptable salt thereof according to claim 6, wherein $R_2$ is selected from the group consisting of hydrogen, deuterium, $C_{1-4}$ alkyl, $C_{3-6}$ cycloalkyl, 3-6 membered heterocyclyl, $C_{6-8}$ aryl and 5-8 membered heteroaryl, and above groups are independently optionally further substituted by one or more substituents selected from the group consisting of deuterium, halogen, cyano, $C_{1-4}$ alkyl, $C_{1-4}$ haloalkyl, $C_{1-4}$ deuterioalkyl, $C_{2-4}$ alkenyl, $C_{2-4}$ alkynyl, $C_{3-6}$ cycloalkyl, 3-6 membered heterocyclyl, $C_{6-8}$ aryl, 5-8 membered heteroaryl, =O, =S, $-SF_5$, $-S(O)_rR_9$, $-O-R_{10}$, $-C(O)OR_{10}$, $-C(O)R_{11}$, $-O-C(O)R_{11}$, $-NR_{12}R_{13}$, $-C(O)NR_{12}R_{13}$ and $-N(R_{12})-C(O)R_{11}$;

**preferably,** $R_2$ is selected from the group consisting of hydrogen, deuterium, methyl, ethyl, n-propyl, isopropyl, n-butyl, isobutyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, sulfinyl, sulfonyl, methanesulfonyl, isopropylsulfonyl, hydroxy, methoxy, ethoxy, n-propoxy, isopropoxy, carboxyl, methoxycarbonyl, ethoxycarbonyl, n-propoxycarbonyl, isopropyloxycarbonyl, formyl, acetyl, n-propionyl, isopropionyl, formyloxy, acetoxy, n-propionyloxy, isopropionyloxy, amino, mono-$C_{1-4}$ alkylamino and di-$C_{1-4}$ alkylamino.

**12.** The compound of formula (I), the stereoisomer or pharmaceutically acceptable salt thereof according to claim 6, wherein

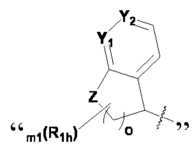

is selected from the following structure:

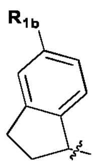 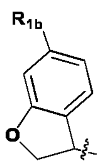 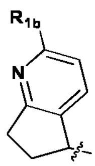 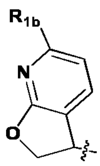 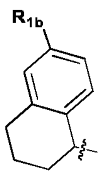 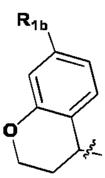 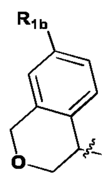 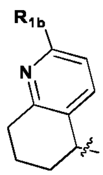 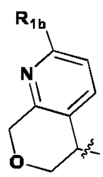 or 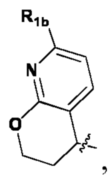,

wherein, each $R_{1b}$ is independently selected from the group consisting of hydrogen, deuterium, fluorine, chlorine, bromine, cyano, methyl, trifluoromethyl, difluoromethyl, trideuteriomethyl, dideuteriomethyl, trifluoromethoxy, difluoromethoxy, trideuteriomethoxy, dideuteriomethoxy, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy, cyclobutoxy, phenyl and methyl-substituted pyrazolyl.

**13.** The compound of formula (I), the stereoisomer or pharmaceutically acceptable salt thereof according to claim 6, wherein

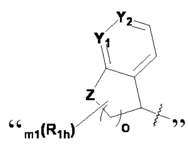

is selected from the following structure:

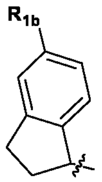 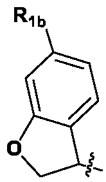 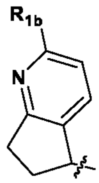 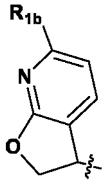 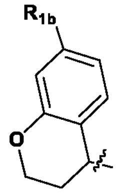 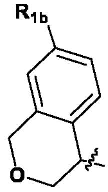 or 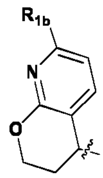,

wherein, each $R_{1b}$ is independently selected from the group consisting of hydrogen, deuterium, fluorine, chlorine, bromine, cyano, methyl, trifluoromethyl, difluoromethyl, trideuteriomethyl, dideuteriomethyl, trifluoromethoxy, difluoromethoxy, trideuteriomethoxy, dideuteriomethoxy, cyclopropyl and methyl-substituted pyrazolyl.

**14.** The compound of formula (I), the stereoisomer or pharmaceutically acceptable salt thereof according to claim 6, wherein the compound of formula (I) is a compound of formula (IVa):

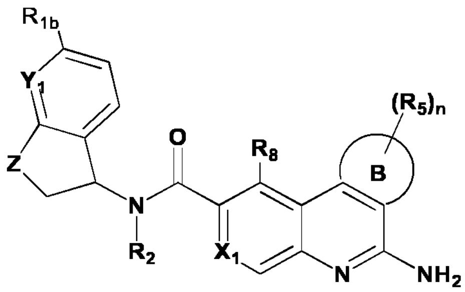

**(IVa)** ,

wherein,

“ 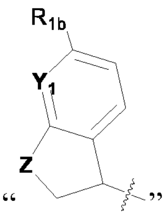 ”

is selected from the following structure:

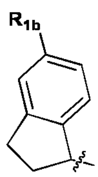 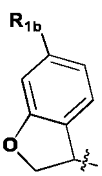 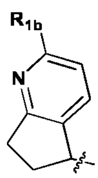

or

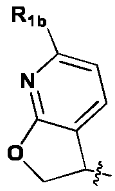,

each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl, and methyl-substituted pyrazolyl;

ring B, together with a moiety

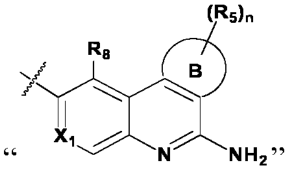

to which it is directly attached thereto, is selected from the following structure:

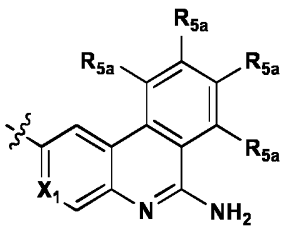
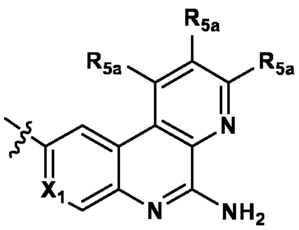
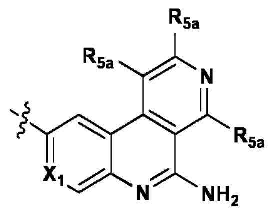
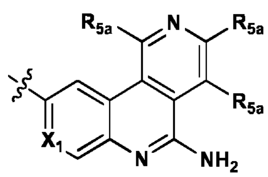

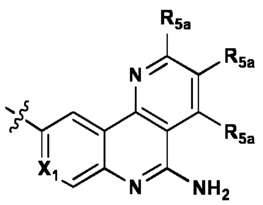
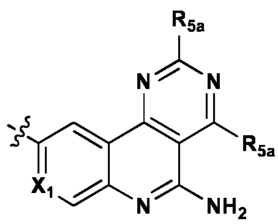
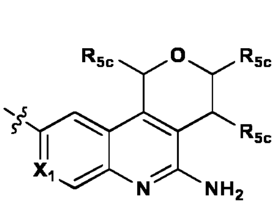
or
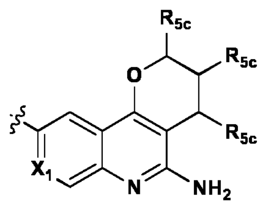
;

each $X_1$ is independently $CR_6$ or N;
each $R_6$ is independently selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;
each $R_{5a}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
each $R_{5c}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
$R_2$ is selected from the group consisting of methyl, ethyl, n-propyl, isopropyl, n-butyl, isobutyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, hydroxy and methoxy; provided that when $R_2$ is methyl,

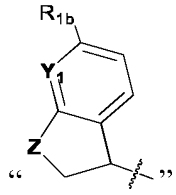

is not

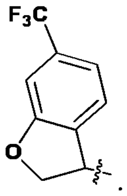

**15.** The compound of formula (I), the stereoisomer or pharmaceutically acceptable salt thereof according to claim 14, wherein each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl and methyl-substituted pyrazolyl;

$R_6$ is selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;
$R_2$ is selected from the group consisting of cyclopropyl-substituted methyl, cyclobutyl-substituted methyl, thiazolyl-substituted methyl, pyrimidinyl-substituted methyl, ethyl, cyclopropyl-substituted ethyl, cyclobutyl-substituted ethyl, 2,2,2-trifluoroethyl, methoxyethyl, thiazolyl-substituted ethyl, pyrimidinyl-substituted ethyl, methoxy-substituted isopropyl, cyclopropyl, methyl-substituted pyrazolyl, thiazolyl, isothiazolyl, thiadiazolyl and pyrimidinyl.

**16.** The compound of formula (I), the stereoisomer or pharmaceutically acceptable salt thereof according to claim 15, wherein the compound of formula (I) is a compound of formula (Va):

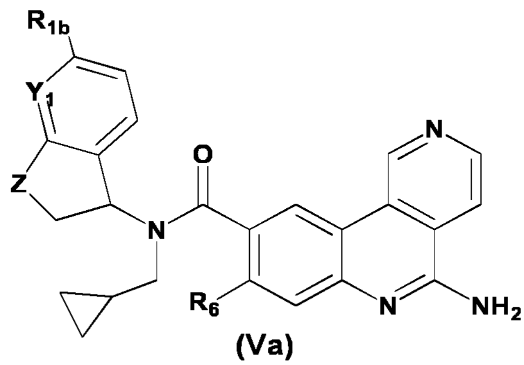

**(Va)**

wherein,

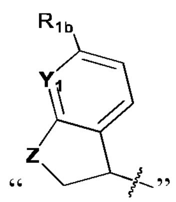

is selected from the following structure:

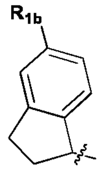 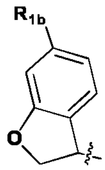 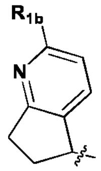

or

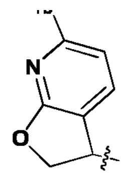,

each $R_{1b}$ is independently selected from the group consisting of hydrogen, deuterium, fluorine, chlorine, methyl, trifluoromethyl, trideuteriomethyl, trifluoromethoxy, trideuteriomethoxy and cyclopropyl;

$R_6$ is selected from the group consisting of hydrogen, deuterium, fluorine, chlorine, methyl, trifluoromethyl, trideuteriomethyl and cyclopropyl.

**17.** The compound of formula (I), the stereoisomer or pharmaceutically acceptable salt thereof according to claim 6, wherein the compound of formula (I) is a compound of formula (IVb):

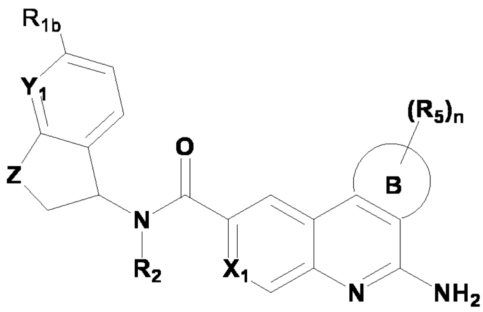

**(IVb)**

wherein,

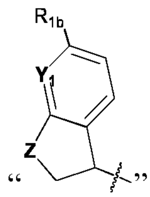

is selected from the following structure:

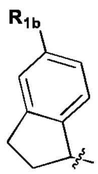 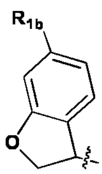 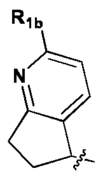

or

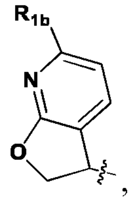,

each $R_{1b}$ is independently selected from the group consisting of hydrogen, deuterium, fluorine, chlorine, bromine, cyano, methyl, trifluoromethyl, trideuteriomethyl, trifluoromethoxy, trideuteriomethoxy, cyclopropyl, and methyl-substituted pyrazolyl;

ring B, together with a moiety

“ 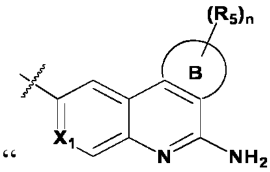 ”

to which it is directly attached thereto, is selected from the following structure:

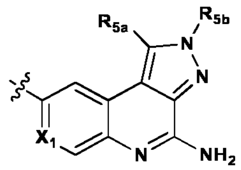 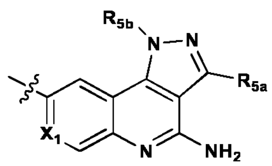 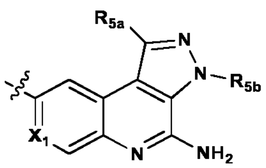

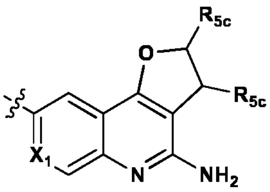 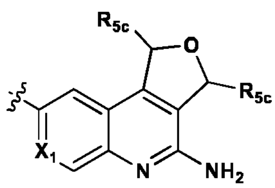 or 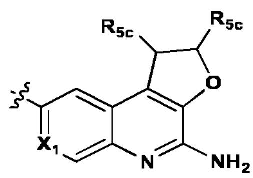;

each $X_1$ is independently $CR_6$ or N;

each $R_6$ is independently selected from the group consisting of hydrogen, deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy;

each $R_{5a}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;

each $R_{5b}$ is independently selected from the group consisting of hydrogen, deuterium, methyl, ethyl, n-propyl, isopropyl, n-butyl, isobutyl, cyclopropyl, cyclobutyl, oxacyclobutyl, azacyclobutyl and hydroxy, and above groups are independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;

each $R_{5c}$ is independently selected from the group consisting of hydrogen, deuterium, fluorine, chlorine, bromine, cyano, methyl, ethyl, n-propyl, isopropyl, n-butyl, isobutyl, cyclopropyl, cyclobutyl, oxacyclobutyl, azacyclobutyl, $-SF_5$, hydroxy, methoxy, ethoxy, n-propoxy, isopropoxy, cyclopropoxy and cyclobutoxy, and above groups are independently optionally further substituted by one or more substituents selected from the group consisting of

deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, vinyl, ethynyl, cyclopropyl, cyclobutyl, oxacyclobutyl, azacyclobutyl, phenyl, =O, =S and $-SF_5$;
wherein, $R_2$ is defined as in claim 6.

**18.** The compound of formula (I), the stereoisomer or pharmaceutically acceptable salt thereof according to claim 17, wherein ring B, together with a moiety

“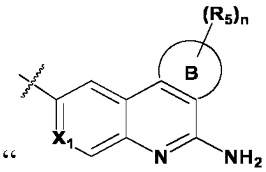”

to which it is directly attached thereto, is selected from the following structure:

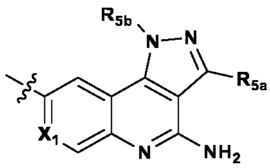 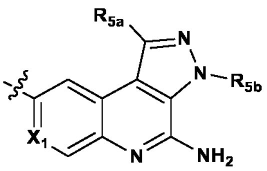 or 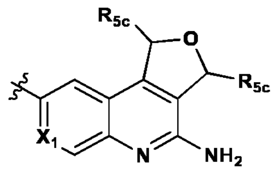 ;

each $X_1$ is independently $CR_6$; each $R_6$ is independently selected from the group consisting of hydrogen, deuterium, fluorine, chlorine, bromine, cyano, methyl, trifluoromethyl, trideuteriomethyl and cyclopropyl;
each $R_{5a}$ is independently selected from the group consisting of hydrogen, deuterium, fluorine, chlorine, bromine, cyano, methyl, trifluoromethyl, trideuteriomethyl and cyclopropyl;
each $R_{5b}$ is independently selected from the group consisting of hydrogen, deuterium, methyl, trifluoromethyl, trideuteriomethyl and cyclopropyl;
each $R_{5c}$ is independently selected from the group consisting of hydrogen, deuterium, fluorine, chlorine, bromine, cyano, methyl, trifluoromethyl, trideuteriomethyl and cyclopropyl.

**19.** The compound of formula (I), the stereoisomer or pharmaceutically acceptable salt thereof according to claim 17, wherein $R_2$ is selected from the group consisting of substituted methyl, substituted ethyl, substituted or unsubstituted n-propyl, substituted isopropyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl,

and the substituents of the substituted methyl, substituted ethyl, substituted n-propyl and substituted isopropyl are each independently one or more substituents selected from the group consisting of fluorine, chlorine, bromine, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, $-SF_5$, hydroxy and methoxy,
and the bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl and pyrimidinyl are each independently optionally further substituted by one or more substituents selected from the group consisting of deuterium, fluorine, chlorine, bromine, cyano, methyl, ethyl, n-propyl, isopropyl, n-butyl, isobutyl, trifluoromethyl, difluoromethyl, trideuteriomethyl, dideuteriomethyl, cyclopropyl, cyclobutyl, bicyclo[1.1.1]pentyl, oxacyclobutyl, azacyclobutyl, phenyl, pyrazolyl, imidazolyl, triazolyl, oxazolyl, thiazolyl, isothiazolyl, thiadiazolyl, pyrimidinyl, =O, =S, $-SF_5$, hydroxy and methoxy.

**20.** The compound of formula (I), the stereoisomer or pharmaceutically acceptable salt thereof according to claim 17, wherein $R_2$ is selected from the group consisting of substituted methyl, substituted ethyl, substituted or unsubstituted n-propyl, substituted isopropyl, phenyl, pyrazolyl, imidazolyl, triazolyl, oxazolyl, thiazolyl, isothiazolyl, thiadiazolyl and pyrimidinyl,

and the substituents of the substituted methyl, substituted ethyl, substituted n-propyl and substituted isopropyl are each independently one or more substituents selected from the group consisting of fluorine, chlorine, bromine, pyrazolyl, imidazolyl, triazolyl, oxazolyl, thiazolyl, isothiazolyl, thiadiazolyl, pyrimidinyl and methoxy,
and the phenyl, pyrazolyl, imidazolyl, triazolyl, oxazolyl, thiazolyl, isothiazolyl, thiadiazolyl and pyrimidinyl are

each independently optionally further substituted by one or more substituents selected from the group consisting of deuterium, fluorine, chlorine, bromine, cyano, methyl, ethyl, trifluoromethyl, difluoromethyl, trideuteriomethyl, dideuteriomethyl, cyclopropyl, $-SF_5$, hydroxy and methoxy.

**21.** The compound of formula (I), the stereoisomer or pharmaceutically acceptable salt thereof according to claim 17, wherein $R_2$ is selected from the group consisting of trifluoromethyl, pyrazolyl-substituted methyl, imidazolyl-substituted methyl, triazolyl-substituted methyl, oxazolyl-substituted methyl, thiazolyl-substituted methyl, isothiazolyl-substituted methyl, thiadiazolyl-substituted methyl, pyrimidinyl-substituted methyl, fluorine-substituted ethyl, methoxyethyl, pyrazolyl-substituted ethyl, imidazolyl-substituted ethyl, triazolyl-substituted ethyl, oxazolyl-substituted ethyl, thiazolyl-substituted ethyl, isothiazolyl-substituted ethyl, thiadiazolyl-substituted ethyl, pyrimidinyl-substituted ethyl, methoxy-substituted isopropyl, pyrazolyl, methyl-substituted pyrazolyl, imidazolyl, methyl-substituted imidazolyl, triazolyl, methyl-substituted triazolyl, oxazolyl, methyl-substituted oxazolyl, thiazolyl, methyl-substituted thiazolyl, isothiazolyl, methyl-substituted isothiazolyl, thiadiazolyl, methyl-substituted thiadiazolyl, pyrimidinyl and methyl-substituted pyrimidinyl.

**22.** The compound of formula (I), the stereoisomer or pharmaceutically acceptable salt thereof according to claim 17, wherein $R_2$ is selected from the group consisting of thiazolyl-substituted methyl, pyrimidinyl-substituted methyl, 2,2,2-trifluoroethyl, methoxyethyl, thiazolyl-substituted ethyl, pyrimidinyl-substituted ethyl, methoxy-substituted isopropyl, methyl-substituted pyrazolyl, thiazolyl, isothiazolyl, thiadiazolyl and pyrimidinyl.

**23.** The compound of formula (I), the stereoisomer or pharmaceutically acceptable salt thereof according to claim 17, wherein $R_2$ is selected from the following structure:

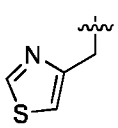 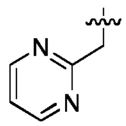 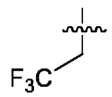 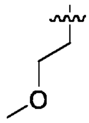 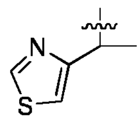 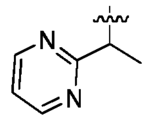 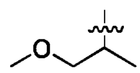

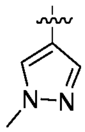 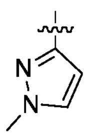 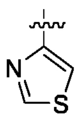 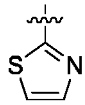 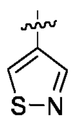 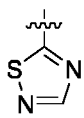 or 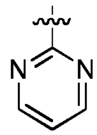 .

**24.** The compound of formula (I), the stereoisomer or pharmaceutically acceptable salt thereof according to claim 1, wherein the compound of formula (I) is a compound of formula (Vb1), formula (Vb2), formula (Vb3) or formula (Vb4):

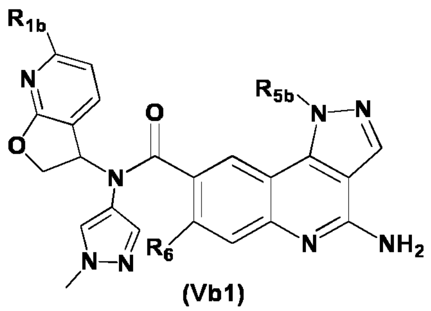
**(Vb1)**

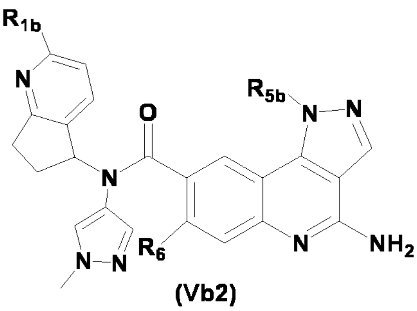
**(Vb2)**

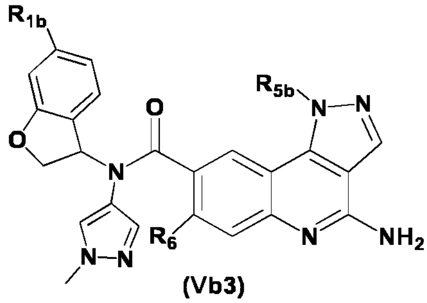

(Vb3) or

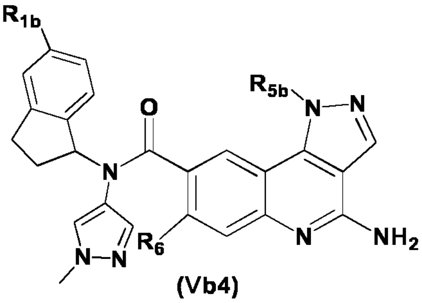

(Vb4) ,

each $R_{1b}$ is independently selected from the group consisting of hydrogen, deuterium, methyl, trifluoromethyl, trideuteriomethyl, trifluoromethoxy, trideuteriomethoxy and cyclopropyl;
each $R_6$ is independently selected from the group consisting of hydrogen, deuterium, fluorine, chlorine, bromine, cyano and methyl;
each $R_{5b}$ is independently selected from the group consisting of hydrogen, deuterium, methyl, trifluoromethyl, trideuteriomethyl and cyclopropyl.

**25.** The compound of formula (I), the stereoisomer or pharmaceutically acceptable salt thereof according to any one of claims 1-24, wherein it is selected from the following compounds:

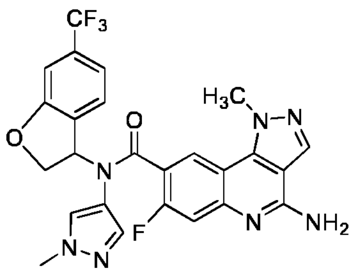
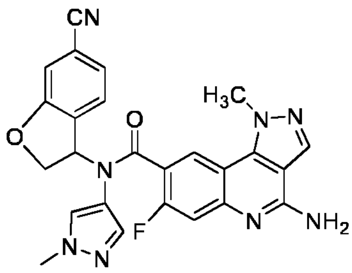
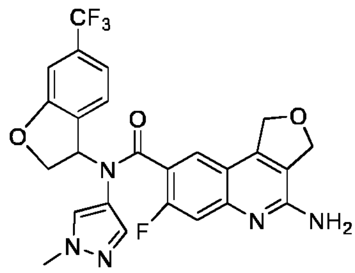

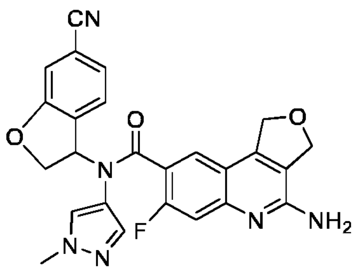
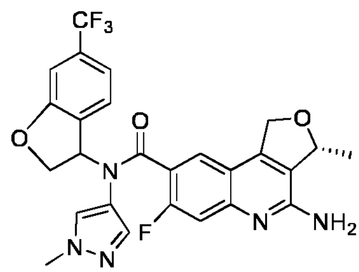
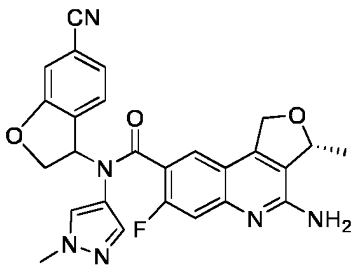

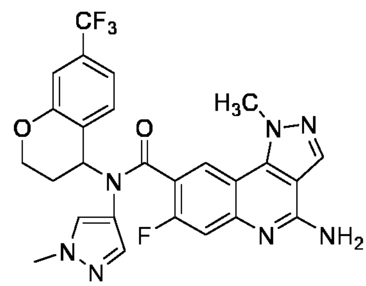
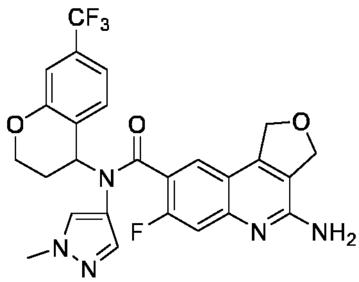
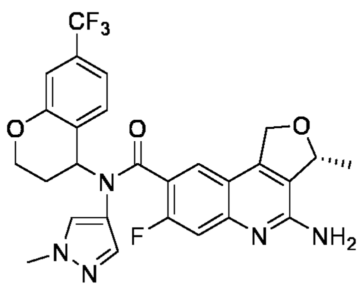

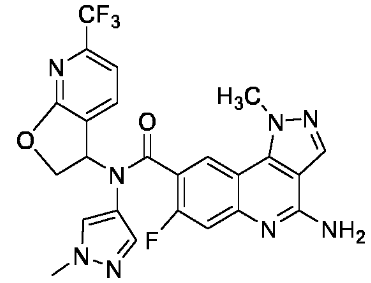

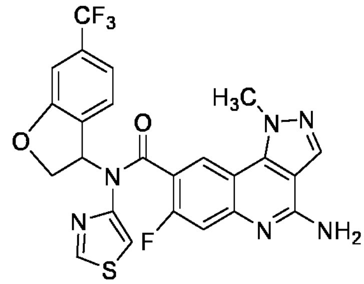

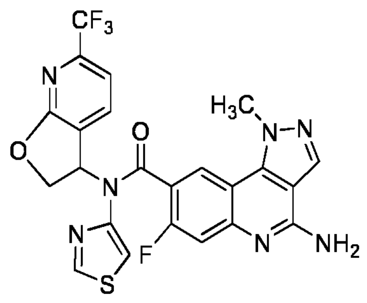

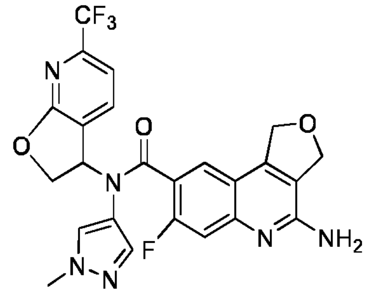

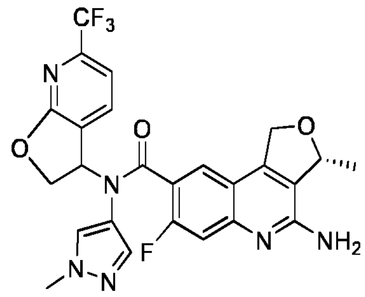

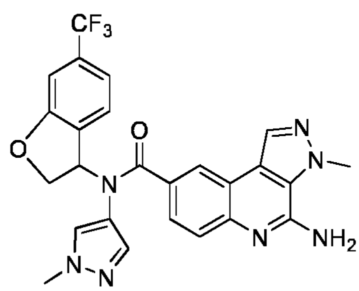

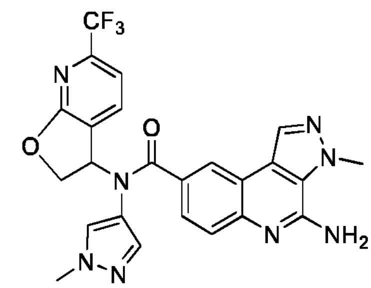

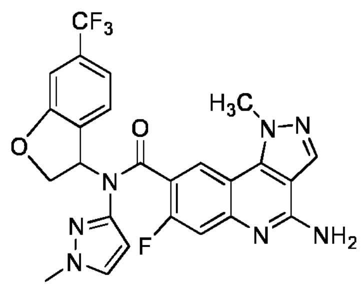

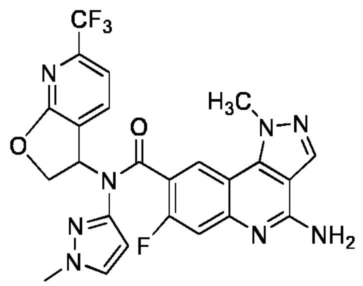

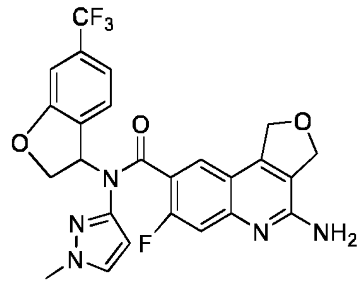

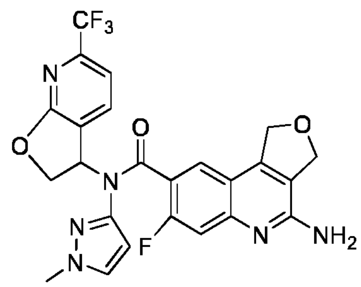

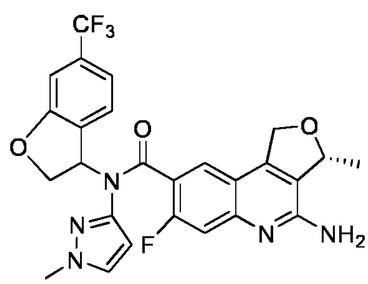

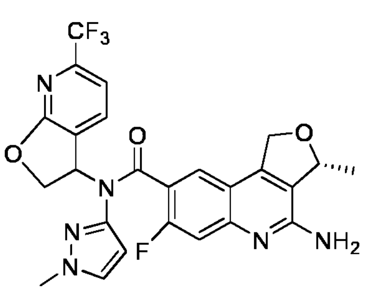

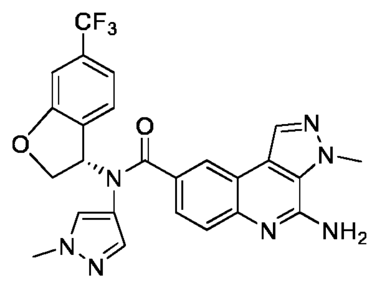

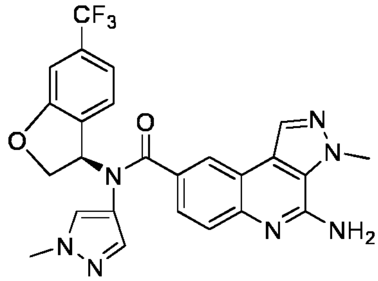
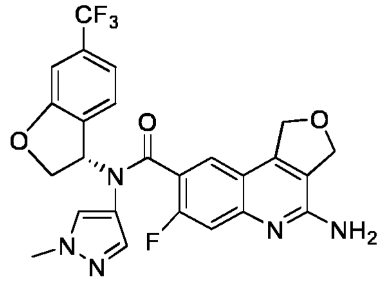

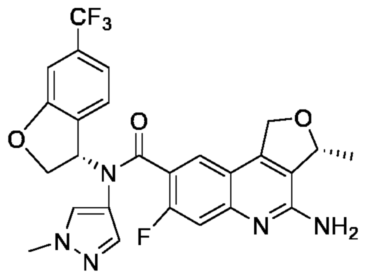
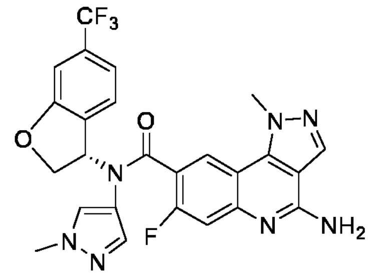
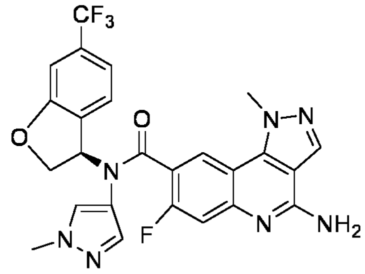

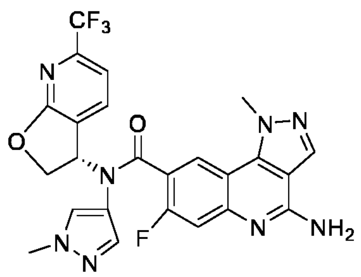
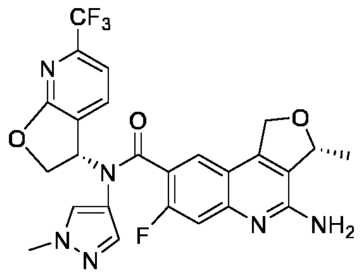
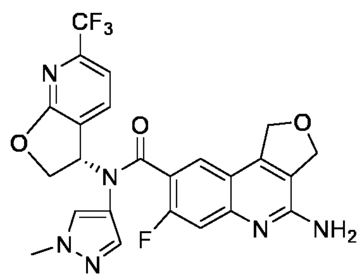

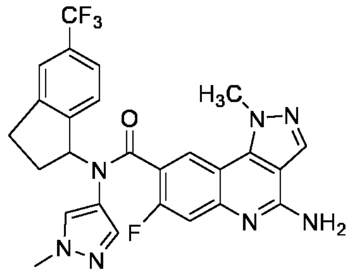
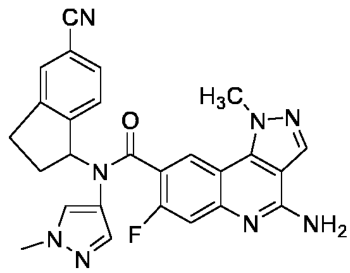
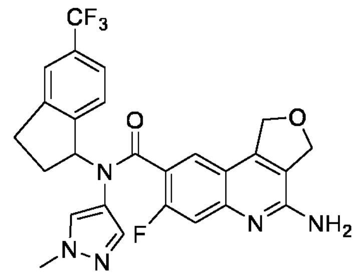

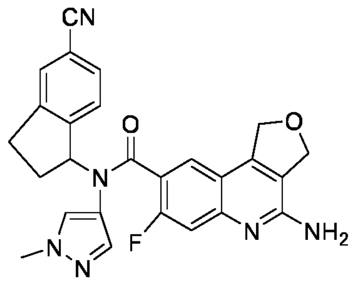
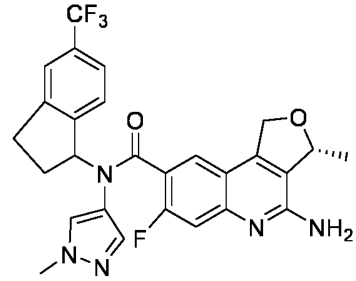
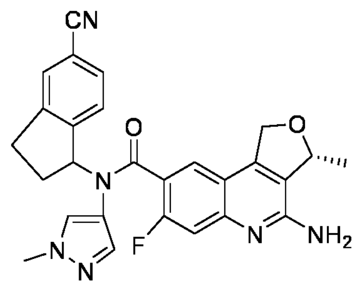

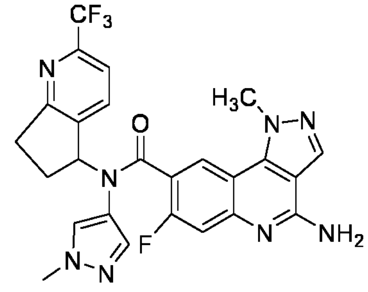
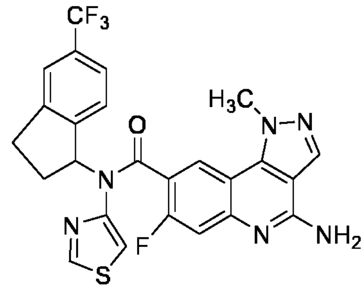

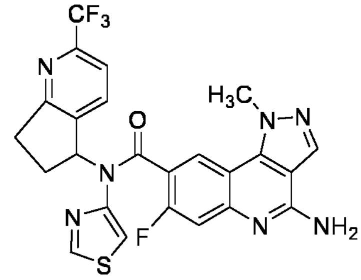
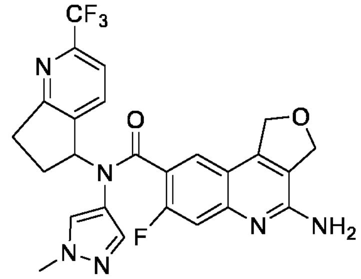
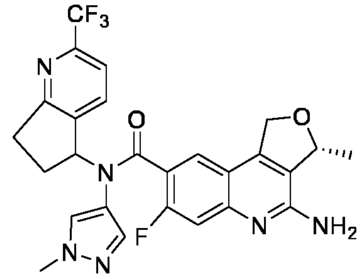

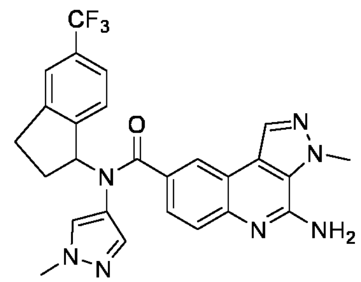
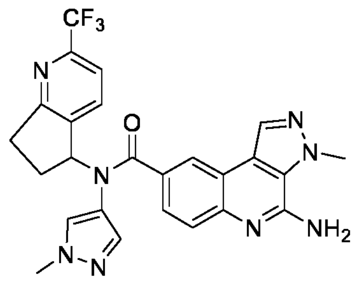
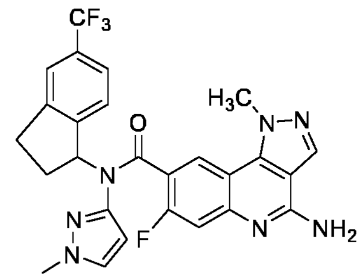

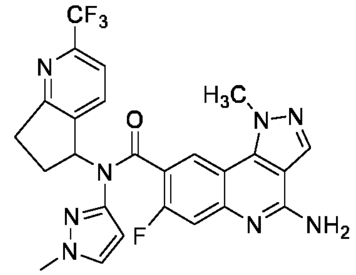
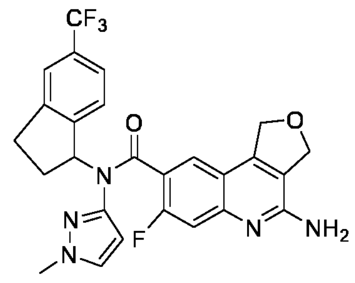
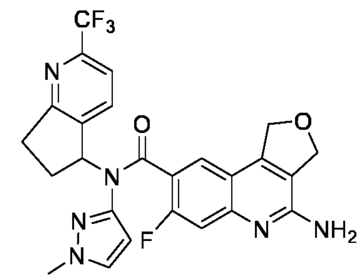

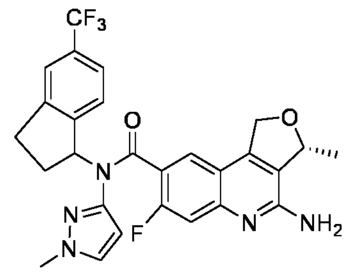
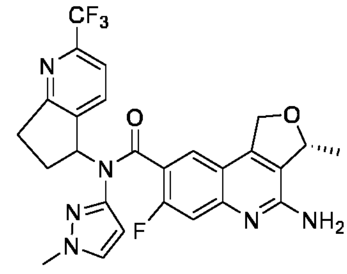
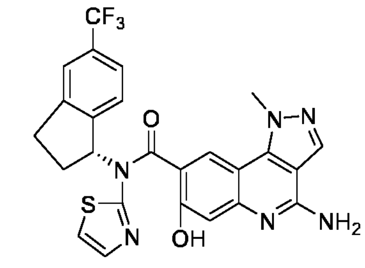

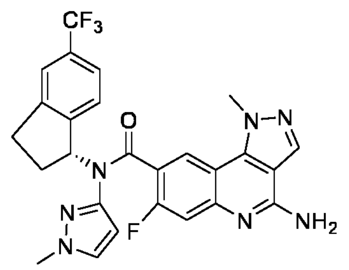
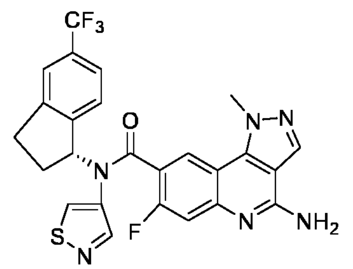
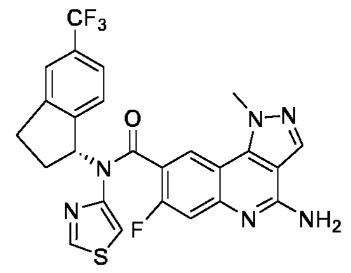

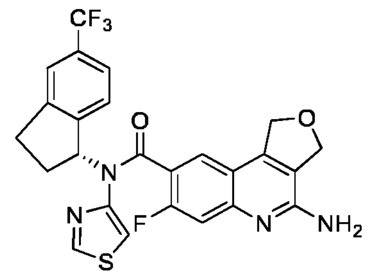
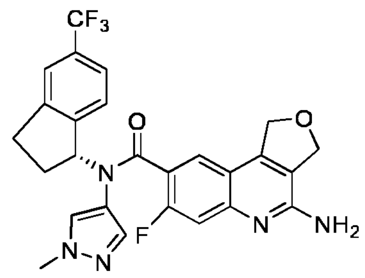

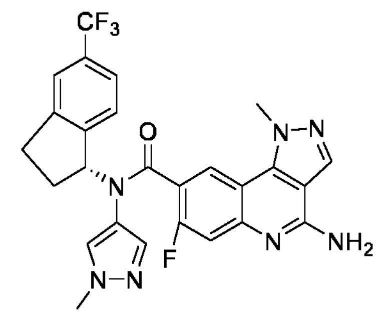
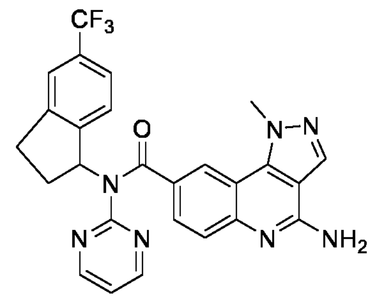

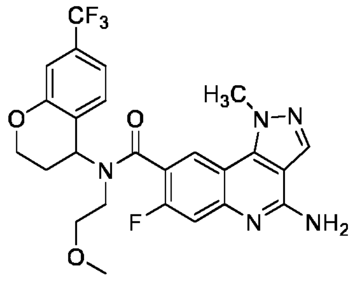
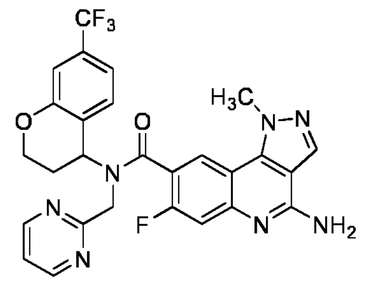
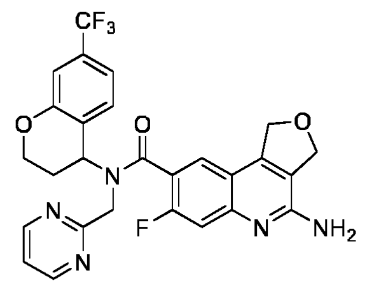

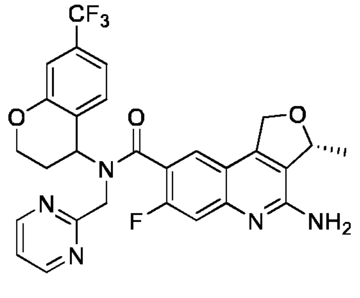
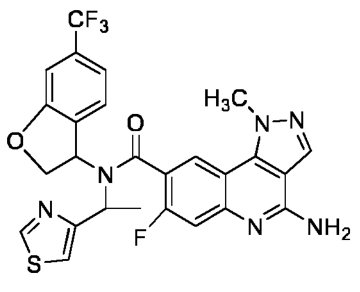
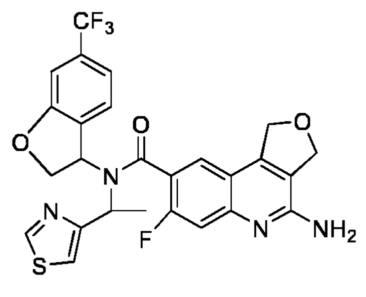

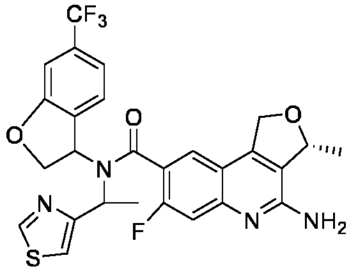

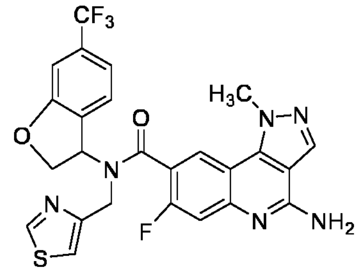

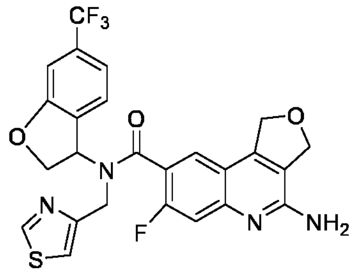

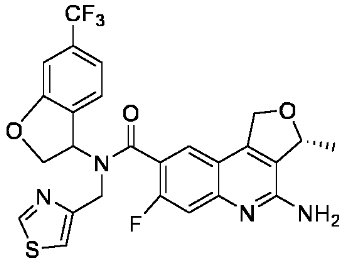

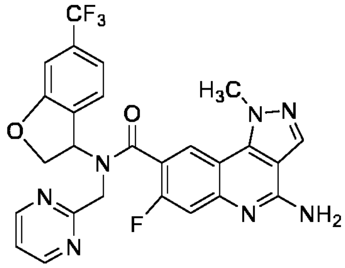

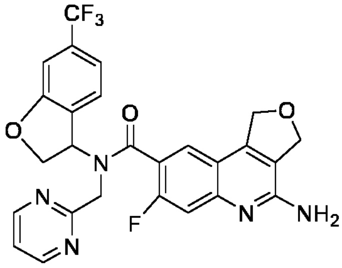

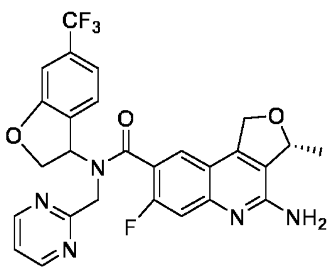

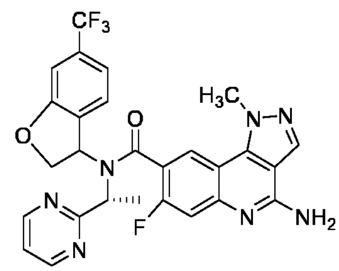

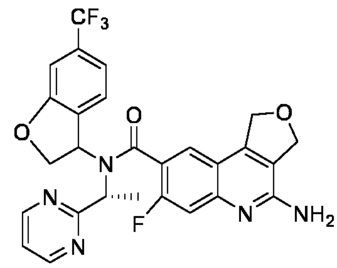

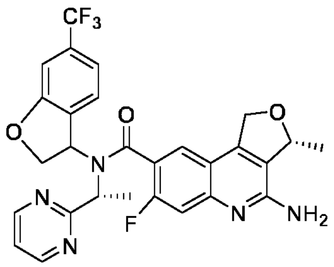

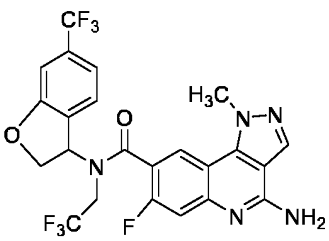

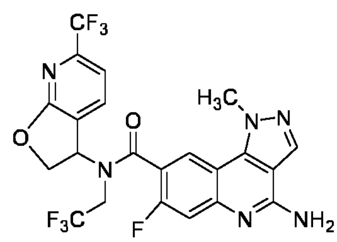

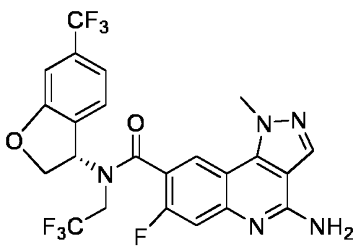

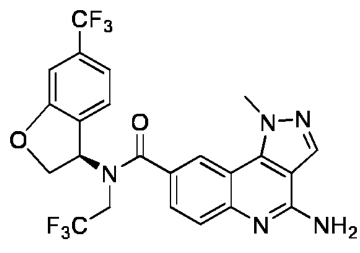

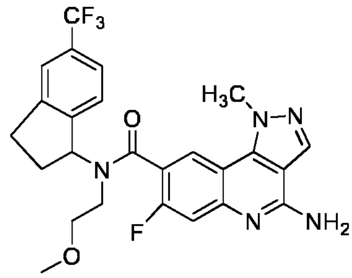

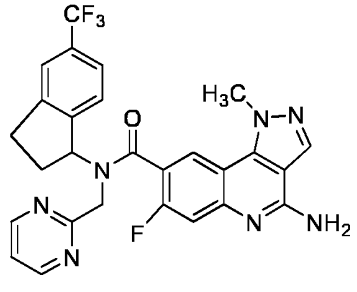
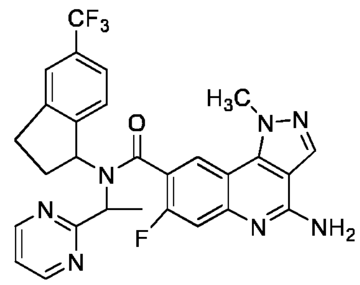
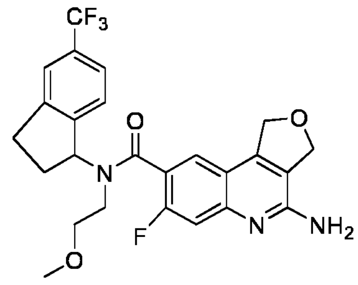

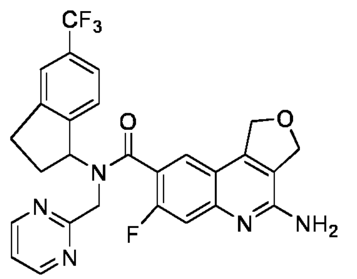
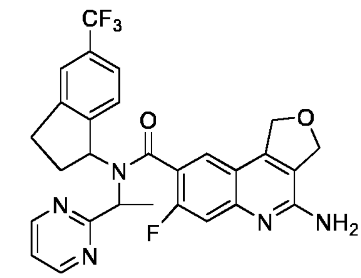
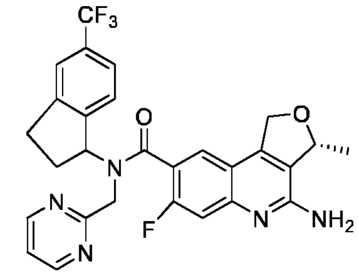

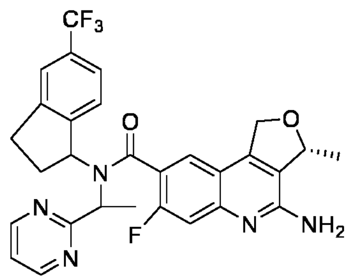
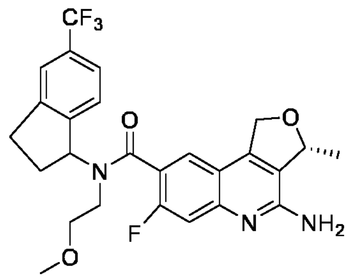
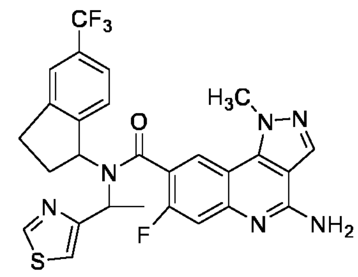

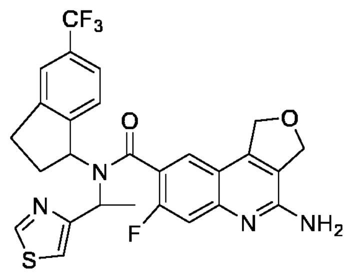
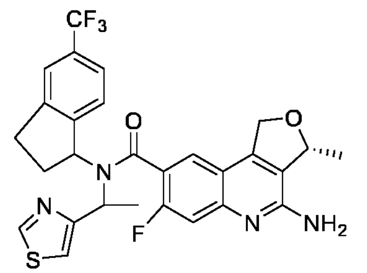
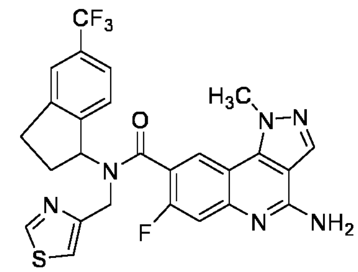

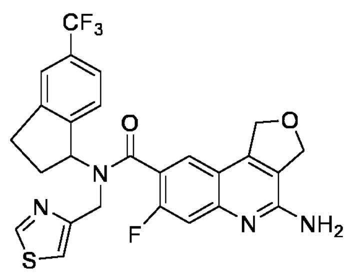
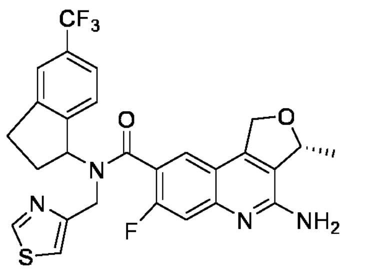
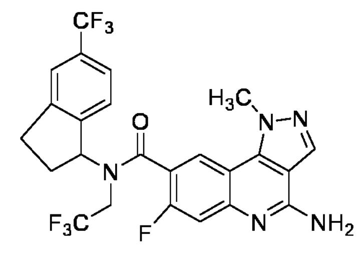

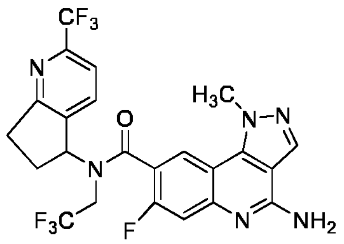
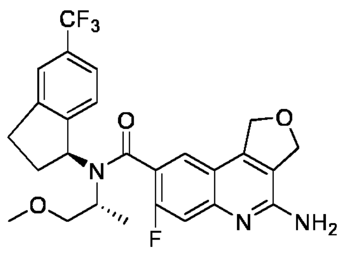
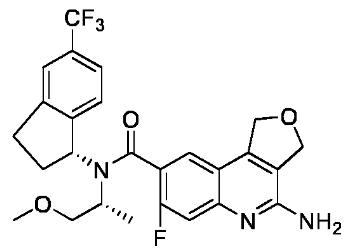

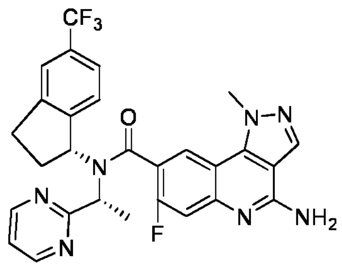
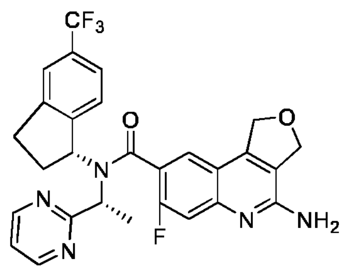
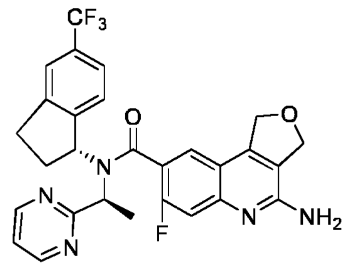

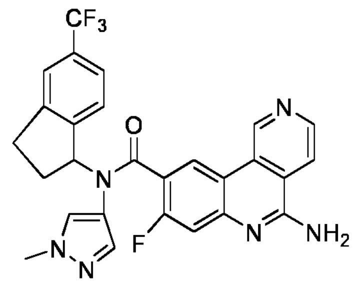
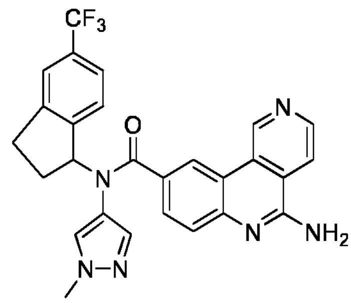
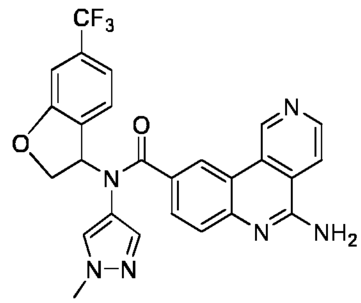

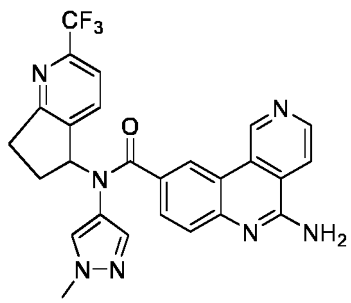
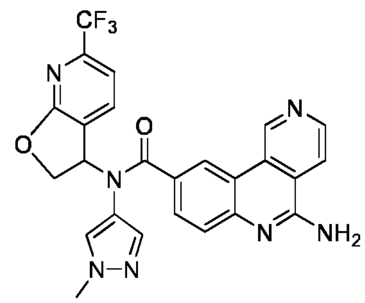
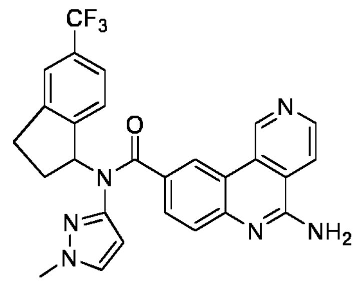

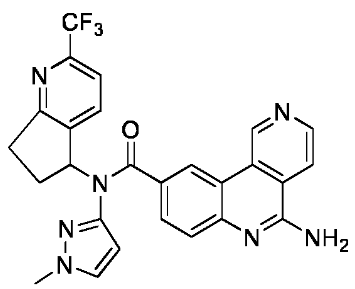
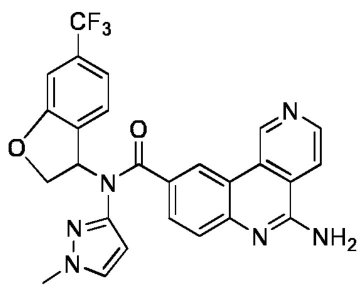
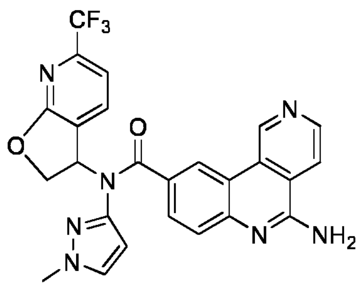

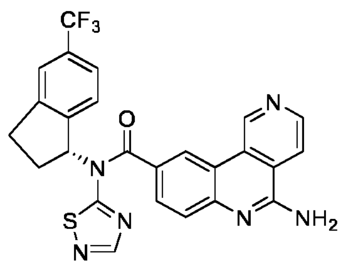
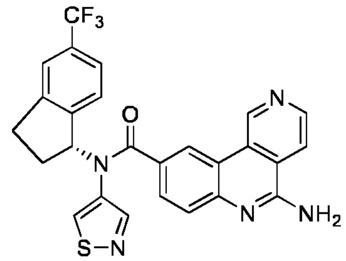
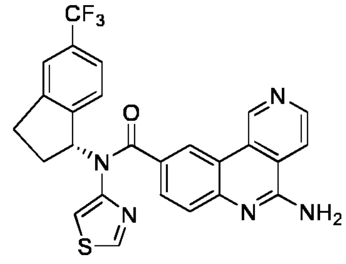

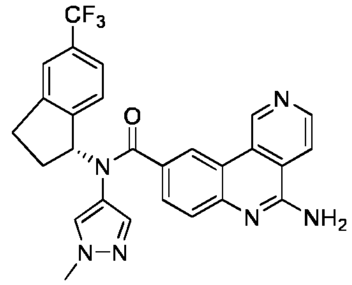
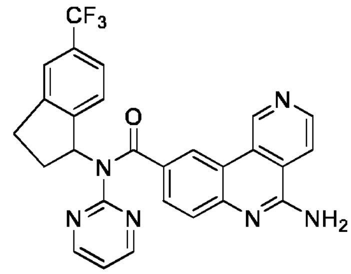

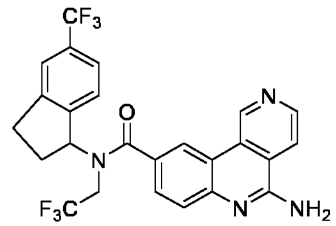
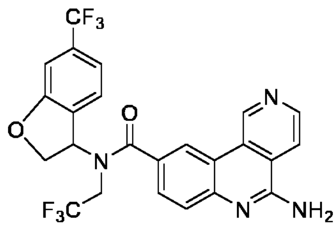
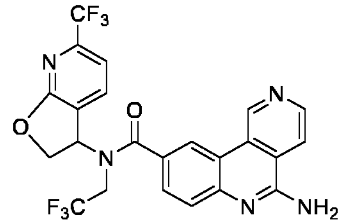

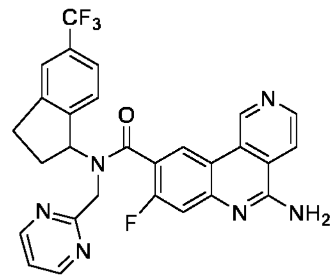
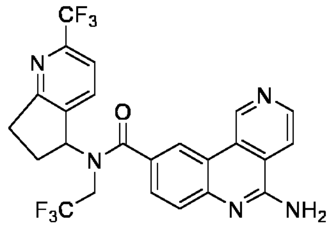
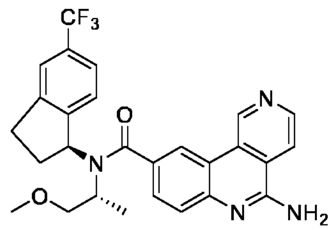

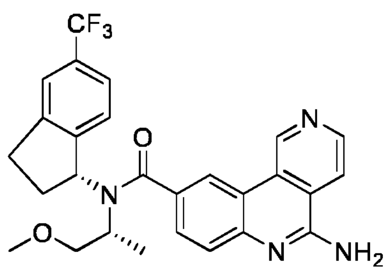
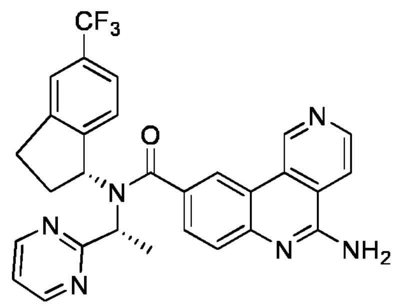

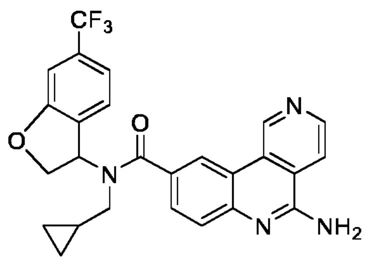

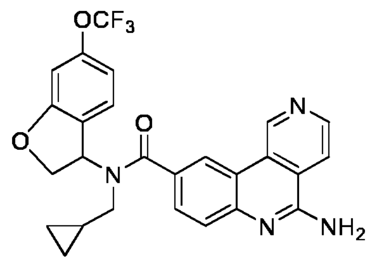

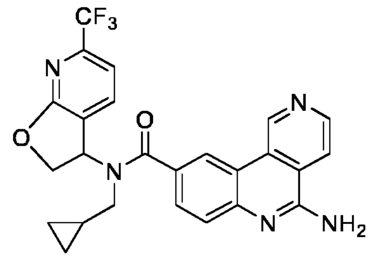

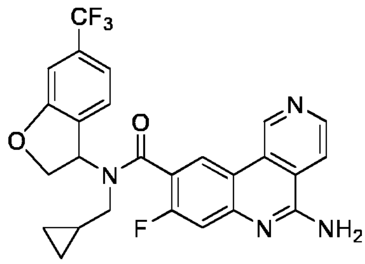

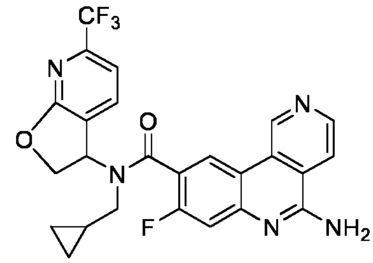

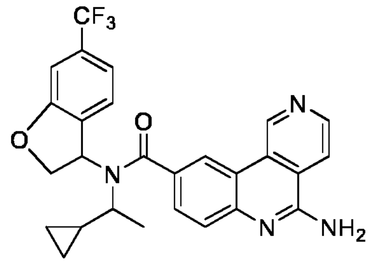

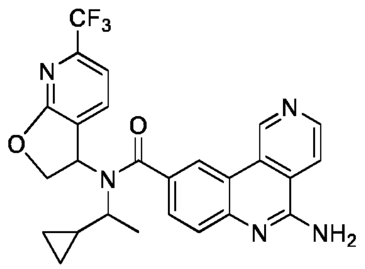

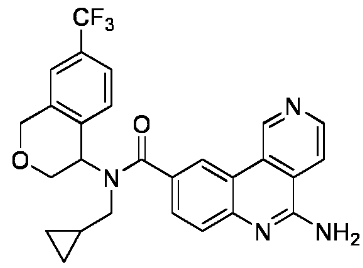

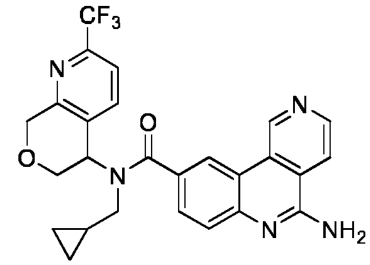

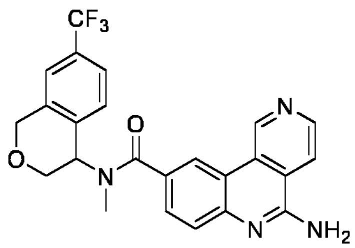

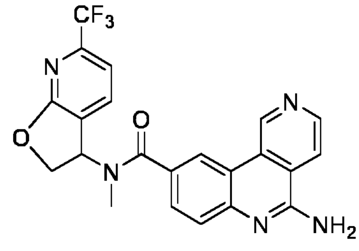

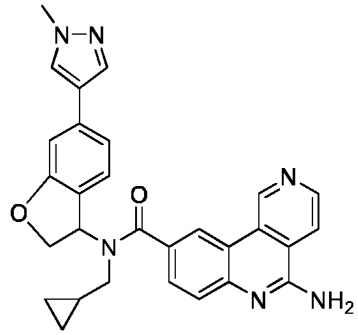

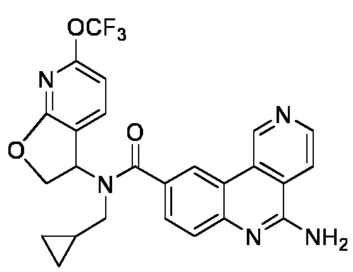

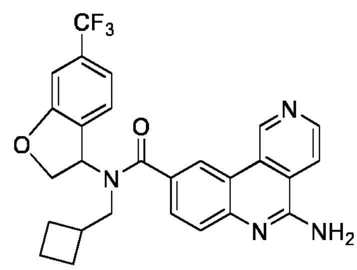

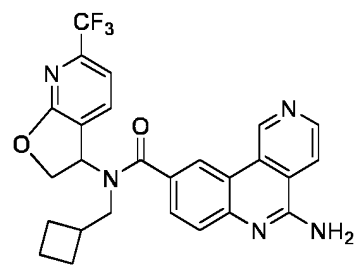

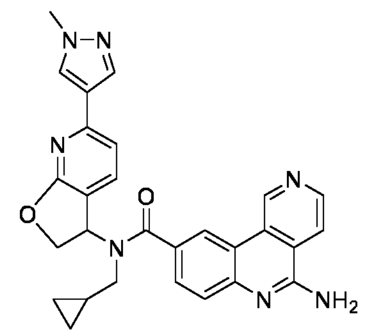

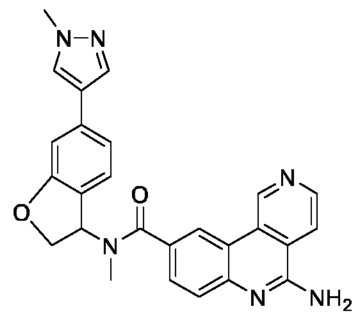

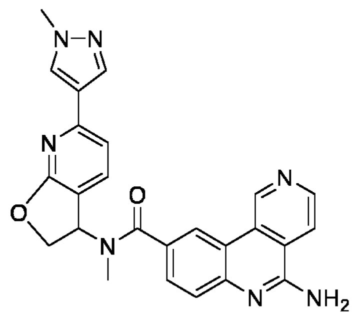

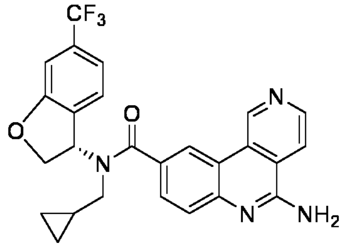

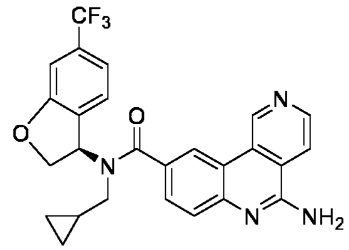

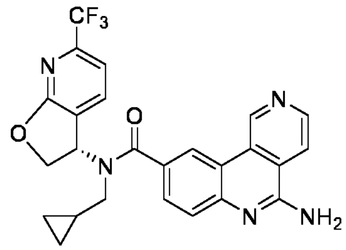

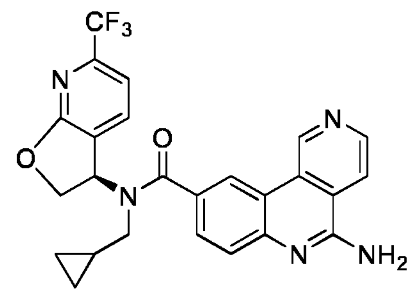

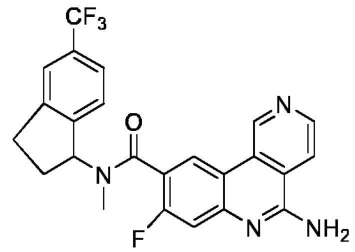

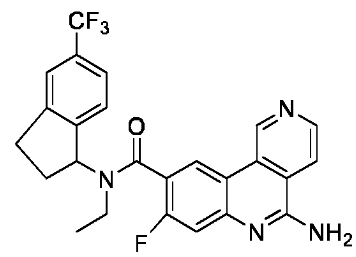

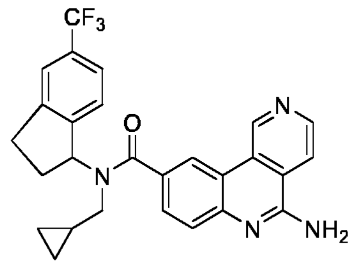

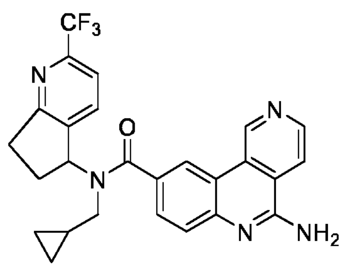

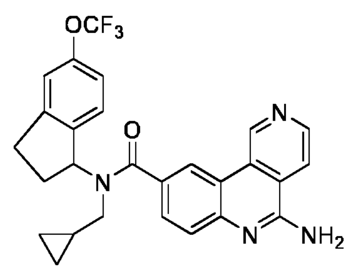

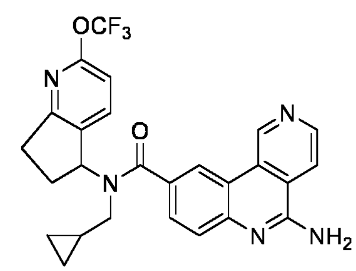

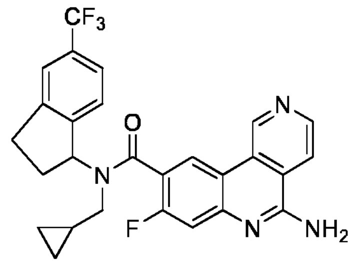
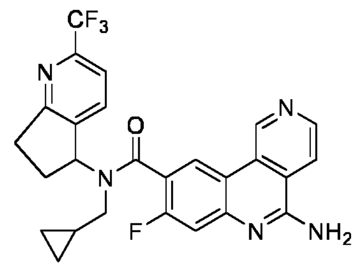
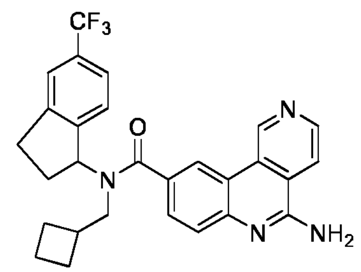

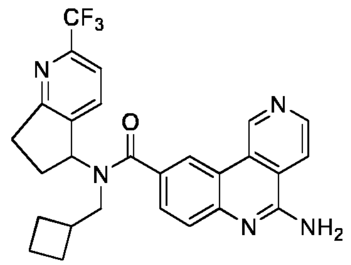
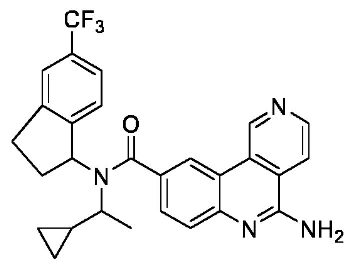
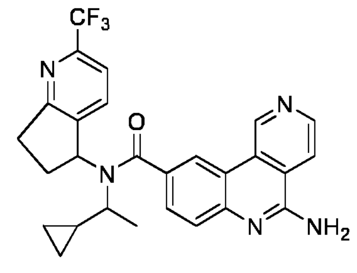

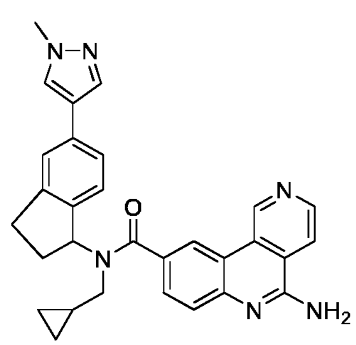
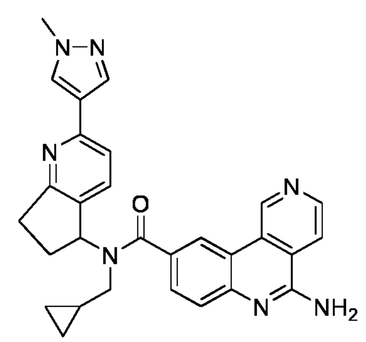
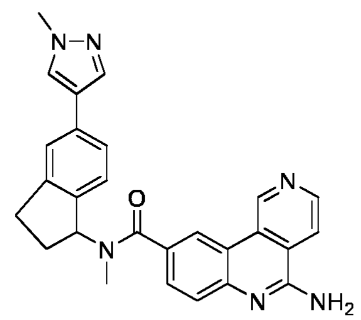

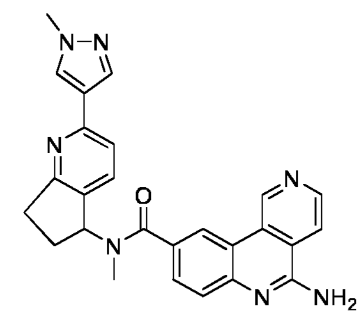
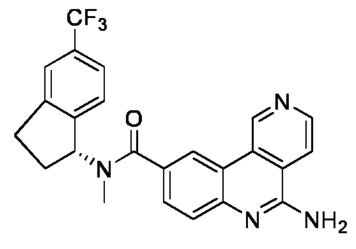
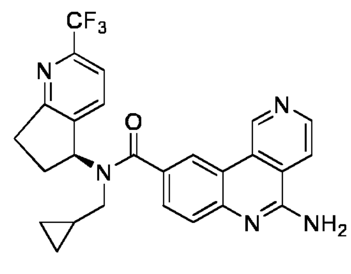

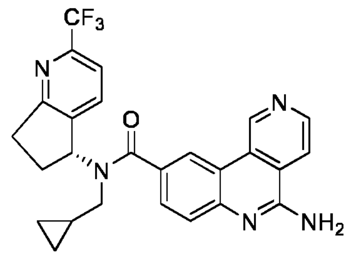
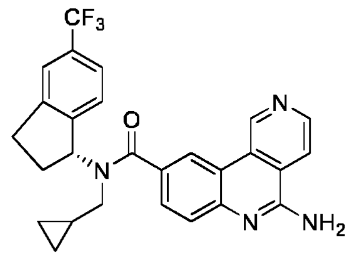
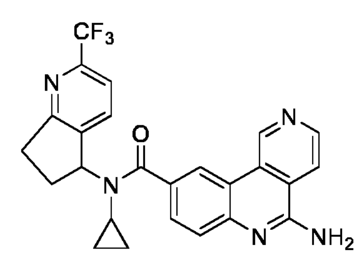

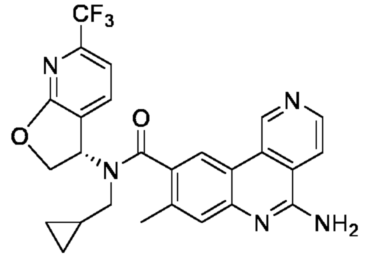

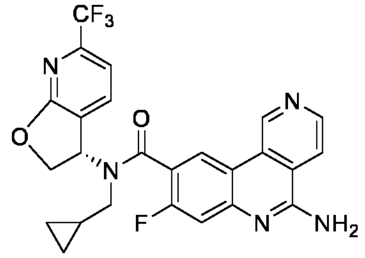

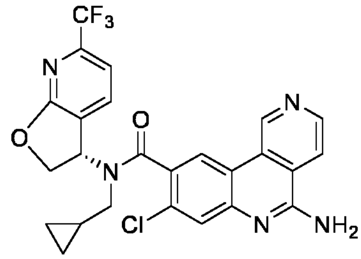

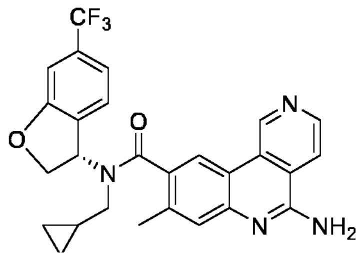

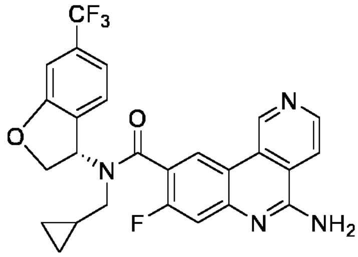

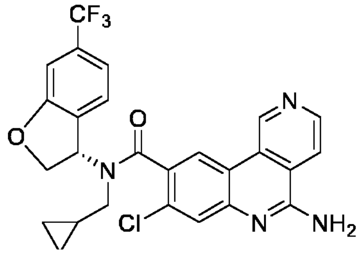

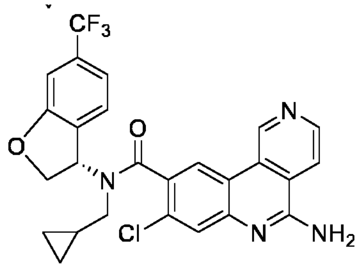

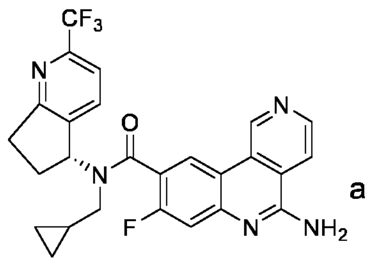

and

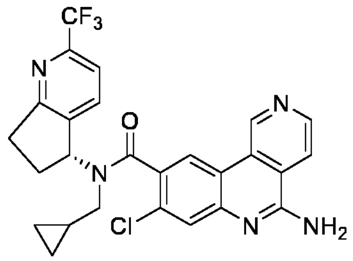

.

**26.** A method of preparing the compound of formula (I), the stereoisomer or pharmaceutically acceptable salt thereof according to claim 1, comprising the following steps:

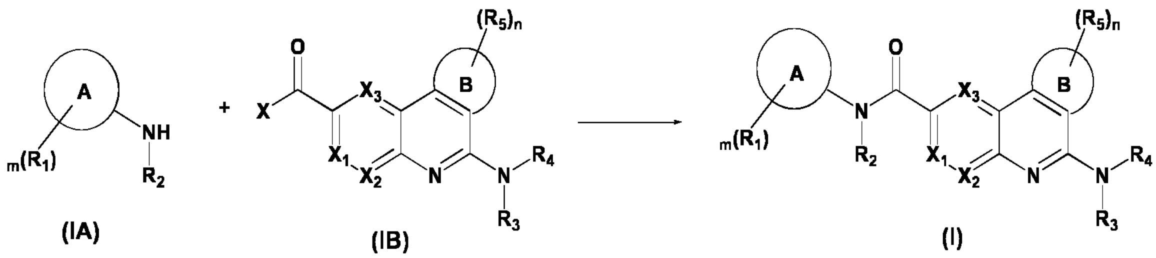

(IA) (IB) (I)

wherein, X is fluorine, chlorine, bromine or hydroxy, and ring A, ring B, $X_1$, $X_2$, $X_3$, $R_1$, $R_2$, $R_3$, $R_4$, $R_5$, m and n are defined as in claim 1.

**27.** A pharmaceutical composition comprising the compound of formula (I), the stereoisomer or pharmaceutically acceptable salt thereof of any one of claims 1-25, and a pharmaceutically acceptable carrier.

**28.** Use of the compound of formula (I), the stereoisomer or pharmaceutically acceptable salt thereof of any one of claims 1-25 in the preparation of a medicament for treating MATP-associated cancer or tumor.

**29.** The use according to claim 28, wherein the tumor or cancer is selected from the group consisting of endometrial carcinoma, granulosa-theca cell tumor, Sertoli-Leydig cell tumor, germinomas, malignant teratoma, squamous cell carcinoma, intraepithelial cancer, adenocarcinoma, fibrosarcoma, melanoma, clear cell carcinoma, squamous cell carcinoma, botryoid sarcoma, fallopian tube cancer, adenocarcinoma, nephroblastoma, lymphoma, leukemia, bladder cancer, squamous cell carcinoma, transitional cell carcinoma, adenocarcinoma, prostate cancer, seminoma,

teratoma, embryonal carcinoma, teratoma, choriocarcinoma, sarcoma, mesenchymal cell carcinoma, fibroma, fibroadenoma, adenomatoid tumor, lipoma, liver cancer, cholangiocarcinoma, hepatoblastoma, hemangiosarcoma, hepatocellular adenoma, hemangioma, gallbladder cancer, ampullary carcinoma, cholangiocarcinoma, malignant melanoma, basal cell carcinoma, squamous cell carcinoma, Kaposi's sarcoma, moles, dysplastic nevus, lipomyoma, hemangioma, acute and chronic myeloid leukemia, acute lymphoblastic leukemia, chronic lymphoblastic leukemia, myeloproliferative disorder, multiple myeloma, myelodysplastic syndrome, Hodgkin's disease, non-Hodgkin's lymphoma, osteosarcoma, fibrosarcoma, malignant fibrous histiocytoma, chondrosarcoma, Ewing's sarcoma, malignant lymphoma, multiple myeloma, malignant giant cell tumor chordoma, osteochondroma, benign chondroma, chondroblastoma, chondromyxoid fibroma, osteoid osteoma, giant cell tumor, angiosarcoma, fibrosarcoma, rhabdomyosarcoma, liposarcoma, myxoma, rhabdomyoma, fibroma, lipomyoma and teratoma, bronchial carcinoma, alveolar carcinoma, bronchial adenoma, sarcoma, lymphoma, chondromatoid hamartoma, mesothelioma, squamous cell carcinoma, adenocarcinoma, leiomyosarcoma, lymphoma, gastric cancer, lymphoma, leiomyosarcoma, ductal adenocarcinoma, insulinoma, glucagonoma, gastrinoma, carcinoid tumor, serpentine tumor, adenocarcinoma, lymphoma, carcinoid tumor, Kaposis sarcoma, leiomyoma, hemangioma, lipomyoma, neurofibroma, fibroma, colorectal adenocarcinoma, tubular adenoma, villous adenoma, hamartoma, leiomyoma, skull tumor, hemangioma, granuloma, xanthoma, osteitis deformans, meningioma, meningeal sarcoma, gliomatosis, astrocytoma, medulloblastoma, glioma, ependymoma, germ cell tumor, glioblastoma multiforme, oligodendroglioma, neurilemmoma, retinoblastoma, congenital tumor, spinal cord neurofibroma, meningioma, glioma and sarcoma.

**30.** The use according to claim 29, wherein the cancer or tumor is selected from the group consisting of breast cancer, pancreatic cancer, skin cancer, bladder cancer, liver cancer and head and neck cancer.

**31.** The compound of formula (I), the stereoisomer or pharmaceutically acceptable salt thereof according to any one of claims 1-25 for use as a PRMT5 inhibitor.

## INTERNATIONAL SEARCH REPORT

International application No. **PCT/CN2023/105579**

**A. CLASSIFICATION OF SUBJECT MATTER**

C07D471/04(2006.01)i; C07D471/14(2006.01)i; C07D491/048(2006.01)i; C07D491/052(2006.01)i; C07D491/147(2006.01)i; C07D495/04(2006.01)i; A61K31/55(2006.01)i; A61K31/4375(2006.01)i; A61K31/519(2006.01)i; A61K31/437(2006.01)i; A61K31/4365(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXTC, VEN, CNKI, STN, 上海和誉生物医药科技有限公司, 喹啉, 吡唑, 酰胺, 吡啶, 呋喃, quinoline, pyrazole, +acylamide, pyridine, furan, PRMT, 结构式检索, search for structural formula

**C. DOCUMENTS CONSIDERED TO BE RELEVANT**

| Category* | Citation of document, with indication, where appropriate, of the relevant passages | Relevant to claim No. |
|---|---|---|
| PX | WO 2022169948 A1 (AMGEN INC.) 11 August 2022 (2022-08-11) claims 1-27, and description, tables 12-22 | 12-31 |
| A | WO 2020205660 A1 (UNIVERSITY OF FLORIDA RESEARCH FOUNDATION, INCORPORATED) 08 October 2020 (2020-10-08) abstract, and claims 1-23 | 1-31 |
| A | WO 2020206289 A1 (PRELUDE THERAPEUTICS, INCORPORATED) 08 October 2020 (2020-10-08) abstract, and claims 1-45 | 1-31 |
| A | WO 2019002074 A1 (BAYER AKTIENGESELLSCHAFT, et al.) 03 January 2019 (2019-01-03) abstract, and claims 1-16 | 1-31 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 September 2023** | **13 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/105579**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31**
because they relate to subject matter not required to be searched by this Authority, namely:

Claim 31 relates to the subject matter of a compound as a drug, which falls within methods for treatment of diseases.

2. ☑ Claims Nos.: **1-5 (in part)**
because they relate to parts of the international application that do not comply with the prescribed requirements to such an extent that no meaningful international search can be carried out, specifically:

Claims 1-5 (in part) are not supported by the description, and no meaningful search can be made. The scope of search is made on the basis of the compound falling within the scope defined in claim 6.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/105579**

| Patent document cited in search report | Publication date (day/month/year) | Patent family member(s) | Publication date (day/month/year) |
|---|---|---|---|
| WO 2022169948 A1 | 11 August 2022 | CA 3210332 A1 | 11 August 2022 |
| | | AU 2022217791 A1 | 17 August 2023 |
| WO 2020205660 A1 | 08 October 2020 | US 2022185792 A1 | 16 June 2022 |
| | | US 2023212143 A9 | 06 July 2023 |
| WO 2020206289 A1 | 08 October 2020 | JP 2022527556 A | 02 June 2022 |
| | | US 2022152072 A1 | 19 May 2022 |
| | | EP 3952874 A1 | 16 February 2022 |
| | | EP 3952874 A4 | 28 December 2022 |
| WO 2019002074 A1 | 03 January 2019 | US 2020123147 A1 | 23 April 2020 |
| | | EP 3645531 A1 | 06 May 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022169948 A1